# EUROPEAN PATENT APPLICATION

(11) **EP 4 446 310 A1**
(43) Date of publication of application: **16.10.2024**
(21) Application number: 22904304.7
(22) Date of filing: 08.12.2022
(51) Int. Cl.: C07C 263/04, C07C 239/08, C07C 265/14, C07C 269/04, C07C 269/08, C07C 271/12, C07C 271/24, C07C 271/28, C07C 271/44, C07C 271/56, C07C 271/58, C07C 273/14, C07C 275/06, C07C 275/14, C07C 275/18, C07C 275/24, C07C 275/26, C07C 275/28

(54) **METHOD FOR PRODUCING BLOCKED ISOCYANATE COMPOUND AND METHOD FOR PRODUCING ISOCYANATE COMPOUND**

(30) Priority: 08.12.2021 JP 2021199646; 08.12.2021 JP 2021199647
(71) Applicant: Asahi Kasei Kabushiki Kaisha, Tokyo 1000006 (JP)
(72) Inventor: INADA, Hiroshi, Tokyo 100-0006 (JP); NAKAOKA, Koichi, Tokyo 100-0006 (JP); ONISHI, Kazuhiro, Tokyo 100-0006 (JP)
(74) Representative: D Young & Co LLP
(86) International application number: PCT/JP2022/045329
(87) International publication number: WO 2023/106377

(57) **Abstract**

Provided is a method for producing a blocked isocyanate compound, the method comprising a reaction step of reacting a primary amine compound, a carbonic acid derivative and a blocking agent by means of heat treatment in the presence of at least one compound selected from the group consisting of a secondary amine compound of general formula (I) and a tertiary amine compound of general formula (II) to obtain a blocked isocyanate compound. Also provided is a method for producing an isocyanate compound, the method comprising a reaction step of decomposing a blocked isocyanate compound into a blocking agent and an isocyanate compound by means of heat treatment in the presence of at least one compound selected from the group consisting of a secondary amine compound of general formula (I) and a tertiary amine compound of general formula (II) to obtain the isocyanate compound.

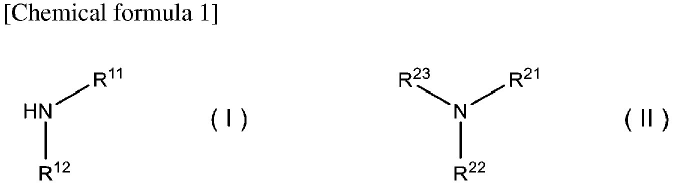

## Description

### [Technical Field]

The present invention relates to a method for producing a blocked isocyanate compound and a method for producing an isocyanate compound.

Priority is claimed on Japanese Patent Application No. 2021-199646 and Japanese Patent Application No. 2021-199647, filed December 8, 2021, the contents of which are incorporated herein by reference.

### [Background Art]

Isocyanates are widely used as production raw materials for polyurethane foams, coating materials, and adhesives and the like. The main industrial method for producing isocyanates is a reaction between an amine compound and phosgene (the phosgene method), with almost the entire world production being produced by the phosgene method. However, the phosgene method has many problems.

Firstly, the phosgene method requires the use of a large amount of phosgene as a raw material. Phosgene is extremely toxic, and therefore special care is required in handling to prevent exposure to workers, and special devices are required for removing waste products.

Secondly, in the phosgene method, because highly corrosive hydrogen chloride is produced in large amounts as a by-product, a process is required to remove the hydrogen chloride. Moreover, in many cases, the produced isocyanate contains hydrolyzable chlorine. As a result, when an isocyanate produced by the phosgene method is used, adverse effects are sometimes observed on the weather resistance and heat resistance of polyurethane products. In light of this type of background, a method for producing isocyanates that does not use phosgene would be desirable.

Further, because isocyanates exhibit high reactivity and react readily with compounds such as water, they are sometimes converted to blocked isocyanates to improve stability, and the blocked isocyanates are heated to remove blocking agents therefrom, thereby reforming the isocyanates at the time of use. Blocked isocyanates have low reactivity with active hydrogen compounds, can be stored stably, and also exhibit low toxicity compared with isocyanates, and are therefore useful as one-pot coating materials, adhesives and molding compounds. Further, because blocked isocyanates can be dissociated into isocyanates and blocking agents by thermal decomposition, they can be used and are useful as raw materials when producing isocyanates, by decomposing the blocked isocyanate by thermal decomposition into an isocyanate and a blocking agent, and then separating the obtained isocyanate and blocking agent, either following or during the thermal decomposition. On the other hand, an isocyanate production process that relies on the thermal decomposition of a blocked isocyanate requires a comparatively high reaction temperature compared with an isocyanate production process using phosgene, and thermal denaturation and coloration can be problematic. Accordingly, when converting the obtained isocyanate to a product, the isocyanate must be purified to a level commensurate with the intended application, and this purification process can be very costly.

Various methods are known for producing blocked isocyanates. Examples thereof include a method for producing a blocked isocyanate by direct reaction of an isocyanate and a blocking agent, a method for producing a blocked isocyanate by reacting a blocking agent with a carbamic chloride obtained by reacting an amine and phosgene, a method for producing a blocked isocyanate by reacting carbamic acid, a blocking agent and a condensation agent, a method for producing a blocked isocyanate containing a compound derived from a carbonic acid derivative by reacting an amine and a carbonic acid derivative, and a method for producing a blocked isocyanate by reacting an amine, a carbonic acid derivative and a blocking agent.

Among these methods, the method for producing a blocked isocyanate by reacting an amine, a carbonic acid derivative and a blocking agent is a useful method in terms of: 1) not requiring the use of an expensive isocyanate, 2) not requiring the use of highly toxic phosgene or carbamic chloride, and 3) not requiring the handling of chemically unstable carbamic acid and not requiring the use of a costly condensation agent. Specific examples of this type of method for producing a blocked isocyanate include a method for producing a carbamate from an amine, urea and an alcohol (for example, see Patent Document 1), a method for producing a trisubstituted urea from an amine, urea and a secondary amine (for example, see Patent Document 2), and a method for producing an N-substituted carbamate ester by reacting a compound having an ureido group obtained by reacting an amine and urea, and an aromatic hydroxy composition containing an aromatic hydroxy compound (for example, see Patent Document 3).

Furthermore, methods in which a raw material blocked isocyanate and a blocking agent are reacted together via an addition-elimination reaction to obtain a blocked isocyanate different from the raw material blocked isocyanate are also known. In a similar manner to the method for producing a blocked isocyanate by reacting an amine, a carbonic acid derivative and a blocking agent, these methods are also useful production methods in terms of the points 1) to 3) mentioned above. Specific examples of this type of method for producing a blocked isocyanate include a method for obtaining an aryl carbamate by reacting an alkyl carbamate and a phenolic compound (for example, see Patent Document 11), and a method for obtaining a carbamate by reacting a compound having a urea linkage and a compound having a hydroxy group (for example, see Non-Patent Document 2).

On the other hand, reactions for obtaining a blocked isocyanate from an amine, a carbonic acid derivative and a blocking agent typically require heat. Further, methods in which a raw material blocked isocyanate and a blocking agent are reacted together via an addition-elimination reaction to obtain a blocked isocyanate different from the raw material blocked isocyanate also generally require heat. In these methods, in order to ensure the reaction progress rapidly on an industrial scale, a high reaction temperature is required. For these reasons, thermal denaturation and coloration can become problematic during production. Accordingly, when converting the obtained blocked isocyanate to a product, or when attempting to obtain an isocyanate with little thermal denaturation and no coloring or very little coloring by thermal decomposition of the blocked isocyanate, the blocked isocyanate or isocyanate must be purified to a level commensurate with the intended application, and this purification process can be very costly. Measures that may be considered for preventing this type of thermal denaturation or coloration of blocked isocyanates include conducting the production of the blocked isocyanate compound under an inert gas atmosphere, or using any of various antioxidants.

Examples of antioxidants which may be used for this purpose include antioxidants used for the purpose of preventing isocyanate coloration, such as phenol-based antioxidants such as BHT, phosphorus-based antioxidants such as triarylphosphine compounds (for example, see Patent Document 4) and trialkyl phosphate compounds (for example, see Patent Document 5). Further, a method for reducing coloration by bringing the isocyanate into contact with an ozone-containing gas (for example, see Patent Document 6), and a method for producing an isocyanate with reduced coloration by irradiating light with a wavelength of 200 to 600 nm onto the isocyanate (for example, see Patent Document 7) are also being investigated.

On the other hand, it is known that compounds having an amino group bonded to a carbon atom having aromaticity are readily oxidized to cause coloration (for example, see Patent Document 8). Accordingly, even if, for example, as disclosed in Patent Document 9, compounds having an amino group bonded to a carbon atom having aromaticity are used in products for which coloration is not a problem, such as rubber degradation inhibitors, these compounds are not used in products for which coloration would be problematic.

However, a process for bringing a compound into contact with an ozone-containing gas or reducing coloration by light irradiation requires special equipment, and not only does this lead to increased complexity of the facilities, but there is still room for improvement in the coloration reduction effect. Further, phenol-based antioxidants such as BHT can generate colored compounds such as stilbene quinone structures upon oxidation, for example as disclosed in Non-Patent Document 1, and therefore there is still room for improvement in the coloration reduction effect. Furthermore, triarylphosphine compounds, which are phosphorus-based antioxidants, have high nucleophilicity, and particularly in the reaction for obtaining a blocked isocyanate from an amine, a carbonic acid derivative and a blocking agent, isocyanate functional groups exist in the reaction system, the triarylphosphine compounds tend to accelerate irreversible denaturation of the isocyanate functional groups, or may accelerate denaturation under the blocked isocyanate thermal decomposition temperature conditions, and are therefore not desirable. Further, trialkyl phosphate compounds are known as carbodiimidization catalysts, and therefore not only promote carbodiimidization under heating in the presence of the isocyanate functional groups produced in the reaction system, but the generated carbodiimides react with the isocyanate to form uretonimine structures, thereby causing an increase in by-products and an increase in the viscosity of the reaction liquid, both of which are undesirable.

As outlined above, the problems of thermal denaturation and coloration in the process for producing a blocked isocyanate from an amine, a carbonic acid derivative and a blocking agent cannot be resolved entirely satisfactorily with conventional methods.

Further, as outlined above, the problems of thermal denaturation and coloration when subjecting a blocked isocyanate to thermal decomposition can also not be resolved with conventional methods.

### [Citation List]

### [Patent Documents]

[Patent Document 1]
   U.S. Patent Publication No. 2,409,712
[Patent Document 2]
   German Patent Publication No. 1064051
[Patent Document 3]
   International Patent Publication No. 2011/021258
[Patent Document 4]
   U.S. Patent Publication No. 2,957,903
[Patent Document 5]
   Japanese Unexamined Patent Application, First Publication No. Sho 49-075505
[Patent Document 6]
   Japanese Unexamined Patent Application, First Publication No. Hei 08-291129
[Patent Document 7]
   Japanese Translation of PCT International Application, Publication No. 2012-506465
[Patent Document 8]
   Japanese Unexamined Patent Application, First Publication No. Sho 59-042346
[Patent Document 9]
   Japanese Patent (Granted) Publication No. 3051523
[Patent Document 10]
   Japanese Patent (Granted) Publication No. 3051523
[Patent Document 11]
   Japanese Patent (Granted) Publication No. 4859255

### [Non-Patent Documents]

[Non-Patent Document 1]
   Cook C. D. et al., "Oxidation of Hindered Phenols. III. The Rearrangement of the 2,6-Di-t-butyl-4-methylphenoxy Radical", J. Am. Chem. Soc., Vol. 77, pp. 1783 to 1785, 1955.
[Non-Patent Document 2]
   Hutchby M et al., "Hindered Ureas as Masked Isocyanates: Facile Carbamoylation of Nucleophiles under Neutral Conditions", Angew. Chem. Int. Ed., Vol. 48, Issue 46, pp. 8721 to 8724, 2009.

### [Summary of Invention]

### [Technical Problem]

The present invention has been developed in light of the above circumstances, and provides a method for producing a blocked isocyanate compound with good suppression of thermal denaturation and coloration.

Further, another aspect of the present invention has also been developed in light of the above circumstances, and provides a method for producing an isocyanate compound with good suppression of thermal denaturation and coloration.

### [Solution to Problem]

In other words, the present invention includes the following aspects.
(1) A method for producing a blocked isocyanate compound, the method including a reaction step of reacting a primary amine compound, a carbonic acid derivative and a blocking agent by conducting a heat treatment in the presence of at least one compound selected from the group consisting of secondary amine compounds of general formula (I) shown below and tertiary amine compounds of general formula (11) shown below, thus obtaining a blocked isocyanate compound. 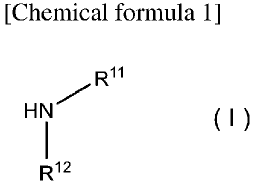 In general formula (I), R¹¹ and R¹² are each independently a monovalent organic group. R¹¹ and R¹² may be bonded together to form a cyclic structure via a carbon-carbon bond, a carbon-oxygen-carbon bond, or a carbon-nitrogen-carbon bond. At least one of R¹¹ and R¹² has an aromatic group. 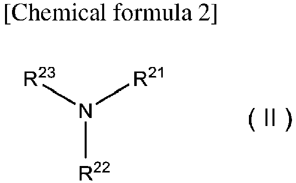 In general formula (II), R²¹, R²² and R²³ are each independently a monovalent organic group. Each combination of R²¹ and R²², R²² and R²³, and R²³ and R²¹ may be independently bonded together to form a cyclic structure via a carbon-carbon bond, a carbon-oxygen-carbon bond, or a carbon-nitrogen-carbon bond. At least one of R²¹, R²² and R²³ has an aromatic group.
(2) The method for producing a blocked isocyanate compound according to (1), wherein the blocking agent includes at least one compound selected from the group consisting of hydroxy compounds, amine compounds and ammonia.
(3) The method for producing a blocked isocyanate compound according to (1) or (2), wherein the primary amine compound is an amine compound of general formula (111) shown below.

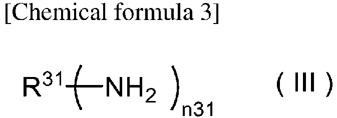

In general formula (111), R³¹ is an n31-valent organic group. Further, n31 is an integer of 1 or more but not more than 12.

(4) The method for producing a blocked isocyanate compound according to any one of (1) to (3), wherein the blocking agent is an aromatic hydroxy compound of general formula (IV-1) shown below.

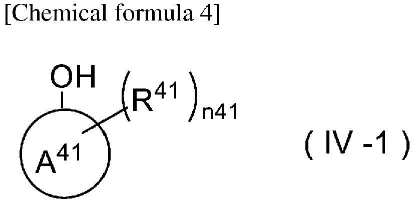

In general formula (IV-1), the ring A⁴¹ is an aromatic hydrocarbon ring of 6 or more but not more than 20 carbon atoms. R⁴¹ is a hydrogen atom, halogen atom, carboxy group, alkyl group of 1 or more but not more than 20 carbon atoms, alkoxy group of 1 or more but not more than 20 carbon atoms, alkyloxycarbonyl group of 1 or more but not more than 20 carbon atoms, alkylcarbonyloxy group of 1 or more but not more than 20 carbon atoms, aryl group of 6 or more but not more than 20 carbon atoms, aryloxy group of 6 or more but not more than 20 carbon atoms, aralkyl group of 7 or more but not more than 20 carbon atoms, or aralkyloxy group of 7 or more but not more than 20 carbon atoms. R⁴¹ may be bonded to the ring A⁴¹ to form a cyclic structure. Further, n41 is an integer of 1 or more but not more than 10.

(5) The method for producing a blocked isocyanate compound according to any one of (1) to (3), wherein the blocking agent is an aliphatic hydroxy compound of general formula (IV-2) shown below.
[Chemical formula 5]

R⁴²-OH ( IV-2 )

In general formula (IV-2), R⁴² is a substituted or unsubstituted aliphatic hydrocarbon group of 1 or more but not more than 24 carbon atoms which may have an ether group, carbonyl group or ester group.

(6) The method for producing a blocked isocyanate compound according to any one of (1) to (3), wherein the blocking agent is a secondary amine compound of general formula (V) shown below.

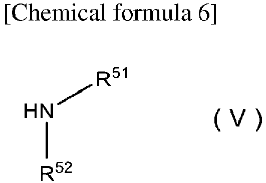

In general formula (V), R⁵¹ and R⁵² are each independently a monovalent organic group. R⁵¹ and R⁵² may be bonded together to form a cyclic structure via a carbon-carbon bond, a carbon-oxygen-carbon bond, or a carbon-nitrogen-carbon bond.

(7) The method for producing a blocked isocyanate compound according to any one of (1) to (6), wherein the carbonic acid derivative is a compound of general formula (VI) shown below.

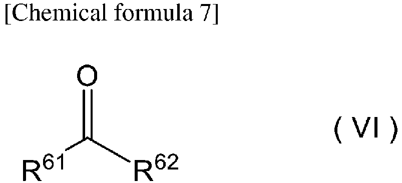

In general formula (VI), R⁶¹ and R⁶² are each independently an amino group, a substituted or unsubstituted alkoxy group of 1 or more but not more than 20 carbon atoms, a substituted or unsubstituted aryloxy group of 6 or more but not more than 20 carbon atoms, a substituted or unsubstituted alkylamino group of 1 or more but not more than 20 carbon atoms or a substituted or unsubstituted arylamino group of 6 or more but not more than 20 carbon atoms.

(8) The method for producing a blocked isocyanate compound according to any one of (1) to (7), wherein in the reaction step, the oxygen concentration within the supplied gas is not more than 21 % by volume relative to the total volume of the gas.

(9) The method for producing a blocked isocyanate compound according to any one of (1) to (8), wherein in the reaction step, the amount present of at least one compound selected from the group consisting of the secondary amine compounds of the general formula (I) and the tertiary amine compounds of the general formula (II) is at least 1 ppm by mass relative to the total mass of the primary amine compound and the blocking agent.

(10) The method for producing a blocked isocyanate compound according to any one of (1) to (9), wherein in the reaction step, the secondary amine compound of the general formula (I) and the tertiary amine compound of the general formula (II) are incorporated simultaneously.

(11) A method for producing an isocyanate compound, the method including a reaction step of decomposing a blocked isocyanate compound into a blocking agent and an isocyanate compound by conducting a heat treatment in the presence of at least one compound selected from the group consisting of secondary amine compounds of general formula (I) shown below and tertiary amine compounds of general formula (II) shown below, thus obtaining the isocyanate compound.

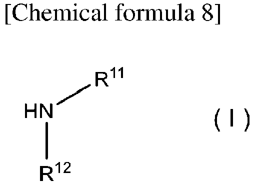

In general formula (1), R¹¹ and R¹² are each independently a monovalent organic group. R¹¹ and R¹² may be bonded together to form a cyclic structure via a carbon-carbon bond, a carbon-oxygen-carbon bond, or a carbon-nitrogen-carbon bond. At least one of R¹¹ and R¹² has an aromatic group.

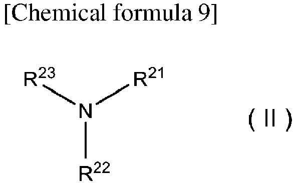

In general formula (11), R²¹, R²² and R²³ are each independently a monovalent organic group. Each combination of R²¹ and R²², R²² and R²³, and R²³ and R²¹ may be independently bonded together to form a cyclic structure via a carbon-carbon bond, a carbon-oxygen-carbon bond, or a carbon-nitrogen-carbon bond. At least one of R²¹, R²² and R²³ has an aromatic group.

(12) The method for producing an isocyanate compound according to (11), wherein the blocking agent includes at least one compound selected from the group consisting of hydroxy compounds, amine compounds and ammonia.

(13) The method for producing an isocyanate compound according to (11) or (12), wherein the isocyanate compound is an isocyanate compound of general formula (VII) shown below.

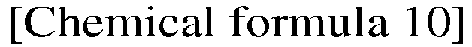

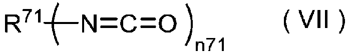

In general formula (VII), R⁷¹ is an n71-valent organic group. Further, n71 is an integer of 1 or more but not more than 12.

(14) The method for producing an isocyanate compound according to any one of (11) to (13), wherein the blocking agent is an aromatic hydroxy compound of general formula (IV-1) shown below.

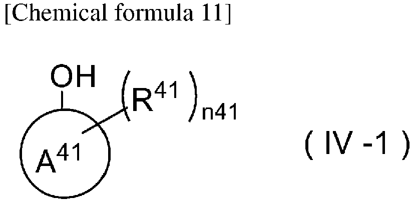

In general formula (IV-1), the ring A⁴¹ is an aromatic hydrocarbon ring of 6 or more but not more than 20 carbon atoms. R⁴¹ is a hydrogen atom, halogen atom, carboxy group, alkyl group of 1 or more but not more than 20 carbon atoms, alkoxy group of 1 or more but not more than 20 carbon atoms, alkyloxycarbonyl group of 1 or more but not more than 20 carbon atoms, alkylcarbonyloxy group of 1 or more but not more than 20 carbon atoms, aryl group of 6 or more but not more than 20 carbon atoms, aryloxy group of 6 or more but not more than 20 carbon atoms, aralkyl group of 7 or more but not more than 20 carbon atoms, or aralkyloxy group of 7 or more but not more than 20 carbon atoms. R⁴¹ may be bonded to the ring A⁴¹ to form a cyclic structure. Further, n41 is an integer of 1 or more but not more than 10.

(15) The method for producing an isocyanate compound according to any one of (11) to (13), wherein the blocking agent is an aliphatic hydroxy compound of general formula (IV-2) shown below.
[Chemical formula 12]

R⁴²-OH ( IV-2 )

In general formula (IV-2), R⁴² is a substituted or unsubstituted aliphatic hydrocarbon group of 1 or more but not more than 24 carbon atoms which may have an ether group, carbonyl group or ester group.

(16) The method for producing an isocyanate compound according to any one of (11) to (13), wherein the blocking agent is a secondary amine compound of general formula (V) shown below.

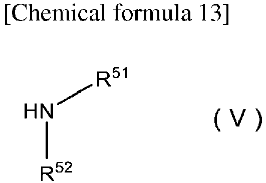

In general formula (V), R⁵¹ and R⁵² are each independently a monovalent organic group. R⁵¹ and R⁵² may be bonded together to form a cyclic structure via a carbon-carbon bond, a carbon-oxygen-carbon bond, or a carbon-nitrogen-carbon bond.

(17) The method for producing an isocyanate compound according to any one of (11) to (16), wherein in the reaction step, the oxygen concentration within the supplied gas is not more than 21 % by volume relative to the total volume of the gas.

(18) The method for producing an isocyanate compound according to any one of (11) to (17), wherein in the reaction step, the amount present of at least one compound selected from the group consisting of the secondary amine compounds of the general formula (I) and the tertiary amine compounds of the general formula (II) is at least 1 ppm by mass relative to the mass of the reaction liquid.

(19) The method for producing an isocyanate compound according to any one of (11) to (18), wherein in the reaction step, the secondary amine compound of the general formula (1) and the tertiary amine compound of the general formula (11) are incorporated simultaneously.

(20) A method for producing an isocyanate compound, the method including:
a first reaction step of reacting a primary amine compound, a carbonic acid derivative and a first blocking agent by conducting a heat treatment in the presence of at least one compound selected from the group consisting of secondary amine compounds of general formula (1) shown below and tertiary amine compounds of general formula (II) shown below, thus obtaining a blocked isocyanate compound, and
a reaction step of decomposing the blocked isocyanate compound into the first blocking agent and an isocyanate compound by conducting a heat treatment in the presence or absence of at least one compound selected from the group consisting of secondary amine compounds of general formula (I) shown below and tertiary amine compounds of general formula (11) shown below, thus obtaining the isocyanate compound.

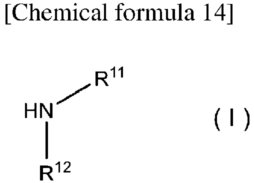

In general formula (I), R¹¹ and R¹² are each independently a monovalent organic group. R¹¹ and R¹² may be bonded together to form a cyclic structure via a carbon-carbon bond, a carbon-oxygen-carbon bond, or a carbon-nitrogen-carbon bond. At least one of R¹¹ and R¹² has an aromatic group.

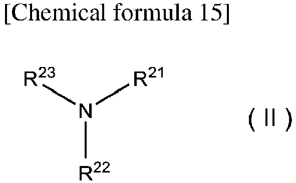

In general formula (II), R²¹, R²² and R²³ are each independently a monovalent organic group. Each combination of R²¹ and R²², R²² and R²³, and R²³ and R²¹ may be independently bonded together to form a cyclic structure via a carbon-carbon bond, a carbon-oxygen-carbon bond, or a carbon-nitrogen-carbon bond. At least one of R²¹, R²² and R²³ has an aromatic group.

(21) A method for producing a blocked isocyanate compound, the method including an addition-elimination reaction step of reacting a first blocked isocyanate compound and a second blocking agent by an addition-elimination reaction in the presence of at least one compound selected from the group consisting of secondary amine compounds of general formula (1) shown below and tertiary amine compounds of general formula (11) shown below, thus obtaining a second blocked isocyanate compound, wherein
the second blocking agent is a compound different from a first blocking agent which blocks isocyanate groups in the first blocked isocyanate compound, and
the first blocked isocyanate compound and the second blocked isocyanate compound have structures different from each other.

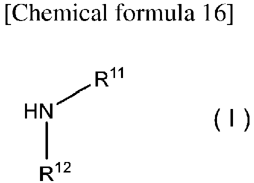

In general formula (I), R¹¹ and R¹² are each independently a monovalent organic group. R¹¹ and R¹² may be bonded together to form a cyclic structure via a carbon-carbon bond, a carbon-oxygen-carbon bond, or a carbon-nitrogen-carbon bond. At least one of R¹¹ and R¹² has an aromatic group.

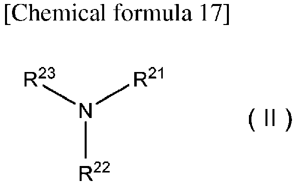

In general formula (II), R²¹, R²² and R²³ are each independently a monovalent organic group. Each combination of R²¹ and R²², R²² and R²³, and R²³ and R²¹ may be independently bonded together to form a cyclic structure via a carbon-carbon bond, a carbon-oxygen-carbon bond, or a carbon-nitrogen-carbon bond. At least one of R²¹, R²² and R²³ has an aromatic group.

(22) The method for producing a blocked isocyanate compound according to (21), wherein an isocyanate compound used in the production of the first blocked isocyanate compound includes an isocyanate compound of general formula (VII) shown below.

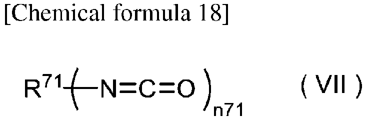

In general formula (VII), R⁷¹ is an n71-valent organic group. Further, n71 is an integer of 1 or more but not more than 12.

(23) The method for producing a blocked isocyanate compound according to (21) or (22), wherein the first blocking agent includes at least one compound selected from the group consisting of hydroxy compounds, amine compounds and ammonia.

(24) The method for producing a blocked isocyanate compound according to any one of (21) to (23), wherein the first blocking agent includes an aromatic hydroxy compound of general formula (IV-1) shown below.

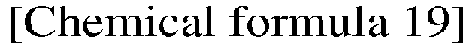

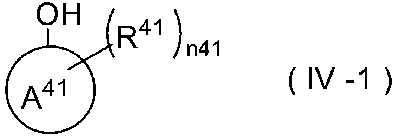

In general formula (IV-1), the ring A⁴¹ is an aromatic hydrocarbon ring of 6 or more but not more than 20 carbon atoms. R⁴¹ is a hydrogen atom, halogen atom, carboxy group, alkyl group of 1 or more but not more than 20 carbon atoms, alkoxy group of 1 or more but not more than 20 carbon atoms, alkyloxycarbonyl group of 1 or more but not more than 20 carbon atoms, alkylcarbonyloxy group of 1 or more but not more than 20 carbon atoms, aryl group of 6 or more but not more than 20 carbon atoms, aryloxy group of 6 or more but not more than 20 carbon atoms, aralkyl group of 7 or more but not more than 20 carbon atoms, or aralkyloxy group of 7 or more but not more than 20 carbon atoms. R⁴¹ may be bonded to the ring A⁴¹ to form a cyclic structure. Further, n41 is an integer of 1 or more but not more than 10.

(25) The method for producing a blocked isocyanate compound according to any one of (21) to (23), wherein the first blocking agent includes an aliphatic hydroxy compound of general formula (IV-2) shown below.
[Chemical formula 20]

R⁴²-OH ( IV-2 )

In general formula (IV-2), R⁴² is a substituted or unsubstituted aliphatic hydrocarbon group of 1 or more but not more than 24 carbon atoms which may have an ether group, carbonyl group or ester group.

(26) The method for producing a blocked isocyanate compound according to any one of (21) to (23), wherein the first blocking agent includes a secondary amine compound of general formula (V) shown below.

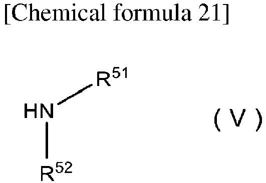

In general formula (V), R⁵¹ and R⁵² are each independently a monovalent organic group. R⁵¹ and R⁵² may be bonded together to form a cyclic structure via a carbon-carbon bond, a carbon-oxygen-carbon bond, or a carbon-nitrogen-carbon bond.

(27) The method for producing a blocked isocyanate compound according to any one of (21) to (26), wherein the second blocking agent includes at least one compound selected from the group consisting of hydroxy compounds and amine compounds.

(28) The method for producing a blocked isocyanate compound according to any one of (21) to (27), wherein the second blocking agent includes an aromatic hydroxy compound of general formula (IV-1) shown below.

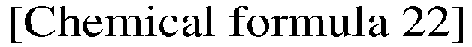

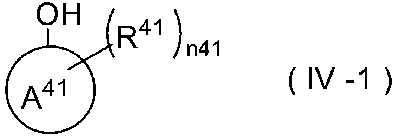

In general formula (IV-1), the ring A⁴¹ is an aromatic hydrocarbon ring of 6 or more but not more than 20 carbon atoms. R⁴¹ is a hydrogen atom, halogen atom, carboxy group, alkyl group of 1 or more but not more than 20 carbon atoms, alkoxy group of 1 or more but not more than 20 carbon atoms, alkyloxycarbonyl group of 1 or more but not more than 20 carbon atoms, alkylcarbonyloxy group of 1 or more but not more than 20 carbon atoms, aryl group of 6 or more but not more than 20 carbon atoms, aryloxy group of 6 or more but not more than 20 carbon atoms, aralkyl group of 7 or more but not more than 20 carbon atoms, or aralkyloxy group of 7 or more but not more than 20 carbon atoms. R⁴¹ may be bonded to the ring A⁴¹ to form a cyclic structure. Further, n41 is an integer of 1 or more but not more than 10.

(29) The method for producing a blocked isocyanate compound according to any one of (21) to (27), wherein the second blocking agent includes an aliphatic hydroxy compound of general formula (1V-2) shown below.
[Chemical formula 23]

R⁴²-OH ( IV-2 )

In general formula (IV-2), R⁴² is a substituted or unsubstituted aliphatic hydrocarbon group of 1 or more but not more than 24 carbon atoms which may have an ether group, carbonyl group or ester group.

(30) The method for producing a blocked isocyanate compound according to any one of (21) to (27), wherein the second blocking agent includes a secondary amine compound of general formula (V) shown below.

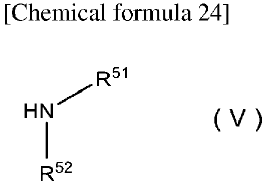

In general formula (V), R⁵¹ and R⁵² are each independently a monovalent organic group. R⁵¹ and R⁵² may be bonded together to form a cyclic structure via a carbon-carbon bond, a carbon-oxygen-carbon bond, or a carbon-nitrogen-carbon bond.

(31) The method for producing a blocked isocyanate compound according to any one of (21) to (30), wherein in the addition-elimination reaction step, the oxygen concentration within the supplied gas is not more than 21% by volume relative to the total volume of the gas.

(32) The method for producing a blocked isocyanate compound according to any one of (21) to (31), wherein in the addition-elimination reaction step, the amount present of at least one compound selected from the group consisting of the secondary amine compounds of the general formula (I) and the tertiary amine compounds of the general formula (11) is at least 1 ppm by mass relative to the total mass of the reaction liquid.

(33) The method for producing a blocked isocyanate compound according to any one of (21) to (32), wherein in the addition-elimination reaction step, the secondary amine compound of the general formula (1) and the tertiary amine compound of the general formula (II) are incorporated simultaneously.

(34) A method for producing a blocked isocyanate compound, the method including:
a reaction step of reacting a primary amine compound, a carbonic acid derivative and a first blocking agent by conducting a heat treatment in the presence of at least one compound selected from the group consisting of secondary amine compounds of general formula (I) and tertiary amine compounds of general formula (II), thus obtaining a first blocked isocyanate compound, and
an addition-elimination reaction step of reacting the first blocked isocyanate compound and a second blocking agent by an addition-elimination reaction in the presence or absence of at least one compound selected from the group consisting of secondary amine compounds of the general formula (I) and tertiary amine compounds of the general formula (11), thus obtaining a second blocked isocyanate compound, wherein
the second blocking agent is a compound different from the first blocking agent and a third blocking agent derived from the carbonic acid derivative, and
the first blocked isocyanate compound and the second blocked isocyanate compound have structures different from each other.

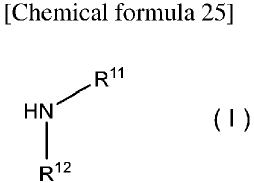

In general formula (I), R¹¹ and R¹² are each independently a monovalent organic group. R¹¹ and R¹² may be bonded together to form a cyclic structure via a carbon-carbon bond, a carbon-oxygen-carbon bond, or a carbon-nitrogen-carbon bond. At least one of R¹¹ and R¹² has an aromatic group.

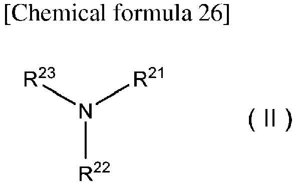

In general formula (II), R²¹, R²² and R²³ are each independently a monovalent organic group. Each combination of R²¹ and R²², R²² and R²³, and R²³ and R²¹ may be independently bonded together to form a cyclic structure via a carbon-carbon bond, a carbon-oxygen-carbon bond, or a carbon-nitrogen-carbon bond. At least one of R²¹, R²² and R²³ has an aromatic group.

(35) A method for producing an isocyanate compound, the method including:
a first reaction step of reacting a primary amine compound, a carbonic acid derivative and a first blocking agent by conducting a heat treatment in the presence of at least one compound selected from the group consisting of secondary amine compounds of general formula (1) and tertiary amine compounds of general formula (11), thus obtaining a blocked isocyanate compound, and
an addition-elimination reaction step of reacting the first blocked isocyanate compound and a second blocking agent by an addition-elimination reaction in the presence or absence of at least one compound selected from the group consisting of secondary amine compounds of the general formula (I) and tertiary amine compounds of the general formula (11), thus obtaining a second blocked isocyanate compound, and
a second reaction step of decomposing the second blocked isocyanate compound into a second blocking agent and an isocyanate compound by conducting a heat treatment in the presence or absence of at least one compound selected from the group consisting of secondary amine compounds of the general formula (I) and tertiary amine compounds of the general formula (II), thus obtaining the isocyanate compound, wherein
the second blocking agent is a compound different from the first blocking agent and a third blocking agent derived from the carbonic acid derivative, and
the first blocked isocyanate compound and the second blocked isocyanate compound have structures different from each other.

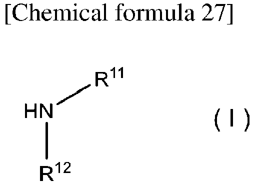

In general formula (1), R¹¹ and R¹² are each independently a monovalent organic group. R¹¹ and R¹² may be bonded together to form a cyclic structure via a carbon-carbon bond, a carbon-oxygen-carbon bond, or a carbon-nitrogen-carbon bond. At least one of R¹¹ and R¹² has an aromatic group.

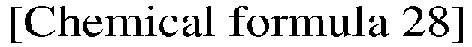

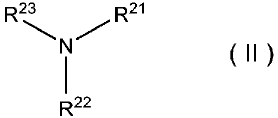

In general formula (II), R²¹, R²² and R²³ are each independently a monovalent organic group. Each combination of R²¹ and R²², R²² and R²³, and R²³ and R²¹ may be independently bonded together to form a cyclic structure via a carbon-carbon bond, a carbon-oxygen-carbon bond, or a carbon-nitrogen-carbon bond. At least one of R²¹, R²² and R²³ has an aromatic group.

### [Advantageous Effects of Invention]

The method for producing a blocked isocyanate compound according to one of the aspects described above makes it possible to provide a method for producing a blocked isocyanate compound with good suppression of thermal denaturation and coloration.

Further, the method for producing an isocyanate compound according to one of the aspects described above makes it possible to provide a method for producing an isocyanate compound with good suppression of thermal denaturation and coloration.

### [Description of Embodiments]

Embodiments for implementing the present invention (hereinafter referred to as "embodiment of the present invention" are described below in detail. However, the following embodiments of the present invention are merely provided to better describe the present invention, and the present invention is in no way limited by the following embodiments. The present invention can be implemented with various modifications within the scope of the invention.

In this description, the term "active hydrogen" refers to a hydrogen atom bonded to an oxygen atom, sulfur atom or nitrogen atom, or a hydrogen atom of an active methylene group. Specific examples thereof include hydrogen atoms incorporated in atom groupings such as an -OH group, -C(=O)OH group, -C(=O)H group, -SH group, - NH₂ group, -NH- group, or -C(=O)-C(-H₂)-C(=O)- group.

The production method according to the present embodiment is a method for producing an isocyanate compound, the method including:
a first reaction step of reacting a primary amine compound, a carbonic acid derivative and a first blocking agent by conducting a heat treatment in the presence of at least one compound selected from the group consisting of secondary amine compounds of general formula (1) shown below and tertiary amine compounds of general formula (II) shown below, thus obtaining a blocked isocyanate compound, and
a second reaction step of decomposing the blocked isocyanate compound into a an isocyanate compound and the first blocking agent by thermal decomposition in the presence or absence of at least one compound selected from the group consisting of secondary amine compounds of general formula (I) shown below and tertiary amine compounds of general formula (11) shown below, thus obtaining the isocyanate compound.

In at least the first reaction step described above, and preferably in both the first reaction step and second reaction step described above, reaction is conducted in the presence of at least one compound selected from the group consisting of secondary amine compounds of general formula (1) shown below and tertiary amine compounds of general formula (II) shown below. As a result, a blocked isocyanate compound with good suppression of thermal denaturation and coloration can be obtained in the first reaction step. Moreover, an isocyanate compound with good suppression of thermal denaturation and coloration can be obtained in the second reaction step.

Alternatively, the production method according to the present embodiment is a method for producing a blocked isocyanate compound including an addition-elimination reaction step of reacting a first blocked isocyanate compound and a second blocking agent by an addition-elimination reaction in the presence of at least one compound selected from the group consisting of secondary amine compounds of general formula (I) shown below and tertiary amine compounds of general formula (II) shown below, thus obtaining a second blocked isocyanate compound.

The second blocking agent is a compound different from a first blocking agent which blocks isocyanate groups in the first blocked isocyanate compound.

The first blocked isocyanate compound and the second blocked isocyanate compound have structures different from each other.

In the above addition-elimination reaction, the reaction is conducted in the presence of at least one compound selected from the group consisting of secondary amine compounds of general formula (1) shown below and tertiary amine compounds of general formula (II) shown below. As a result, a blocked isocyanate compound with good suppression of thermal denaturation and coloration can be obtained in the addition-elimination reaction step.

Alternatively, the production method according to the present embodiment may be conducted by combining at least two steps selected from the group consisting of the above first reaction step, the above addition-elimination reaction step, and the above second reaction step.

In other words, the production method according to the present embodiment is a method for producing a blocked isocyanate compound, the method including:
a reaction step of reacting a primary amine compound, a carbonic acid derivative and a first blocking agent by conducting a heat treatment in the presence of at least one compound selected from the group consisting of secondary amine compounds of general formula (I) shown below and tertiary amine compounds of general formula (11) shown below, thus obtaining a first blocked isocyanate compound, and
an addition-elimination reaction step of reacting the first blocked isocyanate compound and a second blocking agent by an addition-elimination reaction in the presence or absence of at least one compound selected from the group consisting of secondary amine compounds of the general formula (I) and tertiary amine compounds of the general formula (II), thus obtaining a second blocked isocyanate compound.

The second blocking agent is a compound different from the first blocking agent and a third blocking agent derived from the carbonic acid derivative.

The first blocked isocyanate compound and the second blocked isocyanate compound have structures different from each other.

The production method according to the present embodiment is a method for producing an isocyanate compound, the method including:
an addition-elimination reaction step of reacting a first blocked isocyanate compound and a second blocking agent by an addition-elimination reaction in the presence of a secondary amine compound of general formula (I) shown below and a tertiary amine compound of general formula (11) shown below, thus obtaining a second blocked isocyanate compound, and
a reaction step of decomposing the second blocked isocyanate compound into a second blocking agent and an isocyanate compound by conducting a heat treatment in the presence or absence of at least one compound selected from the group consisting of secondary amine compounds of the general formula (I) and tertiary amine compounds of the general formula (II), thus obtaining the isocyanate compound.

The second blocking agent is a compound different from the first blocking agent which blocks isocyanate groups in the first blocked isocyanate compound.

The first blocked isocyanate compound and the second blocked isocyanate compound have structures different from each other.

Alternatively, the production method according to the present embodiment is a method for producing an isocyanate compound, the method including:
a first reaction step of reacting a primary amine compound, a carbonic acid derivative and a first blocking agent by conducting a heat treatment in the presence of at least one compound selected from the group consisting of secondary amine compounds of general formula (1) shown below and tertiary amine compounds of general formula (II) shown below, thus obtaining a blocked isocyanate compound,
an addition-elimination reaction step of reacting the first blocked isocyanate compound and a second blocking agent by an addition-elimination reaction in the presence or absence of at least one compound selected from the group consisting of secondary amine compounds of the general formula (I) and tertiary amine compounds of the general formula (11), thus obtaining a second blocked isocyanate compound, and
a second reaction step of decomposing the second blocked isocyanate compound into a second blocking agent and an isocyanate compound by conducting a heat treatment in the presence or absence of at least one compound selected from the group consisting of secondary amine compounds of the general formula (1) and tertiary amine compounds of the general formula (II), thus obtaining the isocyanate compound.

The second blocking agent is a compound different from the first blocking agent and a third blocking agent derived from the carbonic acid derivative. The third blocking agent derived from the carbonic acid derivative is described below in detail as a compound derived from the carbonic acid derivative.

The first blocked isocyanate compound and the second blocked isocyanate compound have structures different from each other.

The first reaction step, the addition-elimination reaction step and the second reaction step are each described below in detail in relation to a method for producing a blocked isocyanate compound according to a first embodiment, a method for producing a blocked isocyanate compound according to a second embodiment, and a method for producing an isocyanate compound according to a third embodiment. Further, a method for producing an isocyanate compound according to a fourth embodiment and a method for producing an isocyanate compound according to a fifth embodiment are also described as examples of methods for producing an isocyanate compound in which the above steps are combined.

### <<Method for Producing Blocked Isocyanate Compound according to First Embodinient>>

The method for producing a blocked isocyanate compound according to the present embodiment (hereinafter sometimes referred to as simply "the production method of the first embodiment") includes a reaction step (hereinafter sometimes referred to as the "first reaction step") of reacting a primary amine compound, a carbonic acid derivative and a blocking agent by conducting a heat treatment in the presence of at least one compound selected from the group consisting of secondary amine compounds of general formula (I) shown below (hereinafter sometimes referred to as "secondary amine compounds (I)") and tertiary amine compounds of general formula (II) shown below (hereinafter sometimes referred to as "tertiary amine compounds (11)"), thus obtaining a blocked isocyanate compound.

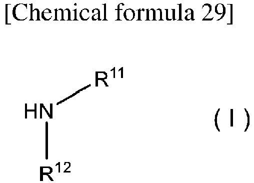

In general formula (I), R¹¹ and R¹² are each independently a monovalent organic group. R¹¹ and R¹² may be bonded together to form a cyclic structure via a carbon-carbon bond, a carbon-oxygen-carbon bond, or a carbon-nitrogen-carbon bond. At least one of R¹¹ and R¹² has an aromatic group.

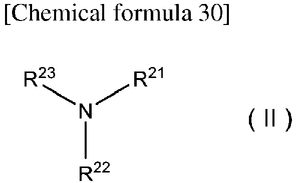

In general formula (II), R²¹, R²² and R²³ are each independently a monovalent organic group. Each combination of R²¹ and R²², R²² and R²³, and R²³ and R²¹ may be independently bonded together to form a cyclic structure via a carbon-carbon bond, a carbon-oxygen-carbon bond, or a carbon-nitrogen-carbon bond. At least one of R²¹, R²² and R²³ has an aromatic group.

The inventors of the present invention discovered that the problems of thermal denaturation and coloration could be resolved by reacting a primary amine compound, a carbonic acid derivative and a blocking agent in the presence of a specific amine compound having an aromatic group to produce a blocked isocyanate, thus enabling them to complete the present invention.

As described above, conventionally, compounds having an amino group bonded to a carbon atom having aromaticity are known to oxidize readily and cause coloration. Accordingly, even though compounds having an amino group bonded to a carbon atom having aromaticity have been used in products such as rubber degradation inhibitors, the coloration of which is not a problem, these compounds have not been used in products, the coloration of whih would be problematic. However, surprisingly, by employing the production method of the first embodiment, and using a specific amine compound having an aromatic group, it has become evident for the first time that not only do coloration problems not occur, but a particularly remarkable effect is achieved wherein the levels of thermal denaturation and coloration are actually improved (suppressed).

Accordingly, the production method of the first embodiment may also be described as a method for improving thermal denaturation and coloration during production of a blocked isocyanate compound, or a method for suppressing thermal denaturation and coloration during production of a blocked isocyanate compound.

Next, each of the steps of the production method of the first embodiment is described below in further detail.

### <First Reaction Step>

In the first reaction step, a primary amine compound, a carbonic acid derivative and a blocking agent are reacted by heat treatment in the presence of at least one compound selected from the group consisting of the secondary amine compounds (I) and the tertiary amine compounds (11), thus obtaining a blocked isocyanate compound.

In the first reaction step, from the viewpoint of reducing coloration of the blocked isocyanate compound and the blocked isocyanate composition, the amount present of the secondary amine compound (I) within the composition is preferably large. Specifically, the amount present of the secondary amine compound (I), relative to the total mass of the primary amine compound and the blocking agent, is preferably greater than 0% by mass, more preferably at least 1 ppm by mass, even more preferably at least 1% by mass, and particularly preferably 50% by mass or more. On the other hand, when the amount present of the secondary amine compound (1) within the composition containing the blocked isocyanate compound is large, there is a case in which at least one denaturation among denaturation of the isocyanate compound and denaturation of the blocked isocyanate compound is accelerated due to the basicity of the secondary amine compound (I). Consequently, the amount present of the secondary amine compound (I), relative to the total mass of the primary amine compound and the blocking agent, is preferably less than 1,000% by mass, more preferably less than 100% by mass, even more preferably not more than 90% by mass, still more preferably not more than 80% by mass, and most preferably 50% by mass or less. Further, a combination of two or more types of the secondary amine compound (I) may be added to the composition containing the primary amine compound, the carbonic acid derivative and the blocking agent. The thermal denaturation and coloration suppression effect of the secondary amine compound (I) varies depending on the chemical structure of the secondary amine compound, and by including two or more types of the secondary amine compound, the denaturation and coloration suppression effect can be enhanced.

In the first reaction step, from the viewpoint of reducing coloration of the blocked isocyanate compound and the blocked isocyanate composition, the amount present of the tertiary amine compound (II) within the composition is preferably large. Specifically, the amount present of the tertiary amine compound (11), relative to the total mass of the primary amine compound and the blocking agent, is preferably greater than 0% by mass, more preferably at least 1 ppm by mass, even more preferably at least 1% by mass, and particularly preferably 50% by mass or more. On the other hand, when the amount present of the tertiary amine compound (II) within the composition containing the blocked isocyanate compound is large, there is a case in which at least one denaturation among denaturation of the isocyanate compound and denaturation of the blocked isocyanate compound is accelerated due to the basicity of the tertiary amine compound (II). Consequently, the amount present of the tertiary amine compound (II), relative to the total mass of the primary amine compound and the blocking agent, is preferably less than 1,000% by mass, more preferably less than 100% by mass, even more preferably not more than 90% by mass, still more preferably not more than 80% by mass, and most preferably 50% by mass or less. Further, a combination of two or more types of the tertiary amine compound (II) may be added to the composition containing the primary amine compound, the carbonic acid derivative and the blocking agent. The thermal denaturation and coloration suppression effect of the tertiary amine compound (1) varies depending on the chemical structure of the tertiary amine compound, and by including two or more types of the tertiary amine compound, the thermal denaturation and coloration suppression effect can be enhanced.

Either of the secondary amine compound (I) and the tertiary amine compound (II) may be added alone to the composition containing the primary amine compound, the carbonic acid derivative and the blocking agent, or both the secondary amine compound (I) and the tertiary amine compound (II) may be added. Comparison of the secondary amine compound (I) and the tertiary amine compound (II) reveals that the secondary amine compound (1) exhibits a greater effect in reducing coloration under high temperatures. On the other hand, the tertiary amine compound (II) exhibits less temperature dependency of the coloration reduction effect, thereby making it possible to exhibit a coloration reduction effect across a wider temperature range.

Further, because the secondary amine compound (I) has an active hydrogen group, a portion of the blocking agent in the blocked isocyanate compound is substituted with the secondary amine compound (I). As a result, there is possibility that urea groups formed by reaction between the secondary amine compound (I) and an isocyanate group may be retained in the blocked isocyanate compound. Because this type of reaction does not occur with the tertiary amine compound (II), the tertiary amine compound (II) is more preferable.

Furthermore, in the case where the secondary amine compound (I) and the tertiary amine compound (II) are included at the same time, the separation operation becomes more complex, when the secondary amine compound (I) and the tertiary amine compound (11) are recovered, for example,. However, the secondary amine compound (I) and the tertiary amine compound (II) exhibit different effects in terms of suppressing thermal denaturation and coloration, and including both compounds produces a synergistic effect in relation to the suppression of thermal denaturation and coloration. Consequently, including the secondary amine compound (I) and the tertiary amine compound (II) simultaneously is preferred.

In the first reaction step, although there are no particular limitations on the total amount present of the secondary amine compound (I) and the tertiary amine compound (11), from the viewpoint of reducing coloration of the blocked isocyanate compound and the blocked isocyanate composition, the amount present within the composition is preferably large. Specifically, the total amount present of the secondary amine compound (1) and the tertiary amine compound (11), relative to the total mass of the primary amine compound and the blocking agent, is preferably greater than 0% by mass, more preferably at least 1 ppm by mass, even more preferably at least 1% by mass, and particularly preferably 50% by mass or more. On the other hand, when the amount present of the secondary amine compound (I) within the composition containing the blocked isocyanate compound is large, there is a case in which at least one denaturation among denaturation of the isocyanate compound and denaturation of the blocked isocyanate compound is accelerated due to the basicity of the secondary amine compound (I) and the tertiary amine compound (II). Consequently, the total amount present of the secondary amine compound (1) and the tertiary amine compound (11), relative to the total mass of the primary amine compound and the blocking agent, is preferably less than 1,000% by mass, more preferably less than 100% by mass, even more preferably not more than 90% by mass, still more preferably not more than 80% by mass, and most preferably 50% by mass or less.

The secondary amine compound (I) and the tertiary amine compound (II) make it possible to further enhance the thermal denaturation and coloration suppression effect by combining at least one thereof with an antioxidant different from the secondary amine compound (I) and the tertiary amine compound (II). Further, the thermal denaturation and coloration suppression effect can be further enhanced by combining the secondary amine compound (I) and the tertiary amine compound (II) with an antioxidant.

There are no particular limitations on the antioxidant different from the secondary amine compound (I) and the tertiary amine compound (II), provided the antioxidant is capable of enhancing the thermal denaturation and coloration suppression effect, but in terms of having a recognized coloration suppression effect, a phenol-based antioxidant or a phosphorus-based antioxidant is preferred. Among these, from the viewpoint of providing a particularly superior thermal denaturation and coloration suppression effect when combined with the secondary amine compound (1) and the tertiary amine compound (11), a phenol-based antioxidant is preferred.

In the first reaction step, although the ratio between the amounts added of the primary amine compound and the blocking agent may be selected as appropriate, the ratio between the molar amount of amino groups in the primary amine compound and the molar amount of active hydrogen groups in the blocking agent is typically either 1:1, of the ratio is adjusted so that the molar amount of active hydrogen groups in the blocking agent is larger than the molar amount of amino groups in the primary amine compound.

Further, in the first reaction step, although the ratio between the amounts added of the primary amine compound and the blocking agent may be selected as appropriate, when expressed as a mass ratio, it is typically preferable that the blocking agent exist in an amount that provides an excess over the primary amine compound, and for example, the mass ratio between the amounts added of the primary amine compound and the blocking agent may be set within a range from 1: 1 to 1:999.

Furthermore, in the first reaction step, although the ratio between the amounts added of the primary amine compound and the carbonic acid derivative may be selected as appropriate, the ratio between the molar amount of amino groups in the primary amine compound and the molar amount of carbonyl groups in the carbonic acid derivative is typically within a range from 1:0.5 to 1:20.

Further, in the first reaction step, although the ratio between the amounts added of the primary amine compound and the carbonic acid derivative may be selected as appropriate, when expressed as a mass ratio, the amount added of the carbonic acid derivative may be selected so that, for example, the mass ratio between the primary amine compound and the carbonic acid derivative falls within a range from 1:0.01 to 1:99.

In the first reaction step, the combination of the primary amine compound, the carbonic acid derivative, the blocking agent, the secondary amine compound (1) and the tertiary amine compound (II) may be selected as appropriate, but in the case where a distillation separation is conducted in the first reaction step, progression of the reaction can be accelerated by actively removing compounds derived from the carbonic acid derivative from the system, and therefore the boiling point of the compounds derived from the carbonic acid derivative are preferably lower than the boiling point of the blocking agent. In addition, in terms of reducing coloration, it is preferable that the secondary amine compound (1) and the tertiary amine compound (11) exist in the obtained blocked isocyanate composition. Further, considering this viewpoint, the boiling points of the secondary amine compound (I) and the tertiary amine compound (II) are preferably higher than the boiling point of the compounds derived from the carbonic acid derivative. Here, these compounds derived from the carbonic acid derivative are compounds in which an active hydrogen is bonded to R⁶¹ or R⁶² in general formula (VI) shown below, the compounds being obtained in a separate form from the carbonic acid derivative, and specific examples thereof include hydroxy compounds, ammonia, and primary or secondary amine compounds.

There are no particular limitations on the reaction temperature, and the reaction temperature may be selected appropriately in accordance with the reaction rate between the primary amine compound, the carbonic acid derivative and the blocking agent, and the levels of thermal denaturation and coloration. From the viewpoint of suppressing denaturation of the blocked isocyanate compound, the reaction temperature is preferably not higher than 350°C, more preferably not higher than 300°C, and even more preferably 260°C or lower. On the other hand, when the reaction temperature is low, the temperature of a condenser must be set to a low temperature, which may sometimes require a new equipment, and therefore the reaction temperature is preferably at least 50°C, more preferably at least 80°C, and even more preferably 100°C or higher. Further, in the case of production of a blocked isocyanate compound in the presence of the secondary amine compound (I), the coloration reduction effect is exhibited more distinctly as the temperature is increased, and therefore the reaction temperature for production of a blocked isocyanate is preferably at least 120°C, more preferably at least 160°C, and even more preferably 200°C or higher.

Furthermore, although the reaction pressure varies depending on the types of compounds used and the reaction temperature, the reaction pressure may be any of a reduced pressure, normal pressure, or a pressurized state, and the reaction is typically conducted at an absolute pressure within a range from at least 20 Pa to not more than 2×10⁷ Pa.

The first reaction step may be conducted in the presence of oxygen. In the case where the first reaction step is carried out in the presence of oxygen, thermal denaturation and coloration of the blocked isocyanate compound tends to be caused, and therefore it is preferable that the amount present of oxygen inside the blocked isocyanate production apparatus be reduced. On the other hand, in the case of a large-scale production facility, reducing the amount present of oxygen inside the production apparatus of the blocked isocyanate compound requires that air leakage into the production equipment is reduced, and therefore the design standards for the facility become more stringent, thereby causing an increase in equipment costs. From this viewpoint, the oxygen concentration in the gas supplied during the first reaction step is preferably controlled under stringent conditions, and is preferably controlled to a level exceeding 0% by volume, more preferably controlled to a level exceeding 0.0001% by volume, and even more preferably controlled to a level exceeding 0.001 % by volume.

In the first reaction step, at least one compound selected from the group consisting of the secondary amine compounds (1) and the tertiary amine compounds (11) is present, and therefore thermal denaturation and coloration of the blocked isocyanate compound can be reduced even in the presence of oxygen. However, as mentioned above, an increase in the amount present of oxygen promotes thermal denaturation and coloration, and therefore the amounts used of the secondary amine compound (I) and the tertiary amine compound (II) are required to be increased to suppress thermal denaturation and coloration, which is undesirable. Accordingly, when considered from this perspective, the first reaction step is preferably operated under conditions in which the oxygen concentration in the gas supplied to the first reaction step is low, and is preferably controlled to 21% by volume or less, more preferably 10% by volume or less, even more preferably 1% by volume or less, still more preferably 0.1% by volume or less, particularly preferably 0.01% by volume or less, and most preferably 0.001% by volume or less, relative to the total volume of gas. Measurement of the oxygen concentration may be conducted using a conventional technique such as gas chromatography or trace oxygen analysis by electrochemical methods.

In the first reaction step, an optional solvent may be used in any desired proportion. The solvent is preferably an inert solvent that exhibits no reactivity with the primary amine compound, the carbonic acid derivative, the blocking agent, the blocked isocyanate compound or the like. Preferred examples of such solvents include ester-based solvents, ether-based solvents, phosphate ester-based solvents, hydrocarbon-based solvents, aromatic hydrocarbon-based solvents, and carbonic acid derivative-based solvents.

In the first reaction step, the reaction mixture containing the primary amine compound, the carbonic acid derivative and the blocking agent may also contain an optional metal in any desired proportion. The form of the metal may be a complex or a solid. Metals reduce the thermal decomposition temperature of blocked isocyanate compounds and accelerate the reaction between the primary amine compound and the carbonic acid derivative, but on the other hand, may sometimes cause thermal denaturation, degradation and coloration, and therefore the amount of the metal relative to the mass of the blocked isocyanate compound is preferably less than 10% by mass, more preferably less than 1% by mass, and even more preferably less than 0.1 ppm by mass.

In the first reaction step, the reaction mixture containing the primary amine compound, the carbonic acid derivative and the blocking agent may also contain an organic acid, inorganic acid, organic base or inorganic base. These acids and bases act as catalysts in the thermal decomposition of the blocked isocyanate, enabling the temperature required for thermal decomposition to be lowered, and also promote the reaction between the primary amine compound and the carbonic acid derivative. On the other hand, these acids and bases also act as catalysts of side reactions in the thermal decomposition of the blocked isocyanate, and from that viewpoint, the amount of these organic acids, inorganic acids, organic bases or inorganic bases, relative to the mass of the blocked isocyanate compound, is preferably less than 10% by mass, more preferably less than 1% by mass, even more preferably less than 0.1% by mass, and particularly preferably less than 1 ppb by mass.

In the first reaction step, the blocked isocyanate compound is obtained as a composition containing the blocked isocyanate compound (hereinafter sometimes referred to as the "blocked isocyanate composition").

The term "blocked isocyanate composition" means a composition containing a blocked isocyanate compound in an amount exceeding 0 wt% but not more than 100 wt% of the composition. There are no particular limitations on the blocked isocyanate composition, except that a blocked isocyanate compound is included therein, and the blocked isocyanate composition may also contain a solvent, blocking agent, isocyanate, catalyst, primary amine compound or the like in any desired proportion.

In light of these circumstances, the blocked isocyanate composition may be used directly as the blocked isocyanate compound, or the blocked isocyanate compound may be used after purifying it from the blocked isocyanate composition.

### (Reaction Apparatus)

There are no particular limitations on the reaction apparatus, and conventional blocked isocyanate production apparatus may be used. Examples of methods that may be used include a method in which the blocked isocyanate compound is synthesized in a batch manner by adding a mixture containing the primary amine compound, the carbonic acid derivative and the blocking agent to a vessel to which at least one device selected from the group consisting of a condenser device and a treatment device has been connected, heating the vessel to produce a blocked isocyanate compound, and then or simultaneously guiding the vapor containing compounds derived from the carbonic acid derivative into the condenser device or treatment device, and a method in which the blocked isocyanate compound is obtained in a continuous manner by continuously introducing a mixture containing the primary amine compound, the carbonic acid derivative and the blocking agent into a distillation column that has been heated to a prescribed reaction temperature, thereby producing the blocked isocyanate compound and simultaneously separating the produced vapor containing compounds derived from the carbonic acid derivative. Further, the primary amine compound and the carbonic acid derivative may also be reacted either partially or completely in the presence or absence of a blocking agent to obtain the blocked isocyanate compound from the obtained reaction product.

In the reaction apparatus, the material of regions that make contact with the mixture containing the primary amine compound, the carbonic acid derivative and the blocking agent, and also the composition containing the blocked isocyanate compound produced by the reaction, may be conventional materials, unless they have any adverse effects such as causing denaturation of the primary amine compound, the carbonic acid derivative, the blocking agent, the blocked isocyanate compound, or any other included component. Specific examples of such materials include steel, stainless steel, ceramics, carbon, and lined versions of these materials.

In a case where a distillation device is used, there are no particular limitations on the form of the distillation device, and various conventional distillation devices such as batch distillation devices, simple distillation devices, distillation devices containing any selected from multistage distillation column, continuous multistage distillation column and packed column, distillation devices in which these are combined may be used.

As the distillation column, any distillation column in which the theoretical number of distillation stages is two or greater may be employed. Further, in the case where the theoretical number of stages is large, a multistage distillation column becomes extremely large, which sometimes makes industrial implementation difficult, and therefore the theoretical number of stages is typically 500 or less.

As the distillation column, any column typically usable as a multistage distillation column, such as plate columns using trays such as a bubble cap tray, porous plate tray, valve tray or cross-flow tray, of packed columns that have been packed with any of various fillers such as Raschig rings, Lessing rings, Pall rings, Berl saddles, interlock saddles, Dickson packing, McMahon packing, Helipack, Sulzer packing or Mellapak, may be used. Moreover, plate-packed mixed columns in which a plate portion and a filler-packed portion are combined may also be used favorably.

Next, the raw materials used and the product obtained in the production method of the first embodiment are described below in further detail.

### <Secondary Amine Compound (1)>

The secondary amine compound (1) is a compound of general formula (I) shown below.

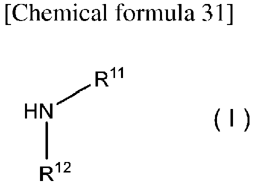

In general formula (I), R¹¹ and R¹² are each independently a monovalent organic group. R¹¹ and R¹² may be bonded together to form a cyclic structure via a carbon-carbon bond, a carbon-oxygen-carbon bond, or a carbon-nitrogen-carbon bond. At least one of R¹¹ and R¹² has an aromatic group.

Compounds having an amino group bonded directly to an aromatic ring are known to cause coloration in the presence of oxygen or the like. However, surprisingly, it became very evident that the secondary amine compound (1) actually exhibited a coloration reduction effect. Although the reasons for this effect are not entirely clear, it is generally known that thermal denaturation and coloration tend to occur in processes of producing a blocked isocyanate compound from an amine, a carbonic acid derivative and a blocking agent, and it is surmised that these issues are due to oxidation and thermal denaturation of either the blocked isocyanate compound itself or isocyanate compounds or the like that exist as reaction intermediates. It is surmised that the secondary amine compound (I) interacts with oxides such as oxygen and functions as an antioxidant that is oxidized instead of isocyanate compounds or derivatives thereof which exist at equilibrium reactions in the process of producing the blocked isocyanate compound by reacting the amine, the carbonic acid derivative and the blocking agent, and is also converted to colorless or lightly colored substances upon heating. Further, although the secondary amine compound (1) is an amine-based compound, which are generally known to accelerate the denaturation of blocked isocyanate compounds and the isocyanate compounds that exist as reaction intermediates, the denaturation acceleration observed with typical aliphatic amine compounds or heterocyclic amine compounds is either not observed, or is very slight. It is surmised that this is due to the facts that the secondary amine compound (I) has an aromatic group bonded directly to the nitrogen atom, which results in a low electron density on the nitrogen atom and poor nucleophilicity, and the substituents bonded to the nitrogen atom produce significant steric hindrance around the nitrogen atom.

### [R¹¹ and R¹²]

R¹¹ and R¹² are each independently a monovalent organic group. R¹¹ and R¹² may be bonded together to form a cyclic structure via a carbon-carbon bond, a carbon-oxygen-carbon bond, or a carbon-nitrogen-carbon bond. At least one of R¹¹ and R¹² has an aromatic group.

Among them, each of R¹¹ and R¹² is preferably a substituted or unsubstituted monovalent aliphatic hydrocarbon group of 1 or more but not more than 70 carbon atoms or substituted or unsubstituted monovalent aromatic hydrocarbon group of 6 or more but not more than 70 carbon atoms, which may have an ether group, carbonyl group or ester group.

The number of carbon atoms in the aliphatic hydrocarbon group as R¹¹ and R¹² is 1 or more but not more than 70, and is preferably 1 or more but not more than 20, more preferably 1 or more but not more than 12, and even more preferably 1 or more but not more than 10.

Specific examples of the aliphatic hydrocarbon group as R¹¹ and R¹² include alkyl groups such as a methyl group, ethyl group, propyl group (each isomer), butyl group (each isomer), pentyl group (each isomer), hexyl group (each isomer), heptyl group (each isomer), octyl group (each isomer), nonyl group (each isomer), decyl group (each isomer), undecyl group (each isomer), dodecyl group (each isomer), tridecyl group (each isomer), tetradecyl group (each isomer), pentadecyl group (each isomer), hexadecyl group (each isomer), heptadecyl group (each isomer), octadecyl group (each isomer), nonadecyl group (each isomer), and eicosyl group (each isomer); and cycloalkyl groups such as a cyclopentyl group, cyclohexyl group, cycloheptyl group, cyclooctyl group, cyclononyl group, and cyclodecyl group.

The number of carbon atoms in the aromatic hydrocarbon group as R¹¹ and R¹² is 6 or more but not more than 70, and is preferably 6 or more but not more than 20, more preferably 6 or more but not more than 12, and even more preferably 6 or more but not more than 10.

Specific examples of the aromatic hydrocarbon group as R¹¹ and R¹² include aryl groups such as a phenyl group, naphthyl group, anthryl group, pyrenyl group, and phenanthryl group.

Examples of substituents of the aliphatic hydrocarbon group as R¹¹ and R¹² include aromatic hydrocarbon groups, a hydroxyl group, a cyano group, and halogen atoms.

Examples of substituents of the aromatic hydrocarbon group as R¹¹ and R¹² include aliphatic hydrocarbon groups, a hydroxyl group, a cyano group, and halogen atoms.

The aromatic hydrocarbon group that may function as a substituent of the aliphatic hydrocarbon group is preferably an aromatic hydrocarbon group of 6 or more but not more than 10 carbon atoms, and specific examples thereof include the groups mentioned above.

The aliphatic hydrocarbon group that may function as a substituent of the aromatic hydrocarbon group is preferably an aliphatic hydrocarbon group of 1 or more but not more than 10 carbon atoms, and specific examples thereof include the groups mentioned above.

Examples of the halogen atom include a fluorine atom, chlorine atom, bromine atom, and iodine atom.

Although there are no particular limitations on preferred compounds as the secondary amine compound (I), compounds of general formulas (I-1) to (I-3) shown below (hereinafter sometimes referred to as "the secondary amine compound (I-1)" or the like, respectively) are preferred from the viewpoint of ease of availability.

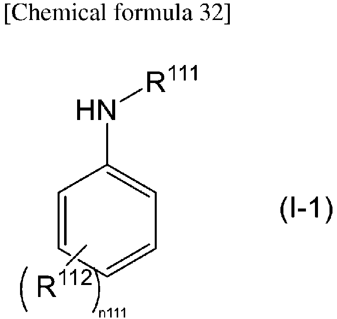

In general formula (I-1), R¹¹¹ is the same as R¹¹ and R¹² described above. R¹¹² is a substituent on the benzene ring, and is a substituted or unsubstituted monovalent aliphatic hydrocarbon group of 1 or more but not more than 12 carbon atoms or substituted or unsubstituted monovalent aromatic hydrocarbon group of 6 or more but not more than 12 carbon atoms, which may have an ether group, carbonyl group or ester group. Further, n111 indicates the number of substituents, and is an integer of at least 0 but not more than 5.

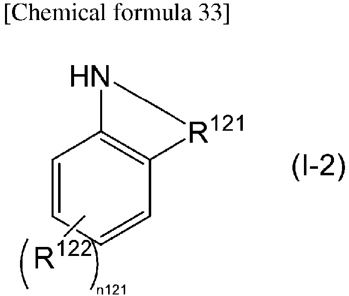

In general formula (1-2), R¹²¹ is a divalent aliphatic hydrocarbon group of 1 or more but not more than 12 carbon atoms or a divalent aromatic hydrocarbon group of 6 or more but not more than 12 carbon atoms. R¹²² is a substituent on the benzene ring, and is a substituted or unsubstituted monovalent aliphatic hydrocarbon group of 1 or more but not more than 12 carbon atoms or substituted or unsubstituted monovalent aromatic hydrocarbon group of 6 or more but not more than 12 carbon atoms, which may have an ether group, carbonyl group or ester group. Further, n121 indicates the number of substituents, and is an integer of at least 0 but not more than 4.

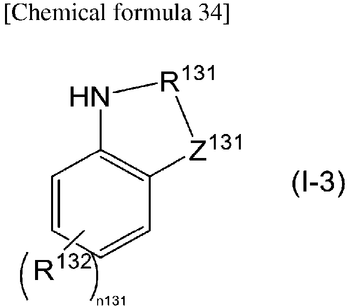

In general formula (1-3), R¹³¹ is a divalent aliphatic hydrocarbon group of 1 or more but not more than 12 carbon atoms or a divalent aromatic hydrocarbon group of 6 or more but not more than 12 carbon atoms. Z¹³¹ is -O-, -NH- or -C(=O)-. R¹³² is a substituent on the benzene ring, and is a substituted or unsubstituted monovalent aliphatic hydrocarbon group of 1 or more but not more than 12 carbon atoms or substituted or unsubstituted monovalent aromatic hydrocarbon group of 6 or more but not more than 12 carbon atoms, which may have an ether group. Further, n131 indicates the number of substituents, and is an integer of at least 0 but not more than 4.

### [R¹¹² and R¹²²]

R¹¹² and R¹²² are each a substituent on a benzene ring.

R¹¹² and R¹²² are each independently a substituted or unsubstituted aliphatic hydrocarbon group of 1 or more but not more than 12 carbon atoms or substituted or unsubstituted aromatic hydrocarbon group of 6 or more but not more than 12 carbon atoms, which may have an ether group, carbonyl group or ester group.

Examples of the aliphatic hydrocarbon group and aromatic hydrocarbon group as R¹¹² and R¹²² include the same groups as those mentioned above as R¹¹ and R¹². Among them, it is preferable that R¹¹² and R¹²² be each independently an unsubstituted monovalent aliphatic hydrocarbon group of 1 or more but not more than 12 carbon atoms, and it is more preferable that R¹¹² and R¹²² be each independently a methyl group, ethyl group, or propyl group (any isomer).

### [R¹³²]

R¹³² is a substituent on a benzene ring.

R¹³² is a substituted or unsubstituted aliphatic hydrocarbon group of 1 or more but not more than 12 carbon atoms or substituted or unsubstituted aromatic hydrocarbon group of 6 or more but not more than 12 carbon atoms, which may have an ether group.

Examples of the aliphatic hydrocarbon group and aromatic hydrocarbon group as R¹³² include the same groups as those mentioned above as R¹¹ and R¹². Among these, R¹³² is preferably an unsubstituted monovalent aliphatic hydrocarbon group of 1 or more but not more than 12 carbon atoms, and more preferably a methyl group, ethyl group, or propyl group (any isomer).

### [n111, n121 and n131]

n111 indicates the number of substituents, and is an integer of at least 0 but not more than 5, preferably an integer of at least 0 but not more than 3, and more preferably 0 or 1.

Each of n121 and n131 indicates a number of substituents, and is an integer of at least 0 but not more than 4, preferably an integer of at least 0 but not more than 3, and more preferably 0 or 1.

### [R¹²¹ and R¹³¹]

The divalent aliphatic hydrocarbon groups as R¹²¹ and R¹³¹ may be saturated or unsaturated.

Examples of the divalent saturated aliphatic hydrocarbon groups as R¹²¹ and R¹³¹ include alkylene groups of 1 or more but not more than 12 carbon atoms, and alkylidene groups of at least 2 but not more than 12 carbon atoms.

Examples of the alkylene groups of 1 or more but not more than 12 carbon atoms include chained alkylene groups of 1 or more but not more than 12 carbon atoms, and cyclic alkylene groups of at least 3 but not more than 12 carbon atoms.

Examples of the chained alkylene groups of 1 or more but not more than 12 carbon atoms include linear or branched chained alkylene groups of 1 or more but not more than 12 carbon atoms. Specific examples of the linear or branched chained alkylene groups of 1 or more but not more than 12 carbon atoms include a methylene group, ethylene group, propylene group, trimethylene group, pentylene group, tetramethylene group, propan-1,2-diyl group, n-hexylene group, pentamethylene group, butan-1,3-diyl group, hexamethylene group, heptamethylene group, octamethylene group, nonamethylene group, decamethylene group, undecamethylene group, and dodecamethylene group.

Examples of the cyclic alkylene groups of at least 3 but not more than 12 carbon atoms include a cyclopropylene group, cyclobutylene group, cyclopentylene group, cyclohexylene group, cycloheptylene group, cyclooctylene group, cyclononylene group, cyclodecylene group, cycloundecylene group, and cyclododecylene group.

Examples of the alkylidene groups of at least 2 but not more than 12 carbon atoms include chained alkylidene groups of at least 2 but not more than 12 carbon atoms, and cyclic alkylidene groups of at least 3 but not more than 12 carbon atoms.

Examples of the chained alkylidene groups of at least 2 but not more than 12 carbon atoms include linear or branched chained alkylidene groups of at least 2 but not more than 12 carbon atoms. Specific examples of the linear or branched chained alkylidene groups of at least 2 but not more than 12 carbon atoms include an ethylidene group, propylidene group, isopropylidene group, butylidene group, pentylidene group, hexylidene group, heptylidene group, octylidene group, nonylidene group, decylidene group, undecylidene group, and dodecylidene group.

Examples of the cyclic alkylidene groups of at least 3 but not more than 12 carbon atoms include a cyclopropylidene group, cyclobutylidene group, cyclohexylidene group, 3,5,5-trimethylcyclohexylidene group, cycloheptylidene group, cyclooctylidene group, cyclononylidene group, cyclodecylidene group, cycloundecylidene group, and cyclododecylidene group.

Examples of the divalent unsaturated aliphatic hydrocarbon group as R¹²¹ and R¹³¹ include alkenylene groups of at least 2 but not more than 12 carbon atoms, alkadienylene groups of at least 2 but not more than 12 carbon atoms, and alkynylene groups of at least 2 but not more than 12 carbon atoms.

Examples of the alkenylene groups of at least 2 but not more than 12 carbon atoms include chained alkenylene groups of at least 2 but not more than 12 carbon atoms, and cyclic alkenylene groups of at least 3 but not more than 12 carbon atoms.

Examples of the chained alkenylene groups of at least 2 but not more than 12 carbon atoms include linear or branched chained alkenylene groups of at least 2 but not more than 12 carbon atoms. Specific examples of the linear or branched chained alkenylene groups of at least 2 but not more than 12 carbon atoms include a vinylene group, propenylene group, isopropenylene group, butenylene group, pentenylene group, hexenylene group, heptenylene group, octenylene group, nonenylene group, decenylene group, undecenylene group, and dodecenylene group.

Examples of the cyclic alkenylene groups of at least 3 but not more than 12 carbon atoms include a cyclopropenylene group, cyclobutenylene group, cyclopentenylene group, cyclohexenylene group, cycloheptenylene group, cyclooctenylene group, cyclononenylene group, cyclodecenylene group, cycloundecenylene group, and cyclododecenylene group.

Examples of the alkadienylene groups of at least 3 but not more than 12 carbon atoms include chained alkadienylene groups of at least 3 but not more than 12 carbon atoms, and cyclic alkadienylene groups of at least 4 but not more than 12 carbon atoms.

Examples of the chained alkadienylene groups of at least 3 but not more than 12 carbon atoms include linear or branched chained alkadienylene groups of at least 3 but not more than 12 carbon atoms. Specific examples of the linear or branched chained alkadienylene groups of at least 3 but not more than 12 carbon atoms include a propadienylene group, butadienylene group, 2-ethylbutadienylene group, pentadienylene group, hexadienylene group, heptadienylene group, octadienylene group, nonadienylene group, decadienylene group, undecadienylene group, and dodecadienylene group.

Examples of the cyclic alkadienylene groups of at least 4 but not more than 12 carbon atoms include a cyclobutadienylene group, cyclopentadienylene group, cyclohexadienylene group, cycloheptadienylene group, cyclooctadienylene group, cyclononadienylene group, cyclodecadienylene group, cycloundecadienylene group, and cyclododecadienylene group.

Examples of the alkynylene group of at least 2 but not more than 12 carbon atoms include chained alkynylene groups of at least 2 but not more than 12 carbon atoms, and cyclic alkynylene groups of at least 4 but not more than 12 carbon atoms.

Examples of the chained alkynylene groups of at least 2 but not more than 12 carbon atoms include linear or branched chained alkynylene groups of at least 2 but not more than 12 carbon atoms. Specific examples of the linear or branched chained alkynylene groups of at least 2 but not more than 12 carbon atoms include a ethynylene group, propynylene group, isopropynylene group, butynylene group, pentynylene group, hexynylene group, heptynylene group, octynylene group, nonynylene group, decynylene group, undecynylene group, and dodecynylene group.

Examples of the cyclic alkynylene groups of at least 4 but not more than 12 carbon atoms include a cyclobutynylene group, cyclopentynylene group, cyclohexynylene group, cycloheptynylene group, cyclooctynylene group, cyclononynylene group, cyclodecynylene group, cycloundecynylene group, and cyclododecynylene group.

Among the secondary amine compounds (I-1) to (I-3) described above, compounds in which the amino group bonded to the aromatic hydrocarbon is bonded to an aliphatic hydrocarbon are preferred in comparison with compounds in which the amino group bonded to the aromatic hydrocarbon is bonded directly to an aromatic hydrocarbon and compounds in which the amino group bonded to the aromatic hydrocarbon is bonded directly to an unsaturated hydrocarbon, in terms of further improving a coloration reduction effect.

Furthermore, structures composed entirely of carbon atoms and hydrogen atoms, with the exception of the monovalent secondary amino group bonded directly to the aromatic hydrocarbon, are preferred from the viewpoint of thermal stability.

In other words, in the secondary amine compound (I-1), it is preferable that R¹¹¹ be a monovalent aliphatic hydrocarbon group, R¹¹² be an unsubstituted aliphatic hydrocarbon group of 1 or more but not more than 12 carbon atoms or an unsubstituted aromatic hydrocarbon group of 6 or more but not more than 12 carbon atoms, and n111 be either 0 or 1.

In the secondary amine compound (I-1), it is more preferable that R¹¹¹ be a monovalent aliphatic hydrocarbon group of 1 or more but not more than 12 carbon atoms, R¹¹² be a methyl group or ethyl group, and n111 be either 0 or 1.

Further, in the secondary amine compound (I-2), it is preferable that R¹²¹ be a divalent aliphatic hydrocarbon group, R¹²² be an unsubstituted aliphatic hydrocarbon group of 1 or more but not more than 12 carbon atoms or an unsubstituted aromatic hydrocarbon group of 6 or more but not more than 12 carbon atoms, and n121 be either 0 or 1.

In the secondary amine compound (I-2), it is more preferable that R¹²¹ be a divalent aliphatic hydrocarbon group of 1 or more but not more than 12 carbon atoms, R¹²² be a methyl group or ethyl group, and n121 be either 0 or 1.

Further, in the secondary amine compound (I-3), it is preferable that R¹³¹ be a divalent aliphatic hydrocarbon group, R¹³² be an unsubstituted aliphatic hydrocarbon group of 1 or more but not more than 12 carbon atoms or an unsubstituted aromatic hydrocarbon group of 6 or more but not more than 12 carbon atoms, and n131 be either 0 or 1.

In the secondary amine compound (I-3), it is more preferable that R¹³¹ be a divalent aliphatic hydrocarbon group of 1 or more but not more than 12 carbon atoms, R¹³² be a methyl group or ethyl group, and n131 be either 0 or 1.

Specific examples of the preferred secondary amine compound (I-1) include N-methylaniline, N-ethylaniline, N-propylaniline (each isomer), N-butylaniline (each isomer), N-pentylaniline (each isomer), N-hexylaniline (each isomer), N-heptylaniline (each isomer), N-octylaniline (each isomer), N-nonylaniline (each isomer), N-decylaniline (each isomer), N-methyltoluidine (each isomer), N-ethyltoluidine (each isomer), N-propyltoluidine (each isomer), N-butyltoluidine (each isomer), N-pentyltoluidine (each isomer), N-hexyltoluidine (each isomer), N-heptyltoluidine (each isomer), N-octyltoluidine (each isomer), N-aryltoluidine (each isomer), N-arylaniline, 3-(phenylamino)propionitrile, 3-(O-toluylamino)propionitrile, 2-anilinoethanol, fluoro-N-methylaniline (each isomer), chloro-N-methylaniline (each isomer), bromo-N-methylaniline (each isomer), iodo-N-methylaniline (each isomer), fluoro-N-ethylaniline (each isomer), chloro-N-ethylaniline (each isomer), bromo-N-ethylaniline (each isomer), iodo-N-ethylaniline (each isomer), fluoro-N-propylaniline (each isomer), chloro-N-propylaniline (each isomer), bromo-N-propylaniline (each isomer), and iodo-N-propylaniline (each isomer).

Specific examples of the preferred secondary amine compound (I-2) include 2,3-dihydro-4(1H)-quinolinone, diphenylamine, indoline, methylindoline (each isomer), 1,2,3,4-tetrahydroquinoline, and methyl-1,2,3,4-tetrahydroquinoline (each isomer).

Specific examples of the preferred secondary amine compound (I-3) include 3,4-dihydro-2H-1,4-benzoxazine.

Among them, examples of the secondary amine compound (I) preferred in terms of ensuring higher thermal stability of the compound as well as a superior coloration suppression effect include: N-alkylaniline compounds such as N-methylaniline, N-ethylaniline, N-propylaniline (each isomer), N-butylaniline (each isomer), N-pentylaniline (each isomer), N-hexylaniline (each isomer), N-heptylaniline (each isomer), N-octylaniline (each isomer), N-nonylaniline (each isomer), and N-decylaniline (each isomer); N-alkyltoluidine compounds such as N-methyltoluidine (each isomer), N-ethyltoluidine (each isomer), N-propyltoluidine (each isomer), N-butyltoluidine (each isomer), N-pentyltoluidine (each isomer), N-hexyltoluidine (each isomer), N-heptyltoluidine (each isomer), N-octyltoluidine (each isomer), N-nonyltoluidine (each isomer), and N-decyltoluidine (each isomer); and aromatic cyclic amine compounds such as indoline, methylindoline (each isomer), 1,2,3,4-tetrahydroquinoline, and methyl-1,2,3,4-tetrahydroquinoline.

### <Tertiary Amine Compound (II)>

The tertiary amine compound (II) is a compound of general formula (II) shown below.

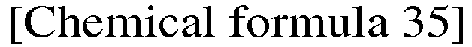

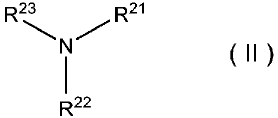

In general formula (II), R²¹, R²² and R²³ are each independently a monovalent organic group. Each combination of R²¹ and R²², R²² and R²³, and R²³ and R²¹ may be independently bonded together to form a cyclic structure via a carbon-carbon bond, a carbon-oxygen-carbon bond, or a carbon-nitrogen-carbon bond. At least one of R²¹, R²² and R²³ has an aromatic group.

Compounds having an amino group bonded directly to an aromatic ring are known to cause coloration in the presence of oxygen or the like. However, surprisingly, it became very evident that the tertiary amine compound (II) actually exhibited a coloration reduction effect. Although the reasons for this effect are not entirely clear, it is generally known that thermal denaturation and coloration tend to occur in processes of producing a blocked isocyanate compound from an amine, a carbonic acid derivative and a blocking agent, and it is surmised that these issues are due to oxidation and thermal denaturation of either the blocked isocyanate compound itself or isocyanate compounds or the like that exist as reaction intermediates. It is surmised that the tertiary amine compound (II) interacts with oxides such as oxygen and functions as an antioxidant that is oxidized instead of isocyanate compounds or derivatives thereof which exist at equilibrium reactions in the process of producing the blocked isocyanate compound by reacting the amine, the carbonic acid derivative and the blocking agent, and is also converted to colorless or lightly colored substances upon heating. Further, although the tertiary amine compound (II) is an amine-based compound, which are generally known to accelerate the denaturation of blocked isocyanate compounds and the isocyanate compounds that exist as reaction intermediates, the denaturation acceleration observed with typical aliphatic amine compounds or heterocyclic amine compounds is either not observed, or is very slight. It is surmised that this is due to the facts that the tertiary amine compound (II) has an aromatic group bonded directly to the nitrogen atom, which results in a low electron density on the nitrogen atom and poor nucleophilicity, and the substituents bonded to the nitrogen atom produce significant steric hindrance around the nitrogen atom.

### [R²¹, R²² and R²³]

R²¹, R²² and R²³ are each independently a monovalent organic group. Each combination of R²¹ and R²², R²² and R²³, and R²³ and R²¹ may be independently bonded together to form a cyclic structure via a carbon-carbon bond, a carbon-oxygen-carbon bond, or a carbon-nitrogen-carbon bond. At least one of R²¹, R²² and R²³ has an aromatic group.

Among them, each R²¹, R²² and R²³ is preferably a substituted or unsubstituted monovalent aliphatic hydrocarbon group of 1 or more but not more than 70 carbon atoms or substituted or unsubstituted monovalent aromatic hydrocarbon group of 6 or more but not more than 70 carbon atoms, which may have an ether group, carbonyl group or ester group.

Examples of the monovalent aliphatic hydrocarbon group and monovalent aromatic hydrocarbon group as R²¹, R²² and R²³ include the same groups as those mentioned above as R¹¹ and R¹².

Although there are no particular limitations on preferred tertiary amine compound (II), examples thereof include compounds of general formulas (II-1) to (II-3) shown below (hereinafter sometimes referred to as "the tertiary amine compound (II-1)" or the like, respectively) from the viewpoint of ease of availability.

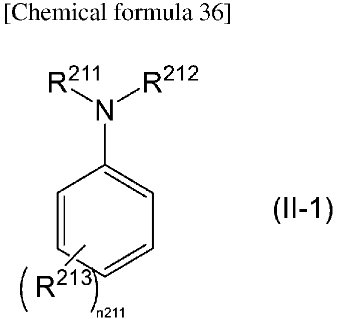

In general formula (II-1), R²¹¹ and R²¹² are the same as R¹¹ and R¹² described above. R²¹³ is a substituent on the benzene ring, and is the same as R¹¹² described above. Further, n211 indicates the number of substituents, and is the same as n111 described above.

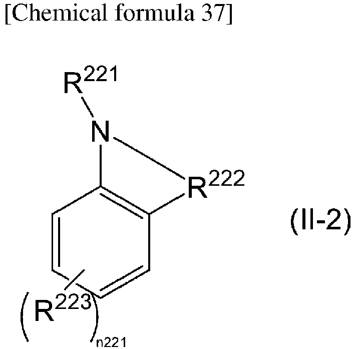

In general formula (11-2), R²²¹ is the same as R¹¹ and R¹² described above. R²²² is the same as R¹²¹ described above. R²²¹ and R²²² may be bonded together to form a carbon-carbon bond. R²²³ is a substituent on the benzene ring, and is the same as R¹²² described above. Further, n221 indicates the number of substituents, and is the same as n121 described above.

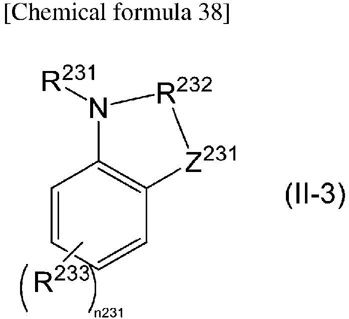

In general formula (11-3), R²³¹ is the same as R¹¹ and R¹² described above. R²³² is the same as R¹³¹ described above. R²³¹ and R²³² may be bonded together to form a carbon-carbon bond. Z²³¹ is the same as Z¹³¹ described above. R²³³ is a substituent on the benzene ring, and is the same as R¹²² described above. Further, n231 indicates the number of substituents, and is the same as n131 described above.

Among the tertiary amine compounds (II-1) to (II-3) described above, compounds in which the amino group bonded to the aromatic hydrocarbon is bonded to an aliphatic hydrocarbon are preferred in comparison with compounds in which the amino group bonded to the aromatic hydrocarbon is bonded directly to an aromatic hydrocarbon and compounds in which the amino group bonded to the aromatic hydrocarbon is bonded directly to an unsaturated hydrocarbon, in terms of achieving a superior coloration reduction effect.

Further, structures composed entirely of carbon atoms and hydrogen atoms, with the exception of the monovalent secondary amino group bonded directly to the aromatic hydrocarbon, are preferred from the viewpoint of thermal stability.

In other words, in the secondary amine compound (II-1), it is preferable that R²¹¹ and R²¹² be monovalent aliphatic hydrocarbon groups, R²¹³ be an unsubstituted aliphatic hydrocarbon group of 1 or more but not more than 12 carbon atoms or an unsubstituted aromatic hydrocarbon group of 6 or more but not more than 12 carbon atoms, and n211 be either 0 or 1.

In the secondary amine compound (11-1), it is more preferable that R²¹¹ and R²¹² be monovalent aliphatic hydrocarbon groups of 1 or more but not more than 12 carbon atoms, R²¹³ be a methyl group or ethyl group, and n211 be either 0 or 1.

Further, in the secondary amine compound (II-2), it is preferable that R²²¹ be a monovalent aliphatic hydrocarbon group, R²²² be a divalent aliphatic hydrocarbon group, R²²³ be an unsubstituted aliphatic hydrocarbon group of 1 or more but not more than 12 carbon atoms or an unsubstituted aromatic hydrocarbon group of 6 or more but not more than 12 carbon atoms, and n221 be either 0 or 1.

In the secondary amine compound (II-2), it is more preferable that R²²¹ be a monovalent aliphatic hydrocarbon group of 1 or more but not more than 12 carbon atoms, R²²² be a divalent aliphatic hydrocarbon group of 1 or more but not more than 12 carbon atoms, R²²¹ and R²²² be bonded together to form a carbon-carbon bond, R²²³ be a methyl group or ethyl group, and n221 be either 0 or 1.

Further, in the secondary amine compound (II-3), it is preferable that R²³¹ be a monovalent aliphatic hydrocarbon group, R²³² be a divalent aliphatic hydrocarbon group, R²³³ be an unsubstituted aliphatic hydrocarbon group of 1 or more but not more than 12 carbon atoms or an unsubstituted aromatic hydrocarbon group of 6 or more but not more than 12 carbon atoms, and n231 be either 0 or 1.

In the secondary amine compound (II-3), it is more preferable that R²³¹ be a monovalent aliphatic hydrocarbon group of 1 or more but not more than 12 carbon atoms, R²³² be a divalent aliphatic hydrocarbon group of 1 or more but not more than 12 carbon atoms, R²²¹ and R²²² be bonded together to form a carbon-carbon bond, R²³³ be a methyl group or ethyl group, and n231 be either 0 or 1.

Specific examples of the preferred tertiary amine compound (II-1) include N,N-dimethylaniline, N-methyl-N-ethylaniline, N,N-diethylaniline, N-methyl-N-propylaniline (each isomer), N-ethyl-N-propylaniline (each isomer), N,N-dipropylaniline (each isomer), N-methyl-N-butylaniline (each isomer), N-ethyl-N-butylaniline (each isomer), N-propyl-N-butylaniline (each isomer), N,N-dibutylaniline (each isomer), N-methyl-N-pentylaniline (each isomer), N-ethyl-N-pentylaniline (each isomer), N-propyl-N-pentylaniline (each isomer), N-butyl-N-pentylaniline (each isomer), N,N-dipentylaniline (each isomer), N-methyl-N-hexylaniline (each isomer), N-ethyl-N-hexylaniline (each isomer), N-propyl-N-hexylaniline (each isomer), N-butyl-N-hexylaniline (each isomer), N-pentyl-N-hexylaniline (each isomer), N,N-dihexylaniline (each isomer), N-methyl-N-heptylaniline (each isomer), N-ethyl-N-heptylaniline (each isomer), N-propyl-N-heptylaniline (each isomer), N-butyl-N-heptylaniline (each isomer), N-pentyl-N-heptylaniline (each isomer), N-hexyl-N-heptylaniline (each isomer), N,N-diheptylaniline (each isomer), N-methyl-N-octylaniline (each isomer), N-ethyl-N-octylaniline (each isomer), N-propyl-N-octylaniline (each isomer), N-butyl-N-octylaniline (each isomer), N-pentyl-N-octylaniline (each isomer), N-hexyl-N-octylaniline (each isomer), N-heptyl-N-octylaniline (each isomer), N,N-dioctylaniline (each isomer), N-methyl-N-nonylaniline (each isomer), N-ethyl-N-nonylaniline (each isomer), N-propyl-N-nonylaniline (each isomer), N-butyl-N-nonylaniline (each isomer), N-pentyl-N-nonylaniline (each isomer), N-hexyl-N-nonylaniline (each isomer), N-heptyl-N-nonylaniline (each isomer), N-octyl-N-nonylaniline (each isomer), N,N-dinonylaniline (each isomer), N-methyl-N-decylaniline (each isomer), N-ethyl-N-decylaniline (each isomer), N-propyl-N-decylaniline (each isomer), N-butyl-N-decylaniline (each isomer), N-pentyl-N-decylaniline (each isomer), N-hexyl-N-decylaniline (each isomer), N-heptyl-N-decylaniline (each isomer), N-octyl-N-decylaniline (each isomer), N-nonyl-N-decylaniline (each isomer), N,N-didecylaniline (each isomer), N,N-dimethyltoluidine (each isomer), N-methyl-N-ethyltoluidine (each isomer), N,N-diethyltoluidine (each isomer), N-methyl-N-propyltoluidine (each isomer), N-ethyl-N-propyltoluidine (each isomer), N,N-dipropyltoluidine (each isomer), N-methyl-N-butyltoluidine (each isomer), N-ethyl-N-butyltoluidine (each isomer), N-propyl-N-butyltoluidine (each isomer), N,N-dibutyltoluidine (each isomer), N-methyl-N-pentyltoluidine (each isomer), N-ethyl-N-pentyltoluidine (each isomer), N-propyl-N-pentyltoluidine (each isomer), N-butyl-N-pentyltoluidine (each isomer), N,N-dipentyltoluidine (each isomer), N-methyl-N-hexyltoluidine (each isomer), N-ethyl-N-hexyltoluidine (each isomer), N-propyl-N-hexyltoluidine (each isomer), N-butyl-N-hexyltoluidine (each isomer), N-pentyl-N-hexyltoluidine (each isomer), N,N-dihexyltoluidine (each isomer), N-methyl-N-heptyltoluidine (each isomer), N-ethyl-N-heptyltoluidine (each isomer), N-propyl-N-heptyltoluidine (each isomer), N-butyl-N-heptyltoluidine (each isomer), N-pentyl-N-heptyltoluidine (each isomer), N-hexyl-N-heptyltoluidine (each isomer), N,N-diheptyltoluidine (each isomer), N-methyl-N-octyltoluidine (each isomer), N-ethyl-N-octyltoluidine (each isomer), N-propyl-N-octyltoluidine (each isomer), N-butyl-N-octyltoluidine (each isomer), N-pentyl-N-octyltoluidine (each isomer), N-hexyl-N-octyltoluidine (each isomer), N-heptyl-N-octyltoluidine (each isomer), N,N-dioctyltoluidine (each isomer), N-methyl-N-nonyltoluidine (each isomer), N-ethyl-N-nonyltoluidine (each isomer), N-propyl-N-nonyltoluidine (each isomer), N-butyl-N-nonyltoluidine (each isomer), N-pentyl-N-nonyltoluidine (each isomer), N-hexyl-N-nonyltoluidine (each isomer), N-heptyl-N-nonyltoluidine (each isomer), N-octyl-N-nonyltoluidine (each isomer), N,N-dinonyltoluidine (each isomer), N-methyl-N-decyltoluidine (each isomer), N-ethyl-N-decyltoluidine (each isomer), N-propyl-N-decyltoluidine (each isomer), N-butyl-N-decyltoluidine (each isomer), N-pentyl-N-decyltoluidine (each isomer), N-hexyl-N-decyltoluidine (each isomer), N-heptyl-N-decyltoluidine (each isomer), N-octyl-N-decyltoluidine (each isomer), N-nonyl-N-decyltoluidine (each isomer), N,N-didecyltoluidine (each isomer), N,N-diarylaniline, N-aryl-N-methylaniline, N,N-diaryltoluidine (each isomer), 3,3'-(phenylazanediyl)dipropanenitrile, 3,3'-(o-tolylazanediyl)dipropanenitrile (each isomer), N-methyl-N-phenylaniline, N-ethyl-N-phenylaniline, N-propyl-N-phenylaniline (each isomer), N-butyl-N-phenylaniline (each isomer), N-pentyl-N-phenylaniline (each isomer), N-hexyl-N-phenylaniline (each isomer), N-heptyl-N-phenylaniline (each isomer), N-octyl-N-phenylaniline (each isomer), N-nonyl-N-phenylaniline (each isomer), N-decyl-N-phenylaniline (each isomer), N-phenyldiethanolamine, fluoro-N,N-dimethylaniline (each isomer), chloro-N,N-dimethylaniline (each isomer), bromo-N,N-dimethylaniline (each isomer), iodo-N,N-dimethylaniline (each isomer), fluoro-N,N-diethylaniline (each isomer), chloro-N,N-diethylaniline (each isomer), bromo-N,N-diethylaniline (each isomer), iodo-N,N-diethylaniline (each isomer), fluoro-N,N-dipropylaniline (each isomer), chloro-N,N-dipropylaniline (each isomer), bromo-N,N-dipropylaniline (each isomer), and iodo-N,N-dipropylaniline (each isomer).

Specific examples of the preferred tertiary amine compound (II-2) include 1-methyl-2,3-dihydro-4(1H)-quinolinone, N-methylindoline, N-ethylindoline, N-propylindoline (each isomer), N-butylindoline (each isomer), N-pentylindoline (each isomer), N-hexylindoline (each isomer), N-heptylindoline (each isomer), N-octylindoline (each isomer), N-heptylindoline (each isomer), N-nonylindoline (each isomer), N-decylindoline (each isomer), N-methyl-1,2,3,4-tetrahydroquinoline, N-ethyl-1,2,3,4-tetrahydroquinoline, N-propyl-1,2,3,4-tetrahydroquinoline (each isomer), N-butyl-1,2,3,4-tetrahydroquinoline (each isomer), N-pentyl-1,2,3,4-tetrahydroquinoline (each isomer), N-hexyl-1,2,3,4-tetrahydroquinoline (each isomer), N-heptyl-1,2,3,4-tetrahydroquinoline (each isomer), N-octyl-1,2,3,4-tetrahydroquinoline (each isomer), N-nonyl-1,2,3,4-tetrahydroquinoline (each isomer), and N-decyl-1,2,3,4-tetrahydroquinoline (each isomer).

Specific examples of the preferred tertiary amine compound (II-3) include 4-methyl-3,4-dihydro-2H-1,4-benzoxazine.

Among them, examples of the tertiary amine compound (II) preferred in terms of ensuring superior thermal stability of the compound as well as a superior coloration suppression effect include N,N-dialkylaniline compounds such as N,N-dimethylaniline, N-methyl-N-ethylaniline, N,N-diethylaniline, N-methyl-N-propylaniline (each isomer), N-ethyl-N-propylaniline (each isomer), N,N-dipropylaniline (each isomer), N-methyl-N-butylaniline (each isomer), N-ethyl-N-butylaniline (each isomer), N-propyl-N-butylaniline (each isomer), N,N-dibutylaniline (each isomer), N-methyl-N-pentylaniline (each isomer), N-ethyl-N-pentylaniline (each isomer), N-propyl-N-pentylaniline (each isomer), N-butyl-N-pentylaniline (each isomer), N,N-dipentylaniline (each isomer), N-methyl-N-hexylaniline (each isomer), N-ethyl-N-hexylaniline (each isomer), N-propyl-N-hexylaniline (each isomer), N-butyl-N-hexylaniline (each isomer), N-pentyl-N-hexylaniline (each isomer), N,N-dihexylaniline (each isomer), N-methyl-N-heptylaniline (each isomer), N-ethyl-N-heptylaniline (each isomer), N-propyl-N-heptylaniline (each isomer), N-butyl-N-heptylaniline (each isomer), N-pentyl-N-heptylaniline (each isomer), N-hexyl-N-heptylaniline (each isomer), N,N-diheptylaniline (each isomer), N-methyl-N-octylaniline (each isomer), N-ethyl-N-octylaniline (each isomer), N-propyl-N-octylaniline (each isomer), N-butyl-N-octylaniline (each isomer), N-pentyl-N-octylaniline (each isomer), N-hexyl-N-octylaniline (each isomer), N-heptyl-N-octylaniline (each isomer), N,N-dioctylaniline (each isomer), N-methyl-N-nonylaniline (each isomer), N-ethyl-N-nonylaniline (each isomer), N-propyl-N-nonylaniline (each isomer), N-butyl-N-nonylaniline (each isomer), N-pentyl-N-nonylaniline (each isomer), N-hexyl-N-nonylaniline (each isomer), N-heptyl-N-nonylaniline (each isomer), N-octyl-N-nonylaniline (each isomer), N,N-dinonylaniline (each isomer), N-methyl-N-decylaniline (each isomer), N-ethyl-N-decylaniline (each isomer), N-propyl-N-decylaniline (each isomer), N-butyl-N-decylaniline (each isomer), N-pentyl-N-decylaniline (each isomer), N-hexyl-N-decylaniline (each isomer), N-heptyl-N-decylaniline (each isomer), N-octyl-N-decylaniline (each isomer), N-nonyl-N-decylaniline (each isomer), and N,N-didecylaniline (each isomer); N,N-dialkyltoluidine compounds such as N,N-dimethyltoluidine (each isomer), N-methyl-N-ethyltoluidine (each isomer), N,N-diethyltoluidine (each isomer), N-methyl-N-propyltoluidine (each isomer), N-ethyl-N-propyltoluidine (each isomer), N,N-dipropyltoluidine (each isomer), N-methyl-N-butyltoluidine (each isomer), N-ethyl-N-butyltoluidine (each isomer), N-propyl-N-butyltoluidine (each isomer), N,N-dibutyltoluidine (each isomer), N-methyl-N-pentyltoluidine (each isomer), N-ethyl-N-pentyltoluidine (each isomer), N-propyl-N-pentyltoluidine (each isomer), N-butyl-N-pentyltoluidine (each isomer), N,N-dipentyltoluidine (each isomer), N-methyl-N-hexyltoluidine (each isomer), N-ethyl-N-hexyltoluidine (each isomer), N-propyl-N-hexyltoluidine (each isomer), N-butyl-N-hexyltoluidine (each isomer), N-pentyl-N-hexyltoluidine (each isomer), N,N-dihexyltoluidine (each isomer), N-methyl-N-heptyltoluidine (each isomer), N-ethyl-N-heptyltoluidine (each isomer), N-propyl-N-heptyltoluidine (each isomer), N-butyl-N-heptyltoluidine (each isomer), N-pentyl-N-heptyltoluidine (each isomer), N-hexyl-N-heptyltoluidine (each isomer), N,N-diheptyltoluidine (each isomer), N-methyl-N-octyltoluidine (each isomer), N-ethyl-N-octyltoluidine (each isomer), N-propyl-N-octyltoluidine (each isomer), N-butyl-N-octyltoluidine (each isomer), N-pentyl-N-octyltoluidine (each isomer), N-hexyl-N-octyltoluidine (each isomer), N-heptyl-N-octyltoluidine (each isomer), N,N-dioctyltoluidine (each isomer), N-methyl-N-nonyltoluidine (each isomer), N-ethyl-N-nonyltoluidine (each isomer), N-propyl-N-nonyltoluidine (each isomer), N-butyl-N-nonyltoluidine (each isomer), N-pentyl-N-nonyltoluidine (each isomer), N-hexyl-N-nonyltoluidine (each isomer), N-heptyl-N-nonyltoluidine (each isomer), N-octyl-N-nonyltoluidine (each isomer), N,N-dinonyltoluidine (each isomer), N-methyl-N-decyltoluidine (each isomer), N-ethyl-N-decyltoluidine (each isomer), N-propyl-N-decyltoluidine (each isomer), N-butyl-N-decyltoluidine (each isomer), N-pentyl-N-decyltoluidine (each isomer), N-hexyl-N-decyltoluidine (each isomer), N-heptyl-N-decyltoluidine (each isomer), N-octyl-N-decyltoluidine (each isomer), N-nonyl-N-decyltoluidine (each isomer), and N,N-didecyltoluidine (each isomer); and N-alkyl aromatic cyclic amines such as N-methylindoline, N-ethylindoline, N-propylindoline (each isomer), N-butylindoline (each isomer), N-pentylindoline (each isomer), N-hexylindoline (each isomer), N-heptylindoline (each isomer), N-octylindoline (each isomer), N-heptylindoline (each isomer), N-nonylindoline (each isomer), N-decylindoline (each isomer), N-methyl-1,2,3,4-tetrahydroquinoline, N-ethyl-1,2,3,4-tetrahydroquinoline, N-propyl-1,2,3,4-tetrahydroquinoline (each isomer), N-butyl-1,2,3,4-tetrahydroquinoline (each isomer), N-pentyl-1,2,3,4-tetrahydroquinoline (each isomer), N-hexyl-1,2,3,4-tetrahydroquinoline (each isomer), N-heptyl-1,2,3,4-tetrahydroquinoline (each isomer), N-octyl-1,2,3,4-tetrahydroquinoline (each isomer), N-nonyl-1,2,3,4-tetrahydroquinoline (each isomer), and N-decyl-1,2,3,4-tetrahydroquinoline (each isomer).

### <Phenol-Based Antioxidant>

There are no particular limitations on the phenol-based antioxidant that may be used in combination with the secondary amine compound (I) and the tertiary amine compound (II), and any compound having at least one hydroxy group bonded to an aromatic ring within the molecule may be used. Specific examples of the phenol-based antioxidant include: phenol; phenol-based blocking agents having a hydrocarbon group such as methylphenol (each isomer), ethylphenol (each isomer), propylphenol (each isomer), butylphenol (each isomer), pentylphenol (each isomer), hexylphenol (each isomer), heptylphenol (each isomer), octylphenol (each isomer), nonylphenol (each isomer), decylphenol (each isomer), undecylphenol (each isomer), tridecylphenol (each isomer), tetradecylphenol (each isomer), octadecylphenol (each isomer), nonadecylphenol (each isomer), eicosylphenol (each isomer), cyclopentylphenol (each isomer), cyclohexylphenol (each isomer), dimethylphenol (each isomer), methylethylphenol (each isomer), diethylphenol (each isomer), methylpropylphenol (each isomer), ethylpropylphenol (each isomer), dipropylphenol (each isomer), methylbutylphenol (each isomer), ethylbutylphenol (each isomer), propylbutylphenol (each isomer), dibutylphenol (each isomer), trimethylphenol (each isomer), methyldiethylphenol (each isomer), dimethylethylphenol (each isomer), triethylphenol (each isomer), methyldipropylphenol (each isomer), dimethylpropylphenol (each isomer), tripropylphenol (each isomer), ethyldipropylphenol (each isomer), diethylpropylphenol (each isomer), methyldibutylphenol (each isomer), dimethylbutylphenol (each isomer), ethyldibutylphenol (each isomer), diethylbutylphenol (each isomer), propyldibutylphenol (each isomer), dipropylbutylphenol (each isomer), tributylphenol (each isomer), naphthol (each isomer), methylnaphthol (each isomer), ethylnaphthol (each isomer), propylnaphthol (each isomer), butylnaphthol (each isomer), tetrahydronaphthalen-1-ol, tetrahydronaphthalen-2-ol, hydroxyphenylphenylmethane (each isomer), hydroxyphenylphenylethane (each isomer), hydroxyphenylphenylpropane (each isomer), hydroxyphenylphenylbutane (each isomer), 4-α-cumylphenol, and 2,4-di-α-cumylphenol; as well as methoxyphenol (each isomer), ethoxyphenol (each isomer), propoxyphenol (each isomer), butoxyphenol (each isomer), pentoxyphenol (each isomer), hextoxyphenol (each isomer), heptoxyphenol (each isomer), octoxyphenol (each isomer), methoxymethylphenol (each isomer), methoxyethylphenol (each isomer), methoxypropylphenol (each isomer), methoxybutylphenol (each isomer), ethoxymethylphenol (each isomer), ethoxyethylphenol (each isomer), ethoxypropylphenol (each isomer), ethoxybutylphenol (each isomer), propoxymethylphenol (each isomer), propoxyethylphenol (each isomer), propoxypropylphenol (each isomer), butoxymethylphenol (each isomer), butoxyethylphenol (each isomer), butoxypropylphenol (each isomer), and butoxybutylphenol (each isomer); phenols having a halogen group such as fluorophenol (each isomer), difluorophenol (each isomer), trifluorophenol (each isomer), tetrafluorophenol (each isomer), pentafluorophenol (each isomer), chlorophenol (each isomer), dichlorophenol (each isomer), trichlorophenol (each isomer), tetrachlorophenol (each isomer), pentachlorophenol (each isomer), bromophenol (each isomer), dibromophenol (each isomer), tribromophenol (each isomer), tetrabromophenol (each isomer), pentabromophenol (each isomer), iodophenol (each isomer), diiodophenol (each isomer), triiodophenol (each isomer), tetraiodophenol (each isomer), and pentaiodophenol (each isomer); and phenol-based blocking agents having a carbonyl group such as methyl salicylate, ethyl salicylate, propyl salicylate (each isomer), butyl salicylate (each isomer), pentyl salicylate (each isomer), hexyl salicylate (each isomer), heptyl salicylate (each isomer), octyl salicylate (each isomer), nonyl salicylate (each isomer), decyl salicylate (each isomer), undecyl salicylate (each isomer), dodecyl salicylate (each isomer), tetradecyl salicylate (each isomer), octadecyl salicylate (each isomer), and acetylphenol.

Among them, phenol, 2,6-di-tertiary-butyl-4-methylphenol, or 3-(tertiary-butyl)-4-methoxyphenol is preferred in terms of ease of availability. Further, 2,6-di-tertiarybutyl-4-methylphenol or 3-(tertiary-butyl)-4-methoxyphenol is particularly preferred from the viewpoint of being readily available and also providing a superior coloration suppression effect.

### <Primary Amine Compound>

A compound of general formula (III) shown below (hereinafter sometimes referred to as the "primary amine compound (III)") may be used favorably as the primary amine compound.

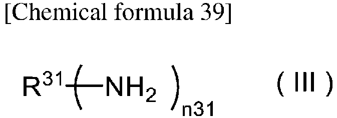

In general formula (III), R³¹ is an n31-valent organic group. Further, n31 is an integer of 1 or more but not more than 12.

R³¹ is an n31-valent organic group. In other words, R³¹ is an organic group having a valency of 1 or more but not more than 12. Among them, R³¹ is preferably an organic group consisting of carbon atoms, oxygen atoms and hydrogen atoms, and is more preferably an organic group that does not have an active hydrogen.

The aliphatic hydrocarbon group as R³¹ is preferably an alkylene group or alkanetriyl group, cycloalkyl group, cycloalkylene group or cycloalkanetriyl group, or a group composed of the above-mentioned alkyl group, alkylene group or alkanetriyl group, and the above-mentioned cycloalkyl group, cycloalkylene group or cycloalkanetriyl group, and more preferably a linear or branched alkylene group or alkanetriyl group, cycloalkylene group or cycloalkanetriyl group, or a group composed of the above alkylene group or alkanetriyl group and the above cycloalkyl group, cycloalkylene group or cycloalkanetriyl group.

Examples of the linear or branched alkylene group include a methylene group, ethylene group, propylene group, trimethylene group, pentylene group, n-hexylene group, and decamethylene group.

Examples of the cycloalkylene group include a cyclobutylene group, cyclopentylene group and cyclohexylene group.

Examples of the linear or branched alkanetriyl group include a hexanetriyl group, nonanetriyl group and decanetriyl group.

Examples of the cycloalkanetriyl group include a cyclopropanetriyl group, cyclobutanetriyl group, cyclopentanetriyl group, and cyclohexanetriyl group.

The aromatic hydrocarbon group as R³¹ is preferably a group having a substituted or unsubstituted aromatic ring of 6 or more but not more than 13 carbon atoms. Examples of substituents include alkyl groups, aryl groups, and aralkyl groups. The aromatic ring may be an aromatic hydrocarbon ring or a heteroaromatic ring, and specific examples thereof include a benzene ring, naphthalene ring, and pyridine ring.

Examples of preferred primary amine compounds include monofunctional primary amine compounds, difunctional primary amine compounds, and polyfunctional primary amine compounds.

Examples of the monofunctional primary amine compounds include primary amine compounds having a saturated hydrocarbon group such as methylamine, ethylamine, propylamine (each isomer), butylamine (each isomer), pentylamine (each isomer), hexylamine (each isomer), heptylamine (each isomer), octylamine (each isomer), nonylamine (each isomer), decylamine (each isomer), undecylamine (each isomer), dodecylamine (each isomer), tridecylamine (each isomer), tetradecylamine (each isomer), pentadecylamine (each isomer), hexadecylamine (each isomer), heptadecylamine (each isomer), octadecylamine (each isomer), nonadecylamine (each isomer), and eicosylamine (each isomer); primary amine compounds having an unsaturated bond such as 2-aminomethyl acrylate, 2-aminoethyl acrylate, 2-aminopropyl acrylate (each isomer), 2-aminobutyl acrylate (each isomer), 2-aminomethyl methacrylate, 2-aminoethyl methacrylate, 2-aminopropyl methacrylate (each isomer), 2-aminobutyl methacrylate (each isomer), phenylamine and benzylamine; primary amine compounds having an ether group such as methyl (S)-2-amino-3-tertiary-butoxypropionate, ethyl (S)-2-amino-3-tertiary-butoxypropionate, propyl (S)-2-amino-3-tertiary-butoxypropionate (each isomer), butyl (S)-2-amino-3-tertiary-butoxypropionate (each isomer), pentyl (S)-2-amino-3-tertiary-butoxypropionate (each isomer), hexyl (S)-2-amino-3-tertiary-butoxypropionate (each isomer), and dodecyl (S)-2-amino-3-tertiary-butoxypropionate (each isomer); primary amine compounds having a halogen group such as fluorophenylamine (each isomer), chlorophenylamine (each isomer), bromophenylamine (each isomer), and iodophenylamine (each isomer); and primary amine compounds having a carbonyl group such as glycine methyl ester, glycine ethyl ester, glycine propyl ester (each isomer), glycine butyl ester (each isomer), glycine pentyl ester (each isomer), glycine hexyl ester (each isomer), glycine dodecyl ester (each isomer), leucine methyl ester, leucine ethyl ester, leucine propyl ester (each isomer), leucine butyl ester (each isomer), leucine pentyl ester (each isomer), leucine hexyl ester (each isomer), and leucine dodecyl ester (each isomer).

Examples of the difunctional primary amine compounds include primary amine compounds having a saturated hydrocarbon group such as dimethylenediamine, trimethylenediamine (each isomer), tetramethylenediamine (each isomer), pentamethylenediamine (each isomer), hexamethylenediamine (each isomer), heptamethylenediamine (each isomer), octamethylenediamine (each isomer), cyclohexanediamine (each isomer), methylcyclohexanediamine (each isomer), isophoronediamine (each isomer), dicyclohexylmethanediamine (each isomer), dimethylpropanediamine (each isomer), dimethylpentanediamine (each isomer), dimethylhexanediamine (each isomer), dimethylheptanediamine (each isomer), dimethyloctanediamine (each isomer), dimethylnonanediamine (each isomer), dimethyldecanediamine (each isomer), methylethylpropanediamine (each isomer), methylethylbutanediamine (each isomer), methylethylpentanediamine (each isomer), methylethylhexanediamine (each isomer), methylethylheptanediamine (each isomer), methylethyloctanediamine (each isomer), diethylpropanediamine (each isomer), diethylpentanediamine (each isomer), diethylhexanediamine (each isomer), diethylheptanediamine (each isomer), diethyloctanediamine (each isomer), and diethylnonanediamine (each isomer); primary amine compounds having an unsaturated hydrocarbon group such as diphenylmethanediamine (each isomer), tolylenediamine (each isomer), naphthalenediamine (each isomer), xylylenediamine (each isomer), tetramethylxylylenediamine (each isomer), methylenebis(diisoamyl-phenylene)diamine (each isomer), oxybis(phenylenediamine) (each isomer), carbonylbis(phenylene)diamine (each isomer), butenediamine (each isomer), and butynylenediamine; and primary amine compounds having an ester group such as lysine methyl ester, lysine ethyl ester, lysine propyl ester (each isomer), lysine butyl ester (each isomer), lysine pentyl ester (each isomer), and lysine hexyl ester (each isomer).

Examples of the polyfunctional amines include primary amine compounds having a saturated hydrocarbon group such as propanetriamine, butanetriamine (each isomer), pentanetriamine (each isomer), hexanetriamine (each isomer), heptanetriamine (each isomer), octanetriamine (each isomer), nonanetriamine (each isomer), decanetriamine (each isomer), undecanetriamine (each isomer), dodecanetriamine (each isomer), tridecanetriamine (each isomer), tetradecanetriamine (each isomer), pentadecanetriamine (each isomer), hexadecanetriamine (each isomer), heptadecanetriamine (each isomer), octadecanetriamine (each isomer), nonadecanetriamine (each isomer), and eicosanetriamine (each isomer); primary amine compounds having an unsaturated hydrocarbon group such as polymeric MDA (each isomer); primary amine compounds having an ester group such as 2-aminoethyl 2,6-diaminohexanoate; and primary amine compounds having a difunctional or more-functional amino group having an isocyanurate group, primary amine compounds having a difunctional or more-functional amino group having a biuret group, and primary amine compounds having a difunctional or more-functional amino group having an allophanate group.

### <Blocking Agent>

The blocking agent may be any compound that has an active hydrogen group, and is capable of reacting with an isocyanate group to form a bond, forming a blocked isocyanate compound from the primary amine compound, the carbonic acid derivative and the blocking agent, and then being decomposed into an isocyanate compound and the blocking agent by thermal decomposition. Examples of such blocking agents include alcohol-based, phenol-based (hereinafter alcohol-based and phenol-based blocking agents are sometimes referred to jointly as "hydroxy compounds"), thiol-based, amine- and ammonia-based, oxime-based, hydroxylamine-based, and active methylene-based blocking agents. Among them, blocking agents formed from hydroxy compounds, and amine- and ammonia-based, or hydroxylamine-based blocking agents are preferred, blocking agents formed from hydroxy compounds, and amine- and ammonia-based blocking agents are more preferred, and blocking agents formed from hydroxy compounds, blocking agents formed from amine compounds and ammonia are even more preferred, from the viewpoint of the stability of the blocking agent in the reaction of obtaining the blocked isocyanate compound by heating the primary amine compound, the carbonic acid derivative and the blocking agent. Further, the use of a blocking agent with high nucleophilicity is particularly preferred in terms of being able to react preferentially with isocyanate groups when obtaining the blocked isocyanate compound, and enabling an improvement in the yield of the blocked isocyanate compound. In the case where phenol-based blocking agents, alcohol-based blocking agents, amine- and ammonia-based blocking agents, and hydroxylamine-based blocking agents are compared from the viewpoint of the nucleophilicity of the blocking agent, the nucleophilicity is typically increased in the order from hydroxylamine-based blocking agents < phenol-based blocking agents < alcohol-based blocking agents < amine- and ammonia-based blocking agents.

### [Phenol-Based Blocking Agent]

There are no particular limitations on the phenol-based blocking agent, provided it is a compound having a hydroxyl group bonded to an aromatic ring, but a blocking agent having a phenol group or naphthol group is preferred from the viewpoint of ease of availability. The blocking agent may be a compound having one hydroxyl group bonded to an aromatic ring within the molecule, or may be a compound having two or more hydroxyl groups bonded to one or more aromatic rings within the molecule. In those cases where a compound having two or more hydroxyl groups bonded to one or more aromatic rings within the molecule is used as the blocking agent, the obtained blocked isocyanate compounds contain blocked isocyanates having a high molecular weight, and the blocked isocyanates increase the viscosity of the blocked isocyanate composition containing the blocked isocyanate compounds, and may sometimes cause gelling of the blocked isocyanate composition, thereby making production of the blocked isocyanate compound and the blocked isocyanate composition difficult. Accordingly, a compound having one hydroxyl group bonded to an aromatic ring within the molecule is preferably used as the phenol-based blocking agent.

Examples of the preferred phenol-based blocking agent include a compound of general formula (IV-1) shown below (hereinafter sometimes referred to as the "phenol-based blocking agent (IV-1)").

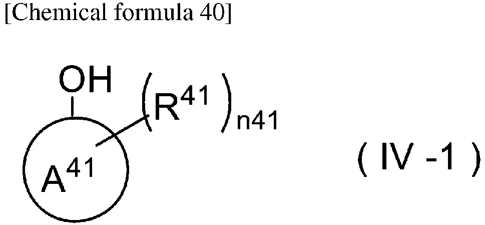

In general formula (IV-1), the ring A⁴¹ is an aromatic hydrocarbon ring of 6 or more but not more than 20 carbon atoms. R⁴¹ is a hydrogen atom, halogen atom, carboxy group, alkyl group of 1 or more but not more than 20 carbon atoms, alkoxy group of 1 or more but not more than 20 carbon atoms, alkyloxycarbonyl group of 1 or more but not more than 20 carbon atoms, alkylcarbonyloxy group of 1 or more but not more than 20 carbon atoms, aryl group of 6 or more but not more than 20 carbon atoms, aryloxy group of 6 or more but not more than 20 carbon atoms, aralkyl group of 7 or more but not more than 20 carbon atoms, or aralkyloxy group of 7 or more but not more than 20 carbon atoms. R⁴¹ may be bonded to the ring A⁴¹ to form a cyclic structure. Further, n41 is an integer of 1 or more but not more than 10.

### (R41)

Examples of the alkyl group of 1 or more but not more than 20 carbon atoms as R⁴¹ include a methyl group, ethyl group, propyl group (each isomer), butyl group (each isomer), pentyl group (each isomer), hexyl group (each isomer), heptyl group (each isomer), octyl group (each isomer), nonyl group (each isomer), decyl group (each isomer), dodecyl group (each isomer), and octadecyl group (each isomer).

Examples of the alkoxy group of 1 or more but not more than 20 carbon atoms as R⁴¹ include a methoxy group, ethoxy group, propoxy group (each isomer), butyloxy group (each isomer), pentyloxy group (each isomer), hexyloxy group (each isomer), heptyloxy group (each isomer), octyloxy group (each isomer), nonyloxy group (each isomer), decyloxy group (each isomer), dodecyloxy group (each isomer), and octadecyloxy group (each isomer).

Examples of the alkyloxycarbonyl group of 1 or more but not more than 20 carbon atoms as R⁴¹ include a methoxycarbonyl group, ethoxycarbonyl group, propoxycarbonyl group (each isomer), butyloxycarbonyl group (each isomer), pentyloxycarbonyl group (each isomer), hexyloxycarbonyl group (each isomer), heptyloxycarbonyl group (each isomer), octyloxycarbonyl group (each isomer), nonyloxycarbonyl group (each isomer), decyloxycarbonyl group (each isomer), dodecyloxycarbonyl group (each isomer), and octadecyloxycarbonyl group (each isomer).

Examples of the alkylcarbonyloxy group of 1 or more but not more than 20 carbon atoms as R⁴¹ include a methylcarbonyloxy group, ethylcarbonyloxy group, propylcarbonyloxy group (each isomer), butylcarbonyloxy group (each isomer), pentylcarbonyloxy group (each isomer), hexylcarbonyloxy group (each isomer), heptylcarbonyloxy group (each isomer), octylcarbonyloxy group (each isomer), nonylcarbonyloxy group (each isomer), decylcarbonyloxy group (each isomer), dodecylcarbonyloxy group (each isomer), and octadecylcarbonyloxy group (each isomer).

Examples of the aryl group of 6 or more but not more than 20 carbon atoms as R⁴¹ include a phenyl group and a naphthyl group.

Examples of aryl groups having an alkyl group as the substituent as R⁴¹ include a methylphenyl group (each isomer), ethylphenyl group (each isomer), propylphenyl group (each isomer), butylphenyl group (each isomer), pentylphenyl group (each isomer), hexylphenyl group (each isomer), heptylphenyl group (each isomer), octylphenyl group (each isomer), nonylphenyl group (each isomer), decylphenyl group (each isomer), biphenyl group (each isomer), dimethylphenyl group (each isomer), diethylphenyl group (each isomer), dipropylphenyl group (each isomer), dibutylphenyl group (each isomer), dipentylphenyl group (each isomer), dihexylphenyl group (each isomer), diheptylphenyl group (each isomer), terphenyl group (each isomer), trimethylphenyl group (each isomer), triethylphenyl group (each isomer), tripropylphenyl group (each isomer), and tributylphenyl group (each isomer).

Examples of the aryloxy group of 6 or more but not more than 20 carbon atoms as R⁴¹ include a phenoxy group, methylphenoxy group (each isomer), ethylphenoxy group (each isomer), propylphenoxy group (each isomer), butylphenoxy group (each isomer), pentylphenoxy group (each isomer), hexylphenoxy group (each isomer), heptylphenoxy group (each isomer), octylphenoxy group (each isomer), nonylphenoxy group (each isomer), decylphenoxy group (each isomer), phenylphenoxy group (each isomer), dimethylphenoxy group (each isomer), diethylphenoxy group (each isomer), dipropylphenoxy group (each isomer), dibutylphenoxy group (each isomer), dipentylphenoxy group (each isomer), dihexylphenoxy group (each isomer), diheptylphenoxy group (each isomer), diphenylphenoxy group (each isomer), trimethylphenoxy group (each isomer), triethylphenoxy group (each isomer), tripropylphenoxy group (each isomer), and tributylphenoxy group (each isomer).

Examples of the aralkyl group of 7 or more but not more than 20 carbon atoms as R⁴¹ include a phenylmethyl group, phenylethyl group (each isomer), phenylpropyl group (each isomer), phenylbutyl group (each isomer), phenylpentyl group (each isomer), phenylhexyl group (each isomer), phenylheptyl group (each isomer), phenyloctyl group (each isomer), and phenylnonyl group (each isomer).

Examples of the aralkyloxy group of 7 or more but not more than 20 carbon atoms as R⁴¹ include a phenylmethoxy group, phenylethoxy group (each isomer), phenylpropoyloxy group (each isomer), phenylbutyloxy group (each isomer), phenylpentyloxy group (each isomer), phenylhexyloxy group (each isomer), phenylheptyloxy group (each isomer), phenyloctyloxy group (each isomer), and phenylnonyloxy group (each isomer).

### (A41)

The ring A⁴¹ is an aromatic hydrocarbon ring of 6 or more but not more than 20 carbon atoms. The ring A⁴¹ may be monocyclic, polycyclic, or a condensed ring structure.

Specific examples of the ring A⁴¹ include a benzene ring, naphthalene ring, anthracene ring, phenanthrene ring, naphthacene ring, chrysene ring, pyrene ring, triphenylene ring, pentalene ring, azulene ring, heptalene ring, indacene ring, biphenylene ring, acenaphthylene ring, aceanthrylene ring, and acephenanthrylene ring. Among these, the ring A⁴¹ is preferably a benzene ring, naphthalene ring or anthracene ring, and is more preferably a benzene ring.

Furthermore, these rings may have a substituent other than the R⁴¹ group described above. Examples of this substituent other than the R⁴¹ group include the same groups as those mentioned above as R⁴¹. R⁴¹ and the substituent other than R⁴¹ are composed of different functional groups.

### (n41)

Further, n41 indicates the number of the substituents R⁴¹, and is an integer of 1 or more but not more than 10.

Examples of the preferred phenol-based blocking agent (IV-1) include phenol, methylphenol (each isomer), ethylphenol (each isomer), propylphenol (each isomer), butylphenol (each isomer), pentylphenol (each isomer), hexylphenol (each isomer), heptylphenol (each isomer), octylphenol (each isomer), nonylphenol (each isomer), decylphenol (each isomer), undecylphenol (each isomer), tridecylphenol (each isomer), tetradecylphenol (each isomer), octadecylphenol (each isomer), nonadecylphenol (each isomer), eicosylphenol (each isomer), cyclopentylphenol (each isomer), cyclohexylphenol (each isomer), dimethylphenol (each isomer), methylethylphenol (each isomer), diethylphenol (each isomer), methylpropylphenol (each isomer), ethylpropylphenol (each isomer), dipropylphenol (each isomer), methylbutylphenol (each isomer), ethylbutylphenol (each isomer), propylbutylphenol (each isomer), dibutylphenol (each isomer), trimethylphenol (each isomer), methyldiethylphenol (each isomer), dimethylethylphenol (each isomer), triethylphenol (each isomer), methyldipropylphenol (each isomer), dimethylpropylphenol (each isomer), tripropylphenol (each isomer), ethyldipropylphenol (each isomer), diethylpropylphenol (each isomer), methyldibutylphenol (each isomer), dimethylbutylphenol (each isomer), ethyldibutylphenol (each isomer), diethylbutylphenol (each isomer), propyldibutylphenol (each isomer), dipropylbutylphenol (each isomer), tributylphenol (each isomer), naphthol (each isomer), methylnaphthol (each isomer), ethylnaphthol (each isomer), propylnaphthol (each isomer), butylnaphthol (each isomer), tetrahydronaphthalen-1-ol, tetrahydronaphthalen-2-ol, hydroxyphenylphenylmethane (each isomer), hydroxyphenylphenylethane (each isomer), hydroxyphenylphenylpropane (each isomer), hydroxyphenylphenylbutane (each isomer); phenol-based blocking agents having an ether group such as methoxyphenol (each isomer), ethoxyphenol (each isomer), propoxyphenol (each isomer), butoxyphenol (each isomer), pentoxyphenol (each isomer), hextoxyphenol (each isomer), heptoxyphenol (each isomer), octoxyphenol (each isomer), methoxymethylphenol (each isomer), methoxyethylphenol (each isomer), methoxypropylphenol (each isomer), methoxybutylphenol (each isomer), ethoxymethylphenol (each isomer), ethoxyethylphenol (each isomer), ethoxypropylphenol (each isomer), ethoxybutylphenol (each isomer), propoxymethylphenol (each isomer), propoxyethylphenol (each isomer), propoxypropylphenol (each isomer), butoxymethylphenol (each isomer), butoxyethylphenol (each isomer), butoxypropylphenol (each isomer), and butoxybutylphenol (each isomer); phenol-based blocking agents having a halogen group such as fluorophenol (each isomer), difluorophenol (each isomer), trifluorophenol (each isomer), tetrafluorophenol (each isomer), pentafluorophenol (each isomer), chlorophenol (each isomer), dichlorophenol (each isomer), trichlorophenol (each isomer), tetrachlorophenol (each isomer), pentachlorophenol (each isomer), bromophenol (each isomer), dibromophenol (each isomer), tribromophenol (each isomer), tetrabromophenol (each isomer), pentabromophenol (each isomer), iodophenol (each isomer), diiodophenol (each isomer), triiodophenol (each isomer), tetraiodophenol (each isomer), and pentaiodophenol (each isomer); and phenol-based blocking agents having a carbonyl group such as methyl salicylate, ethyl salicylate, propyl salicylate (each isomer), butyl salicylate (each isomer), pentyl salicylate (each isomer), hexyl salicylate (each isomer), heptyl salicylate (each isomer), octyl salicylate (each isomer), nonyl salicylate (each isomer), decyl salicylate (each isomer), undecyl salicylate (each isomer), dodecyl salicylate (each isomer), tetradecyl salicylate (each isomer), octadecyl salicylate (each isomer), and acetylphenol.

### [Alcohol-Based Blocking Agent]

There are no particular limitations on the alcohol-based blocking agent, provided it is an aliphatic compound having a hydroxyl group, and either an aliphatic compound having one hydroxyl group within the molecule, or an aliphatic compound having two or more hydroxyl groups within the molecule may be used. In those cases where an aliphatic compound having two or more hydroxyl groups within the molecule is used as the blocking agent, the obtained blocked isocyanates contain blocked isocyanates having a high molecular weight, and the blocked isocyanates increase the viscosity of the blocked isocyanate composition containing the blocked isocyanates, and sometimes cause gelling of the blocked isocyanate composition, thereby making production of the blocked isocyanate compound and the blocked isocyanate composition difficult. Accordingly, an aliphatic compound having one hydroxyl group within the molecule is preferably used.

Examples of the preferred alcohol-based blocking agent include a compound of general formula (IV-2) shown below (hereinafter sometimes referred to as the "alcohol-based blocking agent (IV-2)").
[Chemical formula 41]

R⁴²-OH ( IV-2)

In general formula (IV-2), R⁴² is a substituted or unsubstituted aliphatic hydrocarbon group of 1 or more but not more than 24 carbon atoms which may have an ether group, carbonyl group or ester group.

### (R⁴²)

The number of carbon atoms in the aliphatic hydrocarbon group as R⁴² is 1 or more but not more than 24, and is preferably 1 or more but not more than 20, and more preferably 1 or more but not more than 12.

The aliphatic hydrocarbon group as R⁴² may be saturated or unsaturated.

Examples of the aliphatic hydrocarbon group as R⁴² include saturated or unsaturated monovalent hydrocarbon groups of 1 or more but not more than 12 carbon atoms.

Examples of the saturated monovalent hydrocarbon groups of 1 or more but not more than 12 carbon atoms include alkyl groups of 1 or more but not more than 12 carbon atoms. Examples of the alkyl groups of 1 or more but not more than 12 carbon atoms include chained alkyl groups of 1 or more but not more than 12 carbon atoms, and cyclic alkyl groups of at least 3 but not more than 12 carbon atoms.

Examples of the chained alkyl groups of 1 or more but not more than 12 carbon atoms include linear or branched chained alkyl groups of 1 or more but not more than 12 carbon atoms. Specific examples of the linear or branched chained alkyl groups of 1 or more but not more than 12 carbon atoms include a methyl group, ethyl group, n-propyl group, isopropyl group, n-butyl group, isobutyl group, sec-butyl group, tert-butyl group, n-pentyl group, isopentyl group, sec-pentyl group, neopentyl group, tert-pentyl group, 2,3-dimethylpropyl group, 1-ethylpropyl group, 1-methylbutyl group, 2-methylbutyl group, n-hexyl group, isohexyl group, hexan-2-yl group, hexan-3-yl group, 2-methylpentyl group, 3-methylpentyl group, 1,1,2-trimethylpropyl group, 3,3-dimethylbutyl group, n-heptyl group, heptan-2-yl group, heptan-3-yl group, heptan-4-yl group, n-octyl group, octan-2-yl group, octan-3-yl group, octan-4-yl group, n-nonyl group, n-decyl group, n-undecyl group, and n-dodecyl group.

Examples of the cyclic alkyl groups of at least 3 but not more than 12 carbon atoms include a cyclopropyl group, cyclobutyl group, cyclopentyl group, cyclohexyl group, cycloheptyl group, cyclooctyl group, cyclononyl group, cyclodecyl group, cycloundecyl group, and cyclododecyl group.

Examples of the unsaturated monovalent hydrocarbon groups of at least 2 but not more than 12 carbon atoms include alkenyl groups of at least 2 but not more than 12 carbon atoms, alkadienyl groups of at least 3 but not more than 12 carbon atoms, and alkynyl groups of at least 2 but not more than 12 carbon atoms.

Examples of the alkenyl groups of at least 2 but not more than 12 carbon atoms include chained alkenyl groups of at least 2 but not more than 18 carbon atoms, and cyclic alkenyl groups of at least 3 but not more than 12 carbon atoms.

Examples of the chained alkenyl groups of at least 2 but not more than 12 carbon atoms include linear or branched chained alkenyl groups of at least 2 but not more than 12 carbon atoms. Specific examples of the linear or branched chained alkenyl groups of at least 2 but not more than 12 carbon atoms include a vinyl group, allyl group, propenyl group, isopropenyl group, 1-methylpropenyl group, 2-methylpropenyl group, butenyl group, pentenyl group, isopentenyl group, hexenyl group, heptenyl group, octenyl group, nonenyl group, decenyl group, undecenyl group, and dodecenyl group.

Examples of the cyclic alkenyl groups of at least 3 but not more than 12 carbon atoms include a cyclopentenyl group, cyclohexenyl group, cycloheptenyl group, methylcyclopentenyl group, methylcyclohexenyl group, methylcycloheptenyl group, methylcyclooctenyl group, methylcyclononenyl group, methylcyclodecenyl group, methylcycloundecenyl group, and methylcyclododecenyl group.

Examples of the alkadienyl groups of at least 3 but not more than 12 carbon atoms include chained alkadienyl groups of at least 3 but not more than 12 carbon atoms, and cyclic alkadienyl groups of at least 3 but not more than 12 carbon atoms.

Examples of the chained alkadienyl groups of at least 3 but not more than 12 carbon atoms include linear or branched alkadienyl groups of at least 3 but not more than 12 carbon atoms. Specific examples of the linear or branched alkadienyl groups of at least 3 but not more than 12 carbon atoms include a propadienyl group, butadienyl group, pentadienyl group, 2-methylpentadienyl group, hexadienyl group, heptadienyl group, octadienyl group, nonadienyl group, decadienyl group, undecadienyl group, and dodecadienyl group.

Examples of the cyclic alkadienyl groups of at least 3 but not more than 12 carbon atoms include a cyclopropadienyl group, cyclobutadienyl group, cyclopentadienyl group, cyclohexadienyl group, cycloheptadienyl group, cyclooctadienyl group, cyclononadienyl group, cyclodecadienyl group, cycloundecadienyl group, and cyclododecadienyl group.

Examples of the alkynyl groups of at least 2 but not more than 12 carbon atoms include chained alkynyl groups of at least 2 but not more than 12 carbon atoms, and cyclic alkynyl groups of at least 3 but not more than 12 carbon atoms.

Examples of the chained alkynyl groups of at least 2 but not more than 12 carbon atoms include linear or branched alkynyl groups of at least 2 but not more than 12 carbon atoms. Specific examples of the linear or branched alkynyl groups of at least 2 but not more than 12 carbon atoms include an ethynyl group, propynyl group, 2-methylpropynyl group, butynyl group, 2-methylbutynyl group, pentynyl group, hexynyl group, heptynyl group, octynyl group, nonynyl group, decynyl group, undecynyl group, and dodecynyl group.

Examples of the cyclic alkynyl groups of at least 3 but not more than 12 carbon atoms include a cyclopropynyl group, cyclobutynyl group, cyclopentynyl group, cyclohexynyl group, cycloheptynyl group, cyclooctynyl group, cyclononynyl group, cyclodecynyl group, cycloundecynyl group, and cyclododecynyl group.

Examples of substituents of the aliphatic hydrocarbon group as R⁴² include halogen atoms and an amino group.

Specific examples of the preferred alcohol-based blocking agent (IV-2) include alcohol-based blocking agents having a saturated hydrocarbon group such as methanol, ethanol, propanol (each isomer), butanol (each isomer), pentanol (each isomer), hexanol (each isomer), octanol (each isomer), nonanol (each isomer), decanol (each isomer), undecanol (each isomer), dodecanol (each isomer), tridecanol (each isomer), tetradecanol (each isomer), pentadecanol (each isomer), hexadecanol (each isomer), heptadecanol (each isomer), octadecanol (each isomer), nonadecanol (each isomer), eicosanol (each isomer), cyclopentanol, cyclohexanol, cycloheptanol, cyclooctanol, decahydronaphthalen-1-ol, decahydronaphthalen-2-ol, methylcyclopentanol (each isomer), ethylcyclohexanol (each isomer), propylcyclohexanol (each isomer), pentylcyclohexanol (each isomer), hexylcyclohexanol (each isomer), dimethylcyclohexanol (each isomer), methylethylcyclohexanol (each isomer), diethylcyclohexanol (each isomer), methylpropylcyclohexanol (each isomer), ethylpropylcyclohexanol (each isomer), dipropylcyclohexanol (each isomer), methylbutylcyclohexanol (each isomer), ethylbutylcyclohexanol (each isomer), propylbutylcyclohexanol (each isomer), dibutylcyclohexanol (each isomer), trimethylcyclohexanol (each isomer), methyldiethylcyclohexanol (each isomer), dimethylethylcyclohexanol (each isomer), triethylcyclohexanol (each isomer), methyldipropylcyclohexanol (each isomer), dimethylpropylcyclohexanol (each isomer), ethyldipropylcyclohexanol (each isomer), diethylpropylcyclohexanol (each isomer), tripropylcyclohexanol (each isomer), methyldibutylcyclohexanol (each isomer), dimethylbutylcyclohexanol (each isomer), ethyldibutylcyclohexanol (each isomer), diethylbutylcyclohexanol (each isomer), propyldibutylcyclohexanol (each isomer), dipropylbutylcyclohexanol (each isomer), and tributylcyclohexanol (each isomer); alcohol-based blocking agents having an unsaturated hydrocarbon group such as 2-propen-1-ol, 2-buten-1-ol, 3-buten-1-ol, 2-penten-1-ol, 3-penten-1-ol, 4-penten-1-ol, 2-hexen-1-ol, 3-hexen-1-ol, 4-hexen-1-ol, 5-hexen-1-ol, and benzyl alcohol; alcohol-based blocking agents having an ether group such as 1-methoxyethanol, 1-ethoxyethanol, 1-propoxyethanol, 1-butoxyethanol, 1-pentoxyethanol, 1-hextoxyethanol, 1-methoxy-2-propanol, 1-ethoxy-2-propanol, 1-propoxy-2-propanol, 1-butoxy-2-propanol, 1-pentoxy-2-propanol, and 1-butoxy-2-propanol; alcohol-based blocking agents having a halogen group such as fluoromethanol, chloromethanol, bromoethanol, iodomethanol, difluoromethanol, dichloromethanol, dibromoethanol, diiodomethanol, trifluoromethanol, trichloromethanol, tribromoethanol, triiodomethanol, 2-fluoroethanol, 2-chloroethanol, 2-bromoethanol, 2-iodoethanol, 2,2-difluoroethanol, 2,2-dichloroethanol, 2,2-dibromoethanol, 2,2-diiodoethanol, 2,2,2-trifluoroethanol, 2,2,2-trichloroethanol, 2,2,2-tribromoethanol, 2,2,2-triiodoethanol, 2,2,3,3,3-pentafluoropropanol, 2,2,3,3,3-pentachloropropanol, 2,2,3,3,3-pentabromopropanol, 2,2,3,3,3-pentaiodopropanol, 2,2,2-trifluoro-1-trifluoromethylethanol, 2,2,2-trichloro-1-trichloromethylethanol, 2,2,2-tribromo-1-tribromomethylethanol, 2,2,2-triiodo-1-triiodomethylethanol, 1,1,1,3,3,3-hexafluoro-2-trifluoromethylpropanol, 1,1,1,3,3,3-hexachloro-2-trichloromethylpropanol, 1,1,1,3,3,3-hexabromo-2-tribromomethylpropanol, 1,1,1,3,3,3-hexaiodo-2-triiodomethylpropanol, 2,2,3,3,4,4,5,5-octafluoro-1-pentanol, 2,2,3,3,4,4,5,5-octachloro-1-pentanol, 2,2,3,3,4,4,5,5-octabromo-1-pentanol, 2,2,3,3,4,4,5,5-octaiodo-1-pentanol, hexafluoroisopropanol, hexachloroisopropanol, hexabromoisopropanol, hexaiodoisopropanol, 2-fluorobenzyl alcohol, 2-chlorobenzyl alcohol, 2-bromobenzyl alcohol, 2-iodobenzyl alcohol, 3-fluorobenzyl alcohol, 3-chlorobenzyl alcohol, 3-bromobenzyl alcohol, 3-iodobenzyl alcohol, 4-fluorobenzyl alcohol, 4-chlorobenzyl alcohol, 4-bromobenzyl alcohol, and 4-iodobenzyl alcohol; alcohol-based blocking agents having a carbonyl group such as hydroxy acid esters, methyl glycolate, ethyl glycolate, propyl glycolate (each isomer), butyl glycolate (each isomer), pentyl glycolate (each isomer), hexyl glycolate (each isomer), dodecyl glycolate (each isomer), methyl lactate, ethyl lactate, propyl lactate (each isomer), butyl glycolate (each isomer), pentyl glycolate (each isomer), hexyl glycolate (each isomer), dodecyl glycolate (each isomer), methyl tartronate, ethyl tartronate, propyl tartronate (each isomer), butyl tartronate (each isomer), pentyl tartronate (each isomer), hexyl tartronate (each isomer), dodecyl tartronate (each isomer), methyl 2-hydroxybutyrate, ethyl 2-hydroxybutyrate, propyl 2-hydroxybutyrate (each isomer), butyl 2-hydroxybutyrate (each isomer), pentyl 2-hydroxybutyrate (each isomer), hexyl 2-hydroxybutyrate (each isomer), dodecyl 2-hydroxybutyrate (each isomer), methyl 3-hydroxybutyrate, ethyl 3-hydroxybutyrate, propyl 3-hydroxybutyrate (each isomer), butyl 3-hydroxybutyrate (each isomer), pentyl 3-hydroxybutyrate (each isomer), hexyl 3-hydroxybutyrate (each isomer), dodecyl 3-hydroxybutyrate (each isomer), methyl 4-hydroxybutyrate, ethyl 4-hydroxybutyrate, propyl 4-hydroxybutyrate (each isomer), butyl 4-hydroxybutyrate (each isomer), pentyl 4-hydroxybutyrate (each isomer), hexyl 4-hydroxybutyrate (each isomer), dodecyl 4-hydroxybutyrate (each isomer), dimethyl malate, diethyl malate, dipropyl malate (each isomer), dipentyl malate (each isomer), dihexyl malate (each isomer), didodecyl malate (each isomer), trimethyl citrate, triethyl citrate, tripropyl citrate (each isomer), tributyl citrate (each isomer), tripentyl citrate (each isomer), trihexyl citrate (each isomer), tridodecyl citrate (each isomer), trimethyl isocitrate, triethyl isocitrate, tripropyl isocitrate (each isomer), tripentyl isocitrate (each isomer), trihexyl isocitrate (each isomer), tridodecyl isocitrate (each isomer), hydroxy-2-propanone, hydroxy-2-butanone, hydroxy-2-pentanone, hydroxy-2-hexanone, hydroxy-3-butanone, hydroxy-3-pentanone, and hydroxy-3-hexanone; and alcohol-based blocking agents having an amino group such as 2-(dimethylamino)ethanol, 3-(dimethylamino)propanol, 4-(dimethylamino)pentanol, and 5-(dimethylamino)hexanol.

### [Amine- and Ammonia-Based Blocking Agents]

Examples of compounds that may be used as the amine- and ammonia-based blocking agents include amine compounds having a primary or secondary amino group as an active hydrogen group, and ammonia. When a primary amine compound or ammonia is used as the blocking agent, there is a case in which the primary compound or ammonia as the blocking agent reacts with the carbonic acid derivative, thereby making it difficult to obtain the target blocked isocyanate compound. Accordingly, it is preferable that an amine compound having a secondary amino group be used.

The amine- and ammonia-based blocking agent may be an amine compound having one amino group within the molecule, or an amine compound having two or more amino groups within the molecule. In those cases where an amine compound having two or more amino groups within the molecule is used as the blocking agent, the obtained blocked isocyanate compounds contain blocked isocyanate compounds having a high molecular weight, and the blocked isocyanate compounds increase the viscosity of the blocked isocyanate composition containing the blocked isocyanate compounds, and may sometimes cause gelling of the blocked isocyanate composition, thereby making production of the blocked isocyanate compound and the blocked isocyanate composition difficult. Accordingly, an amine compound having one amino group within the molecule is preferably used as the amine- and ammonia-based blocking agent.

Examples of the preferred amine- and ammonia-based blocking agent include a secondary amine compound of general formula (V) shown below (hereinafter sometimes referred to as the "secondary amine compound (V)").

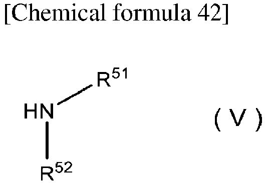

In general formula (V), R⁵¹ and R⁵² are each independently a monovalent organic group. R⁵¹ and R⁵² may be bonded together to form a cyclic structure via a carbon-carbon bond, a carbon-oxygen-carbon bond, or a carbon-nitrogen-carbon bond.

### (R⁵¹ and R⁵²)

R⁵¹ and R⁵² are each independently a monovalent organic group. R⁵¹ and R⁵² may be bonded together to form a cyclic structure via a carbon-carbon bond, a carbon-oxygen-carbon bond, or a carbon-nitrogen-carbon bond.

Among them, it is preferable that R⁵¹ and R⁵² be each a substituted or unsubstituted monovalent aliphatic hydrocarbon group of 1 or more but not more than 70 carbon atoms or a monovalent aromatic hydrocarbon group of 6 or more but not more than 70 carbon atoms which may have an ether group, carbonyl group or ester group.

The number of carbon atoms in the aliphatic hydrocarbon group as R⁵¹ and R⁵² is 1 or more but not more than 70, and is preferably 1 or more but not more than 20, more preferably 1 or more but not more than 12, and even more preferably 1 or more but not more than 10.

Specific examples of the aliphatic hydrocarbon group as R⁵¹ and R⁵² include alkyl groups such as a methyl group, ethyl group, propyl group (each isomer), butyl group (each isomer), pentyl group (each isomer), hexyl group (each isomer), heptyl group (each isomer), octyl group (each isomer), nonyl group (each isomer), decyl group (each isomer), undecyl group (each isomer), dodecyl group (each isomer), tridecyl group (each isomer), tetradecyl group (each isomer), pentadecyl group (each isomer), hexadecyl group (each isomer), heptadecyl group (each isomer), octadecyl group (each isomer), nonadecyl group (each isomer), and eicosyl group (each isomer); and cycloalkyl groups such as a cyclopentyl group, cyclohexyl group, cycloheptyl group, cyclooctyl group, cyclononyl group, and cyclodecyl group.

The number of carbon atoms in the aromatic hydrocarbon group as R⁵¹ and R⁵² is 6 or more but not more than 70, and is preferably 6 or more but not more than 20, more preferably 6 or more but not more than 12, and even more preferably 6 or more but not more than 10.

Specific examples of the aromatic hydrocarbon group as R⁵¹ and R⁵² include aryl groups such as a phenyl group, naphthyl group, anthryl group, pyrenyl group, and phenanthryl group.

Examples of substituents on the aliphatic hydrocarbon group as R⁵¹ and R⁵² include aromatic hydrocarbon groups, a hydroxyl group, a cyano group, and halogen atoms.

Examples of substituents on the aromatic hydrocarbon group as R⁵¹ and R⁵² include aliphatic hydrocarbon groups, a hydroxyl group, a cyano group, and halogen atoms.

The aromatic hydrocarbon group as a substituent on the aliphatic hydrocarbon group is preferably an aromatic hydrocarbon group of 6 or more but not more than 10 carbon atoms, and specific examples thereof include the aromatic hydrocarbon groups mentioned above.

The aliphatic hydrocarbon group as a substituent on the aromatic hydrocarbon group is preferably an aliphatic hydrocarbon group of 1 or more but not more than 10 carbon atoms, and specific examples thereof include the aliphatic hydrocarbon groups mentioned above.

Examples of the halogen atoms include a fluorine atom, chlorine atom, bromine atom, and iodine atom.

Examples of the preferred secondary amine compound (V) in which R⁵¹ and R⁵² are aliphatic hydrocarbon groups include secondary amine compounds having saturated aliphatic hydrocarbon groups, such as dimethylamine, methylethylamine, diethylamine, methylpropylamine (each isomer), ethylpropylamine (each isomer), dipropylamine (each isomer), methylbutylamine (each isomer), ethylbutylamine (each isomer), propylbutylamine (each isomer), dibutylamine (each isomer), methylpentylamine (each isomer), ethylpentylamine (each isomer), propylpentylamine (each isomer), butylpentylamine (each isomer), dipentylamine (each isomer), methylhexylamine (each isomer), ethylhexylamine (each isomer), propylhexylamine (each isomer), pentylhexylamine (each isomer), dihexylamine (each isomer), methylheptylamine (each isomer), ethylheptylamine (each isomer), propylheptylamine (each isomer), pentylheptylamine (each isomer), hexylheptylamine (each isomer), diheptylamine (each isomer), methyloctylamine (each isomer), ethyloctylamine (each isomer), propyloctylamine (each isomer), pentyloctylamine (each isomer), hexyloctylamine (each isomer), heptyloctylamine (each isomer), dioctylamine (each isomer), didecylamine (each isomer), and didodecylamine (each isomer); secondary amine compounds having a saturated aliphatic hydrocarbon group with an ether group, such as N-(2-methoxyethyl)methylamine, N-(2-ethoxyethyl)methylamine, N-(2-propoxyethyl)methylamine, N-(2-butoxyethyl)methylamine, N-(2-pentoxyethyl)methylamine, N-(2-hextoxyethyl)methylamine, N-(2-methoxyethyl)ethylamine, N-(2-ethoxyethyl)ethylamine, N-(2-propoxyethyl)ethylamine, N-(2-butoxyethyl)ethylamine, N-(2-pentoxyethyl)ethylamine, and N-(2-hextoxyethyl)ethylamine; secondary amine compounds having a halogen atom, such as bis(fluoromethyl)amine, bis(chloromethyl)amine, bis(bromomethyl)amine, bis(iodomethyl)amine, bis(difluoromethyl)amine, bis(dichloromethyl)amine, bis(dibromomethyl)amine, bis(diiodomethyl)amine, bis(trifluoromethyl)amine, bis(trichloromethyl)amine, bis(tribromomethyl)amine, bis(triiodomethyl)amine, bis(2,2,2-trifluoroethyl)amine, bis(2,2,2-trichloroethyl)amine, bis(2,2,2-tribromoethyl)amine, bis(2,2,2-triiodoethyl)amine, bis(2,2,3,3,3-pentafluoropropyl)amine, bis(2,2,3,3,3-pentachloropropyl)amine, bis(2,2,3,3,3-pentabromopropyl)amine, bis(2,2,3,3,3-pentaiodopropyl)amine, bis(2,2,2-trifluoro-1-trifluoromethylethyl)amine, bis(2,2,2-trichloro-1-trichloromethylethyl)amine, bis(2,2,2-tribromo-1-tribromomethylethyl)amine, bis(2,2,2-triiodo-1-triiodomethylethyl)amine, bis(1,1,1,3,3,3-hexafluoro-2-trifluoromethylpropyl)amine, bis(1,1,1,3,3,3-hexachloro-2-trichloromethylpropyl)amine, bis(1,1,1,3,3,3-hexabromo-2-tribromomethylpropyl)amine, and bis(1,1,1,3,3,3-hexaiodo-2-triiodomethylpropyl)amine; and secondary amine compounds having a carbonyl group, such as dimethyl iminodiacetate, diethyl iminodiacetate, dipropyl iminodiacetate, dibutyl iminodiacetate, dipentyl iminodiacetate, dihexyl iminodiacetate, N-methylglycine ethyl ester, and N-ethylglycine ethyl ester.

Further, examples of other amine-based blocking agents besides the compounds listed above include pyrrolidine, methylpyrrolidine (each isomer), ethylpyrrolidine (each isomer), propylpyrrolidine (each isomer), butylpyrrolidine (each isomer), dimethylpyrrolidine (each isomer), methylethylpyrrolidine (each isomer), diethylpyrrolidine (each isomer), piperidine, methylpiperidine (each isomer), ethylpiperidine (each isomer), propylpiperidine (each isomer), butylpiperidine (each isomer), dimethylpiperidine (each isomer), methylethylpiperidine (each isomer), diethylpiperidine (each isomer), azepane, methylazepane (each isomer), ethylazepane (each isomer), propylazepane (each isomer), butylazepane (each isomer), dimethylazepane (each isomer), methylethylazepane (each isomer), diethylazepane (each isomer), imidazole, methylpiperazine, and pyrrole; methylbenzylamine, ethylbenzylamine, propylbenzylamine (each isomer), butylbenzylamine (each isomer), pentylbenzylamine (each isomer), and hexylbenzylamine (each isomer); and amine-based blocking agents having an ether group, such as morpholine, methylmorpholine (each isomer), ethylmorpholine (each isomer), propylmorpholine (each isomer), butylmorpholine (each isomer), dimethylmorpholine (each isomer), methylethylmorpholine (each isomer), diethylmorpholine (each isomer), methylpropylmorpholine (each isomer), ethylpropylmorpholine (each isomer), dipropylmorpholine (each isomer), methylbutylmorpholine (each isomer), ethylbutylmorpholine (each isomer), propylbutylmorpholine (each isomer), and dibutylmorpholine (each isomer).

Furthermore, the compounds mentioned above as the secondary amine compound (I) may also be used as amine-based blocking agents other than the compounds listed above. Compared with the compound (V), in which R⁵¹ and R⁵² are aliphatic hydrocarbon groups, the compounds mentioned above as the secondary amine compound (I) have lower basicity, and have comparatively less effect on denaturation of the blocked isocyanate compound and the isocyanate compound. In those cases where the secondary amine compound (1) described above is used as the amine-based blocking agent, the secondary amine compound used to improve thermal denaturation and coloration and the secondary amine compound used as the blocking agent may be identical to or different from each other.

### [Hydroxylamine-Based Blocking Agent]

There are no particular limitations on the hydroxylamine-based blocking agent, and the blocking agent may be a hydroxylamine compound having one hydroxylamine structure within the molecule, or a hydroxylamine compound having two or more hydroxylamine structures within the molecule. In those cases where a hydroxylamine compound having two or more hydroxylamine structures within the molecule is used as the blocking agent, the obtained blocked isocyanate compounds contain blocked isocyanate compounds having a high molecular weight, and the blocked isocyanate compounds increase the viscosity of the blocked isocyanate composition containing the blocked isocyanate compounds, and may sometimes cause gelling of the blocked isocyanate composition, thereby making production of the blocked isocyanate compound and the blocked isocyanate composition difficult. Accordingly, a compound having one hydroxylamine structure within the molecule is preferably used as the hydroxylamine-based blocking agent.

Examples of the hydroxylamine compound having one hydroxylamine structure within the molecule include dimethylhydroxylamine, methylethylhydroxylamine, diethylhydroxylamine, methylpropylhydroxylamine (each isomer), ethylpropylhydroxylamine (each isomer), dipropylhydroxylamine (each isomer), methylbutylhydroxylamine (each isomer), ethylbutylhydroxylamine (each isomer), dibutylhydroxylamine (each isomer), N-hydroxysuccinimide, and N-hydroxyphthalimide.

Further, although one of the blocking agents described above may be used alone, a combination of two or more thereof may also be used. For example, when a combination of a blocking agent with low nucleophilicity and a blocking agent with high nucleophilicity is used, the blocking agent with high nucleophilicity reacts with blocked isocyanate groups formed by blocking isocyanate groups with the carbonic acid derivative (hereinafter, sometimes referred to as "blocked isocyanate groups derived from the carbonic acid derivative"), thereby contributing to a reduction in blocked isocyanate functional groups derived from the carbonic acid derivative, and therefore the blocking agent with high nucleophilicity is more preferable. Furthermore, it is more preferable that a blocking agent having nucleophilicity that is at least as high as that of a compound derived from at least one carbonic acid derivative be used. Examples of the blocking agent with high nucleophilicity are as described above.

### <Carbonic Acid Derivative>

Although there are no particular limitations on the carbonic acid derivative, a compound of general formula (VI) shown below (hereinafter sometimes referred to as the "carbonic acid derivative (VI)") is preferably used in terms of ease of availability.

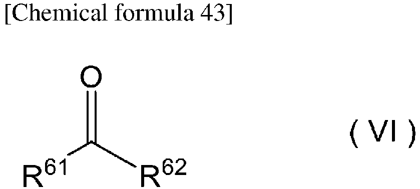

In general formula (VI), R⁶¹ and R⁶² are each independently an amino group, a substituted or unsubstituted alkoxy group of 1 or more but not more than 20 carbon atoms, a substituted or unsubstituted aryloxy group of 6 or more but not more than 20 carbon atoms, a substituted or unsubstituted alkylamino group of 1 or more but not more than 20 carbon atoms or a substituted or unsubstituted arylamino group of 6 or more but not more than 20 carbon atoms.

### [R⁶¹ and R⁶²]

R⁶¹ and R⁶² may be identical to or different from each other.

Examples of the alkoxy group of 1 or more but not more than 20 carbon atoms as R⁶¹ and R⁶² include a methoxy group, ethoxy group, propyloxy group (each isomer), butoxy group (each isomer), and hexyloxy group (each isomer).

Examples of the aryloxy group of 6 or more but not more than 20 carbon atoms as R⁶¹ and R⁶² include a phenoxy group and a naphthyloxy group.

Examples of the alkylamino group of 1 or more but not more than 20 carbon atoms as R⁶¹ and R⁶² include a methylamino group, ethylamino group, propylamino group (each isomer), butylamino group (each isomer), and hexylamino group (each isomer).

Examples of the arylamino group of 6 or more but not more than 20 carbon atoms as R⁶¹ and R⁶² include a phenylamino group and a naphthylamino group.

Examples of substituents on the alkoxy group, aryloxy group, alkylamino group or arylamino group include alkyl groups and alkoxy groups.

Among them, it is preferable that R⁶¹ and R⁶² be each independently an amino group, a substituted or unsubstituted aryloxy group of 6 or more but not more than 20 carbon atoms, or a substituted or unsubstituted arylamino group of 6 or more but not more than 20 carbon atoms.

Examples of the preferred carbonic acid derivative (VI) include urea compounds, N-unsubstituted carbamate esters, carbonate esters, and N-substituted carbamate esters.

### [Urea Compounds]

Urea compounds are compounds having at least one urea linkage within the molecule. There are no particular limitations on the urea compound, and urea compounds having one urea linkage within the molecule, or urea compounds having two or more urea linkages within the molecule may be used. In those cases where a urea compound having two or more urea linkages within the molecule is used, the reaction product contains components of high molecular weight, thereby making production of the blocked isocyanate compound and the blocked isocyanate composition difficult. Accordingly, a compound having one urea linkage within the molecule is preferably used as the urea compound. Among the compounds having one urea linkage within the molecule, a compound of general formula (VI-1) shown below (hereinafter sometimes referred to as the "urea compound (VI-1)") is particularly preferred.

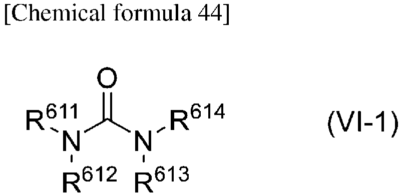

In general formula (VI-1), R⁶¹¹, R⁶¹², R⁶¹³ and R⁶¹⁴ are each independently an alkyl group of 1 or more but not more than 20 carbon atoms, an aralkyl group of 7 or more but not more than 20 carbon atoms, an aryl group of 6 or more but not more than 20 carbon atoms, or a hydrogen atom. The total number of carbon atoms constituting R⁶¹¹ and R⁶¹² is an integer of at least 0 but not more than 20, and the total number of carbon atoms constituting R⁶¹³ and R⁶¹⁴ is an integer of at least 0 but not more than 20.

### (R⁶¹¹, R⁶¹², R⁶¹³ and R⁶¹⁴)

R⁶¹¹, R⁶¹², R⁶¹³ and R⁶¹⁴ may be identical to or different from each other.

Examples of the alkyl group of 1 or more but not more than 20 carbon atoms as R⁶¹¹, R⁶¹², R⁶¹³ and R⁶¹⁴ include alkyl groups such as a methyl group, ethyl group, propyl group (each isomer), butyl group (each isomer), pentyl group (each isomer), hexyl group (each isomer), heptyl group (each isomer), octyl group (each isomer), nonyl group (each isomer), decyl group (each isomer), undecyl group (each isomer), dodecyl group (each isomer), tridecyl group (each isomer), tetradecyl group (each isomer), pentadecyl group (each isomer), hexadecyl group (each isomer), heptadecyl group (each isomer), octadecyl group (each isomer), nonadecyl group (each isomer), and eicosyl group (each isomer); and cycloalkyl groups such as a cyclopentyl group, cyclohexyl group, cycloheptyl group, cyclooctyl group, cyclononyl group, and cyclodecyl group.

Examples of the aralkyl group of 1 or more but not more than 20 carbon atoms as R⁶¹¹, R⁶¹², R⁶¹³ and R⁶¹⁴ include a benzyl group.

Examples of the aryl group of 6 or more but not more than 20 carbon atoms as R⁶¹¹, R⁶¹², R⁶¹³ and R⁶¹⁴ include a phenyl group, naphthyl group, anthryl group, pyrenyl group, and phenanthryl group.

Examples of the preferred urea compound (VI-1) include urea, methylurea, ethylurea, propylurea (each isomer), butylurea (each isomer), pentylurea (each isomer), hexylurea (each isomer), heptylurea (each isomer), octylurea (each isomer), nonylurea (each isomer), decylurea (each isomer), undecylurea (each isomer), dodecylurea (each isomer), tridecylurea (each isomer), tetradecylurea (each isomer), pentadecylurea (each isomer), hexadecylurea (each isomer), heptadecylurea (each isomer), octadecylurea (each isomer), nonadecylurea (each isomer), eicosylurea (each isomer), phenylurea, N-(methylphenyl)urea (each isomer), N-(ethylphenyl)urea (each isomer), N-(propylphenyl)urea (each isomer), N-(butylphenyl)urea (each isomer), N-(pentylphenyl)urea (each isomer), N-(hexylphenyl)urea (each isomer), N-(heptylphenyl)urea (each isomer), N-(octylphenyl)urea (each isomer), N-(nonylphenyl)urea (each isomer), N-(decylphenyl)urea (each isomer), N-(biphenyl)urea (each isomer), N-(dimethylphenyl)urea (each isomer), N-(diethylphenyl)urea (each isomer), N-(dipropylphenyl)urea (each isomer), N-(dibutylphenyl)urea (each isomer), N-(dipentylphenyl)urea (each isomer), N-(dihexylphenyl)urea (each isomer), N-(diheptylphenyl)urea (each isomer), N-terphenylurea (each isomer), N-(trimethylphenyl)urea (each isomer), N-(triethylphenyl)urea (each isomer), N-(tripropylphenyl)urea (each isomer), N-(tributylphenyl)urea (each isomer), N-(phenylmethyl)urea (each isomer), N-(phenylethyl)urea (each isomer), N-(phenylpropyl)urea (each isomer), N-(phenylbutyl)urea (each isomer), N-(phenylpentyl)urea (each isomer), N-(phenylhexyl)urea (each isomer), N-(phenylheptyl)urea (each isomer), N-(phenyloctyl)urea (each isomer), N-(phenylnonyl)urea (each isomer), N,N'-dimethylurea, N-methyl-N'-ethylurea, N,N'-diethylurea, N-methyl-N'-propylurea (each isomer), N-ethyl-N'-propylurea (each isomer), N,N'-dipropylurea (each isomer), N-methyl-N'-butylurea (each isomer), N-ethyl-N'-butylurea (each isomer), N-propyl-N'-butylurea (each isomer), N,N'-dibutylurea (each isomer), N-methyl-N'-pentylurea (each isomer), N-ethyl-N'-pentylurea (each isomer), N-propyl-N'-pentylurea (each isomer), N-butyl-N'-pentylurea (each isomer), N,N'-dipentylurea (each isomer), N-methyl-N'-hexylurea (each isomer), N-ethyl-N'-hexylurea (each isomer), N-propyl-N'-hexylurea (each isomer), N-butyl-N'-hexylurea (each isomer), N-pentyl-N'-hexylurea (each isomer), N,N'-dihexylurea (each isomer), N-methyl-N'-heptylurea (each isomer), N-ethyl-N'-heptylurea (each isomer), N-propyl-N'-heptylurea (each isomer), N-butyl-N'-heptylurea (each isomer), N-pentyl-N'-heptylurea (each isomer), N-hexyl-N'-heptylurea (each isomer), N,N'-diheptylurea (each isomer), N-methyl-N'-octylurea (each isomer), N-ethyl-N'-octylurea (each isomer), N-propyl-N'-octylurea (each isomer), N-butyl-N'-octylurea (each isomer), N-pentyl-N'-octylurea (each isomer), N-hexyl-N'-octylurea (each isomer), N-heptyl-N'-octylurea (each isomer), N,N'-dioctylurea (each isomer), N-methyl-N'-nonylurea (each isomer), N-ethyl-N'-nonylurea (each isomer), N-propyl-N'-nonylurea (each isomer), N-butyl-N'-nonylurea (each isomer), N-pentyl-N'-nonylurea (each isomer), N-hexyl-N'-nonylurea (each isomer), N-heptyl-N'-nonylurea (each isomer), N-octyl-N'-nonylurea (each isomer), N,N'-dinonylurea (each isomer), N-methyl-N'-decylurea (each isomer), N-ethyl-N'-decylurea (each isomer), N-propyl-N'-decylurea (each isomer), N-butyl-N'-decylurea (each isomer), N-pentyl-N'-decylurea (each isomer), N-hexyl-N'-decylurea (each isomer), N-heptyl-N'-decylurea (each isomer), N-octyl-N'-decylurea (each isomer), N-nonyl-N'-decylurea (each isomer), N,N'-didecylurea (each isomer), N-methyl-N'-undecylurea (each isomer), N-ethyl-N'-undecylurea (each isomer), N-propyl-N'-undecylurea (each isomer), N-butyl-N'-undecylurea (each isomer), N-pentyl-N'-undecylurea (each isomer), N-hexyl-N'-undecylurea (each isomer), N-heptyl-N'-undecylurea (each isomer), N-octyl-N'-undecylurea (each isomer), N-nonyl-N'-undecylurea (each isomer), N-decyl-N'-undecylurea (each isomer), N,N'-diundecylurea (each isomer), N-methyl-N'-dodecylurea (each isomer), N-ethyl-N'-dodecylurea (each isomer), N-propyl-N'-dodecylurea (each isomer), N-butyl-N'-dodecylurea (each isomer), N-pentyl-N'-dodecylurea (each isomer), N-hexyl-N'-dodecylurea (each isomer), N-heptyl-N'-dodecylurea (each isomer), N-octyl-N'-dodecylurea (each isomer), N-nonyl-N'-dodecylurea (each isomer), N-decyl-N'-dodecylurea (each isomer), N-undecyl-N'-dodecylurea (each isomer), N,N'-didodecylurea (each isomer), N-methyl-N'-phenylurea, N-ethyl-N'-phenylurea, N-propyl-N'-phenylurea (each isomer), N-butyl-N'-phenylurea (each isomer), N-pentyl-N'-phenylurea (each isomer), N-hexyl-N'-phenylurea (each isomer), N,N'-diphenylurea, N-methyl-N'-methylphenylurea, N-ethyl-N'-methylphenylurea, N-propyl-N'-methylphenylurea (each isomer), N-butyl-N'-methylphenylurea (each isomer), N-pentyl-N'-methylphenylurea (each isomer), N-hexyl-N'-methylphenylurea (each isomer), N-phenyl-N'-methylphenylurea, N,N'-dimethylphenylurea, N-methyl-N'-ethylphenylurea (each isomer), N-ethyl-N'-ethylphenylurea (each isomer), N-propyl-N'-ethylphenylurea (each isomer), N-butyl-N'-ethylphenylurea (each isomer), N-pentyl-N'-ethylphenylurea (each isomer), N-hexyl-N'-ethylphenylurea (each isomer), N-phenyl-N'-ethylphenylurea (each isomer), N-methylphenyl-N'-ethylphenylurea (each isomer), N,N'-diethylphenylurea (each isomer), N,N-dimethylurea, N-methyl-N-ethylurea, N,N-diethylurea, N-methyl-N-propylurea (each isomer), N-ethyl-N-propylurea (each isomer), N,N-dipropylurea (each isomer), N-methyl-N-butylurea (each isomer), N-ethyl-N-butylurea (each isomer), N-propyl-N-butylurea (each isomer), N,N-dibutylurea (each isomer), N-methyl-N-pentylurea (each isomer), N-ethyl-N-pentylurea (each isomer), N-propyl-N-pentylurea (each isomer), N-butyl-N-pentylurea (each isomer), N,N-dipentylurea (each isomer), N-methyl-N-hexylurea (each isomer), N-ethyl-N-hexylurea (each isomer), N-propyl-N-hexylurea (each isomer), N-butyl-N-hexylurea (each isomer), N-pentyl-N-hexylurea (each isomer), N,N-dihexylurea (each isomer), N-methyl-N-heptylurea (each isomer), N-ethyl-N-heptylurea (each isomer), N-propyl-N-heptylurea (each isomer), N-butyl-N-heptylurea (each isomer), N-pentyl-N-heptylurea (each isomer), N-hexyl-N-heptylurea (each isomer), N,N-diheptylurea (each isomer), N-methyl-N-octylurea (each isomer), N-ethyl-N-octylurea (each isomer), N-propyl-N-octylurea (each isomer), N-butyl-N-octylurea (each isomer), N-pentyl-N-octylurea (each isomer), N-hexyl-N-octylurea (each isomer), N-heptyl-N-octylurea (each isomer), N,N-dioctylurea (each isomer), N-methyl-N-nonylurea (each isomer), N-ethyl-N-nonylurea (each isomer), N-propyl-N-nonylurea (each isomer), N-butyl-N-nonylurea (each isomer), N-pentyl-N-nonylurea (each isomer), N-hexyl-N-nonylurea (each isomer), N-heptyl-N-nonylurea (each isomer), N-octyl-N-nonylurea (each isomer), N,N-dinonylurea (each isomer), N-methyl-N-decylurea (each isomer), N-ethyl-N-decylurea (each isomer), N-propyl-N-decylurea (each isomer), N-butyl-N-decylurea (each isomer), N-pentyl-N-decylurea (each isomer), N-hexyl-N-decylurea (each isomer), N-heptyl-N-decylurea (each isomer), N-octyl-N-decylurea (each isomer), N-nonyl-N-decylurea (each isomer), N,N-didecylurea (each isomer), N-methyl-N-undecylurea (each isomer), N-ethyl-N-undecylurea (each isomer), N-propyl-N-undecylurea (each isomer), N-butyl-N-undecylurea (each isomer), N-pentyl-N-undecylurea (each isomer), N-hexyl-N-undecylurea (each isomer), N-heptyl-N-undecylurea (each isomer), N-octyl-N-undecylurea (each isomer), N-nonyl-N-undecylurea (each isomer), N-decyl-N-undecylurea (each isomer), N,N-diundecylurea (each isomer), N-methyl-N-dodecylurea (each isomer), N-ethyl-N-dodecylurea (each isomer), N-propyl-N-dodecylurea (each isomer), N-butyl-N-dodecylurea (each isomer), N-pentyl-N-dodecylurea (each isomer), N-hexyl-N-dodecylurea (each isomer), N-heptyl-N-dodecylurea (each isomer), N-octyl-N-dodecylurea (each isomer), N-nonyl-N-dodecylurea (each isomer), N-decyl-N-dodecylurea (each isomer), N-undecyl-N-dodecylurea (each isomer), N,N-didodecylurea (each isomer), N-methyl-N-phenylurea, N-ethyl-N-phenylurea, N-propyl-N-phenylurea (each isomer), N-butyl-N-phenylurea (each isomer), N-pentyl-N-phenylurea (each isomer), N-hexyl-N-phenylurea (each isomer), N,N-diphenylurea, N-methyl-N-methylphenylurea, N-ethyl-N-methylphenylurea, N-propyl-N-methylphenylurea (each isomer), N-butyl-N-methylphenylurea (each isomer), N-pentyl-N-methylphenylurea (each isomer), N-hexyl-N-methylphenylurea (each isomer), N-phenyl-N-methylphenylurea, N,N-dimethylphenylurea, N-methyl-N-ethylphenylurea (each isomer), N-ethyl-N-ethylphenylurea (each isomer), N-propyl-N-ethylphenylurea (each isomer), N-butyl-N-ethylphenylurea (each isomer), N-pentyl-N-ethylphenylurea (each isomer), N-hexyl-N-ethylphenylurea (each isomer), N-phenyl-N-ethylphenylurea (each isomer), N-methylphenyl-N-ethylphenylurea (each isomer), N,N-diethylphenylurea (each isomer), morpholine-4-carboxamide, piperidine-4-carboxamide, pyrrolidine-4-carboxamide, 1H-imidazole-1-carboxamide, N,N,N'-trimethylurea, N-ethyl-N,N'-dimethylurea, N-ethyl-N,N'-dimethylurea, N,N,N'- trimethylurea, N-ethyl-N',N'-dimethylurea, N,N,N'-triethylurea, N-propyl-N',N'-dimethylurea (each isomer), N-propyl-N',N'-diethylurea (each isomer), N,N,N'-tripropylurea (each isomer), N-butyl-N',N'-dimethylurea (each isomer), N-butyl-N',N'-diethylurea (each isomer), N-butyl-N',N'-dipropylurea (each isomer), N,N,N'-tributylurea (each isomer), N-pentyl-N',N'-dimethylurea (each isomer), N-pentyl-N',N'-diethylurea (each isomer), N-pentyl-N',N'-dipropylurea (each isomer), N-pentyl-N',N'-dibutylurea (each isomer), N,N,N'-tripentylurea (each isomer), N-hexyl-N',N'-dimethylurea (each isomer), N-hexyl-N',N'-diethylurea (each isomer), N-hexyl-N',N'-dipropylurea (each isomer), N-hexyl-N',N'-dibutylurea (each isomer), N-hexyl-N',N'-dipentylurea (each isomer), N,N,N'-trihexylurea (each isomer), N-heptyl-N',N'-dimethylurea (each isomer), N-heptyl-N',N'-diethylurea (each isomer), N-heptyl-N',N'-dipropylurea (each isomer), N-heptyl-N',N'-dibutylurea (each isomer), N-heptyl-N',N'-dipentylurea (each isomer), N-heptyl-N',N'-dihexylurea (each isomer), N,N,N'-triheptylurea (each isomer), N-octyl-N',N'-dimethylurea (each isomer), N-octyl-N',N'-diethylurea (each isomer), N-octyl-N',N'-dipropylurea (each isomer), N-octyl-N',N'-dibutylurea (each isomer), N-octyl-N',N'-dipentylurea (each isomer), N-octyl-N',N'-dihexylurea (each isomer), N-octyl-N',N'-diheptylurea (each isomer), N,N,N'-trioctylurea (each isomer), N-nonyl-N',N'-dimethylurea (each isomer), N-nonyl-N',N'-diethylurea (each isomer), N-nonyl-N',N'-dipropylurea (each isomer), N-nonyl-N',N'-dibutylurea (each isomer), N-nonyl-N',N'-dipentylurea (each isomer), N-nonyl-N',N'-dihexylurea (each isomer), N-nonyl-N',N'-diheptylurea (each isomer), N-nonyl-N',N'-dioctylurea (each isomer), N,N,N'-trinonylurea (each isomer), N-decyl-N',N'-dimethylurea (each isomer), N-decyl-N',N'-diethylurea (each isomer), N-decyl-N',N'-dipropylurea (each isomer), N-decyl-N',N'-dibutylurea (each isomer), N-decyl-N',N'-dipentylurea (each isomer), N-decyl-N',N'-dihexylurea (each isomer), N-decyl-N',N'-diheptylurea (each isomer), N-decyl-N',N'-dioctylurea (each isomer), N-decyl-N',N'-dinonylurea (each isomer), N,N,N'-tridecylurea (each isomer), N-undecyl-N',N'-dimethylurea (each isomer), N-undecyl-N',N'-diethylurea (each isomer), N-undecyl-N',N'-dipropylurea (each isomer), N-undecyl-N',N'-dibutylurea (each isomer), N-undecyl-N',N'-dipentylurea (each isomer), N-undecyl-N',N'-dihexylurea (each isomer), N-undecyl-N',N'-diheptylurea (each isomer), N-undecyl-N',N'-dioctylurea (each isomer), N-undecyl-N',N'-dinonylurea (each isomer), N-undecyl-N',N'-didecylurea (each isomer), N,N,N'-triundecylurea (each isomer), N-dodecyl-N',N'-dimethylurea (each isomer), N-dodecyl-N',N'-diethylurea (each isomer), N-dodecyl-N',N'-dipropylurea (each isomer), N-dodecyl-N',N'-dibutylurea (each isomer), N-dodecyl-N',N'-dipentylurea (each isomer), N-dodecyl-N',N'-dihexylurea (each isomer), N-dodecyl-N',N'-diheptylurea (each isomer), N-dodecyl-N',N'-dioctylurea (each isomer), N-dodecyl-N',N'-dinonylurea (each isomer), N-dodecyl-N',N'-didecylurea (each isomer), N-dodecyl-N',N'-diundecylurea (each isomer), N,N,N'-tridodecylurea (each isomer), N-phenyl-N',N'-dimethylurea (each isomer), N-phenyl-N',N'-diethylurea (each isomer), N-phenyl-N',N'-dipropylurea (each isomer), N-phenyl-N',N'-dibutylurea (each isomer), N-phenyl-N',N'-dipentylurea (each isomer), N-phenyl-N',N'-dihexylurea (each isomer), N,N,N'-triphenylurea (each isomer), N-methylphenyl-N',N'-dimethylurea (each isomer), N-methylphenyl-N',N'-diethylurea (each isomer), N-methylphenyl-N',N'-dipropylurea (each isomer), N-methylphenyl-N',N'-dibutylurea (each isomer), N-methylphenyl-N',N'-dipentylurea (each isomer), N-methylphenyl-N',N'-dihexylurea (each isomer), N-methylphenyl-N',N'-diphenylurea (each isomer), N,N,N'-trimethylphenylurea, N-ethylphenyl-N',N'-dimethylurea (each isomer), N-ethylphenyl-N',N'-diethylurea (each isomer), N-ethylphenyl-N',N'-dipropylurea (each isomer), N-ethylphenyl-N',N'-dibutylurea (each isomer), N-ethylphenyl-N',N'-dipentylurea (each isomer), N-ethylphenyl-N',N'-dihexylurea (each isomer), N-ethylphenyl-N',N'-diphenylurea (each isomer), N-ethylphenyl-N',N'-dimethylphenylurea (each isomer), N,N,N'-triethylphenylurea (each isomer), N,N,N',N'-tetramethylurea, N,N,N',N'-tetraethylurea, N,N,N',N'-tetraethylurea, N,N,N',N'-tetrapropylurea (each isomer), N,N,N',N'-tetrapropylurea (each isomer), N,N,N',N'-tetrabutylurea (each isomer), N,N,N',N'-tetrabutylurea (each isomer), N,N,N',N'-tetrapentylurea (each isomer), N,N,N',N'-tetrapentylurea (each isomer), N,N,N',N'-tetrahexylurea (each isomer), N,N,N',N'-tetrahexylurea (each isomer), N,N,N',N'-tetraheptylurea (each isomer), N,N,N',N'-tetraheptylurea (each isomer), N,N,N',N'-tetraoctylurea (each isomer), N,N,N',N'-tetraoctylurea (each isomer), N,N,N',N'-tetranonylurea (each isomer), N,N,N',N'-tetranonylurea (each isomer), N,N,N',N'-tetradecylurea (each isomer), N,N,N',N'-tetradecylurea (each isomer), N,N,N',N'-tetraundecylurea (each isomer), N,N,N',N'-tetraundecylurea (each isomer), N,N,N',N'-tetradodecylurea (each isomer), N,N,N',N'-tetradodecylurea (each isomer), 4,4'-carbonyldimorpholine, 1,1'-carbonyldipiperidine, 1,1'-carbonyldipyrrolidine, and 1,1'-carbonyldiimidazole.

Among them, urea is preferable in terms of ease of availability and lower cost, and 1-substituted urea compounds or 1,2-disubstituted urea compounds are more preferable because they can be easily obtained by reacting urea with a primary amine, or 1,1-disubstituted urea compounds are more preferable because they can be easily obtained by reacting urea with a secondary amine.

### [Carbamate Esters]

Carbamate esters are compounds having at least one carbamate ester structure within the molecule. There are no particular limitations on the carbamate ester, and carbamate esters having one carbamate ester structure within the molecule, or carbamate esters having two or more carbamate ester structures within the molecule may be used. In those cases where a carbamate ester having two or more carbamate ester structures within the molecule is used, the reaction product contains components of high molecular weight, thereby making production of the blocked isocyanate compound and the blocked isocyanate composition difficult. Accordingly, a compound having one carbamate ester structure within the molecule is preferably used as the carbamate ester.

Amongst the compounds having one carbamate ester structure within the molecule, a compound of general formula (VI-2) shown below (hereinafter sometimes referred to as the "carbamate ester (VI-2)") is preferred.

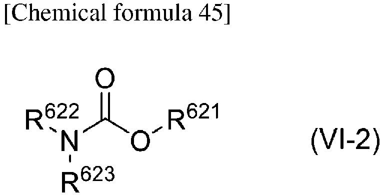

In general formula (VI-2), R⁶²¹ is an alkyl group of 1 or more but not more than 20 carbon atoms, an aralkyl group of 7 or more but not more than 20 carbon atoms, or an aryl group of 6 or more but not more than 20 carbon atoms. R⁶²² and R⁶²³ are each independently a hydrogen atom, an alkyl group of 1 or more but not more than 20 carbon atoms, an aralkyl group of 7 or more but not more than 20 carbon atoms, or an aryl group of 6 or more but not more than 20 carbon atoms.

### (R⁶²¹, R⁶²² and R⁶²³)

R⁶²² and R⁶²³ may be identical to or different from each other.

Examples of the alkyl group, aralkyl group and aryl group as R⁶²¹, R⁶²² and R⁶²³ include the same groups as those mentioned above as R⁶¹¹, R⁶¹², R⁶¹³ and R⁶¹⁴.

Examples of the preferred carbamate ester (VI-2) include N-unsubstituted carbamate esters such as methyl carbamate, ethyl carbamate, propyl carbamate (each isomer), butyl carbamate (each isomer), pentyl carbamate (each isomer), hexyl carbamate (each isomer), heptyl carbamate (each isomer), octyl carbamate (each isomer), nonyl carbamate (each isomer), decyl carbamate (each isomer), undecyl carbamate (each isomer), dodecyl carbamate (each isomer), tridecyl carbamate (each isomer), tetradecyl carbamate (each isomer), pentadecyl carbamate (each isomer), hexadecyl carbamate (each isomer), heptadecyl carbamate (each isomer), octadecyl carbamate (each isomer), nonadecyl carbamate (each isomer), eicosyl carbamate (each isomer), phenyl carbamate, (methylphenyl) carbamate (each isomer), (ethylphenyl) carbamate (each isomer), (propylphenyl) carbamate (each isomer), (butylphenyl) carbamate (each isomer), (pentylphenyl) carbamate (each isomer), (hexylphenyl) carbamate (each isomer), (heptylphenyl) carbamate (each isomer), (octylphenyl) carbamate (each isomer), (nonylphenyl) carbamate (each isomer), (decylphenyl) carbamate (each isomer), (undecylphenyl) carbamate (each isomer), (dodecylphenyl) carbamate (each isomer), (biphenyl) carbamate (each isomer), (terphenyl) carbamate (each isomer), (dimethylphenyl) carbamate (each isomer), (diethylphenyl) carbamate (each isomer), (dipropylphenyl) carbamate (each isomer), (dibutylphenyl) carbamate (each isomer), (dipentylphenyl) carbamate (each isomer), (dihexylphenyl) carbamate (each isomer), (diheptylphenyl) carbamate (each isomer), (dioctylphenyl) carbamate (each isomer), (dinonylphenyl) carbamate (each isomer), (didecylphenyl) carbamate (each isomer), (diundecylphenyl) carbamate (each isomer), (didodecylphenyl) carbamate (each isomer), (trimethylphenyl) carbamate (each isomer), (triethylphenyl) carbamate (each isomer), (tripropylphenyl) carbamate (each isomer), (tributylphenyl) carbamate (each isomer), (tripentylphenyl) carbamate (each isomer), (trihexylphenyl) carbamate (each isomer), (triheptylphenyl) carbamate (each isomer), (trioctylphenyl) carbamate (each isomer), (trinonylphenyl) carbamate (each isomer), (tridecylphenyl) carbamate (each isomer), (triundecylphenyl) carbamate (each isomer), (tridodecylphenyl) carbamate (each isomer), (phenylmethyl) carbamate, (phenylethyl) carbamate (each isomer), (phenylpropyl) carbamate (each isomer), (phenylbutyl) carbamate (each isomer), (2-methoxyethyl) carbamate, (2-ethoxyethyl) carbamate, (2-propoxyethyl) carbamate (each isomer), (2-butoxyethyl) carbamate (each isomer), (1-methoxypropan-2-yl) carbamate, (1-ethoxypropan-2-yl) carbamate, (1-propoxypropan-2-yl) carbamate (each isomer), (1-butoxypropan-2-yl) carbamate (each isomer), (2-methoxypropyl) carbamate, (2-ethoxypropyl) carbamate, (2-propoxypropyl) carbamate (each isomer), (2-butoxypropyl) carbamate (each isomer), and (2-phenoxyethyl) carbamate (each isomer); and N-substituted carbamate esters such as methyl N-methylcarbamate, ethyl N-methylcarbamate, ethyl N-ethylcarbamate, propyl N-methylcarbamate (each isomer), propyl N-ethylcarbamate (each isomer), propyl N-propylcarbamate (each isomer), butyl N-methylcarbamate (each isomer), butyl N-ethylcarbamate (each isomer), butyl N-propylcarbamate (each isomer), butyl N-butylcarbamate (each isomer), pentyl N-methylcarbamate (each isomer), pentyl N-ethylcarbamate (each isomer), pentyl N-propylcarbamate (each isomer), pentyl N-butylcarbamate (each isomer), pentyl N-pentylcarbamate (each isomer), hexyl N-methylcarbamate (each isomer), hexyl N-ethylcarbamate (each isomer), hexyl N-propylcarbamate (each isomer), hexyl N-butylcarbamate (each isomer), hexyl N-pentylcarbamate (each isomer), hexyl N-hexylcarbamate (each isomer), heptyl N-methylcarbamate (each isomer), heptyl N-ethylcarbamate (each isomer), heptyl N-propylcarbamate (each isomer), heptyl N-butylcarbamate (each isomer), heptyl N-pentylcarbamate (each isomer), heptyl N-hexylcarbamate (each isomer), heptyl N-heptylcarbamate (each isomer), octyl N-methylcarbamate (each isomer), octyl N-ethylcarbamate (each isomer), octyl N-propylcarbamate (each isomer), octyl N-butylcarbamate (each isomer), octyl N-pentylcarbamate (each isomer), octyl N-hexylcarbamate (each isomer), octyl N-heptylcarbamate (each isomer), octyl N-octylcarbamate (each isomer), nonyl N-methylcarbamate (each isomer), nonyl N-ethylcarbamate (each isomer), nonyl N-propylcarbamate (each isomer), nonyl N-butylcarbamate (each isomer), nonyl N-pentylcarbamate (each isomer), nonyl N-hexylcarbamate (each isomer), nonyl N-heptylcarbamate (each isomer), nonyl N-octylcarbamate (each isomer), nonyl N-nonylcarbamate (each isomer), decyl N-methylcarbamate (each isomer), decyl N-ethylcarbamate (each isomer), decyl N-propylcarbamate (each isomer), decyl N-butylcarbamate (each isomer), decyl N-pentylcarbamate (each isomer), decyl N-hexylcarbamate (each isomer), decyl N-heptylcarbamate (each isomer), decyl N-octylcarbamate (each isomer), decyl N-nonylcarbamate (each isomer), decyl N-decylcarbamate (each isomer), undecyl N-methylcarbamate (each isomer), undecyl N-ethylcarbamate (each isomer), undecyl N-propylcarbamate (each isomer), undecyl N-butylcarbamate (each isomer), undecyl N-pentylcarbamate (each isomer), undecyl N-hexylcarbamate (each isomer), undecyl N-heptylcarbamate (each isomer), undecyl N-octylcarbamate (each isomer), undecyl N-nonylcarbamate (each isomer), undecyl N-decylcarbamate (each isomer), undecyl N-undecylcarbamate (each isomer), dodecyl N-methylcarbamate (each isomer), dodecyl N-ethylcarbamate (each isomer), dodecyl N-propylcarbamate (each isomer), dodecyl N-butylcarbamate (each isomer), dodecyl N-pentylcarbamate (each isomer), dodecyl N-hexylcarbamate (each isomer), dodecyl N-heptylcarbamate (each isomer), dodecyl N-octylcarbamate (each isomer), dodecyl N-nonylcarbamate (each isomer), dodecyl N-decylcarbamate (each isomer), dodecyl N-undecylcarbamate (each isomer), dodecyl N-dodecylcarbamate (each isomer), phenyl N-methylcarbamate, phenyl N-ethylcarbamate, phenyl N-propylcarbamate (each isomer), phenyl N-butylcarbamate (each isomer), phenyl N-pentylcarbamate (each isomer), phenyl N-hexylcarbamate (each isomer), phenyl N-phenylcarbamate, methylphenyl N-methylcarbamate, methylphenyl N-ethylcarbamate, methylphenyl N-propylcarbamate (each isomer), methylphenyl N-butylcarbamate (each isomer), methylphenyl N-pentylcarbamate (each isomer), methylphenyl N-hexylcarbamate (each isomer), methylphenyl N-phenylcarbamate, methylphenyl N-methylphenylcarbamate, ethylphenyl N-methylcarbamate (each isomer), ethylphenyl N-ethylcarbamate (each isomer), ethylphenyl N-propylcarbamate (each isomer), ethylphenyl N-butylcarbamate (each isomer), ethylphenyl N-pentylcarbamate (each isomer), ethylphenyl N-hexylcarbamate (each isomer), ethylphenyl N-phenylcarbamate (each isomer), ethylphenyl N-methylphenylcarbamate (each isomer), ethylphenyl N-ethylphenylcarbamate (each isomer), methyl N,N-dimethylcarbamate, methyl N,N-diethylcarbamate, methyl N,N-dipropylcarbamate, methyl N,N-dibutylcarbamate, methyl N,N-dipentylcarbamate, methyl N,N-dihexylcarbamate, methyl N,N-diheptylcarbamate, methyl N,N-dioctylcarbamate, methyl N,N-dinonylcarbamate, methyl N,N-didecylcarbamate, methyl N,N-diundecylcarbamate, methyl N,N-didodecylcarbamate, ethyl N,N-dimethylcarbamate, ethyl N,N-diethylcarbamate, ethyl N,N-dipropylcarbamate, ethyl N,N-dibutylcarbamate, ethyl N,N-dipentylcarbamate, ethyl N,N-dihexylcarbamate, ethyl N,N-diheptylcarbamate, ethyl N,N-dioctylcarbamate, ethyl N,N-dinonylcarbamate, ethyl N,N-didecylcarbamate, ethyl N,N-diundecylcarbamate, ethyl N,N-didodecylcarbamate, propyl N,N-dimethylcarbamate, propyl N,N-diethylcarbamate, propyl N,N-dipropylcarbamate, propyl N,N-dibutylcarbamate, propyl N,N-dipentylcarbamate, propyl N,N-dihexylcarbamate, propyl N,N-diheptylcarbamate, propyl N,N-dioctylcarbamate, propyl N,N-dinonylcarbamate, propyl N,N-didecylcarbamate, propyl N,N-diundecylcarbamate, propyl N,N-didodecylcarbamate, butyl N,N-dimethylcarbamate, butyl N,N-diethylcarbamate, butyl N,N-dipropylcarbamate, butyl N,N-dibutylcarbamate, butyl N,N-dipentylcarbamate, butyl N,N-dihexylcarbamate, butyl N,N-diheptylcarbamate, butyl N,N-dioctylcarbamate, butyl N,N-dinonylcarbamate, butyl N,N-didecylcarbamate, butyl N,N-diundecylcarbamate, butyl N,N-didodecylcarbamate, pentyl N,N-dimethylcarbamate, pentyl N,N-diethylcarbamate, pentyl N,N-dipropylcarbamate, pentyl N,N-dibutylcarbamate, pentyl N,N-dipentylcarbamate, pentyl N,N-dihexylcarbamate, pentyl N,N-diheptylcarbamate, pentyl N,N-dioctylcarbamate, pentyl N,N-dinonylcarbamate, pentyl N,N-didecylcarbamate, pentyl N,N-diundecylcarbamate, pentyl N,N-didodecylcarbamate, hexyl N,N-dimethylcarbamate, hexyl N,N-diethylcarbamate, hexyl N,N-dipropylcarbamate, hexyl N,N-dibutylcarbamate, hexyl N,N-dipentylcarbamate, hexyl N,N-dihexylcarbamate, hexyl N,N-diheptylcarbamate, hexyl N,N-dioctylcarbamate, hexyl N,N-dinonylcarbamate, hexyl N,N-didecylcarbamate, hexyl N,N-diundecylcarbamate, hexyl N,N-didodecylcarbamate, heptyl N,N-dimethylcarbamate, heptyl N,N-diethylcarbamate, heptyl N,N-dipropylcarbamate, heptyl N,N-dibutylcarbamate, heptyl N,N-dipentylcarbamate, heptyl N,N-dihexylcarbamate, heptyl N,N-diheptylcarbamate, heptyl N,N-dioctylcarbamate, heptyl N,N-dinonylcarbamate, heptyl N,N-didecylcarbamate, heptyl N,N-diundecylcarbamate, heptyl N,N-didodecylcarbamate, octyl N,N-dimethylcarbamate, octyl N,N-diethylcarbamate, octyl N,N-dipropylcarbamate, octyl N,N-dibutylcarbamate, octyl N,N-dipentylcarbamate, octyl N,N-dihexylcarbamate, octyl N,N-diheptylcarbamate, octyl N,N-dioctylcarbamate, octyl N,N-dinonylcarbamate, octyl N,N-didecylcarbamate, octyl N,N-diundecylcarbamate, and octyl N,N-didodecylcarbamate.

Among them, N-unsubstituted carbamate esters are more preferable in terms of ease of availability and being easily obtainable by reacting inexpensive urea with an alcohol.

### [Carbonate Esters]

Carbonate esters are compounds having at least one carbonate ester structure within the molecule. There are no particular limitations on the carbonate ester, and carbonate esters having one carbonate ester structure within the molecule, or carbonate esters having two or more carbonate ester structures within the molecule may be used. In those cases where a carbonate ester having two or more carbonate ester structures within the molecule is used, the reaction product contains components of high molecular weight, thereny making production of the blocked isocyanate compound and the blocked isocyanate composition difficult. Accordingly, a compound having one carbonate ester structure within the molecule is preferably used as the carbonate ester. Amongst the compounds having one carbonate ester structure within the molecule, a compound of general formula (VI-3) shown below (hereinafter sometimes referred to as the "carbonate ester (VI-3)") is more preferable.

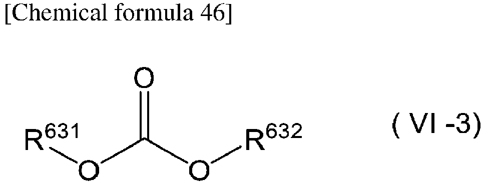

In general formula (VI-3), R⁶³¹ and R⁶³² are each independently an aralkyl group of 7 or more but not more than 20 carbon atoms, an alkyl group of 1 or more but not more than 20 carbon atoms, or an aryl group of 6 or more but not more than 20 carbon atoms.

### (R⁶³¹ and R⁶³²)

R⁶³¹ and R⁶³² may be identical to or different from each other.

Examples of the alkyl group, aralkyl group and aryl group as R⁶³¹ and R⁶³² include the same groups as those mentioned above as R⁶¹¹, R⁶¹², R⁶¹³ and R⁶¹⁴.

Examples of the preferred carbonate ester (VI-3) include dimethyl carbonate, methyl ethyl carbonate, diethyl carbonate, methyl propyl carbonate (each isomer), ethyl propyl carbonate (each isomer), dipropyl carbonate (each isomer), methyl butyl carbonate (each isomer), ethyl butyl carbonate (each isomer), propyl butyl carbonate (each isomer), dibutyl carbonate (each isomer), methyl pentyl carbonate (each isomer), ethyl pentyl carbonate (each isomer), propyl pentyl carbonate (each isomer), butyl pentyl carbonate (each isomer), dipentyl carbonate (each isomer), methyl hexyl carbonate (each isomer), ethyl hexyl carbonate (each isomer), propyl hexyl carbonate (each isomer), butyl hexyl carbonate (each isomer), pentyl hexyl carbonate (each isomer), dihexyl carbonate (each isomer), methyl heptyl carbonate (each isomer), ethyl heptyl carbonate (each isomer), propyl heptyl carbonate (each isomer), butyl heptyl carbonate (each isomer), pentyl heptyl carbonate (each isomer), hexyl heptyl carbonate (each isomer), diheptyl carbonate (each isomer), methyl octyl carbonate (each isomer), ethyl octyl carbonate (each isomer), propyl octyl carbonate (each isomer), butyl octyl carbonate (each isomer), pentyl octyl carbonate (each isomer), hexyl octyl carbonate (each isomer), heptyl octyl carbonate (each isomer), dioctyl carbonate (each isomer), methyl nonyl carbonate (each isomer), ethyl nonyl carbonate (each isomer), propyl nonyl carbonate (each isomer), butyl nonyl carbonate (each isomer), pentyl nonyl carbonate (each isomer), hexyl nonyl carbonate (each isomer), heptyl nonyl carbonate (each isomer), octyl nonyl carbonate (each isomer), dinonyl carbonate (each isomer), methyl decyl carbonate (each isomer), ethyl decyl carbonate (each isomer), propyl decyl carbonate (each isomer), butyl decyl carbonate (each isomer), pentyl decyl carbonate (each isomer), hexyl decyl carbonate (each isomer), heptyl decyl carbonate (each isomer), octyl decyl carbonate (each isomer), nonyl decyl carbonate (each isomer), didecyl carbonate (each isomer), methyl undecyl carbonate (each isomer), ethyl undecyl carbonate (each isomer), propyl undecyl carbonate (each isomer), butyl undecyl carbonate (each isomer), pentyl undecyl carbonate (each isomer), hexyl undecyl carbonate (each isomer), heptyl undecyl carbonate (each isomer), octyl undecyl carbonate (each isomer), nonyl undecyl carbonate (each isomer), decyl undecyl carbonate (each isomer), diundecyl carbonate (each isomer), methyl dodecyl carbonate (each isomer), ethyl dodecyl carbonate (each isomer), propyl dodecyl carbonate (each isomer), butyl dodecyl carbonate (each isomer), pentyl dodecyl carbonate (each isomer), hexyl dodecyl carbonate (each isomer), heptyl dodecyl carbonate (each isomer), octyl dodecyl carbonate (each isomer), nonyl dodecyl carbonate (each isomer), decyl dodecyl carbonate (each isomer), undecyl dodecyl carbonate (each isomer), didodecyl carbonate (each isomer), methyl phenyl carbonate, ethyl phenyl carbonate, propyl phenyl carbonate (each isomer), butyl phenyl carbonate (each isomer), pentyl phenyl carbonate (each isomer), hexyl phenyl carbonate (each isomer), diphenyl carbonate, methyl methylphenyl carbonate, ethyl methylphenyl carbonate, propyl methylphenyl carbonate (each isomer), butyl methylphenyl carbonate (each isomer), pentyl methylphenyl carbonate (each isomer), hexyl methylphenyl carbonate (each isomer), phenyl methylphenyl carbonate, dimethylphenyl carbonate, methyl ethylphenyl carbonate (each isomer), ethyl ethylphenyl carbonate (each isomer), propyl ethylphenyl carbonate (each isomer), butyl ethylphenyl carbonate (each isomer), pentyl ethylphenyl carbonate (each isomer), hexyl ethylphenyl carbonate (each isomer), phenyl ethylphenyl carbonate (each isomer), methylphenyl ethylphenyl carbonate (each isomer), and diethylphenyl carbonate.

### <Blocked Isocyanate Compound>

As illustrated in the reaction formula below, a blocked isocyanate compound is a compound having a blocked isocyanate group that can be dissociated by heating into a blocking agent and an isocyanate group.

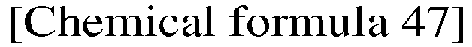

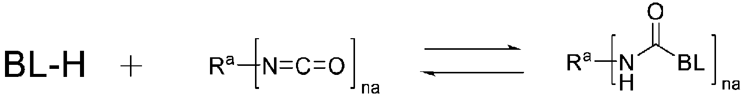

In the above reaction formula, BL-H is the blocking agent, and is an organic compound having an active hydrogen group. R^{a} is an na-valent organic group, and is the same as R³¹ described above.

The blocked isocyanate compound may be any compound in which one or more isocyanate groups of an isocyanate compound derived from the primary amine compound described above have been blocked with a blocking agent. Further, in those cases where the secondary amine compound (I) is used, the blocked isocyanate compound may have a structure in which a bond is formed between an isocyanate group and the secondary amine compound (1).

There are no particular limitations on the isocyanate compound prior to blocking, and examples thereof include pentamethylene diisocyanate, hexamethylene diisocyanate, isophorone diisocyanate, 4,4'-methylenebis(cyclohexane isocyanate), 4-isocyanatomethyl-1,8-octamethylene diisocyanate, diisocyanatotoluene, and 4,4'-diphenylmethane diisocyanate. Accordingly, examples of the preferred blocked isocyanate compound include compounds in which at least one isocyanate group in one of these compounds has been blocked with the blocking agent described above.

### <<Method for Producing Blocked Isocyanate Compound according to Second Embodiment>>

The method for producing a blocked isocyanate compound according to the present embodiment (hereinafter sometimes referred to as simply "the production method of the second embodiment") includes an addition-elimination reaction step of reacting a first blocked isocyanate compound and a second blocking agent by an addition-elimination reaction in the presence of at least one compound selected from the group consisting of the secondary amine compounds (I) and the tertiary amine compounds (II), thus obtaining a second blocked isocyanate compound.

The second blocking agent is a compound different from a first blocking agent which blocks isocyanate groups in the first blocked isocyanate compound.

The first blocked isocyanate compound and the second blocked isocyanate compound have structures different from each other.

The inventors of the present invention discovered that the problems of thermal denaturation and coloration could be resolved by conducting a heat treatment to react a first blocked isocyanate compound with a second blocking agent different from a first blocking agent that blocks isocyanate groups of the first blocked isocyanate compound in the presence of a specific amine compound having an aromatic group, thereby producing a second blocked isocyanate compound having a structure different from that of the first blocked isocyanate compound, and thus the inventors completed the present invention.

Accordingly, the production method of the second embodiment may also be described as a method of improving thermal denaturation and coloration during production of a blocked isocyanate compound, or a method of suppressing thermal denaturation and coloration during production of a blocked isocyanate compound.

Next, each of the steps of the production method of the second embodiment is described below in further detail.

### <Addition-Elimination Reaction Step>

As illustrated in the reaction formula below, an addition-elimination reaction is a reaction in which a blocking agent BLₐ-H and a blocking agent BL_{b}-H are interchanged by heat. In those cases where the blocked isocyanate compound on the left side of the following reaction formula is the first blocked isocyanate compound that functions as a raw material, the blocking agent BLₐ-H on the right side of the reaction formula corresponds to the first blocking agent that blocks the isocyanate groups of the first blocked isocyanate compound, the blocking agent BL_{b}-H on the left side of the reaction formula corresponds to the second blocking agent, and the blocked isocyanate compound on the right side of the reaction formula corresponds to the second blocked isocyanate compound. The second blocking agent is a compound different from the first blocking agent. Further, the first blocked isocyanate compound and the second blocked isocyanate compound have structures different from each other.

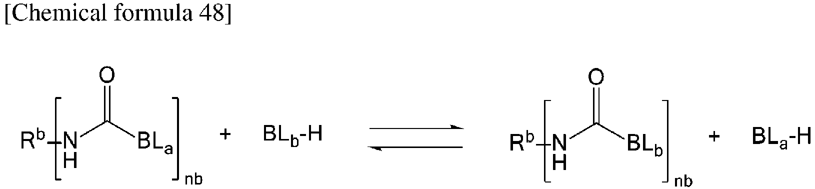

In the above reaction formula, BLₐ-H and BL_{b}-H are blocking agents having chemical structures different from each other, but are both organic compounds having an active hydrogen group. Rb is an nb-valent organic group.

In the addition-elimination reaction step, the amount present of the secondary amine compound (I) in the composition is preferably large from the viewpoint of reducing coloration of the second blocked isocyanate compound and the blocked isocyanate composition. Specifically, the amount present of the secondary amine compound (1), relative to the total mass of the first blocked isocyanate compound and the second blocking agent, preferably exceeds 0% by mass, is more preferably at least 1 ppm by mass, even more preferably at least 1% by mass, and particularly preferably 50% by mass or greater. On the other hand, when the amount present of the secondary amine compound (I) in the composition containing the blocked isocyanate compound is large, there is a case in which at least one denaturation among denaturation of the isocyanate compound and denaturation of the blocked isocyanate compound is accelerated due to the basicity of the secondary amine compound (I). Consequently, the amount present of the secondary amine compound (I), relative to the total mass of the primary amine compound and the second blocking agent, is preferably less than 1,000% by mass, more preferably less than 100% by mass, even more preferably not more than 90% by mass, still more preferably not more than 80% by mass, and most preferably 50% by mass or less. Furthermore, two or more different types of the secondary amine compound (I) may be combined and added to the composition containing the first blocked isocyanate compound and the second blocking agent. The thermal denaturation and coloration suppression effect of the secondary amine compound (I) varies depending on the chemical structure of the secondary amine compound (1), and the thermal denaturation and coloration suppression effect can be enhanced by including two or more types of the secondary amine compound (I).

In the addition-elimination reaction step, the amount present of the tertiary amine compound (II) in the composition is preferably large from the viewpoint of reducing coloration of the second blocked isocyanate compound and the blocked isocyanate composition. Specifically, the amount present of the tertiary amine compound (II), relative to the total mass of the reaction solution, preferably exceeds 0% by mass, is more preferably at least 1 ppm by mass, even more preferably at least 1% by mass, and particularly preferably 50% by mass or greater. On the other hand, when the amount present of the tertiary amine compound (II) in the composition containing the blocked isocyanate compound is large, there is a case in which at least one denaturation among denaturation of the isocyanate compound and denaturation of the blocked isocyanate compound is accelerated due to the basicity of the tertiary amine compound (II). Consequently, the amount present of the tertiary amine compound (II), relative to the total mass of the reaction solution, is preferably less than 1,000% by mass, more preferably less than 100% by mass, even more preferably not more than 90% by mass, still more preferably not more than 80% by mass, and most preferably 50% by mass or less. Furthermore, two or more types of the tertiary amine compound (II) may be combined and added to the composition containing the first blocked isocyanate compound and the second blocking agent. The thermal denaturation and coloration suppression effect of the tertiary amine compound (II) varies depending on the chemical structure of the tertiary amine compound, and the thermal denaturation and coloration suppression effect can be enhanced by including two or more types of the tertiary amine compound.

Either of the secondary amine compound (I) and the tertiary amine compound (11) may be added alone to the composition containing the first blocked isocyanate compound and the second blocking agent, or both of the secondary amine compound (I) and the tertiary amine compound (II) may be added thereto. Comparison of the secondary amine compound (I) and the tertiary amine compound (11) reveals that the secondary amine compound (I) has a greater effect in reducing coloration under high temperatures. On the other hand, the tertiary amine compound (II) exhibits less temperature dependency of the coloration reduction effect, thereby exhibiting a coloration reduction effect across a wider temperature range.

Further, because the secondary amine compound (1) has an active hydrogen group, a portion of the blocking agent in the second blocked isocyanate compound is substituted with the secondary amine compound (I). As a result, there is possibility that urea groups formed by reaction between the secondary amine compound (1) and an isocyanate group may be retained in the blocked isocyanate compound. Because this type of reaction does not occur with the tertiary amine compound (II), the tertiary amine compound (II) is more preferable.

Furthermore, if the secondary amine compound (I) and the tertiary amine compound (II) are included at the same time, the separation operation becomes more complex, when the secondary amine compound (1) and the tertiary amine compound (11) are recovered, for example However, the secondary amine compound (I) and the tertiary amine compound (II) exhibit different effects in terms of suppressing thermal denaturation and coloration, and further exhibit a synergistic effect in relation to the suppression of thermal denaturation and coloration. Consequently, it is preferable that the secondary amine compound (I) and the tertiary amine compound (II) be included simultaneously.

In the addition-elimination reaction step, there are no particular limitations on the total amount present of the secondary amine compound (1) and the tertiary amine compound (II), but the total amount present within the composition is preferably large from the viewpoint of reducing coloration of the second blocked isocyanate compound and the blocked isocyanate composition. Specifically, the total amount present of the secondary amine compound (I) and the tertiary amine compound (II), relative to the total mass of the first blocked isocyanate compound and the second blocking agent, is preferably greater than 0% by mass, more preferably at least 1 ppm by mass, even more preferably at least 1% by mass, and particularly preferably 50% by mass or more. On the other hand, when the amount of the secondary amine compound (I) within the composition containing the second blocked isocyanate compound is large, there is a case in which at least one denaturation among denaturation of the isocyanate compound and denaturation of the second blocked isocyanate compound is accelerated due to the basicity of the secondary amine compound (1) and the tertiary amine compound (II). Consequently, the total amount present of the secondary amine compound (1) and the tertiary amine compound (II), relative to the total mass of the first blocked isocyanate compound and the second blocking agent, is preferably less than 1,000% by mass, more preferably less than 100% by mass, even more preferably not more than 90% by mass, still more preferably not more than 80% by mass, and most preferably 50% by mass or less.

The thermal denaturation and coloration suppression effect can be further enhanced by combining at least one compound selected from the group consisting of the secondary amine compound (I) and the tertiary amine compound (11) with an antioxidant different from the secondary amine compound (I) and the tertiary amine compound (II). Further, the thermal denaturation and coloration suppression effect can be further enhanced by combining the secondary amine compound (1) and the tertiary amine compound (II) with an antioxidant.

There are no particular limitations on the antioxidant different from the secondary amine compound (I) and the tertiary amine compound (II), provided the antioxidant is capable of enhancing the thermal denaturation and coloration suppression effect, but a phenol-based antioxidant or a phosphorus-based antioxidant is preferred in terms of having a recognized coloration suppression effect. Among them, a phenol-based antioxidant is preferred, from the viewpoint of providing a particularly superior thermal denaturation and coloration suppression effect when combined with the secondary amine compound (I) and the tertiary amine compound (II).

In the addition-elimination reaction step, the ratio between the amounts added of the first blocked isocyanate compound and the second blocking agent may be selected as appropriate, but the ratio between the molar amount of carbonyl groups in the first blocked isocyanate compound and the molar amount of active hydrogen groups in the first blocking agent is typically either 1:1, or the ratio is adjusted so that the molar amount of active hydrogen groups in the second blocking agent is larger than the molar amount of carbonyl groups in the first blocked isocyanate compound.

Further, in the addition-elimination reaction step, the weight ratio between the amounts added of first blocked isocyanate compound and the second blocking agent may be selected as appropriate.

In the addition-elimination reaction step, the combination of the first blocked isocyanate compound, the second blocking agent, the secondary amine compound (1), and the tertiary amine compound (II) may be selected as appropriate, but the boiling point of the first blocking agent is preferably lower than the boiling point of the second blocking agent from the viewpoint that the progression of the reaction can be accelerated by actively removing the first blocking agent from the system when a distillation separation is conducted during the reaction step.

There are no particular limitations on the reaction temperature, and the reaction temperature may be selected appropriately in accordance with the reaction rates and the levels of thermal denaturation and coloration of the first blocked isocyanate compound and the second blocking agent. The reaction temperature is preferably not higher than 350°C, more preferably not higher than 300°C, and even more preferably 260°C or lower from the viewpoint of suppressing denaturation of the first blocked isocyanate compound and/or the second blocked isocyanate compound. On the other hand, when the reaction temperature is low, the temperature of the condenser must be set to a low temperature, which may sometimes require new equipment, and from that viewpoint, the reaction temperature is preferably at least 50°C, more preferably at least 80°C, and even more preferably 100°C or higher. Further, in the case of production of a blocked isocyanate compound in the presence of the secondary amine compound (I), the coloration reduction effect is exhibited more distinctly as the temperature is increased. From that viewpoint, the reaction temperature at which the blocked isocyanate is produced is preferably at least 120°C, more preferably at least 160°C, and even more preferably 200°C or higher.

Furthermore, the reaction pressure varies depending on the types of compounds used and the reaction temperature, but may be any of a reduced pressure, normal pressure, or a pressurized state, and the reaction is typically conducted at an absolute pressure within a range from at least 20 Pa to not more than 2×10⁷ Pa.

The addition-elimination reaction step may be conducted in the presence of oxygen. Carrying out the reaction step in the presence of oxygen tends to cause thermal denaturation and coloration of the blocked isocyanate compound, and therefore it is preferable that the amount present of oxygen inside the blocked isocyanate production apparatus be reduced. On the other hand, in the case of a large-scale production facility, reducing the amount present of oxygen inside the production apparatus of the blocked isocyanate compound requires that air leakage into the production apparatus is reduced, and therefore the design standards for the facility become more stringent, thereby causing an increase in equipment costs. From this viewpoint, the oxygen concentration in the gas supplied during the reaction step is preferably controlled under stringent conditions, and is preferably controlled to a level exceeding 0% by volume, more preferably controlled to a level exceeding 0.0001% by volume, and even more preferably controlled to a level exceeding 0.001% by volume.

In the addition-elimination reaction step, because at least one compound selected from the group consisting of the secondary amine compounds (1) and the tertiary amine compounds (II) is present, thermal denaturation and coloration of the blocked isocyanate compounds can be reduced even in the presence of oxygen. However, an increase in the amount present of oxygen promotes thermal denaturation and coloration, as mentioned above, and therefore the amounts used of the secondary amine compound (I) and the tertiary amine compound (II) are required to be increased to suppress thermal denaturation and coloration, which is undesirable. Accordingly, from this viewpoint, the reaction step is preferably carried out under conditions in which the oxygen concentration in the gas supplied during the reaction step is low, and relative to the total volume of gas, the oxygen concentration is preferably controlled to a level of not more than 21% by volume, more preferably controlled to a level of not more than 10% by volume, even more preferably controlled to a level of not more than 1 % by volume, still more preferably controlled to a level of not more than 0.1% by volume, particularly preferably controlled to a level of not more than 0.01% by volume, and most preferably controlled to a level of 0.001 % by volume or lower. Measurement of the oxygen concentration may be conducted using a conventional technique such as gas chromatography or trace oxygen analysis by electrochemical methods.

In the addition-elimination reaction step, an optional solvent may be used in any desired proportion. The solvent is preferably an inert solvent that exhibits no reactivity with the first blocked isocyanate compound, the first blocking agent, the second blocking agent, or the second blocked isocyanate compound. Preferred examples of such a solventdeterio include ester-based solvents, ether-based solvents, phosphate ester-based solvents, hydrocarbon-based solvents, aromatic hydrocarbon-based solvents, and carbonic acid derivative-based solvents.

In the addition-elimination reaction step, the reaction mixture containing the first blocked isocyanate compound and the second blocking agent may also contain an optional metal in any desired proportion. The form of the metal may be a complex or a solid. Metals accelerate the addition-elimination reaction between the first blocked isocyanate compound and the second blocking agent, but on the other hand, may sometimes cause thermal denaturation, deterioration and coloration, and therefore the amount of the metal relative to the mass of the first blocked isocyanate compound is preferably less than 10% by mass, more preferably less than 1% by mass, and even more preferably less than 0.1 ppm by mass.

In the addition-elimination reaction step, the reaction mixture containing the first blocked isocyanate compound and the second blocking agent may also contain an organic acid, inorganic acid, organic base or inorganic base. These acids and bases act as catalysts in the addition-elimination reaction between the first blocked isocyanate compound and the second blocking agent, and make it possible to lower the temperature required for the reaction. On the other hand, these acids and bases also act as catalysts in denaturation reactions of the blocked isocyanates, and from that viewpoint, the amount of these organic acids, inorganic acids, organic bases or inorganic bases, relative to the mass of the first blocked isocyanate compound, is preferably less than 10% by mass, more preferably less than 1% by mass, even more preferably less than 0.1% by mass, and particularly preferably less than 1 ppb by mass.

In the addition-elimination reaction step, the second blocked isocyanate compound is obtained as a composition containing the second blocked isocyanate compound (hereinafter sometimes referred to as the "blocked isocyanate composition").

The term "blocked isocyanate composition" means a composition containing a blocked isocyanate compound in an amount exceeding 0 wt% but not more than 100 wt% of the composition. There are no particular limitations on the blocked isocyanate composition, except that a blocked isocyanate compound is included therein, and the composition may also contain a solvent, blocking agent, isocyanate, catalyst, primary amine compound or the like in any desired proportion.

Thus, the blocked isocyanate composition may be used directly as the blocked isocyanate compound, or the blocked isocyanate compound may be purified from the blocked isocyanate composition prior to use.

### (Reaction Apparatus)

There are no particular limitations on the reaction apparatus, and conventional reaction apparatus for addition-elimination reactions may be used. Examples of methods that may be used include a method in which the second blocked isocyanate compound is synthesized in a batch manner by adding a mixture containing the first blocked isocyanate compound and the second blocking agent to a vessel to which at least one device selected from the group consisting of a condenser device and a treatment device has been connected, heating the vessel to produce the second blocked isocyanate compound, and then or simultaneously guiding the vapor containing the first blocking agent into the condenser device or treatment device, and a method in which the second blocked isocyanate compound is obtained in a continuous manner by continuously introducing a mixture containing the first blocked isocyanate compound and the second blocking agent into a distillation column that has been heated to a prescribed reaction temperature, thereby producing the second blocked isocyanate compound and simultaneously separating the produced vapor containing the first blocking agent.

In the reaction apparatus, the material of regions that make contact with the mixture containing the first blocked isocyanate compound, the first blocking agent, the second blocking agent and the second blocked isocyanate compound, and also the composition containing the second blocked isocyanate compound produced by the reaction, may be a conventional material, provided the material exhibits no adverse effects on the first blocked isocyanate compound, the first blocking agent, the second blocking agent, and the second blocked isocyanate compound. Specific examples of such a material include steel, stainless steel, ceramics, carbon, and lined versions of these materials.

In those cases where a distillation device is used, there are no particular limitations on the form of the distillation device, and various conventional distillation devices such as distillation devices containing any of a batch distillation device, simple distillation device, multistage distillation device, continuous multistage distillation device, packed column, and a combination thereof may be used,.

As the distillation column, any distillation column in which the theoretical number of distillation stages is two or greater may be employed. Further, in the case where the theoretical number of stages is large, a multistage distillation column becomes extremely large, which sometimes makes industrial implementation difficult, and therefore the theoretical number of stages is typically 500 or less.

As the distillation column, any column typically usable as a multistage distillation column, such as plate columns using trays such as a bubble cap tray, porous plate tray, valve tray or cross-flow tray, or packed columns that have been packed with any of various fillers such as Raschig rings, Lessing rings, Pall rings, Berl saddles, interlock saddles, Dickson packing, McMahon packing, Helipack, Sulzer packing or Mellapak, may be used. Moreover, plate-packed mixed columns in which a plate portion and a filler-packed portion are combined may also be used favorably.

Next, the raw materials used and the product obtained in the production method of the second embodiment are described below in further detail.

### <First Blocked Isocyanate Compound and First Blocking Agent>

As illustrated in the reaction formula below, the first blocked isocyanate compound is a compound having a blocked isocyanate group that can be dissociated by heating into a first blocking agent and an isocyanate group. When the first blocked isocyanate compound is described on the right side of the following reaction formula, the first blocking agent that blocks an isocyanate group of the first blocked isocyanate compound is a blocking agent described as BL-H on the left side of the reaction formula.

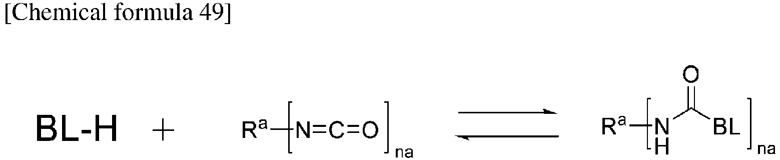

In the above reaction formula, BL-H is the blocking agent, and is an organic compound having an active hydrogen group. R^{a} is an na-valent organic group, and is the same as R³¹ described above.

### [First Blocked Isocyanate Compound]

Examples of the first blocked isocyanate compound include blocked isocyanate compounds obtained by reacting the isocyanate compound that is a raw material of the second blocked isocyanate compound with an aforementioned blocking agent (such as an alcohol-based, phenol-based, thiol-based, amine- and ammonia-based, oxime-based, hydroxylamine-based or active methylene-based blocking agent). Additional examples thereof include blocked isocyanate compounds produced by a method of producing a blocked isocyanate compound, the method including a reaction step of obtaining a blocked isocyanate compound by reacting a primary amine compound, a carbonic acid derivative and a blocking agent by a heat treatment.

### [First Blocking Agent]

The first blocking agent may be any compound that has an active hydrogen group, and is capable of reacting with an isocyanate group to form a bond, and then being decomposed into an isocyanate compound and the blocking agent by thermal decomposition. Examples of such a blocking agent include alcohol-based, phenol-based (hereinafter alcohol-based and phenol-based blocking agents are sometimes referred to jointly as "hydroxy compounds"), thiol-based, amine- and ammonia-based, oxime-based, hydroxylamine-based, and active methylene-based blocking agents.

Among them, blocking agents formed from hydroxy compounds, and amine- and ammonia-based, or hydroxylamine-based blocking agents are preferred from the viewpoint of the stability of the blocking agent during thermal decomposition. Further, phenol-based, amine-based or hydroxylamine-based blocking agents or ammonia are more preferred in terms of limiting denaturation of the isocyanate compound during thermal decomposition. Furthermore, a phenol-based blocking agent is even more preferred in terms of achieving particularly superior stability of the blocking agent during thermal decomposition and limiting denaturation of the isocyanate compound during thermal decomposition.

When obtaining the second blocked isocyanate compound by addition-elimination reaction, it is particularly preferable to use a blocking agent having a lower nucleophilicity than the second blocking agent as the first blocking agent in order to improve the reactivity between the first blocked isocyanate compound and the second blocking agent and improve the yield of the second blocked isocyanate compound.

In the case where phenol-based blocking agents, alcohol-based blocking agents, amine- and ammonia-based blocking agents, and hydroxylamine-based blocking agents are compared from the viewpoint of the nucleophilicity of the blocking agent, the nucleophilicity is typically increased in the order from hydroxylamine-based blocking agents < phenol-based blocking agents < alcohol-based blocking agents < amine- and ammonia-based blocking agents.

### [Phenol-Based Blocking Agent]

There are no particular limitations on the phenol-based blocking agent, provided it is a compound having a hydroxyl group bonded to an aromatic ring, but a blocking agent having a phenol group or naphthol group is preferred from the viewpoint of ease of availability. The blocking agent may be a compound having one hydroxyl group bonded to an aromatic ring within the molecule, or may be a compound having two or more hydroxyl groups bonded to one or more aromatic rings within the molecule. In those cases where a compound having two or more hydroxyl groups bonded to one or more aromatic rings within the molecule is used as the blocking agent, separation of the isocyanate compound and the compound having the hydroxyl groups bonded to the aromatic ring becomes more difficult. Accordingly, a compound having one hydroxyl group bonded to an aromatic ring within the molecule is preferably used as the phenol-based blocking agent.

Examples of the preferred phenol-based blocking agent include the same compounds as those exemplified in the above section entitled "Method for Producing Blocked Isocyanate Compound according to First Embodiment".

### [Alcohol-Based Blocking Agent]

There are no particular limitations on the alcohol-based blocking agent, provided it is an aliphatic compound having a hydroxyl group, and either an aliphatic compound having one hydroxyl group within the molecule, or an aliphatic compound having two or more hydroxyl groups within the molecule may be used. In those cases where an aliphatic compound having two or more hydroxyl groups within the molecule is used as the blocking agent, separation of the isocyanate compound and the aliphatic compound having the hydroxyl groups becomes more difficult. Accordingly, an aliphatic compound having one hydroxyl group within the molecule is preferably used.

Examples of the preferred alcohol-based blocking agent include the same compounds as those exemplified in the above section entitled "Method for Producing Blocked Isocyanate Compound according to First Embodiment".

### [Amine- and Ammonia-Based Blocking Agents]

Examples of compounds that may be used as the amine- and ammonia-based blocking agents include amine compounds having a primary or secondary amino group as an active hydrogen group, and ammonia. In those cases where a primary amine compound or ammonia is used as the blocking agent, the urea linkage formed by the reaction of the primary amine compound or ammonia with the isocyanate compound has two possible cleavage pathways during thermal decomposition, and therefore there is a case in which an isocyanate compound is formed on the primary amine compound used as the blocking agent, thereby making it more difficult to obtain the target isocyanate compound. Accordingly, it is preferable that an amine compound having a secondary amino group be used.

The amine- and ammonia-based blocking agent may be an amine compound having one amino group within the molecule, or an amine compound having two or more amino groups within the molecule. In those cases where an amine compound having two or more amino groups within the molecule is used as the blocking agent, separation of the isocyanate compound and the amine compound becomes more difficult. Accordingly, an amine compound having one amino group within the molecule is preferably used as the amine- and ammonia-based blocking agent.

Examples of the preferred amine- and ammonia-based blocking agent include the same compounds as those exemplified in the above section entitled "Method for Producing Blocked Isocyanate Compound according to First Embodiment".

### [Hydroxylamine-Based Blocking Agent]

There are no particular limitations on the hydroxylamine-based blocking agent, and the blocking agent may be a hydroxylamine compound having one hydroxylamine structure within the molecule, or a hydroxylamine compound having two or more hydroxylamine structures within the molecule. In those cases where a hydroxylamine compound having two or more hydroxylamine structures within the molecule is used as the blocking agent, separation of the isocyanate compound and the hydroxylamine compound becomes more difficult. Accordingly, a compound having one hydroxylamine structure within the molecule is preferably used as the hydroxylamine-based blocking agent.

Examples of the hydroxylamine compound having one hydroxylamine structure within the molecule include the same compounds as those exemplified in the above section entitled "Method for Producing Blocked Isocyanate Compound according to First Embodiment".

### <Second Blocking Agent>

The second blocking agent may be any compound that has an active hydrogen group, and is capable of reacting with an isocyanate group to form a bond, and then being decomposed into an isocyanate compound and the blocking agent by thermal decomposition.

Examples of such a blocking agent include alcohol-based, phenol-based (hereinafter alcohol-based and phenol-based blocking agents are sometimes referred to jointly as "hydroxy compounds"), thiol-based, amine- and ammonia-based, oxime-based, hydroxylamine-based, and active methylene-based blocking agents.

Among them, blocking agents formed from hydroxy compounds, and amine- and ammonia-based, or hydroxylamine-based blocking agents are preferred from the viewpoint of the stability of the blocking agent during thermal decomposition. Further, phenol-based, amine-based or hydroxylamine-based blocking agents or ammonia are more preferred in terms of limiting denaturation of the isocyanate compound during thermal decomposition. Furthermore, a phenol-based blocking agent is even more preferred in terms of achieving particularly superior stability of the blocking agent during thermal decomposition and limiting denaturation of the isocyanate compound during thermal decomposition.

On the other hand, when obtaining the second blocked isocyanate compound by addition-elimination reaction, in order to improve the reactivity between the first blocked isocyanate compound and the second blocking agent and improve the yield of the second blocked isocyanate compound, it is particularly preferable to use a blocking agent having a higher nucleophilicity than the first blocking agent as the second blocking agent.

In the case where phenol-based blocking agents, alcohol-based blocking agents, amine- and ammonia-based blocking agents, and hydroxylamine-based blocking agents are compared from the viewpoint of the nucleophilicity of the blocking agent, the nucleophilicity is typically increased in the order from hydroxylamine-based blocking agents < phenol-based blocking agents < alcohol-based blocking agents < amine- and ammonia-based blocking agents.

In those cases where either a method in which the second blocked isocyanate compound is synthesized in a batch manner by conducting production of the second blocked isocyanate compound by an addition-elimination reaction, and then or simultaneously guiding the vapor containing the first blocking agent into a condenser device or treatment device, or a method in which the second blocked isocyanate compound is obtained in a continuous manner by continuously introducing a mixture containing the first blocked isocyanate compound and the second blocking agent into a distillation column that has been heated to a prescribed reaction temperature, thereby producing the second blocked isocyanate compound and simultaneously separating the vapor containing the first blocking agent is used, the boiling point of the second blocking agent is preferably equal to or higher than the boiling point of the first blocking agent.

### [Phenol-Based Blocking Agent]

There are no particular limitations on the phenol-based blocking agent, provided it is a compound having a hydroxyl group bonded to an aromatic ring, but a blocking agent having a phenol group or naphthol group is preferred from the viewpoint of ease of availability. The blocking agent may be a compound having one hydroxyl group bonded to an aromatic ring within the molecule, or may be a compound having two or more hydroxyl groups bonded to one or more aromatic rings within the molecule. In those cases where a compound having two or more hydroxyl groups bonded to one or more aromatic rings within the molecule is used as the blocking agent, separation of the isocyanate compound and the compound having the hydroxyl groups bonded to the aromatic ring becomes more difficult. Accordingly, a compound having one hydroxyl group bonded to an aromatic ring within the molecule is preferably used as the phenol-based blocking agent.

Examples of the preferred phenol-based blocking agent include the same compounds as those exemplified in the above section entitled "Method for Producing Blocked Isocyanate Compound according to First Embodiment".

### [Alcohol-Based Blocking Agent]

There are no particular limitations on the alcohol-based blocking agent, provided it is an aliphatic compound having a hydroxyl group, and either an aliphatic compound having one hydroxyl group within the molecule, or an aliphatic compound having two or more hydroxyl groups within the molecule may be used. In those cases where an aliphatic compound having two or more hydroxyl groups within the molecule is used as the blocking agent, separation of the isocyanate compound and the aliphatic compound having the hydroxyl groups becomes more difficult. Accordingly, an aliphatic compound having one hydroxyl group within the molecule is preferably used.

Examples of the preferred alcohol-based blocking agent include the same compounds as those exemplified in the above section entitled "Method for Producing Blocked Isocyanate Compound according to First Embodiment".

### [Amine- and Ammonia-Based Blocking Agents]

### [Amine- and Ammonia-Based Blocking Agents]

Examples of compounds that may be used as the amine- and ammonia-based blocking agents include amine compounds having a primary or secondary amino group as an active hydrogen group, and ammonia. In those cases where a primary amine compound or ammonia is used as the blocking agent, the urea linkage formed by the reaction of the primary amine compound or ammonia with the isocyanate compound has two possible cleavage pathways during thermal decomposition, and therefore there is a case in which an isocyanate compound is formed on the primary amine compound used as the blocking agent, thereby making it more difficult to obtain the target isocyanate compound. Accordingly, it is preferable that an amine compound having a secondary amino group be used.

The amine- and ammonia-based blocking agent may be an amine compound having one amino group within the molecule, or an amine compound having two or more amino groups within the molecule. In those cases where an amine compound having two or more amino groups within the molecule is used as the blocking agent, separation of the isocyanate compound and the amine compound becomes more difficult. Accordingly, an amine compound having one amino group within the molecule is preferably used as the amine- and ammonia-based blocking agent.

Examples of the preferred amine- and ammonia-based blocking agent include the same compounds as those exemplified in the above section entitled "Method for Producing Blocked Isocyanate Compound according to First Embodiment".

### [Hydroxylamine-Based Blocking Agent]

There are no particular limitations on the hydroxylamine-based blocking agent, and the blocking agent may be a hydroxylamine compound having one hydroxylamine structure within the molecule, or a hydroxylamine compound having two or more hydroxylamine structures within the molecule. In those cases where a hydroxylamine compound having two or more hydroxylamine structures within the molecule is used as the blocking agent, separation of the isocyanate compound and the hydroxylamine compound becomes more difficult. Accordingly, a compound having one hydroxylamine structure within the molecule is preferably used as the hydroxylamine-based blocking agent.

Examples of the hydroxylamine compound having one hydroxylamine structure within the molecule include the same compounds as those exemplified in the above section entitled "Method for Producing Blocked Isocyanate Compound according to First Embodiment".

Although any of the blocking agents described above may be used alone, using a combination of two or more blocking agents can also be effective. For example, when a combination of a blocking agent with low nucleophilicity and a blocking agent with high nucleophilicity is used, the blocking agent with high nucleophilicity reacts with blocked isocyanate groups formed by the first blocking agent blocking the isocyanate groups (hereinafter, sometimes referred to as "blocked isocyanate groups derived from the first blocked isocyanate"), thereby contributing to a reduction in blocked isocyanate groups derived from the first blocked isocyanate. Accordingly, it is preferable that at least one, and preferably all of the blocking agents have a higher nucleophilicity than the first blocking agent. Examples of blocking agents having high nucleophilicity are as described above.

### <<Method for Producing Isocyanate Compound according to Third Embodiment>>

The method for producing an isocyanate compound according to the present embodiment (hereinafter sometimes referred to as simply "the production method of the third embodiment") includes a reaction step (hereinafter sometimes referred to as the "thermal decomposition step" or the "second reaction step") of decomposing a blocked isocyanate compound into a blocking agent and an isocyanate compound by subjecting the blocked isocyanate compound to a heat treatment in the presence of at least one compound selected from the group consisting of secondary amine compounds of general formula (I) shown below (hereinafter sometimes referred to as the "secondary amine compounds (I)") and tertiary amine compounds of general formula (II) shown below (hereinafter sometimes referred to as the "tertiary amine compounds (11)"), thereby obtaining the isocyanate compound.

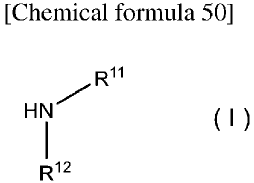

In general formula (I), R¹¹ and R¹² are each independently a monovalent organic group. R¹¹ and R¹² may be bonded together to form a cyclic structure via a carbon-carbon bond, a carbon-oxygen-carbon bond, or a carbon-nitrogen-carbon bond. At least one of R¹¹ and R¹² has an aromatic group.

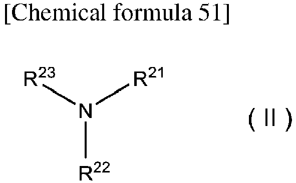

In general formula (II), R²¹, R²² and R²³ are each independently a monovalent organic group. Each combination of R²¹ and R²², R²² and R²³, and R²³ and R²¹ may be independently bonded together to form a cyclic structure via a carbon-carbon bond, a carbon-oxygen-carbon bond, or a carbon-nitrogen-carbon bond. At least one of R²¹, R²² and R²³ has an aromatic group.

The inventors of the present invention discovered that the problems of thermal denaturation and coloration could be resolved by thermally decomposing a blocked isocyanate compound in the presence of a specific amine compound having an aromatic group, thereby producing a free blocking agent and isocyanate compound, and thus the present invention has been completed.

As described above, conventionally, compounds having an amino group bonded to a carbon atom having aromaticity are known to oxidize readily and cause coloration. Accordingly, even though compounds having an amino group bonded to a carbon atom having aromaticity have been used in products such as rubber degradation inhibitors, the coloration of which is not a problem, these compounds have not been used in products, the coloration of whih would be problematic. However, surprisingly, by employing the production method of the third embodiment, and using a specific amine compound having an aromatic group, it has become evident for the first time that not only do coloration problems not occur, but a particularly remarkable effect is achieved wherein the levels of thermal denaturation and coloration are actually improved (suppressed).

Accordingly, the production method of the third embodiment may also be described as a method for improving thermal denaturation and coloration during production of an isocyanate compound from a blocked isocyanate compound, or a method for suppressing thermal denaturation and coloration during production of an isocyanate compound from a blocked isocyanate compound.

Next, each of the steps of the production method of the third embodiment is described below in further detail.

### <Thermal Decomposition Step>

In the thermal decomposition step, a blocked isocyanate compound is subjected to a heat treatment in the presence of at least one compound selected from the group consisting of the secondary amine compounds (I) and the tertiary amine compounds (II), thereby decomposing the blocked isocyanate compound into a blocking agent and an isocyanate compound, and obtaining the isocyanate compound.

In the thermal decomposition step, the amount present of the secondary amine compound (I) within the composition is preferably large from the viewpoint of reducing coloration of the isocyanate compound and the isocyanate composition. Specifically, the amount present of the secondary amine compound (I), relative to the total mass of the reaction solution, is preferably greater than 0% by mass, more preferably at least 1 ppm by mass, even more preferably at least 1% by mass, and particularly preferably 50% by mass or more. On the other hand, when the amount present of the secondary amine compound (I) within the composition containing the isocyanate compound is large, there is a case in which denaturation of the isocyanate compound is accelerated due to the basicity of the secondary amine compound (I). Consequently, the amount present of the secondary amine compound (I), relative to the total mass of the reaction solution, is preferably less than 100% by mass, more preferably not more than 90% by mass, even more preferably not more than 80% by mass, and most preferably 50% by mass or less. Further, a combination of two or more types of the secondary amine compound (I) may be added to the reaction solution. The thermal denaturation and coloration suppression effect of the secondary amine compound (I) varies depending on the chemical structure of the secondary amine compound, and the denaturation and coloration suppression effect can be enhanced by including two or more types of the secondary amine compound.

In the thermal decomposition step, the amount present of the tertiary amine compound (II) within the composition is preferably large from the viewpoint of reducing coloration of the isocyanate compound and the isocyanate composition. Specifically, the amount present of the tertiary amine compound (II), relative to the total mass of the reaction solution, is preferably greater than 0% by mass, more preferably at least 1 ppm by mass, even more preferably at least 1% by mass, and particularly preferably 50% by mass or more. On the other hand, when the amount present of the tertiary amine compound (II) within the composition containing the blocked isocyanate compound is large, there is a case in which denaturation of the isocyanate compound is accelerated due to the basicity of the tertiary amine compound (II). Consequently, the amount present of the tertiary amine compound (II), relative to the total mass of the reaction solution, is preferably less than 100% by mass, more preferably not more than 90% by mass, even more preferably not more than 80% by mass, and particularly preferably 50% by mass or less. Further, a combination of two or more types of the tertiary amine compound (II) may be added to the reaction solution. The thermal denaturation and coloration suppression effect of the tertiary amine compound (II) varies depending on the chemical structure of the tertiary amine compound, and the thermal denaturation and coloration suppression effect can be enhanced by including two or more types of the tertiary amine compound.

Either of the secondary amine compound (I) and the tertiary amine compound (II) may be added alone to the reaction solution, or both the secondary amine compound (I) and the tertiary amine compound (II) may be added. Comparison of the secondary amine compound (1) and the tertiary amine compound (II) reveals that the secondary amine compound (I) exhibits a greater effect in reducing coloration under high temperatures. On the other hand, the tertiary amine compound (II) exhibits less temperature dependency of the coloration reduction effect, and therefore exhibits a coloration reduction effect across a wider temperature range.

Further, because the secondary amine compound (I) has an active hydrogen group, a portion of the blocking agent in the blocked isocyanate compound is substituted with the secondary amine compound (I). As a result, there is possibility that urea groups formed by reaction between the secondary amine compound (I) and an isocyanate group may be retained in the blocked isocyanate compound. Because such as reaction does not occur with the tertiary amine compound (II), the tertiary amine compound (II) is more preferred.

Furthermore, if the secondary amine compound (I) and the tertiary amine compound (II) are included at the same time, the separation operation becomes more complex, when the secondary amine compound (I) and the tertiary amine compound (II) are recovered, for example However, the secondary amine compound (I) and the tertiary amine compound (II) exhibit different effects in terms of suppressing thermal denaturation and coloration, and further exhibit a synergistic effect in relation to the suppression of thermal denaturation and coloration. Consequently, it is preferable that the secondary amine compound (I) and the tertiary amine compound (II) be included simultaneously.

In the thermal decomposition step, although there are no particular limitations on the total amount present of the secondary amine compound (I) and the tertiary amine compound (II), the total amount present within the composition is preferably large from the viewpoint of reducing coloration of the isocyanate compound and the isocyanate composition. Specifically, the total amount present of the secondary amine compound (I) and the tertiary amine compound (II), relative to the total mass of the reaction solution, is preferably greater than 0% by mass, more preferably at least 1 ppm by mass, even more preferably at least 1% by mass, and particularly preferably 50% by mass or more. On the other hand, when the amount present of the secondary amine compound (I) within the composition containing the blocked isocyanate compound is large, there is a case in which at least one denaturation among denaturation of the isocyanate compound and denaturation of the blocked isocyanate compound is accelerated due to the basicity of the secondary amine compound (I) and the tertiary amine compound (II). Consequently, the total amount present of the secondary amine compound (I) and the tertiary amine compound (II), relative to the total mass of the reaction solution, is preferably less than 100% by mass, more preferably not more than 90% by mass, even more preferably not more than 80% by mass, and particularly preferably 50% by mass or less.

Furthermore, in those cases where the secondary amine compound (I) and the tertiary amine compound (II) are added simultaneously to the reaction solution, the mass ratio between the secondary amine compound (I) and the tertiary amine compound (II) may be adjusted as appropriate in accordance with the intended purpose. When suppression of coloration under high temperature conditions is being targeted, the amount of the secondary amine compound (I) is preferably larger than that of the tertiary amine compound (II). On the other hand, when there is a concern that urea groups formed by reaction between the active hydrogen group of the secondary amine compound (I) and an isocyanate group may be retained in the isocyanate compound obtained by replacing a portion of the blocking agent in the blocked isocyanate compound with the secondary amine compound (I), the amount of the tertiary amine compound (II) is preferably larger than that of the secondary amine compound (I).

Further, the thermal denaturation and coloration suppression effect can be further enhanced by combining one or more compounds selected from the group consisting of the secondary amine compounds (I) and the tertiary amine compounds (II) with an antioxidant different from the secondary amine compound (I) and the tertiary amine compound (II). Further, the thermal denaturation and coloration suppression effect can be further enhanced by combining the secondary amine compound (I) and the tertiary amine compound (II) with an antioxidant.

There are no particular limitations on the antioxidant different from the secondary amine compound (I) and the tertiary amine compound (II), provided the antioxidant is capable of enhancing the thermal denaturation and coloration suppression effect, but in terms of having a recognized coloration suppression effect, a phenol-based antioxidant or a phosphorus-based antioxidant is preferred. Among these, from the viewpoint of providing a particularly superior thermal denaturation and coloration suppression effect when combined with the secondary amine compound (I) and the tertiary amine compound (II), a phenol-based antioxidant is preferred.

In the thermal decomposition step, the combination of the blocked isocyanate compound and one or more compounds selected from the group consisting of the secondary amine compounds (I) and the tertiary amine compounds (II) may be selected as appropriate, and the combination of the blocking agent used in the blocked isocyanate compound, the blocked isocyanate compound, and one or more compounds selected from the group consisting of the secondary amine compounds (I) and the tertiary amine compounds (II) may also be selected as appropriate. For example, in those cases where distillation and separation of the isocyanate compound and the blocking agent are conducted simultaneously with the thermal decomposition of the blocked isocyanate compound, ensuring that one or more compounds selected from the group consisting of the secondary amine compounds (I) and the tertiary amine compounds (II) are present within the composition containing the obtained isocyanate compound can reduce coloration, and is consequently desirable. Further, from that viewpoint, one or more compounds selected from the group consisting of the secondary amine compounds (I) and the tertiary amine compounds (II) are preferably compounds having a different boiling point from that of the blocking agent. Furthermore, among them, in those cases where the blocking agent has a lower boiling point that the isocyanate compound obtained by thermal decomposition, one or more compounds selected from the group consisting of the secondary amine compounds (1) and the tertiary amine compounds (II) preferably have a higher boiling point than that of the blocking agent. On the other hand, in those cases where the blocking agent has a higher boiling point that the isocyanate compound obtained by thermal decomposition, one or more compounds selected from the group consisting of the secondary amine compounds (I) and the tertiary amine compounds (II) preferably have a lower boiling point than that of the blocking agent. In the case where the boiling point relationship between one or more compounds selected from the group consisting of the secondary amine compounds (I) and the tertiary amine compounds (II) and the blocking agent satisfies the above-mentioned relationship, the blocking agent can be separated while retaining the presence of one or more compounds selected from the group consisting of the secondary amine compounds (I) and the tertiary amine compounds (II) in the composition containing the isocyanate compound, when the isocyanate compound and the blocking agent undergo distillation separation simultaneously with the thermal decomposition.

There are no particular limitations on the reaction temperature (the thermal decomposition temperature of the blocked isocyanate compound), and the reaction temperature may be selected appropriately in accordance with the rate at which the blocked isocyanate compound is decomposed into the blocking agent and the isocyanate compound, and the levels of thermal denaturation and coloration. The reaction temperature is preferably not higher than 350°C, more preferably not higher than 300°C, and more preferably 260°C or lower from the viewpoint of suppressing denaturation of the isocyanate compound. On the other hand, when the reaction temperature is low, the temperature of the condenser must be set to a low temperature, which may sometimes require new equipment, and from that viewpoint, the reaction temperature is preferably at least 50°C, more preferably at least 80°C, and even more preferably 100°C or higher. Further, when the blocked isocyanate compound is subjected to thermal decomposition in the presence of the secondary amine compound (I) to obtain the isocyanate compound and the blocking agent, the coloration reduction effect is exhibited more distinctly as the temperature is increased. From that viewpoint, the reaction temperature is preferably at least 120°C, more preferably at least 160°C, and even more preferably 200°C or higher.

Furthermore, the reaction pressure varies depending on the types of compounds used and the reaction temperature, but may be any of a reduced pressure, normal pressure, or a pressurized state, and the reaction is typically conducted at an absolute pressure within a range from at least 20 Pa to not more than 2×10⁷ Pa.

The thermal decomposition step may be conducted in the presence of oxygen. Carrying out the thermal decomposition step in the presence of oxygen tends to cause thermal denaturation and coloration of the isocyanate compound, and therefore it is preferable that the amount of oxygen inside the blocked isocyanate compound thermal decomposition apparatus be reduced. On the other hand, in the case of a large-scale production facility, reducing the amount of oxygen inside the blocked isocyanate compound thermal decomposition apparatus requires that air leakage into the production equipment is reduced, and therefore the design standards for the facility become more stringent, thereby causing an increase in equipment costs. From this viewpoint, the oxygen concentration within the gas supplied during the thermal decomposition step is preferably controlled under stringent conditions, and is preferably controlled to a level exceeding 0% by volume, more preferably controlled to a level exceeding 0.0001% by volume, and even more preferably controlled to a level exceeding 0.001% by volume.

In the thermal decomposition step, because at least one compound selected from the group consisting of the secondary amine compounds (I) and the tertiary amine compounds (II) is present, thermal denaturation and coloration of the isocyanate compounds can be reduced even in the presence of oxygen. However, an increase in the amount present of oxygen promotes thermal denaturation and coloration, as mentioned above, and therefore the amounts used of the secondary amine compound (I) and the tertiary amine compound (II) are required to be increased to suppress thermal denaturation and coloration, which is undesirable. Accordingly, from this viewpoint, the thermal decomposition step is preferably carried out under conditions in which the oxygen concentration in the gas supplied during the thermal decomposition step is low, and relative to the total volume of gas, the oxygen concentration is preferably controlled to a level of not more than 21% by volume, more preferably controlled to a level of not more than 10% by volume, even more preferably controlled to a level of not more than 1% by volume, still more preferably controlled to a level of not more than 0.1% by volume, particularly preferably controlled to a level of not more than 0.01% by volume, and most preferably controlled to a level of 0.001% by volume or lower. Measurement of the oxygen concentration may be conducted using a conventional technique such as gas chromatography or trace oxygen analysis by electrochemical methods.

In the thermal decomposition step, an optional solvent may be used in any desired proportion. The solvent is preferably an inert solvent that exhibits no reactivity with the blocked isocyanate compound. Preferred examples of such a solventdeterio include ester-based solvents, ether-based solvents, phosphate ester-based solvents, hydrocarbon-based solvents, aromatic hydrocarbon-based solvents, and carbonic acid derivative-based solvents. Although there are no particular limitations on the amount used of the solvent, the amount of the solvent relative to the total mass of the reaction solution may be greater than 0% by mass but less than 100% by mass, may be at least 10% by mass but not more than 90% by mass, may be at least 20% by mass but not more than 85% by mass, or may be at least 30% by mass but not more than 80% by mass, for example.

In the thermal decomposition step, the reaction solution may contain an optional metal in any desired proportion. The form of the metal may be a complex or a solid. Metals reduce the thermal decomposition temperature of the blocked isocyanate compound, but on the other hand, may sometimes cause thermal denaturation, degradation and coloration, and therefore the amount of the metal relative to the mass of the blocked isocyanate compound is preferably less than 10% by mass, more preferably less than 1 % by mass, and even more preferably less than 0.1 ppm by mass.

In the thermal decomposition step, the reaction solution may contain an organic acid, inorganic acid, organic base or inorganic base. These acids and bases act as catalysts in the thermal decomposition of the blocked isocyanate, therefore the temperature required to realize thermal decomposition can be lowered. On the other hand, these acids and bases also act as catalysts of side reactions in the thermal decomposition of the blocked isocyanate, and from that viewpoint, the amount of these organic acids, inorganic acids, organic bases or inorganic bases, relative to the mass of the blocked isocyanate compound, is preferably less than 10% by mass, more preferably less than 1% by mass, even more preferably less than 0.1% by mass, and particularly preferably less than 1 ppb by mass.

In the thermal decomposition step, the isocyanate compound is obtained as a composition containing the isocyanate compound (hereinafter sometimes referred to as the "isocyanate composition").

The term "isocyanate composition" means a composition containing an isocyanate compound in an amount exceeding 0 wt% but not more than 100 wt% of the composition. There are no particular limitations on the isocyanate composition, except that an isocyanate compound is included therein, and the composition may contain, in addition to the isocyanate compound, the secondary amine compound (I), the tertiary amine compound (II), a solvent, blocking agent, blocked isocyanate compound, catalyst or the like in any desired proportion.

In light of these circumstances, the isocyanate composition may be used directly as the isocyanate compound, or the isocyanate compound may be purified from the isocyanate composition prior to use.

### (Thermal Decomposition Apparatus)

There are no particular limitations on the blocked isocyanate compound thermal decomposition apparatus, and conventional thermal decomposition apparatus may be used. Examples of methods that may be used include a method in which an isocyanate compound and a blocking agent are separated in a bath manner by introducing the composition containing the blocked isocyanate compound into a vessel to which a condenser device has been connected, heating the vessel to thermally decompose the blocked isocyanate compound, and then or simultaneously guiding the vapor containing the produced blocking agent into the condenser device, a method in which the free isocyanate compound and blocking agent are obtained in a continuous manner by continuously introducing the composition containing the blocked isocyanate compound into a distillation column that has been heated to at least the thermal decomposition temperature of the blocked isocyanate compound, thereby thermally decomposing the blocked isocyanate compound and simultaneously separating the produced blocking agent and isocyanate compound, and a method in which the blocking agent and the isocyanate compound are separated inside a distillation column by introducing the blocked isocyanate compound into an evaporator or thin film that has been heated to at least the thermal decomposition temperature of the blocked isocyanate compound, and guiding the vapor containing the blocking agent and isocyanate compound produced by thermal decomposition into the distillation column.

In the blocked isocyanate compound thermal decomposition apparatus, the material of regions that make contact with the composition containing the blocked isocyanate compound, and the blocking agent, the isocyanate compound and any other components produced during the thermal decomposition, may be conventional materials, unless they have any adverse effects such as causing denaturation of the blocked isocyanate compound, the blocking agent, the isocyanate compound and any other components. Specific examples of such materials include steel, stainless steel, ceramics, carbon, and lined versions of these materials.

In a case where a distillation device is used, there are no particular limitations on the form of the distillation device, and various conventional distillation devices such as batch distillation devices, simple distillation devices, distillation devices containing any selected from multistage distillation column, continuous multistage distillation column and packed column, distillation devices in which these are combined may be used.

As the distillation column, any distillation column in which the theoretical number of distillation stages is two or greater may be employed. Further, in the case where the theoretical number of stages is large, a multistage distillation column becomes extremely large, which sometimes makes industrial implementation difficult, and therefore the theoretical number of stages is typically 500 or less.

As the distillation column, any column typically usable as a multistage distillation column, such as plate columns using trays such as a bubble cap tray, porous plate tray, valve tray or cross-flow tray, or packed columns that have been packed with any of various fillers such as Raschig rings, Lessing rings, Pall rings, Berl saddles, interlock saddles, Dickson packing, McMahon packing, Helipack, Sulzer packing or Mellapak, may be used. Moreover, plate-packed mixed columns in which a plate portion and a filler-packed portion are combined may also be used favorably.

Next, the raw materials used and the product obtained in the production method of the third embodiment are described below in further detail.

### <Secondary Amine Compound (I)>

The secondary amine compound (I) is a compound of general formula (I) shown below.

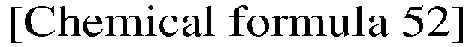

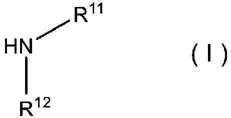

In general formula (I), R¹¹ and R¹² are each independently a monovalent organic group. R¹¹ and R¹² may be bonded together to form a cyclic structure via a carbon-carbon bond, a carbon-oxygen-carbon bond, or a carbon-nitrogen-carbon bond. At least one of R¹¹ and R¹² has an aromatic group.

Compounds having an amino group bonded directly to an aromatic ring are known to cause coloration in the presence of oxygen or the like. However, surprisingly, it became very evident that the secondary amine compound (I) actually exhibited a coloration reduction effect. Although the reasons for this effect are not entirely clear, it is generally known that isocyanate compounds undergo thermal denaturation and coloration when subjected to heat treatment, it is surmised that these issues are due to oxidation and degradation of the isocyanate compound or derivatives thereof. It is surmised that the secondary amine compound (I) interacts with oxides such as oxygen and functions as an antioxidant that is oxidized instead of isocyanate compounds or derivatives thereof, and is also converted to colorless or lightly colored substances upon heating. Further, although the secondary amine compound (I) is an amine-based compound, which are generally known to accelerate the denaturation of isocyanate compounds, the denaturation acceleration observed with typical aliphatic amine compounds or heterocyclic amine compounds is either not observed, or is very slight. It is surmised that this is due to the facts that the secondary amine compound (I) has an aromatic group bonded directly to the nitrogen atom, which results in a low electron density on the nitrogen atom and poor nucleophilicity, and the substituents bonded to the nitrogen atom produce significant steric hindrance around the nitrogen atom.

Details regarding the secondary amine compound (I) are as described in the above section entitled "Method for Producing Blocked Isocyanate Compound according to First Embodiment".

### <Tertiary Amine Compound (II)>

The tertiary amine compound (II) is a compound of general formula (II) shown below.

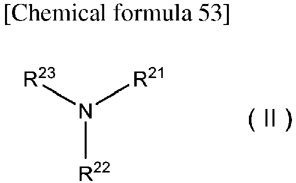

In general formula (II), R²¹, R²² and R²³ are each independently a monovalent organic group. Each combination of R²¹ and R²², R²² and R²³, and R²³ and R²¹ may be independently bonded together to form a cyclic structure via a carbon-carbon bond, a carbon-oxygen-carbon bond, or a carbon-nitrogen-carbon bond. At least one of R²¹, R²² and R²³ has an aromatic group.

Compounds having an amino group bonded directly to an aromatic ring are known to cause coloration in the presence of oxygen or the like. However, surprisingly, it became very evident that the tertiary amine compound (II) actually exhibited a coloration reduction effect. Although the reasons for this effect are not entirely clear, it is generally known that isocyanate compounds undergo thermal denaturation and coloration when subjected to heat treatment, it is surmised that these issues are due to oxidation and degradation of the isocyanate compound or derivatives thereof. It is surmised that the tertiary amine compound (II) interacts with oxides such as oxygen and functions as an antioxidant that is oxidized instead of isocyanate compounds or derivatives thereof, and is also converted to colorless or lightly colored substances upon heating. Further, although the tertiary amine compound (II) is an amine-based compound, which are generally known to accelerate the denaturation of isocyanate compounds, the denaturation acceleration observed with typical aliphatic amine compounds or heterocyclic amine compounds is either not observed, or is very slight. It is surmised that this is due to the facts that the tertiary amine compound (II) has an aromatic group bonded directly to the nitrogen atom, which results in a low electron density on the nitrogen atom and poor nucleophilicity, and the substituents bonded to the nitrogen atom produce significant steric hindrance around the nitrogen atom.

Details regarding the tertiary amine compound (II) are as described in the above section entitled "Method for Producing Blocked Isocyanate Compound according to First Embodiment".

### <Phenol-Based Antioxidant>

There are no particular limitations on the phenol-based antioxidant that may be used in combination with the secondary amine compound (I) and the tertiary amine compound (II), and any compound having at least one hydroxy group bonded to an aromatic ring within the molecule may be used. Specific examples of the phenol-based antioxidant include the same compounds as those exemplified in the above section entitled "Method for Producing Blocked Isocyanate Compound according to First Embodiment".

### <Blocked Isocyanate Compound>

As illustrated in the reaction formula below, a blocked isocyanate compound is a compound having a blocked isocyanate group that can be dissociated by heating into a blocking agent and an isocyanate group.

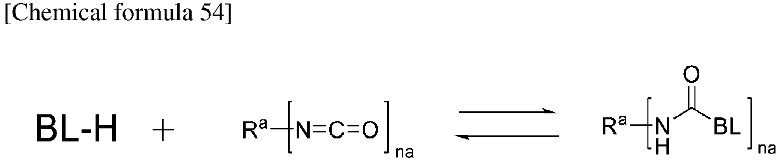

In the above reaction formula, BL-H is the blocking agent, and is an organic compound having an active hydrogen group. R^{a} is an na-valent organic group, and is the same as R³¹ described below.

There are no particular limitations on the blocked isocyanate compound, and any compound may be used that is a reaction product obtained by reaction of an isocyanate compound and a blocking agent, wherein the compound has at least one isocyanate group within the molecule of the isocyanate compound that has been blocked with the blocking agent. Here, the expression "obtained by reaction of an isocyanate compound and a blocking agent" means the compound is obtained by reaction of the reaction formula shown above, and there are no particular limitations on the method used for producing the blocked isocyanate compound. Accordingly, a blocked isocyanate compound produced using any method may be used, such as a blocked isocyanate compound obtained from a blocking agent, a carbonic acid derivative and an amine, a second blocked isocyanate compound obtained by reacting a first blocked isocyanate compound and a second blocking agent by an addition-elimination reaction, a blocked isocyanate compound obtained by reacting phosgene and an amine, followed by reaction with a blocking agent, or a blocked isocyanate compound obtained by reacting an isocyanate compound and a blocking agent.

### <Isocyanate Compound>

Examples of the preferred isocyanate compound obtained in the production method of the third embodiment, which is also the raw material of the above-mentioned blocked isocyanate compound, include a compound of general formula (VII) shown below (hereinafter sometimes referred to as the "isocyanate compound (VII)").

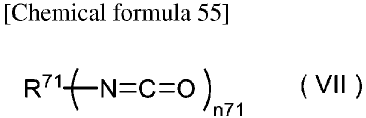

In general formula (VII), R⁷¹ is an n71-valent organic group. Further, n71 is an integer of 1 or more but not more than 12.

### [R⁷¹]

R⁷¹ is an n71-valent organic group. In other words, R⁷¹ is an organic group having a valency of 1 or more but not more than 12. Among them, R⁷¹ is preferably an organic group consisting of carbon atoms, oxygen atoms and hydrogen atoms, and is more preferably an organic group that does not have an active hydrogen.

The aliphatic hydrocarbon group as R⁷¹ is preferably an alkylene group or alkanetriyl group, cycloalkyl group, cycloalkylene group or cycloalkanetriyl group, or a group composed of the above-mentioned alkyl group, alkylene group or alkanetriyl group, and the above-mentioned cycloalkyl group, cycloalkylene group or cycloalkanetriyl group, and more preferably a linear or branched alkylene group or alkanetriyl group, cycloalkylene group or cycloalkanetriyl group, or a group composed of the above alkylene group or alkanetriyl group and the above cycloalkyl group, cycloalkylene group or cycloalkanetriyl group.

Examples of the linear or branched alkylene group include a methylene group, ethylene group, propylene group, trimethylene group, pentylene group, n-hexylene group, and decamethylene group.

Examples of the cycloalkylene group include a cyclobutylene group, cyclopentylene group and cyclohexylene group.

Examples of the linear or branched alkanetriyl group include a hexanetriyl group, nonanetriyl group and decanetriyl group.

Examples of the cycloalkanetriyl group include a cyclopropanetriyl group, cyclobutanetriyl group, cyclopentanetriyl group, and cyclohexanetriyl group.

The aromatic hydrocarbon group as R⁷¹ is preferably a group having a substituted or unsubstituted aromatic ring of 6 or more but not more than 13 carbon atoms. Examples of substituents include alkyl groups, aryl groups, and aralkyl groups. The aromatic ring may be an aromatic hydrocarbon ring or a heteroaromatic ring, and specific examples thereof include a benzene ring, naphthalene ring, and pyridine ring.

Examples of the preferred isocyanate compound (VII) include monofunctional isocyanate compounds, difunctional isocyanate compounds, and polyfunctional isocyanate compounds.

Examples of the monofunctional isocyanate compounds include isocyanate compounds having a saturated hydrocarbon group, such as methyl isocyanate, ethyl isocyanate, propyl isocyanate (each isomer), butyl isocyanate (each isomer), pentyl isocyanate (each isomer), hexyl isocyanate (each isomer), octyl isocyanate (each isomer), nonyl isocyanate (each isomer), decyl isocyanate (each isomer), undecyl isocyanate (each isomer), dodecyl isocyanate (each isomer), tridecyl isocyanate (each isomer), tetradecyl isocyanate (each isomer), pentadecyl isocyanate (each isomer), hexadecyl isocyanate (each isomer), heptadecyl isocyanate (each isomer), octadecyl isocyanate (each isomer), nonadecyl isocyanate (each isomer), and eicosyl isocyanate (each isomer); isocyanate compounds having an unsaturated bond, such as 2-isocyanatomethyl acrylate, 2-isocyanatoethyl acrylate, 2-isocyanatopropyl acrylate (each isomer), 2-isocyanatobutyl acrylate (each isomer), 2-isocyanatomethyl methacrylate, 2-isocyanatoethyl methacrylate, 2-isocyanatopropyl methacrylate (each isomer), 2-isocyanatobutyl methacrylate (each isomer), phenyl isocyanate, and benzyl isocyanate; isocyanate compounds having an ether group, such as methyl (S)-2-isocyanato-3-tertiary-butoxypropionate, ethyl (S)-2-isocyanato-3-tertiary-butoxypropionate, propyl (S)-2-isocyanato-3-tertiary-butoxypropionate (each isomer), butyl (S)-2-isocyanato-3-tertiary-butoxypropionate (each isomer), pentyl (S)-2-isocyanato-3-tertiary-butoxypropionate (each isomer), hexyl (S)-2-isocyanato-3-tertiary-butoxypropionate (each isomer), and dodecyl (S)-2-isocyanato-3-tertiary-butoxypropionate (each isomer); isocyanate compounds having a halogen group, such as fluorophenyl isocyanate (each isomer), chlorophenyl isocyanate (each isomer), bromophenyl isocyanate (each isomer), and iodophenyl isocyanate (each isomer); and isocyanate compounds having a carbonyl group, such as glycine methyl ester isocyanate, glycine ethyl ester isocyanate, glycine propyl ester isocyanate (each isomer), glycine butyl ester isocyanate (each isomer), glycine pentyl ester isocyanate (each isomer), glycine hexyl ester isocyanate (each isomer), glycine dodecyl ester isocyanate (each isomer), leucine methyl ester isocyanate (methyl 2-isocyanato-4-methyl pentanoate), leucine ethyl ester isocyanate (ethyl 2-isocyanato-4-methyl pentanoate), leucine propyl ester isocyanate (each isomer), leucine butyl ester isocyanate (each isomer), leucine pentyl ester isocyanate (each isomer), leucine hexyl ester isocyanate (each isomer), leucine dodecyl ester isocyanate (each isomer), and ethyl isocyanatoacetate.

Examples of the difunctional isocyanate compounds include isocyanate compounds having a saturated hydrocarbon group, such as dimethylene diisocyanate, trimethylene diisocyanate (each isomer), tetramethylene diisocyanate (each isomer), pentamethylene diisocyanate (each isomer), hexamethylene diisocyanate (each isomer), heptamethylene diisocyanate (each isomer), octamethylene diisocyanate (each isomer), diisocyanatocyclohexane (each isomer), bisisocyanatomethylcyclohexane (each isomer), isophorone diisocyanate (each isomer), dicyclohexylmethane diisocyanate (each isomer), diisocyanatodimethylpropane (each isomer), diisocyanatodimethylpentane (each isomer), diisocyanatodimethylhexane (each isomer), diisocyanatodimethylheptane (each isomer), diisocyanatodimethyloctane (each isomer), diisocyanatodimethylnonane (each isomer), diisocyanatodimethyldecane (each isomer), diisocyanatomethylethylpropane (each isomer), diisocyanatomethylethylbutane (each isomer), diisocyanatomethylethylpentane (each isomer), diisocyanatomethylethylhexane (each isomer), diisocyanatomethylethylheptane (each isomer), diisocyanatomethylethyloctane (each isomer), diisocyanatodiethylpropane (each isomer), diisocyanatodiethylpentane (each isomer), diisocyanatodiethylhexane (each isomer), diisocyanatodiethylheptane (each isomer), diisocyanatodiethyloctane (each isomer), and diisocyanatodiethylnonane (each isomer); isocyanate compounds having an unsaturated hydrocarbon group, such as diphenylmethane diisocyanate (each isomer), tolylene diisocyanate (each isomer), naphthalene diisocyanate (each isomer), xylylene diisocyanate (each isomer), tetramethylxylylene diisocyanate (each isomer), methylenebis(diisoamyl-phenylene) diisocyanate (each isomer), oxybis(phenylene diisocyanate) (each isomer), carbonylbis(phenylene) diisocyanate (each isomer), butene diisocyanate (each isomer), and butynylene diisocyanate (each isomer); and isocyanate compounds having an ester group, such as lysine methyl ester isocyanate, lysine ethyl ester isocyanate, lysine propyl ester isocyanate, lysine butyl ester isocyanate, lysine pentyl ester isocyanate, and lysine hexyl ester isocyanate.

Examples of the polyfunctional isocyanate compounds include isocyanate compounds having a saturated hydrocarbon group, such as propane triisocyanate, butane triisocyanate (each isomer), pentane triisocyanate (each isomer), hexane triisocyanate (each isomer), heptane triisocyanate (each isomer), octane triisocyanate (each isomer), nonane triisocyanate (each isomer), decane triisocyanate (each isomer), undecane triisocyanate (each isomer), dodecane triisocyanate (each isomer), tridecane triisocyanate (each isomer), tetradecane triisocyanate (each isomer), pentadecane triisocyanate (each isomer), hexadecane triisocyanate (each isomer), heptadecane triisocyanate (each isomer), octadecane triisocyanate (each isomer), nonadecane triisocyanate (each isomer), and eicosane triisocyanate (each isomer); isocyanate compounds having an unsaturated hydrocarbon group such as polymeric MDI (each isomer); isocyanate compounds having an ester group such as 2-isocyanatoethyl 2,6-diisocyanatohexanoate; as well as difunctional or higher-functional isocyanate compounds having an isocyanurate group, difunctional or higher-functional isocyanate compounds having a biuret group, difunctional or higher-functional isocyanate compounds having an allophanate group, and isocyanate compounds obtained by crosslinking a difunctional or higher-functional isocyanate compound having isocyanate groups with a difunctional or higher-functional compound having active hydrogens.

### <Blocking Agent>

The blocking agent which is produced as a by-product of the production method of the third embodiment and is also a raw material of the above blocked isocyanate compound, may be any compound that has an active hydrogen group, and is capable of reacting with an isocyanate group to form a bond, and then being decomposed into an isocyanate compound and the blocking agent by thermal decomposition. Examples of such a blocking agent include alcohol-based, phenol-based (hereinafter alcohol-based and phenol-based blocking agents are sometimes referred to jointly as "hydroxy compounds"), thiol-based, amine- and ammonia-based, oxime-based, hydroxylamine-based, and active methylene-based blocking agents. Among them, blocking agents formed from hydroxy compounds, and amine- and ammonia-based, or hydroxylamine-based blocking agents are preferred from the viewpoint of the stability of the blocking agent during thermal decomposition. Further, phenol-based, amine-based or hydroxylamine-based blocking agents or ammonia are more preferred in terms of limiting denaturation of the isocyanate compound during thermal decomposition. Furthermore, a phenol-based blocking agent is even more preferred in terms of achieving particularly superior stability of the blocking agent during thermal decomposition and limiting denaturation of the isocyanate compound during thermal decomposition.

### [Phenol-Based Blocking Agent]

There are no particular limitations on the phenol-based blocking agent, provided it is a compound having a hydroxyl group bonded to an aromatic ring, but a blocking agent having a phenol group or naphthol group is preferred from the viewpoint of ease of availability. The blocking agent may be a compound having one hydroxyl group bonded to an aromatic ring within the molecule, or may be a compound having two or more hydroxyl groups bonded to one or more aromatic rings within the molecule. In those cases where a compound having two or more hydroxyl groups bonded to one or more aromatic rings within the molecule is used as the blocking agent, separation of the isocyanate compound and the compound having the hydroxyl groups bonded to the aromatic ring becomes more difficult. Accordingly, a compound having one hydroxyl group bonded to an aromatic ring within the molecule is preferably used as the phenol-based blocking agent.

Examples of the preferred phenol-based blocking agent include the same compounds as those exemplified in the above section entitled "Method for Producing Blocked Isocyanate Compound according to First Embodiment".

### [Alcohol-Based Blocking Agent]

There are no particular limitations on the alcohol-based blocking agent, provided it is an aliphatic compound having a hydroxyl group, and either an aliphatic compound having one hydroxyl group within the molecule, or an aliphatic compound having two or more hydroxyl groups within the molecule may be used. In those cases where an aliphatic compound having two or more hydroxyl groups within the molecule is used as the blocking agent, separation of the isocyanate compound and the aliphatic compound having the hydroxyl groups becomes more difficult. Accordingly, an aliphatic compound having one hydroxyl group within the molecule is preferably used.

Examples of the preferred alcohol-based blocking agent include the same compounds as those exemplified in the above section entitled "Method for Producing Blocked Isocyanate Compound according to First Embodiment".

### [Amine- and Ammonia-Based Blocking Agents]

Examples of compounds that may be used as the amine- and ammonia-based blocking agents include amine compounds having a primary or secondary amino group as an active hydrogen group, and ammonia. In those cases where a primary amine compound or ammonia is used as the blocking agent, the urea linkage formed by the reaction of the primary amine compound or ammonia with the isocyanate compound has two possible cleavage pathways during thermal decomposition, and therefore there is a case in which an isocyanate compound is formed on the primary amine compound used as the blocking agent, thereby making it more difficult to obtain the target isocyanate compound. Accordingly, it is preferable that an amine compound having a secondary amino group be used.

The amine- and ammonia-based blocking agent may be an amine compound having one amino group within the molecule, or an amine compound having two or more amino groups within the molecule. In those cases where an amine compound having two or more amino groups within the molecule is used as the blocking agent, separation of the isocyanate compound and the amine compound becomes more difficult. Accordingly, an amine compound having one amino group within the molecule is preferably used as the amine- and ammonia-based blocking agent.

Examples of the preferred amine- and ammonia-based blocking agent include the same compounds as those exemplified in the above section entitled "Method for Producing Blocked Isocyanate Compound according to First Embodiment".

### [Hydroxylamine-Based Blocking Agent]

There are no particular limitations on the hydroxylamine-based blocking agent, and the blocking agent may be a hydroxylamine compound having one hydroxylamine structure within the molecule, or a hydroxylamine compound having two or more hydroxylamine structures within the molecule. In those cases where a hydroxylamine compound having two or more hydroxylamine structures within the molecule is used as the blocking agent, separation of the isocyanate compound and the hydroxylamine compound becomes more difficult. Accordingly, a compound having one hydroxylamine structure within the molecule is preferably used as the hydroxylamine-based blocking agent.

Examples of the hydroxylamine compound having one hydroxylamine structure within the molecule include the same compounds as those exemplified in the above section entitled "Method for Producing Blocked Isocyanate Compound according to First Embodiment".

### <<Method for Producing Isocyanate Compound according to Fourth Embodiment and Fifth Embodiment>>

The method for producing an isocyanate compound according to the present embodiment (hereinafter sometimes referred to as simply "the production method of the fourth embodiment") includes:
a first reaction step of reacting a primary amine compound, a carbonic acid derivative and a first blocking agent by conducting a heat treatment in the presence of at least one compound selected from the group consisting of the secondary amine compounds (I) and the tertiary amine compounds (II), thus obtaining a blocked isocyanate compound, and
a second reaction step of decomposing the blocked isocyanate compound into an isocyanate compound and the first blocking agent by thermal decomposition in the presence or absence of at least one compound selected from the group consisting of the secondary amine compounds (I) and the tertiary amine compounds (II), thus obtaining the isocyanate compound.

Alternatively, the method for producing an isocyanate compound according to the present embodiment (hereinafter sometimes referred to as simply "the production method of the fifth embodiment") includes:
a first reaction step of reacting a primary amine compound, a carbonic acid derivative and a first blocking agent by conducting a heat treatment in the presence of at least one compound selected from the group consisting of the secondary amine compounds (I) and the tertiary amine compounds (II), thus obtaining a blocked isocyanate compound,
an addition-elimination reaction step of reacting the first blocked isocyanate compound and a second blocking agent by an addition-elimination reaction in the presence or absence of at least one compound selected from the group consisting of the secondary amine compounds (I) and the tertiary amine compounds (II), thus obtaining a second blocked isocyanate compound, and
a second reaction step of decomposing the second blocked isocyanate compound into the second blocking agent and an isocyanate compound by conducting a heat treatment in the presence or absence of at least one compound selected from the group consisting of the secondary amine compounds (I) and the tertiary amine compounds (II), thus obtaining the isocyanate compound.

The second blocking agent is a compound different from the first blocking agent and a third blocking agent derived from the carbonic acid derivative. The third blocking agent derived from the carbonic acid derivative is as described above in relation to the compounds derived from the carbonic acid derivative.

The first blocked isocyanate compound and the second blocked isocyanate compound have structures different from each other.

As described above, the blocked isocyanate compound used in the method for producing an isocyanate compound according to the third embodiment may employ a blocked isocyanate compound produced using any method, such as a blocked isocyanate compound obtained from a blocking agent, a carbonic acid derivative and an amine, a blocked isocyanate compound obtained by reacting phosgene and an amine, followed by reaction with a blocking agent, or a blocked isocyanate compound obtained by reacting an isocyanate compound and a blocking agent.

Among them, a method in which a blocked isocyanate compound is produced from a blocking agent, a carbonic acid derivative and an amine, and the blocked isocyanate composition containing the resultant blocked isocyanate compound is used as a raw material in the above second reaction step, namely, the production method of the fourth embodiment, or a method in which a blocked isocyanate compound is produced from a blocking agent, a carbonic acid derivative and an amine, a second blocking agent is added to the blocked isocyanate composition containing the resultant blocked isocyanate compound, an addition-elimination reaction is conducted to produce a second blocked isocyanate compound, and the blocked isocyanate composition containing the resultant second blocked isocyanate compound is used as a raw material in the above second reaction step, namely, the production method of the fifth embodiment, are very useful production methods in terms that extremely toxic phosgene or very expensive reagents are not used.

In those cases where the blocked isocyanate composition containing the first blocked isocyanate compound obtained in the first reaction step is used as a raw material in the second reaction step, the first blocked isocyanate compound may sometimes include blocked isocyanate groups bonded to a third blocking agent derived from the carbonic acid derivative.

In particular, if the carbonic acid derivative is a compound of the above general formula (VI-1) in which R⁶¹¹ and R⁶¹² are both hydrogen atoms, or a compound of the above general formula (VI-2) in which R⁶¹¹ and R⁶¹² are both hydrogen atoms, and heating is conducted in the presence of a blocking agent, ammonia is produced. Accordingly, in those cases where a blocked isocyanate compound is produced from a blocking agent, a carbonic acid derivative that is either a compound of the above general formula (VI-1) in which R⁶¹¹ and R⁶¹² are both hydrogen atoms and/or a compound of the above general formula (VI-2) in which R⁶²¹ and R¹²² are both hydrogen atoms, and an amine, the obtained first blocked isocyanate compound may sometimes contain a blocked isocyanate group (hereinafter referred to as an ureido group) obtained from reaction of ammonia and an isocyanate group. There is a case in which a blocked isocyanate compound containing an ureido group produces a polymer component having a crosslinkable functional group such as an ureylene group (-NHCONH-) upon thermal denaturation, and therefore the amount present of blocked isocyanate containing an ureido group within the raw material in the second reaction step is preferably reduced. Further, it is preferable that the thermal decomposition reaction be conducted in the presence of at least one compound selected from the group consisting of the secondary amine compounds (I) and the tertiary amine compounds (II), since thermal denaturation is further reduced. As the reason for this, a compsound having an ureido group can be decomposed into an amine and isocyanic acid by thermal dissociation, but isocyanic acid and cyanic acid which exists in a state of equilibrium with isocyanic acid are acidic substances, and may function as acid catalysts which promote denaturation of the isocyanate groups. Accordingly, it is surmised that the presence of at least one compound selected from the group consisting of the secondary amine compounds (I) and the tertiary amine compounds (II) causes a neutralization reaction with the isocyanic acid or the cyanic acid which exists in a state of equilibrium with isocyanic acid, thereby reducing the degree of acidity increased by the presence of the acid components in the reaction system and suppressing denaturation.

In those cases where the blocked isocyanate composition containing the second blocked isocyanate compound obtained following the first reaction step and the addition-elimination reaction step is used as the raw material in an isocyanate production process, the first blocked isocyanate compound from the addition-elimination reaction step may sometimes include blocked isocyanate groups in which a third blocking agent derived from the carbonic acid derivative is bonded to an isocyanate group.

In particular, if the carbonic acid derivative is a compound of the above general formula (VI-1) in which R⁶¹¹ and R⁶¹² are both hydrogen atoms, or a compound of the above general formula (VI-2) in which R⁶¹¹ and R⁶¹² are both hydrogen atoms, and heating is conducted in the presence of a blocking agent, ammonia is produced. Accordingly, in those cases where a first blocked isocyanate compound is produced from a blocking agent, a carbonic acid derivative that is either a compound of the above general formula (VI-1) in which R⁶¹¹ and R⁶¹² are both hydrogen atoms and/or a compound of the above general formula (VI-2) in which R⁶²¹ and R⁶²² are both hydrogen atoms, and an amine, the obtained first blocked isocyanate compound may sometimes contain a blocked isocyanate group (hereinafter referred to as an ureido group) obtained from reaction of ammonia and an isocyanate group. There is a case in which a blocked isocyanate containing an ureido group produces a polymer component having a crosslinkable functional group such as an ureylene group (-NHCONH-) upon thermal denaturation, and therefore the amount present of blocked isocyanate containing an ureido group within the raw material in the addition-elimination reaction step is preferably reduced. Further, it is preferable that the thermal decomposition reaction be conducted in the presence of at least one compound selected from the group consisting of the secondary amine compounds (I) and the tertiary amine compounds (II), since thermal denaturation is further reduced. As the reason for this, a compsound having an ureido group can be decomposed into an amine and isocyanic acid by thermal dissociation, but isocyanic acid and cyanic acid which exists in a state of equilibrium with isocyanic acid are acidic substances, and may function as acid catalysts which promote denaturation of the isocyanate groups. Accordingly, it is surmised that the presence of at least one compound selected from the group consisting of the secondary amine compounds (I) and the tertiary amine compounds (II) causes a neutralization reaction with the isocyanic acid or the cyanic acid which exists in a state of equilibrium with isocyanic acid, thereby reducing the degree of acidity increased by the presence of the acid components in the reaction system and suppressing denaturation.

### [Examples]

The present invention is described below in further detail by way of examples, but the present invention is not limited to these examples. Refined products were used for all of the reagents used as raw materials.

### <<First Test>>

### [Physical Property 1-1]

### (Amino Group Content)

The amino group content (mol%) within each obtained blocked isocyanate composition was determined by titration of the amino groups with 1 N hydrochloric acid using the obtained blocked isocyanate composition as the measurement sample. Further, for this test, any secondary amine compound (I), tertiary amine compound (II) and blocking agent contained in the blocked isocyanate composition was removed in advance by distillation under reduced pressure.

### [Physical Property 1-2]

### (Blocked Isocyanate Group Content)

The blocked isocyanate group content (mol%) within each obtained blocked isocyanate composition (hereinafter sometimes abbreviated as "NCO-BL mol%") was determined in accordance with the formula shown below, from the molar amount of amino groups in the primary amine compound contained within the reaction mixture containing the primary amine compound, the carbonic acid derivative and the blocking agent prior to reaction, the molar amount being calculated from the amount added of the primary amine compound, and the molar amount of blocked isocyanate groups produced following the reaction. The molar amount of blocked isocyanate groups was determined from the concentration of blocked isocyanate groups as determined from the NMR spectrum, and the total mass of the obtained blocked isocyanate composition. [NCO-BL mol%] = (molar amount of blocked isocyanate groups) × 100 (molar amount of amino groups in primary amine compound)

### [Physical Property 1-3]

### (Amount of Isocyanate Groups bonded to Secondary Amine Compound (I))

The amount (mol%) of isocyanate groups bonded to the secondary amine compound (I) within each obtained blocked isocyanate composition (hereinafter sometimes abbreviated as "NCO-compound (I) mol%") was determined in accordance with the formula shown below, from the molar amount of amino groups in the primary amine compound contained within the reaction mixture containing the organic primary amine compound, the carbonic acid derivative and the blocking agent prior to reaction, the molar amount being calculated from the amount added of the primary amine compound, and the molar amount of residual NCO-compound (I) following thermal decomposition. The molar amount of NCO-compound (I) was determined from the concentration of NCO-compound (I) as determined from the NMR spectrum, and the total mass of the obtained blocked isocyanate composition. [NCO-compound (I) mol%] = (molar amount of NCO-compound (I)) × 100 / (molar amount of amino groups in primary amine compound)

### [Physical Property 1-4]

### (Oxygen Concentration)

A synthesis gas supplied to the reaction apparatus was produced from a nitrogen cylinder and an oxygen cylinder, and the oxygen concentration (% by volume) of the synthesis gas was measured using a galvanic cell oxygen concentration meter.

### [Evaluation 1-1]

### (Mass Balance)

The sum, within the obtained blocked isocyanate composition, of the amino group content (mol%), NCO-BL mol% and NCO-compound (1) mol% was determined as the mass balance (hereinafter sometimes abbreviated as "MB mol%").

### [Evaluation 1-2]

### (Hazen Color Number)

The Hazen color number was determined by dissolving, in 2 g of benzyltoluene, 1 g of the blocked isocyanate composition obtained following removal by distillation of any free compound (I), compound (II) and blocking agent contained in the obtained reaction solution. Based on the value measured with a Hazen meter, the resultant was ranked in accordance with the following evaluation criteria.

### (Evaluation Criteria)

Rank 1: APHA of at least 0 but less than 5
Rank 2: APHA of at least 5 but less than 10
Rank 3: APHA of at least 10 but less than 15
Rank 4: APHA of at least 15 but less than 20
Rank 5: APHA of at least 20 but less than 25
Rank 6: APHA of at least 25 but less than 30
Rank 7: APHA of at least 30 but less than 35
Rank 8: APHA of at least 35 but less than 40
Rank 9: APHA of at least 40 but less than 45
Rank 10: APHA of at least 45 but less than 50

### [Example 1-1]

10 parts by mass of hexamethylenediamine (HDA), 15.5 parts by mass of urea, 90 parts by mass of phenol, and 1 part by mass of N-methylaniline (NMA) were placed in a 500 mL SUS pressure-resistant vessel equipped with a condenser tube packed with a filler (the condenser tube having a structure that allows the condensed liquid from the condenser tube to fall into the 500 mL SUS pressure-resistant vessel) and an ammonia trap, the internal temperature of the 500 mL pressure-resistant vessel was set to 240°C, and the temperature of the condenser tube was set to 60°C. After a synthesis gas was produced from a nitrogen cylinder and an oxygen cylinder and then the oxygen concentration in the synthesis gas was confirmed to be 0.1 vol% with an oxygen concentration meter, the synthesis gas was supplied to the 500 mL SUS pressure-resistant vessel to adjust the absolute pressure to 405 kPa, followed by allowing the reaction to proceed for two hours. As a result, 103.3 parts by mass of a reaction liquid was obtained in the 500 mL SUS pressure-resistant vessel. The urea, free phenol and free N-methylaniline contained within the obtained reaction liquid were removed by distillation under reduced pressure, thereby obtaining a blocked isocyanate composition. The results of analyses revealed an amino group content of 0 mol%, and, based on ¹H NMR integral values, NCO-BL mol% of 84 mol%, NCO-compound (I) mol% of 5 mol%, and MB mol% of 89 mol%. Further, when 1 g of the blocked isocyanate composition was dissolved in 2 g of benzyltoluene and the Hazen color number was determined, the result was evaluated as rank 3.

### [Examples 1-2 to 1-125, and Comparative Examples 1-1 to 1-11]

In each of Examples 1-2 to 1-125 and Comparative Examples 1-1 to 1-11, blocked isocyanate compounds were produced using the same method as that described in Example 1-1, except that the raw materials and reaction conditions are altered as shown in the following tables.

In the following tables, the abbreviations used refer to the following compounds.

### (Blocking Agents)

PhOH: phenol
o-cresol: ortho-cresol
m-cresol: meta-cresol
p-cresol: para-cresol
OCP: o-chlorophenol
n-BuOH: normal butanol
DBA: dibutylamine

### (Secondary Amine Compounds (1))

NMA: N-methyl-aniline
NEA: N-ethyl-aniline
NBA: N-butyl-aniline
DPA: N,N-diphenylamine

### (Tertiary Amine Compounds (II))

NNDMA: N,N-dimethylaniline
NNDEA: N,N-diethylaniline
NMDPA: N-methyl-N.N-diphenylamine
TPA: triphenylamine

### (Primary Amine Compounds)

HDA: 1,6-hexamethylenediamine
IPDA: isophoronediamine (isomeric mixture)
MBCA: 4,4'-methylenebis(cyclohexylamine) (isomeric mixture)
DAT: diaminotoluene (isomeric mixture)
DPM: 4,4'-diphenylmethanediamine

### (Other Compounds)

BL-NCO: blocked isocyanate

**[Table 1-1]**

| | Secondary amine compound (I) | | Tertiary amine compound (II) | | Primary amine compound | | Blocking agent | | Carbonic acid derivative | |
|---|---|---|---|---|---|---|---|---|---|---|
| | type | parts by mass | type | parts by mass | type | parts by mass | type | parts by mass | type | parts by mass |
| Example 1-1 | NMA | 1 | - | 0 | HDA | 10 | PhOH | 90 | urea | 15.5 |
| Example 1-2 | NEA | 1 | - | 0 | HDA | 10 | PhOH | 90 | urea | 15.5 |
| Example 1-3 | NBA | 1 | - | 0 | HDA | 10 | PhOH | 90 | urea | 15.5 |
| Example 1-4 | DPA | 1 | - | 0 | HDA | 10 | PhOH | 90 | urea | 15.5 |
| Example 1-5 | - | 0 | NNDMA | 1 | HDA | 10 | PhOH | 90 | urea | 15.5 |
| Example 1-6 | - | 0 | NNDEA | 1 | HDA | 10 | PhOH | 90 | urea | 15.5 |
| Example 1-7 | - | 0 | NMDPA | 1 | HDA | 10 | PhOH | 90 | urea | 15.5 |
| Example 1-8 | - | 0 | TPA | 1 | HDA | 10 | PhOH | 90 | urea | 15.5 |
| Example 1-9 | NEA | 1 | - | 0 | HDA | 10 | o-cresol | 90 | urea | 15.5 |
| Example 1-10 | NEA | 1 | - | 0 | HDA | 10 | m-cresol | 90 | urea | 15.5 |
| Example 1-11 | NEA | 1 | - | 0 | HDA | 10 | p-cresol | 90 | urea | 15.5 |
| Example 1-12 | NEA | 1 | - | 0 | HDA | 10 | OCP | 90 | urea | 15.5 |
| Example 1-13 | NEA | 1 | - | 0 | HDA | 10 | n-BuOH | 90 | urea | 15.5 |
| Example 1-14 | NEA | 1 | - | 0 | HDA | 10 | DBA | 90 | urea | 15.5 |
| Example 1-15 | NEA | 1 | - | 0 | IPDA | 10 | PhOH | 90 | urea | 10.6 |
| Example 1-16 | NEA | 1 | - | 0 | IPDA | 10 | o-cresol | 90 | urea | 10.6 |
| Example 1-17 | NEA | 1 | - | 0 | IPDA | 10 | m-cresol | 90 | urea | 10.6 |
| Example 1-18 | NEA | 1 | - | 0 | IPDA | 10 | p-cresol | 90 | urea | 10.6 |
| Example 1-19 | NEA | 1 | - | 0 | IPDA | 10 | OCP | 90 | urea | 10.6 |
| Example 1-20 | NEA | 1 | - | 0 | IPDA | 10 | n-BuOH | 90 | urea | 10.6 |
| Example 1-21 | NEA | 1 | - | 0 | IPDA | 10 | DBA | 90 | urea | 10.6 |
| Example 1-22 | NEA | 1 | - | 0 | MBCA | 10 | PhOH | 90 | urea | 8.6 |
| Example 1-23 | NEA | 1 | - | 0 | MBCA | 10 | o-cresol | 90 | urea | 8.6 |
| Example 1-24 | NEA | 1 | - | 0 | MBCA | 10 | m-cresol | 90 | urea | 8.6 |
| Example 1-25 | NEA | 1 | - | 0 | MBCA | 10 | p-cresol | 90 | urea | 8.6 |
| Example 1-26 | NEA | 1 | - | 0 | MBCA | 10 | OCP | 90 | urea | 8.6 |
| Example 1-27 | NEA | 1 | - | 0 | MBCA | 10 | n-BuOH | 90 | urea | 8.6 |
| Example 1-28 | NEA | 1 | - | 0 | MBCA | 10 | DBA | 90 | urea | 8.6 |
| Example 1-29 | NEA | 1 | - | 0 | DAT | 10 | PhOH | 90 | urea | 14.7 |
| Example 1-30 | NEA | 1 | - | 0 | DAT | 10 | o-cresol | 90 | urea | 14.7 |
| Example 1-31 | NEA | 1 | - | 0 | DAT | 10 | m-cresol | 90 | urea | 14.7 |
| Example 1-32 | NEA | 1 | - | 0 | DAT | 10 | p-cresol | 90 | urea | 14.7 |
| Example 1-33 | NEA | 1 | - | 0 | DAT | 10 | OCP | 90 | urea | 14.7 |
| Example 1-34 | NEA | 1 | - | 0 | DAT | 10 | n-BuOH | 90 | urea | 14.7 |
| Example 1-35 | NEA | 1 | - | 0 | DAT | 10 | DBA | 90 | urea | 14.7 |

**[Table 1-2]**

| | Secondary amine compound (I) | | Tertiary amine compound (II) | | Primary amine compound | | Blocking agent | | Carbonic acid derivative | |
|---|---|---|---|---|---|---|---|---|---|---|
| | type | parts by mass | type | parts by mass | type | parts by mass | type | parts by mass | type | parts by mass |
| Example 1-36 | NEA | 1 | - | 0 | DPM | 10 | PhOH | 90 | urea | 9.1 |
| Example 1-37 | NEA | 1 | - | 0 | DPM | 10 | o-cresol | 90 | urea | 9.1 |
| Example 1-38 | NEA | 1 | - | 0 | DPM | 10 | m-cresol | 90 | urea | 9.1 |
| Example 1-39 | NEA | 1 | - | 0 | DPM | 10 | p-cresol | 90 | urea | 9.1 |
| Example 1-40 | NEA | 1 | - | 0 | DPM | 10 | OCP | 90 | urea | 9.1 |
| Example 1-41 | NEA | 1 | - | 0 | DPM | 10 | n-BuOH | 90 | urea | 9.1 |
| Example 1-42 | NEA | 1 | - | 0 | DPM | 10 | DBA | 90 | urea | 9.1 |
| Example 1-43 | - | 0 | NNDEA | 1 | HDA | 10 | o-cresol | 90 | urea | 15.5 |
| Example 1-44 | - | 0 | NNDEA | 1 | HDA | 10 | m-cresol | 90 | urea | 15.5 |
| Example 1-45 | - | 0 | NNDEA | 1 | HDA | 10 | p-cresol | 90 | urea | 15.5 |
| Example 1-46 | - | 0 | NNDEA | 1 | HDA | 10 | OCP | 90 | urea | 15.5 |
| Example 1-47 | - | 0 | NNDEA | 1 | HDA | 10 | n-BuOH | 90 | urea | 15.5 |
| Example 1-48 | - | 0 | NNDEA | 1 | HDA | 10 | DBA | 90 | urea | 15.5 |
| Example 1-49 | - | 0 | NNDEA | 1 | IPDA | 10 | PhOH | 90 | urea | 10.6 |
| Example 1-50 | - | 0 | NNDEA | 1 | IPDA | 10 | o-cresol | 90 | urea | 10.6 |
| Example 1-51 | - | 0 | NNDEA | 1 | IPDA | 10 | m-cresol | 90 | urea | 10.6 |
| Example 1-52 | - | 0 | NNDEA | 1 | IPDA | 10 | p-cresol | 90 | urea | 10.6 |
| Example 1-53 | - | 0 | NNDEA | 1 | IPDA | 10 | OCP | 90 | urea | 10.6 |
| Example 1-54 | - | 0 | NNDEA | 1 | IPDA | 10 | n-BuOH | 90 | urea | 10.6 |
| Example 1-55 | - | 0 | NNDEA | 1 | IPDA | 10 | DBA | 90 | urea | 10.6 |
| Example 1-56 | - | 0 | NNDEA | 1 | MBCA | 10 | PhOH | 90 | urea | 8.6 |
| Example 1-57 | - | 0 | NNDEA | 1 | MBCA | 10 | o-cresol | 90 | urea | 8.6 |
| Example 1-58 | - | 0 | NNDEA | 1 | MBCA | 10 | m-cresol | 90 | urea | 8.6 |
| Example 1-59 | - | 0 | NNDEA | 1 | MBCA | 10 | p-cresol | 90 | urea | 8.6 |
| Example 1-60 | - | 0 | NNDEA | 1 | MBCA | 10 | OCP | 90 | urea | 8.6 |
| Example 1-61 | - | 0 | NNDEA | 1 | MBCA | 10 | n-BuOH | 90 | urea | 8.6 |
| Example 1-62 | - | 0 | NNDEA | 1 | MBCA | 10 | DBA | 90 | urea | 8.6 |
| Example 1-63 | - | 0 | NNDEA | 1 | DAT | 10 | PhOH | 90 | urea | 14.7 |
| Example 1-64 | - | 0 | NNDEA | 1 | DAT | 10 | o-cresol | 90 | urea | 14.7 |
| Example 1-65 | - | 0 | NNDEA | 1 | DAT | 10 | m-cresol | 90 | urea | 14.7 |
| Example 1-66 | - | 0 | NNDEA | 1 | DAT | 10 | p-cresol | 90 | urea | 14.7 |
| Example 1-67 | - | 0 | NNDEA | 1 | DAT | 10 | OCP | 90 | urea | 14.7 |
| Example 1-68 | - | 0 | NNDEA | 1 | DAT | 10 | n-BuOH | 90 | urea | 14.7 |
| Example 1-69 | - | 0 | NNDEA | 1 | DAT | 10 | DBA | 90 | urea | 14.7 |

**[Table 1-3]**

| | Secondary amine compound (I) | | Tertiary amine compound (II) | | Primary amine compound | | Blocking agent | | Carbonic acid derivative | |
|---|---|---|---|---|---|---|---|---|---|---|
| | type | parts by mass | type | parts by mass | type | parts by mass | type | parts by mass | type | parts by mass |
| Example 1-70 | - | 0 | NNDEA | 1 | DPM | 10 | PhOH | 90 | urea | 9.1 |
| Example 1-71 | - | 0 | NNDEA | 1 | DPM | 10 | o-cresol | 90 | urea | 9.1 |
| Example 1-72 | - | 0 | NNDEA | 1 | DPM | 10 | m-cresol | 90 | urea | 9.1 |
| Example 1-73 | - | 0 | NNDEA | 1 | DPM | 10 | p-cresol | 90 | urea | 9.1 |
| Example 1-74 | - | 0 | NNDEA | 1 | DPM | 10 | OCP | 90 | urea | 9.1 |
| Example 1-75 | - | 0 | NNDEA | 1 | DPM | 10 | n-BuOH | 90 | urea | 9.1 |
| Example 1-76 | - | 0 | NNDEA | 1 | DPM | 10 | DBA | 90 | urea | 9.1 |
| Example 1-77 | NMA | 0.0001 | - | 0 | HDA | 10 | PhOH | 90 | urea | 15.5 |
| Example 1-78 | - | 0 | NNDMA | 0.0001 | HDA | 10 | PhOH | 90 | urea | 15.5 |
| Example 1-79 | NMA | 100 | - | 0 | HDA | 10 | PhOH | 90 | urea | 15.5 |
| Example 1-80 | - | 0 | NNDMA | 100 | HDA | 10 | PhOH | 90 | urea | 15.5 |
| Example 1-81 | NMA | 1 | NNDMA | 1 | HDA | 10 | PhOH | 90 | urea | 15.5 |
| Example 1-82 | NMA | 1 | NNDMA | 0.0001 | HDA | 10 | PhOH | 90 | urea | 15.5 |
| Example 1-83 | NMA | 0.0001 | NNDMA | 1 | HDA | 10 | PhOH | 90 | urea | 15.5 |
| Example 1-84 | NMA | 100 | NNDMA | 0.0001 | HDA | 10 | PhOH | 90 | urea | 15.5 |
| Example 1-85 | NMA | 90 | NNDMA | 0.1 | HDA | 10 | PhOH | 90 | urea | 15.5 |
| Example 1-86 | NMA | 80 | NNDMA | 20 | HDA | 10 | PhOH | 90 | urea | 15.5 |
| Example 1-87 | NMA | 60 | NNDMA | 40 | HDA | 10 | PhOH | 90 | urea | 15.5 |
| Example 1-88 | NMA | 40 | NNDMA | 60 | HDA | 10 | PhOH | 90 | urea | 15.5 |
| Example 1-89 | NMA | 20 | NNDMA | 80 | HDA | 10 | PhOH | 90 | urea | 15.5 |
| Example 1-90 | NMA | 0.1 | NNDMA | 90 | HDA | 10 | PhOH | 90 | urea | 15.5 |
| Example 1-91 | NMA | 0.0001 | NNDMA | 100 | HDA | 10 | PhOH | 90 | urea | 15.5 |
| Example 1-92 | NMA | 0.0001 | NNDMA | 1 | HDA | 10 | n-BuOH | 90 | urea | 15.5 |
| Example 1-93 | NMA | 100 | NNDMA | 0.0001 | HDA | 10 | n-BuOH | 90 | urea | 15.5 |
| Example 1-94 | NMA | 90 | NNDMA | 0.1 | HDA | 10 | n-BuOH | 90 | urea | 15.5 |
| Example 1-95 | NMA | 80 | NNDMA | 20 | HDA | 10 | n-BuOH | 90 | urea | 15.5 |
| Example 1-96 | NMA | 60 | NNDMA | 40 | HDA | 10 | n-BuOH | 90 | urea | 15.5 |
| Example 1-97 | NMA | 40 | NNDMA | 60 | HDA | 10 | n-BuOH | 90 | urea | 15.5 |
| Example 1-98 | NMA | 20 | NNDMA | 80 | HDA | 10 | n-BuOH | 90 | urea | 15.5 |
| Example 1-99 | NMA | 0.1 | NNDMA | 90 | HDA | 10 | n-BuOH | 90 | urea | 15.5 |
| Example 1-100 | NMA | 0.0001 | NNDMA | 100 | HDA | 10 | n-BuOH | 90 | urea | 15.5 |
| Example 1-101 | NMA | 0.0001 | NNDMA | 1 | HDA | 10 | DBA | 90 | urea | 15.5 |
| Example 1-102 | NMA | 100 | NNDMA | 0.0001 | HDA | 10 | DBA | 90 | urea | 15.5 |
| Example 1-103 | NMA | 90 | NNDMA | 0.1 | HDA | 10 | DBA | 90 | urea | 15.5 |
| Example 1-104 | NMA | 80 | NNDMA | 20 | HDA | 10 | DBA | 90 | urea | 15.5 |
| Example 1-105 | NMA | 60 | NNDMA | 40 | HDA | 10 | DBA | 90 | urea | 15.5 |
| Example 1-106 | NMA | 40 | NNDMA | 60 | HDA | 10 | DBA | 90 | urea | 15.5 |
| Example 1-107 | NMA | 20 | NNDMA | 80 | HDA | 10 | DBA | 90 | urea | 15.5 |
| Example 1-108 | NMA | 0.1 | NNDMA | 90 | HDA | 10 | DBA | 90 | urea | 15.5 |
| Example 1-109 | NMA | 0.0001 | NNDMA | 100 | HDA | 10 | DBA | 90 | urea | 15.5 |

**[Table 1-4]**

| | Secondary amine compound (I) | | Tertiary amine compound (II) | | Primary amine compound | | Blocking agent | | Carbonic acid derivative | |
|---|---|---|---|---|---|---|---|---|---|---|
| | type | parts by mass | type | parts by mass | type | parts by mass | type | parts by mass | type | parts by mass |
| Example | NMA | 1 | - | 0 | HDA | 10 | PhOH | 90 | urea | 15.5 |
| 1-110 | | | | | | | | | | |
| Example 1-111 | NMA | 1 | - | 0 | HDA | 10 | PhOH | 90 | urea | 15.5 |
| Example 1-112 | NMA | 1 | - | 0 | HDA | 10 | PhOH | 90 | urea | 15.5 |
| Example 1-113 | NMA | 1 | - | 0 | HDA | 10 | PhOH | 90 | urea | 15.5 |
| Example 1-114 | NMA | 1 | - | 0 | HDA | 10 | PhOH | 90 | urea | 15.5 |
| Example 1-115 | NMA | 1 | - | 0 | HDA | 10 | PhOH | 90 | urea | 15.5 |
| Example 1-116 | - | 0 | NNDMA | 1 | HDA | 10 | PhOH | 90 | urea | 15.5 |
| Example 1-117 | - | 0 | NNDMA | 1 | HDA | 10 | PhOH | 90 | urea | 15.5 |
| Example 1-118 | - | 0 | NNDMA | 1 | HDA | 10 | PhOH | 90 | urea | 15.5 |
| Example 1-119 | - | 0 | NNDMA | 1 | HDA | 10 | PhOH | 90 | urea | 15.5 |
| Example 1-120 | - | 0 | NNDMA | 1 | HDA | 10 | PhOH | 90 | urea | 15.5 |
| Example 1-121 | - | 0 | NNDMA | 1 | HDA | 10 | PhOH | 90 | urea | 15.5 |
| Example 1-122 | NMA | 1 | - | 0 | HDA | 10 | PhOH | 90 | urea | 15.5 |
| Example 1-123 | NMA | 1 | - | 0 | HDA | 10 | PhOH | 90 | urea | 15.5 |
| Example 1-124 | - | 0 | NNDMA | 1 | HDA | 10 | PhOH | 90 | urea | 15.5 |
| Example 1-125 | - | 0 | NNDMA | 1 | HDA | 10 | PhOH | 90 | urea | 15.5 |
| Comparative Example 1-1 | - | 0 | - | 0 | HDA | 10 | PhOH | 90 | urea | 15.5 |
| Comparative Example 1-2 | - | 0 | - | 0 | HDA | 10 | o-cresol | 90 | urea | 15.5 |
| Comparative Example 1-3 | - | 0 | - | 0 | HDA | 10 | m-cresol | 90 | urea | 15.5 |
| Comparative Example 1-4 | - | 0 | - | 0 | HDA | 10 | p-cresol | 90 | urea | 15.5 |
| Comparative Example 1-5 | - | 0 | - | 0 | HDA | 10 | OCP | 90 | urea | 15.5 |
| Comparative Example 1-6 | - | 0 | - | 0 | HDA | 10 | n-BuOH | 90 | urea | 15.5 |
| Comparative Example 1-7 | - | 0 | - | 0 | HDA | 10 | DBA | 90 | urea | 15.5 |
| Comparative Example 1-8 | - | 0 | - | 0 | IPDA | 10 | PhOH | 90 | urea | 10.6 |
| Comparative Example 1-9 | - | 0 | - | 0 | MBCA | 10 | PhOH | 90 | urea | 8.6 |
| Comparative Example 1-10 | - | 0 | - | 0 | DAT | 10 | PhOH | 90 | urea | 14.7 |
| Comparative Example 1-11 | - | 0 | - | 0 | DPM | 10 | PhOH | 90 | urea | 9.1 |

**[Table 1-5]**

| | Reaction temperature | Absolute pressure | Condenser tube temperature | Reaction time | Oxygen concentration |
|---|---|---|---|---|---|
| | °C | kPa | °C | hours | vol% |
| Example 1-1 | 240 | 405 | 60 | 2 | 0.1 |
| Example 1-2 | 240 | 405 | 60 | 2 | 0.1 |
| Example 1-3 | 240 | 405 | 60 | 2 | 0.1 |
| Example 1-4 | 240 | 405 | 60 | 2 | 0.1 |
| Example 1-5 | 240 | 405 | 60 | 2 | 0.1 |
| Example 1-6 | 240 | 405 | 60 | 2 | 0.1 |
| Example 1-7 | 240 | 405 | 60 | 2 | 0.1 |
| Example 1-8 | 240 | 405 | 60 | 2 | 0.1 |
| Example 1-9 | 240 | 405 | 60 | 2 | 0.1 |
| Example 1-10 | 240 | 405 | 60 | 2 | 0.1 |
| Example 1-11 | 240 | 405 | 60 | 2 | 0.1 |
| Example 1-12 | 240 | 405 | 60 | 2 | 0.1 |
| Example 1-13 | 240 | 405 | 60 | 2 | 0.1 |
| Example 1-14 | 240 | 405 | 60 | 2 | 0.1 |
| Example 1-15 | 240 | 405 | 60 | 2 | 0.1 |
| Example 1-16 | 240 | 405 | 60 | 2 | 0.1 |
| Example 1-17 | 240 | 405 | 60 | 2 | 0.1 |
| Example 1-18 | 240 | 405 | 60 | 2 | 0.1 |
| Example 1-19 | 240 | 405 | 60 | 2 | 0.1 |
| Example 1-20 | 240 | 405 | 60 | 2 | 0.1 |
| Example 1-21 | 240 | 405 | 60 | 2 | 0.1 |
| Example 1-22 | 240 | 405 | 60 | 2 | 0.1 |
| Example 1-23 | 240 | 405 | 60 | 2 | 0.1 |
| Example 1-24 | 240 | 405 | 60 | 2 | 0.1 |
| Example 1-25 | 240 | 405 | 60 | 2 | 0.1 |
| Example 1-26 | 240 | 405 | 60 | 2 | 0.1 |
| Example 1-27 | 240 | 405 | 60 | 2 | 0.1 |
| Example 1-28 | 240 | 405 | 60 | 2 | 0.1 |
| Example 1-29 | 240 | 405 | 60 | 2 | 0.1 |
| Example 1-30 | 240 | 405 | 60 | 2 | 0.1 |
| Example 1-31 | 240 | 405 | 60 | 2 | 0.1 |
| Example 1-32 | 240 | 405 | 60 | 2 | 0.1 |
| Example 1-33 | 240 | 405 | 60 | 2 | 0.1 |
| Example 1-34 | 240 | 405 | 60 | 2 | 0.1 |
| Example 1-35 | 240 | 405 | 60 | 2 | 0.1 |

**[Table 1-6]**

| | Reaction temperature | Absolute pressure | Condenser tube temperature | Reaction time | Oxygen concentration |
|---|---|---|---|---|---|
| | °C | kPa | °C | hours | vol% |
| Example 1-36 | 240 | 405 | 60 | 2 | 0.1 |
| Example 1-37 | 240 | 405 | 60 | 2 | 0.1 |
| Example 1-38 | 240 | 405 | 60 | 2 | 0.1 |
| Example 1-39 | 240 | 405 | 60 | 2 | 0.1 |
| Example 1-40 | 240 | 405 | 60 | 2 | 0.1 |
| Example 1-41 | 240 | 405 | 60 | 2 | 0.1 |
| Example 1-42 | 240 | 405 | 60 | 2 | 0.1 |
| Example 1-43 | 240 | 405 | 60 | 2 | 0.1 |
| Example 1-44 | 240 | 405 | 60 | 2 | 0.1 |
| Example 1-45 | 240 | 405 | 60 | 2 | 0.1 |
| Example 1-46 | 240 | 405 | 60 | 2 | 0.1 |
| Example 1-47 | 240 | 405 | 60 | 2 | 0.1 |
| Example 1-48 | 240 | 405 | 60 | 2 | 0.1 |
| Example 1-49 | 240 | 405 | 60 | 2 | 0.1 |
| Example 1-50 | 240 | 405 | 60 | 2 | 0.1 |
| Example 1-51 | 240 | 405 | 60 | 2 | 0.1 |
| Example 1-52 | 240 | 405 | 60 | 2 | 0.1 |
| Example 1-53 | 240 | 405 | 60 | 2 | 0.1 |
| Example 1-54 | 240 | 405 | 60 | 2 | 0.1 |
| Example 1-55 | 240 | 405 | 60 | 2 | 0.1 |
| Example 1-56 | 240 | 405 | 60 | 2 | 0.1 |
| Example 1-57 | 240 | 405 | 60 | 2 | 0.1 |
| Example 1-58 | 240 | 405 | 60 | 2 | 0.1 |
| Example 1-59 | 240 | 405 | 60 | 2 | 0.1 |
| Example 1-60 | 240 | 405 | 60 | 2 | 0.1 |
| Example 1-61 | 240 | 405 | 60 | 2 | 0.1 |
| Example 1-62 | 240 | 405 | 60 | 2 | 0.1 |
| Example 1-63 | 240 | 405 | 60 | 2 | 0.1 |
| Example 1-64 | 240 | 405 | 60 | 2 | 0.1 |
| Example 1-65 | 240 | 405 | 60 | 2 | 0.1 |
| Example 1-66 | 240 | 405 | 60 | 2 | 0.1 |
| Example 1-67 | 240 | 405 | 60 | 2 | 0.1 |
| Example 1-68 | 240 | 405 | 60 | 2 | 0.1 |
| Example 1-69 | 240 | 405 | 60 | 2 | 0.1 |

**[Table 1-7]**

| | Reaction temperature | Absolute pressure | Condenser tube temperature | Reaction time | Oxygen concentration |
|---|---|---|---|---|---|
| | °C | kPa | °C | hours | vol% |
| Example 1-70 | 240 | 405 | 60 | 2 | 0.1 |
| Example 1-71 | 240 | 405 | 60 | 2 | 0.1 |
| Example 1-72 | 240 | 405 | 60 | 2 | 0.1 |
| Example 1-73 | 240 | 405 | 60 | 2 | 0.1 |
| Example 1-74 | 240 | 405 | 60 | 2 | 0.1 |
| Example 1-75 | 240 | 405 | 60 | 2 | 0.1 |
| Example 1-76 | 240 | 405 | 60 | 2 | 0.1 |
| Example 1-77 | 240 | 405 | 60 | 2 | 0.1 |
| Example 1-78 | 240 | 405 | 60 | 2 | 0.1 |
| Example 1-79 | 240 | 405 | 60 | 2 | 0.1 |
| Example 1-80 | 240 | 405 | 60 | 2 | 0.1 |
| Example 1-81 | 240 | 405 | 60 | 2 | 0.1 |
| Example 1-82 | 240 | 405 | 60 | 2 | 0.1 |
| Example 1-83 | 240 | 405 | 60 | 2 | 0.1 |
| Example 1-84 | 240 | 405 | 60 | 2 | 0.1 |
| Example 1-85 | 240 | 405 | 60 | 2 | 0.1 |
| Example 1-86 | 240 | 405 | 60 | 2 | 0.1 |
| Example 1-87 | 240 | 405 | 60 | 2 | 0.1 |
| Example 1-88 | 240 | 405 | 60 | 2 | 0.1 |
| Example 1-89 | 240 | 405 | 60 | 2 | 0.1 |
| Example 1-90 | 240 | 405 | 60 | 2 | 0.1 |
| Example 1-91 | 240 | 405 | 60 | 2 | 0.1 |
| Example 1-92 | 240 | 405 | 60 | 2 | 0.1 |
| Example 1-93 | 240 | 405 | 60 | 2 | 0.1 |
| Example 1-94 | 240 | 405 | 60 | 2 | 0.1 |
| Example 1-95 | 240 | 405 | 60 | 2 | 0.1 |
| Example 1-96 | 240 | 405 | 60 | 2 | 0.1 |
| Example 1-97 | 240 | 405 | 60 | 2 | 0.1 |
| Example 1-98 | 240 | 405 | 60 | 2 | 0.1 |
| Example 1-99 | 240 | 405 | 60 | 2 | 0.1 |
| Example 1-100 | 240 | 405 | 60 | 2 | 0.1 |
| Example 1-101 | 240 | 405 | 60 | 2 | 0.1 |
| Example 1-102 | 240 | 405 | 60 | 2 | 0.1 |
| Example 1-103 | 240 | 405 | 60 | 2 | 0.1 |
| Example 1-104 | 240 | 405 | 60 | 2 | 0.1 |
| Example 1-105 | 240 | 405 | 60 | 2 | 0.1 |
| Example 1-106 | 240 | 405 | 60 | 2 | 0.1 |
| Example 1-107 | 240 | 405 | 60 | 2 | 0.1 |
| Example 1-108 | 240 | 405 | 60 | 2 | 0.1 |
| Example 1-109 | 240 | 405 | 60 | 2 | 0.1 |

**[Table 1-8]**

| | Reaction temperature | Absolute pressure | Condenser tube temperature | Reaction time | Oxygen concentration |
|---|---|---|---|---|---|
| | °C | kPa | °C | hours | vol% |
| Example 1-110 | 160 | 405 | 60 | 2 | 0.1 |
| Example 1-111 | 180 | 405 | 60 | 2 | 0.1 |
| Example 1-112 | 200 | 405 | 60 | 2 | 0.1 |
| Example 1-113 | 220 | 405 | 60 | 2 | 0.1 |
| Example 1-114 | 230 | 405 | 60 | 2 | 0.1 |
| Example 1-115 | 260 | 405 | 60 | 2 | 0.1 |
| Example 1-116 | 160 | 405 | 60 | 2 | 0.1 |
| Example 1-117 | 180 | 405 | 60 | 2 | 0.1 |
| Example 1-118 | 200 | 405 | 60 | 2 | 0.1 |
| Example 1-119 | 220 | 405 | 60 | 2 | 0.1 |
| Example 1-120 | 230 | 405 | 60 | 2 | 0.1 |
| Example 1-121 | 260 | 405 | 60 | 2 | 0.1 |
| Example 1-122 | 240 | 405 | 60 | 2 | 1 |
| Example 1-123 | 240 | 405 | 60 | 2 | 0.01 |
| Example 1-124 | 240 | 405 | 60 | 2 | 1 |
| Example 1-125 | 240 | 405 | 60 | 2 | 0.01 |
| Comparative Example 1-1 | 240 | 405 | 60 | 2 | 0.1 |
| Comparative Example 1-2 | 240 | 405 | 60 | 2 | 0.1 |
| Comparative Example 1-3 | 240 | 405 | 60 | 2 | 0.1 |
| Comparative Example 1-4 | 240 | 405 | 60 | 2 | 0.1 |
| Comparative Example 1-5 | 240 | 405 | 60 | 2 | 0.1 |
| Comparative Example 1-6 | 240 | 405 | 60 | 2 | 0.1 |
| Comparative Example 1-7 | 240 | 405 | 60 | 2 | 0.1 |
| Comparative Example 1-8 | 240 | 405 | 60 | 2 | 0.1 |
| Comparative Example 1-9 | 240 | 405 | 60 | 2 | 0.1 |
| Comparative Example 1-10 | 240 | 405 | 60 | 2 | 0.1 |
| Comparative Example 1-11 | 240 | 405 | 60 | 2 | 0.1 |

**[Table 1-9]**

| | Reaction liquid | | | | | |
|---|---|---|---|---|---|---|
| | parts by mass | Amino groups | NCO-BL | NCO-compound (I) | MB | Hazen color number |
| | | mol% | mol% | mol% | mol% | APHA |
| Example 1-1 | 103.3 | 0 | 84 | 5 | 89 | 3 |
| Example 1-2 | 103.3 | 0 | 86 | 5 | 91 | 3 |
| Example 1-3 | 103.3 | 0 | 87 | 4 | 91 | 3 |
| Example 1-4 | 103.3 | 0 | 86 | 3 | 89 | 3 |
| Example 1-5 | 103.3 | 0 | 90 | 0 | 90 | 4 |
| Example 1-6 | 103.3 | 0 | 91 | 0 | 91 | 4 |
| Example 1-7 | 103.3 | 0 | 91 | 0 | 91 | 4 |
| Example 1-8 | 103.3 | 0 | 90 | 0 | 90 | 4 |
| Example 1-9 | 103.3 | 0 | 80 | 5 | 85 | 3 |
| Example 1-10 | 103.3 | 0 | 85 | 5 | 90 | 3 |
| Example 1-11 | 103.3 | 0 | 84 | 5 | 89 | 3 |
| Example 1-12 | 103.3 | 0 | 81 | 5 | 86 | 3 |
| Example 1-13 | 103.3 | 0 | 90 | 5 | 95 | 4 |
| Example 1-14 | 103.3 | 0 | 81 | 1 | 82 | 5 |
| Example 1-15 | 102.6 | 0 | 83 | 7 | 90 | 3 |
| Example 1-16 | 102.6 | 0 | 79 | 7 | 86 | 3 |
| Example 1-17 | 102.6 | 0 | 83 | 7 | 90 | 3 |
| Example 1-18 | 102.6 | 0 | 84 | 7 | 91 | 3 |
| Example 1-19 | 102.6 | 0 | 77 | 7 | 84 | 3 |
| Example 1-20 | 102.6 | 0 | 88 | 7 | 95 | 4 |
| Example 1-21 | 102.6 | 0 | 79 | 2 | 81 | 5 |
| Example 1-22 | 102.3 | 0 | 80 | 9 | 89 | 3 |
| Example 1-23 | 102.3 | 0 | 76 | 9 | 85 | 3 |
| Example 1-24 | 102.3 | 0 | 81 | 9 | 90 | 3 |
| Example 1-25 | 102.3 | 0 | 81 | 9 | 90 | 3 |
| Example 1-26 | 102.3 | 0 | 76 | 9 | 84 | 3 |
| Example 1-27 | 102.3 | 0 | 86 | 9 | 95 | 4 |
| Example 1-28 | 102.3 | 0 | 77 | 1 | 78 | 5 |
| Example 1-29 | 103.2 | 0 | 84 | 5 | 89 | 4 |
| Example 1-30 | 103.2 | 0 | 79 | 5 | 84 | 4 |
| Example 1-31 | 103.2 | 0 | 84 | 5 | 89 | 4 |
| Example 1-32 | 103.2 | 0 | 85 | 5 | 90 | 4 |
| Example 1-33 | 103.2 | 0 | 80 | 5 | 85 | 4 |
| Example 1-34 | 103.2 | 0 | 90 | 5 | 95 | 5 |
| Example 1-35 | 103.2 | 0 | 81 | 1 | 82 | 6 |

**[Table 1-10]**

| | Reaction liquid | | | | | |
|---|---|---|---|---|---|---|
| | parts by mass | Amino groups | NCO-BL | NCO-compound (I) | MB | Hazen color number |
| | | mol% | mol% | mol% | mol% | APHA |
| Example 1-36 | 102.4 | 0 | 81 | 8 | 89 | 3 |
| Example 1-37 | 102.4 | 0 | 78 | 8 | 86 | 3 |
| Example 1-38 | 102.4 | 0 | 82 | 8 | 90 | 3 |
| Example 1-39 | 102.4 | 0 | 82 | 8 | 91 | 3 |
| Example 1-40 | 102.4 | 0 | 78 | 8 | 86 | 3 |
| Example 1-41 | 102.4 | 0 | 88 | 8 | 96 | 4 |
| Example 1-42 | 102.4 | 0 | 78 | 2 | 80 | 5 |
| Example 1-43 | 103.3 | 0 | 86 | 0 | 86 | 4 |
| Example 1-44 | 103.3 | 0 | 90 | 0 | 90 | 4 |
| Example 1-45 | 103.3 | 0 | 91 | 0 | 91 | 4 |
| Example 1-46 | 103.3 | 0 | 85 | 0 | 85 | 4 |
| Example 1-47 | 103.3 | 0 | 95 | 0 | 95 | 5 |
| Example 1-48 | 103.3 | 0 | 86 | 0 | 86 | 6 |
| Example 1-49 | 102.6 | 0 | 89 | 0 | 89 | 4 |
| Example 1-50 | 102.6 | 0 | 86 | 0 | 86 | 4 |
| Example 1-51 | 102.6 | 0 | 90 | 0 | 90 | 4 |
| Example 1-52 | 102.6 | 0 | 91 | 0 | 91 | 4 |
| Example 1-53 | 102.6 | 0 | 84 | 0 | 84 | 4 |
| Example 1-54 | 102.6 | 0 | 94 | 0 | 94 | 5 |
| Example 1-55 | 102.6 | 0 | 85 | 0 | 85 | 6 |
| Example 1-56 | 102.3 | 0 | 90 | 0 | 90 | 4 |
| Example 1-57 | 102.3 | 0 | 84 | 0 | 84 | 4 |
| Example 1-58 | 102.3 | 0 | 90 | 0 | 90 | 4 |
| Example 1-59 | 102.3 | 0 | 90 | 0 | 90 | 4 |
| Example 1-60 | 102.3 | 0 | 84 | 0 | 84 | 4 |
| Example 1-61 | 102.3 | 0 | 95 | 0 | 95 | 5 |
| Example 1-62 | 102.3 | 0 | 85 | 0 | 85 | 6 |
| Example 1-63 | 103.2 | 0 | 91 | 0 | 91 | 5 |
| Example 1-64 | 103.2 | 0 | 85 | 0 | 85 | 5 |
| Example 1-65 | 103.2 | 0 | 89 | 0 | 89 | 5 |
| Example 1-66 | 103.2 | 0 | 91 | 0 | 91 | 5 |
| Example 1-67 | 103.2 | 0 | 85 | 0 | 85 | 5 |
| Example 1-68 | 103.2 | 0 | 95 | 0 | 95 | 6 |
| Example 1-69 | 103.2 | 0 | 85 | 0 | 85 | 7 |

**[Table 1-11]**

| | Reaction liquid | | | | | |
|---|---|---|---|---|---|---|
| | parts by mass | Amino groups | NCO-BL | NCO-compound (I) | MB | Hazen color number |
| | | mol% | mol% | mol% | mol% | APHA |
| Example 1-70 | 102.4 | 0 | 91 | 0 | 91 | 4 |
| Example 1-71 | 102.4 | 0 | 84 | 0 | 84 | 4 |
| Example 1-72 | 102.4 | 0 | 91 | 0 | 91 | 4 |
| Example 1-73 | 102.4 | 0 | 89 | 0 | 89 | 4 |
| Example 1-74 | 102.4 | 0 | 84 | 0 | 84 | 4 |
| Example 1-75 | 102.4 | 0 | 95 | 0 | 95 | 5 |
| Example 1-76 | 102.4 | 0 | 85 | 0 | 85 | 6 |
| Example 1-77 | 102.3 | 0 | 90 | 0 | 90 | 4 |
| Example 1-78 | 102.3 | 0 | 91 | 0 | 91 | 5 |
| Example 1-79 | 202.3 | 0 | 90 | 1 | 91 | 3 |
| Example 1-80 | 202.3 | 0 | 90 | 0 | 90 | 4 |
| Example 1-81 | 104.3 | 0 | 85 | 5 | 90 | 1 |
| Example 1-82 | 103.3 | 0 | 85 | 5 | 90 | 2 |
| Example 1-83 | 103.3 | 0 | 91 | 0 | 91 | 2 |
| Example 1-84 | 202.3 | 0 | 1 | 79 | 80 | 1 |
| Example 1-85 | 192.4 | 0 | 4 | 75 | 79 | 1 |
| Example 1-86 | 202.3 | 0 | 9 | 70 | 79 | 1 |
| Example 1-87 | 202.3 | 0 | 15 | 65 | 80 | 1 |
| Example 1-88 | 202.3 | 0 | 49 | 30 | 79 | 1 |
| Example 1-89 | 202.3 | 0 | 61 | 20 | 81 | 1 |
| Example 1-90 | 192.4 | 0 | 79 | 1 | 80 | 1 |
| Example 1-91 | 202.3 | 0 | 90 | 0 | 90 | 2 |
| Example 1-92 | 103.3 | 0 | 90 | 0 | 90 | 2 |
| Example 1-93 | 202.3 | 0 | 10 | 80 | 90 | 2 |
| Example 1-94 | 192.4 | 0 | 15 | 75 | 90 | 1 |
| Example 1-95 | 202.3 | 0 | 19 | 70 | 89 | 1 |
| Example 1-96 | 202.3 | 0 | 24 | 65 | 89 | 1 |
| Example 1-97 | 202.3 | 0 | 60 | 30 | 90 | 1 |
| Example 1-98 | 202.3 | 0 | 70 | 20 | 90 | 1 |
| Example 1-99 | 192.4 | 0 | 89 | 0 | 89 | 1 |
| Example 1-100 | 202.3 | 0 | 90 | 0 | 90 | 2 |
| Example 1-101 | 103.3 | 0 | 89 | 0 | 89 | 2 |
| Example 1-102 | 202.3 | 0 | 59 | 30 | 89 | 2 |
| Example 1-103 | 192.4 | 0 | 64 | 25 | 89 | 2 |
| Example 1-104 | 202.3 | 0 | 70 | 20 | 90 | 2 |
| Example 1-105 | 202.3 | 0 | 80 | 10 | 90 | 2 |
| Example 1-106 | 202.3 | 0 | 84 | 5 | 89 | 2 |
| Example 1-107 | 202.3 | 0 | 88 | 2 | 90 | 2 |
| Example 1-108 | 192.4 | 0 | 88 | 1 | 89 | 2 |
| Example 1-109 | 202.3 | 0 | 89 | 0 | 89 | 2 |

**[Table 1-12]**

| | Reaction liquid | | | | | |
|---|---|---|---|---|---|---|
| | parts by mass | Amino groups | NCO-BL | NCO-compound (I) | MB | Hazen color number |
| | | mol% | mol% | mol% | mol% | APHA |
| Example 1-110 | 103.3 | 0 | 86 | 5 | 91 | 5 |
| Example 1-111 | 103.3 | 0 | 84 | 5 | 89 | 5 |
| Example 1-112 | 103.3 | 0 | 85 | 5 | 90 | 4 |
| Example 1-113 | 103.3 | 0 | 85 | 5 | 90 | 4 |
| Example 1-114 | 103.3 | 0 | 86 | 5 | 91 | 4 |
| Example 1-115 | 103.3 | 0 | 85 | 5 | 90 | 3 |
| Example 1-116 | 103.3 | 0 | 90 | 0 | 90 | 4 |
| Example 1-117 | 103.3 | 0 | 89 | 0 | 89 | 4 |
| Example 1-118 | 103.3 | 0 | 90 | 0 | 90 | 4 |
| Example 1-119 | 103.3 | 0 | 89 | 0 | 89 | 4 |
| Example 1-120 | 103.3 | 0 | 91 | 0 | 91 | 4 |
| Example 1-121 | 103.3 | 0 | 90 | 0 | 90 | 4 |
| Example 1-122 | 103.3 | 0 | 84 | 5 | 89 | 4 |
| Example 1-123 | 103.3 | 0 | 84 | 5 | 89 | 2 |
| Example 1-124 | 103.3 | 0 | 90 | 0 | 90 | 5 |
| Example 1-125 | 103.3 | 0 | 90 | 0 | 90 | 4 |
| Comparative Example 1-1 | 102.3 | 0 | 90 | 0 | 90 | 8 |
| Comparative Example 1-2 | 102.3 | 0 | 85 | 0 | 85 | 8 |
| Comparative Example 1-3 | 102.3 | 0 | 89 | 0 | 89 | 8 |
| Comparative Example 1-4 | 102.3 | 0 | 90 | 0 | 90 | 8 |
| Comparative Example 1-5 | 102.3 | 0 | 85 | 0 | 85 | 8 |
| Comparative Example 1-6 | 102.3 | 0 | 96 | 0 | 96 | 9 |
| Comparative Example 1-7 | 102.3 | 0 | 85 | 0 | 85 | 10 |
| Comparative Example 1-8 | 101.6 | 0 | 90 | 0 | 90 | 8 |
| Comparative Example 1-9 | 101.3 | 0 | 90 | 0 | 90 | 8 |
| Comparative Example 1-10 | 102.2 | 0 | 89 | 0 | 89 | 9 |
| Comparative Example 1-11 | 101.4 | 0 | 90 | 0 | 90 | 9 |

As shown in the above tables, the blocked isocyanate compositions produced by adding at least one compound selected from the group consisting of the secondary amine compounds (1) and the tertiary amine compounds (11) exhibited the Hazen color number (APHA) evaluated as rank 7 or lower, which was favorable.

In contrast, the blocked isocyanate compositions produced without adding the secondary amine compound (I) and the tertiary amine compound (II) exhibited the Hazen color numbers evaluated as rank 8 or higher, which were unfavorable.

Further, among the blocked isocyanate compositions obtained in the presence of the secondary amine compound (I) using a phenol-based, alcohol-based, or aliphatic amine-based blocking agent (Examples 1-2 and 1-9 to 1-42), the blocked isocyanate compositions obtained using a phenol-based blocking agent (Examples 1-2, 1-9 to 1-12, 1-15 to 1-19, 1-22 to 1-26, 1-29 to 1-33, and 1-36 to 1-40) exhibited the Hazen color numbers evaluated as the lowest rank, which were particularly favorable.

Further, among the blocked isocyanate compositions obtained in the presence of the tertiary amine compound (II) using a phenol-based, alcohol-based, or aliphatic amine-based blocking agent (Examples 1-6 and 1-43 to 1-76), the blocked isocyanate compositions obtained using a phenol-based blocking agent (Examples 1-6, 1-43 to 1-46, 1-49 to 1-53, 1-56 to 1-60, 1-63 to 1-67, and 1-70 to 1-74) exhibited the Hazen color numbers evaluated as the lowest rank, which were particularly favorable.

Furthermore, among the blocked isocyanate compositions in which the amounts added of the secondary amine compound (1) or the tertiary amine compound (II) were different (Examples 1-1, 1-5, and 1-77 to 1-80), the blocked isocyanate compositions in which the secondary amine compound (I) or the tertiary amine compound (II) was added in an amount of at least 1 ppm by mass relative to the total mass of the primary amine compound and the blocking agent (Examples 1-77 and 1-78) exhibited the Hazen color numbers evaluated as rank 5 or lower, which were favorable.

Further, among the blocked isocyanate compositions in which the amounts added of the secondary amine compound (I) or the tertiary amine compound (II) were different (Examples 1-1, 1-5, and 1-77 to 1-80), the blocked isocyanate compositions in which the secondary amine compound (I) or the tertiary amine compound (II) was added in an amount of at least 1% by mass relative to the total mass of the primary amine compound and the blocking agent (Examples 1-1, 1-5, 1-79 and 1-80) exhibited the Hazen color numbers evaluated as rank 4 or lower, which were particularly favorable.

Furthermore, in the case of the blocked isocyanate compositions to which both the secondary amine compound (I) and the tertiary amine compound (II) were added (Examples 1-81 to 1-109), the Hazen color numbers thereof were evaluated as rank 2 or lower, which were the most favorable.

Furthermore, comparison of blocked isocyanate compositions prepared at different reaction temperatures (Examples 1-1, and 1-110 to 1-115) revealed that the ranks of the Hazen color numbers of the blocked isocyanate compositions obtained by reaction in the presence of the secondary amine compound (I) depended on the reaction temperature and the coloration reduction effect was improved as the reaction temperature was increased.

In contrast, comparison of blocked isocyanate compositions prepared at different reaction temperatures (Examples 1-5, and 1-116 to 1-121) revealed that the ranks of the Hazen color numbers of the blocked isocyanate compositions obtained by reaction in the presence of the tertiary amine compound (II) did not significantly depend on the reaction temperature.

Further, comparison of blocked isocyanate compositions prepared under different oxygen concentrations (Examples 1-1, 1-122 and 1-123) revealed that the ranks of the Hazen color numbers of the blocked isocyanate compositions obtained by reaction in the presence of the secondary amine compound (I) depended on the oxygen concentration and the coloration reduction effect was improved as the oxygen concentration was decreased.

Furthermore, comparison of blocked isocyanate compositions prepared under different oxygen concentrations (Examples 1-5, 1-124 and 1-125) revealed that the ranks of the Hazen color numbers of the blocked isocyanate compositions obtained by reaction in the presence of the tertiary amine compound (II) depended on the oxygen concentration and the coloration reduction effect was improved as the oxygen concentration was decreased.

### <<Second Test>>

### [Physical Property 2-1]

### (Isocyanate Group Content (NCO%))

The isocyanate group content (NCO%) is the amount of isocyanate functional groups (% by mass) contained in the reaction liquid obtained by thermal decomposition of a blocked isocyanate compound. After the obtained reaction liquid was used as a measurement sample to neutralize isocyanate groups with excess 2 N amine, a back titration is conducted with 1 N hydrochloric acid to determine the isocyanate group content as a % by mass value.

### [Physical Property 2-2]

### (Isocyanate Group Yield (NCO mol%))

The isocyanate group yield (hereinafter sometimes referred to as "NCO mol%") in the reaction liquid obtained by thermal decomposition of a blocked isocyanate compound was determined in accordance with the formula shown below, from the molar amount of blocked isocyanate groups (hereinafter sometimes referred to as "NCO-BL") contained in the blocked isocyanate compound prior to thermal decomposition, which was calculated from the amount added of the blocked isocyanate compound, and the molar amount of isocyanate groups obtained following thermal decomposition. The molar amount of isocyanate groups was determined from the NCO% described above and the total mass of the isocyanate composition obtained by thermal decomposition.[NCO mol%] = (molar amount of isocyanate groups) × 100 / (molar amount of blocked isocyanate groups)

### [Physical Property 2-3]

### (Residual Percentage of Blocked Isocyanate Groups)

The residual percentage of blocked isocyanate groups (hereinafter sometimes referred to as "NCO-BL mol%") in the reaction liquid obtained by thermal decomposition of a blocked isocyanate compound was determined in accordance with the formula shown below, from the molar amount of blocked isocyanate groups (NCO-BL) contained in the blocked isocyanate compound prior to thermal decomposition, which was calculated from the amount added of the blocked isocyanate compound, and the molar amount of residual blocked isocyanate groups (NCO-BL) following the thermal decomposition. Each molar amount of blocked isocyanate groups (NCO-BL) was determined from the NCO-BL concentration determined from an NMR spectrum and the total mass of the isocyanate composition obtained by thermal decomposition.[NCO-BL mol%] = (molar amount of residual NCO-BL following thermal decomposition) × 100 / (molar amount of NCO-BL contained in blocked isocyanate compound prior to thermal decomposition)

### [Physical Property 2-4]

### (Proportion of Isocyanate Groups bonded to Secondary Amine Compound (I))

The proportion (mol%) of isocyanate groups bonded to the secondary amine compound (I) (hereinafter sometimes abbreviated as "NCO-compound (I)") within the obtained isocyanate composition (hereinafter sometimes abbreviated as "NCO-compound (I) mol%") was determined in accordance with the formula shown below, from the molar amount of blocked isocyanate groups (NCO-BL) contained in the blocked isocyanate compound prior to thermal decomposition, which was calculated from the amount added of the blocked isocyanate compound, and the molar amount of residual NCO-compound (I) following the thermal decomposition. The molar amount of NCO-compound (I) was determined from the concentration of NCO-compound (I) which was determined from an NMR spectrum and the total mass of the obtained isocyanate composition.[NCO-compound (I) mol%] = (molar amount of NCO-compound (I)) × 100 / (molar amount of NCO-BL contained in blocked isocyanate compound prior to thermal decomposition)

### [Physical Property 2-5]

### (Oxygen Concentration)

A synthesis gas for supply to the reaction apparatus was produced from a nitrogen cylinder and an oxygen cylinder, and the oxygen concentration (% by volume) of the synthesis gas was measured using a galvanic cell oxygen concentration meter.

### [Physical Property 2-6]

### (Concentrations of Blocking Agent, Secondary Amine Compound (I) and Tertiary Amine Compound (II) in TOP liquid)

The concentrations of the blocking agent, the secondary amine compound (1) and the tertiary amine compound (II) in the TOP liquid were quantified by gas chromatography.

### [Evaluation 2-1]

### (Mass Balance)

The sum, within the obtained isocyanate composition, of the yield of isocyanate groups (NCO mol%), the residual percentage of blocked isocyanate groups (NCO-BL mol%), and the proportion of isocyanate groups bonded to the secondary amine compound (I) (NCO-compound (1) mol%) was determined as the mass balance (hereinafter sometimes abbreviated as "MB mol%").

### [Evaluation 2-2]

### (Hazen Color Number)

The molar amount added of the blocked isocyanate compound was divided by the mass of the reaction liquid following thermal decomposition to determine the concentration (mmol/g) of skeletons derived from the blocked isocyanate compound contained within the reaction liquid following thermal decomposition. Subsequently, a solvent was added to the thermally decomposed reaction liquid based on the concentration (mmol/g) of skeletons derived from the blocked isocyanate compound contained within the reaction liquid following thermal decomposition so as to adjust the concentration (mmol/g) of skeletons derived from the blocked isocyanate compound to a value of 0.40 (mmol/g), thus preparing a Hazen color number measurement sample.

The thus obtained Hazen color number measurement sample was measured with a Hazen meter, and the obtained value was ranked in accordance with the following evaluation criteria.

### (Evaluation Criteria)

Rank 1: APHA of at least 0 but less than 5
Rank 2: APHA of at least 5 but less than 10
Rank 3: APHA of at least 10 but less than 15
Rank 4: APHA of at least 15 but less than 20
Rank 5: APHA of at least 20 but less than 25
Rank 6: APHA of at least 25 but less than 30
Rank 7: APHA of at least 30 but less than 35
Rank 8: APHA of at least 35 but less than 40
Rank 9: APHA of at least 40 but less than 45
Rank 10: APHA of at least 45 but less than 50

### <Production of Blocked Isocyanate Compounds>

### [Production Example 2-1]

### (Production of Blocked Isocyanate Compound BL-1)

20 parts by mass of hexamethylene diisocyanate and 80 parts by mass of phenol were mixed and reacted at 150°C for 20 hours to synthesize diphenylhexane-1,6-diyldicarbamate. Subsequently, excess phenol was removed by distillation under reduced pressure at 100°C and 1 Pa. The results of liquid chromatography (LC) analysis revealed that diphenylhexane-1,6-diyldicarbamate (hereinafter sometimes referred to as the "blocked isocyanate compound BL-1") having a purity of ≥ 99% by mass had been obtained.

### [Production Example 2-2]

### (Production of Blocked Isocyanate Compounds BL-2 to BL-40)

Bocked isocyanate compounds BL-2 to BL-40 were produced by mixing raw material isocyanate compounds and blocking agents by the same method as that described in Production Example 2-1 except that raw materials shown in the following tables were used. The purity of each samples was quantified by LC analysis. The results are shown in the tables below.

In the following tables, the abbreviations used refer to the following compounds.

### (Isocyanate Compounds)

HD1: 1,6-hexamethylene diisocyanate
IPDI: isophorone diisocyanate (isomeric mixture)
HMDI: 4,4'-methylenebis(cyclohexyl isocyanate) (isomeric mixture)
TDI: toluene diisocyanate (isomeric mixture)
MDI: 4,4'-diphenylmethane diisocyanate

### (Blocking Agents)

PhOH: phenol
o-cresol: ortho-cresol
m-cresol: meta-cresol
p-cresol: para-cresol
OCP: o-chlorophenol
n-BuOH: normal butanol
DEHA: diethylhydroxylamine
DBA: dibutylamine

**[Table 2-1]**

| Blocked isocyanate compound | Raw material isocyanate compound | Blocking agent | LC analysis purity |
|---|---|---|---|
| BL-1 | HDI | PhOH | ≥99% by mass |
| BL-2 | | o-cresol | ≥99% by mass |
| BL-3 | | m-cresol | ≥99% by mass |
| BL-4 | | p-cresol | ≥99% by mass |
| BL-5 | | OCP | ≥99% by mass |
| BL-6 | | n-BuOH | ≥99% by mass |
| BL-7 | | DEHA | ≥99% by mass |
| BL-8 | | DBA | ≥99% by mass |
| BL-9 | IPDI | PhOH | ≥99% by mass |
| BL-10 | | o-cresol | ≥99% by mass |
| BL-11 | | m-cresol | ≥99% by mass |
| BL-12 | | p-cresol | ≥99% by mass |
| BL-13 | | OCP | ≥99% by mass |
| BL-14 | | n-BuOH | ≥99% by mass |
| BL-15 | | DEHA | ≥99% by mass |
| BL-16 | | DBA | ≥99% by mass |
| BL-17 | HMDI | PhOH | ≥99% by mass |
| BL-18 | | o-cresol | ≥99% by mass |
| BL-19 | | m-cresol | ≥99% by mass |
| BL-20 | | p-cresol | ≥99% by mass |
| BL-21 | | OCP | ≥99% by mass |
| BL-22 | | n-BuOH | ≥99% by mass |
| BL-23 | | DEHA | ≥99% by mass |
| BL-24 | | DBA | ≥99% by mass |
| BL-25 | TDI | PhOH | ≥99% by mass |
| BL-26 | | o-cresol | ≥99% by mass |
| BL-27 | | m-cresol | ≥99% by mass |
| BL-28 | | p-cresol | ≥99% by mass |
| BL-29 | | OCP | ≥99% by mass |
| BL-30 | | n-BuOH | ≥99% by mass |
| BL-31 | | DEHA | ≥99% by mass |
| BL-32 | | DBA | ≥99% by mass |
| BL-33 | MDI | PhOH | ≥99% by mass |
| BL-34 | | o-cresol | ≥99% by mass |
| BL-35 | | m-cresol | ≥99% by mass |
| BL-36 | | p-cresol | ≥99% by mass |
| BL-37 | | OCP | ≥99% by mass |
| BL-38 | | n-BuOH | ≥99% by mass |
| BL-39 | | DEHA | ≥99% by mass |
| BL-40 | | DBA | ≥99% by mass |

### [Example 2-1]

20 parts by mass of the blocked isocyanate compound BL-1 described above, 79 parts by mass of dibenzyltoluene, and 1 part by mass of N-methylaniline were placed in a 300 mL glass vessel equipped with a condenser tube 1 packed with a filler (the condenser tube 1 having a structure that allows the condensed liquid from the condenser tube 1 to fall into the 300 mL glass vessel) and a condenser 2 (having a structure that allow the condensed liquid from the condenser tube 2 to be collected as a TOP liquid without entering the 300 mL glass vessel) equipped with a pressure reduction device at the tip thereof. Subsequently, the internal temperature of the 300 mL glass vessel was set to 250°C, the temperature of the condenser tube 1 was set to 165°C, and the temperature of the condenser tube 2 was set to 70°C. Further, a synthesis gas was produced from a nitrogen cylinder and an oxygen cylinder, and the oxygen concentration in the synthesis gas was confirmed to be 0.1 vol% using an oxygen concentration meter, followed by adjusting the absolute pressure inside the reaction vessel to 380 mmHg using the pressure reduction device while supplying the synthesis gas to the 300 mL glass vessel at a flow rate of 20 NmL/min to allow the reaction to proceed for two hours. As a result, 90.5 parts by mass of a thermally decomposed reaction liquid was obtained inside the 300 mL glass vessel, and 9.5 parts by mass of the TOP liquid were collected from the condenser tube 2. The result of calculating the NCO mol% from the NCO% of the obtained reaction liquid following thermal decomposition revealed an NCO mol% of 70 mol%, and the results determined from ¹H NMR integral values revealed an NCO-BL mol% of 10 mol%, NCO-compound (I) mol% of 8 mol%, and MB mol% of 88 mol%. On the other hand, the results of GC analysis revealed that the TOP liquid contained 100% by mass of PhOH. Further, the concentration of skeletons derived from the blocked isocyanate compound contained within the reaction liquid following the thermal decomposition was 0.620 mmol/g, and therefore the reaction liquid obtained following the thermal decomposition was diluted 1.55-fold with dibenzyltoluene to prepare a Hazen color number measurement sample having a concentration of skeletons derived from the blocked isocyanate compound of 0.40 mmol/g. The Hazen color number measurement sample was measured using a Hazen meter, and the obtained value was evaluated as rank 3 in accordance with the evaluation criteria mentioned above.

### [Examples 2-2 to 2-116 and Comparative Examples 2-1 to 2-12]

In each of Examples 2-2 to 2-116 and Comparative Examples 2-1 to 2-12, isocyanate compounds were produced using the same method as that described in Example 2-1, except that the raw materials and reaction conditions were altered as shown in the following tables.

In the following tables, the abbreviations used refer to the following compounds.

### (Secondary Amine Compounds (I))

NMA: N-methyl-aniline
NEA: N-ethyl-aniline
NBA: N-butyl-aniline
DPA: N,N-diphenylamine

### (Tertiary Amine Compounds (II))

NNDMA: N,N-dimethylaniline
NNDEA: N,N-diethylaniline
NMDPA: N-methyl-N.N-diphenylamine
TPA: triphenylamine

### (Blocked Isocyanate Compound)

BL-NCO: blocked isocyanate compound

### (Solvent)

DBT: dibenzyltoluene

**[Table 2-2]**

| | Secondary amine compound (I) | | Tertiary amine compound (II) | | | BL-NCO | | Solvent | |
|---|---|---|---|---|---|---|---|---|---|
| | type | parts by mass | type | Mw | parts by mass | type | parts by mass | type | parts by mass |
| Example 2-1 | NMA | 1 | - | - | 0 | BL-1 | 20 | DBT | 79 |
| Example 2-2 | NEA | 1 | - | - | 0 | BL-1 | 20 | DBT | 79 |
| Example 2-3 | NBA | 1 | - | - | 0 | BL-1 | 20 | DBT | 79 |
| Example 2-4 | DPA | 1 | - | - | 0 | BL-1 | 20 | DBT | 79 |
| Example 2-5 | - | 0 | NNDMA | 121.18 | 1 | BL-1 | 20 | DBT | 79 |
| Example 2-6 | - | 0 | NNDEA | 149.24 | 1 | BL-1 | 20 | DBT | 79 |
| Example 2-7 | - | 0 | NMDPA | 183.25 | 1 | BL-1 | 20 | DBT | 79 |
| Example 2-8 | - | 0 | TPA | 245.33 | 1 | BL-1 | 20 | DBT | 79 |
| Example 2-9 | NEA | 1 | - | - | 0 | BL-2 | 20 | DBT | 79 |
| Example 2-10 | NEA | 1 | - | - | 0 | BL-3 | 20 | DBT | 79 |
| Example 2-11 | NEA | 1 | - | - | 0 | BL-4 | 20 | DBT | 79 |
| Example 2-12 | NEA | 1 | - | - | 0 | BL-5 | 20 | DBT | 79 |
| Example 2-13 | NEA | 1 | - | - | 0 | BL-6 | 20 | DBT | 79 |
| Example 2-14 | NEA | 1 | - | - | 0 | BL-7 | 20 | DBT | 79 |
| Example 2-15 | NEA | 1 | - | - | 0 | BL-8 | 20 | DBT | 79 |
| Example 2-16 | NEA | 1 | - | - | 0 | BL-9 | 20 | DBT | 79 |
| Example 2-17 | NEA | 1 | - | - | 0 | BL-10 | 20 | DBT | 79 |
| Example 2-18 | NEA | 1 | - | - | 0 | BL-11 | 20 | DBT | 79 |
| Example 2-19 | NEA | 1 | - | - | 0 | BL-12 | 20 | DBT | 79 |
| Example 2-20 | NEA | 1 | - | - | 0 | BL-13 | 20 | DBT | 79 |
| Example 2-2 | NEA | 1 | - | - | 0 | BL-14 | 20 | DBT | 79 |
| Example 2-22 | NEA | 1 | - | - | 0 | BL-15 | 20 | DBT | 79 |
| Example 2-23 | NEA | 1 | - | - | 0 | BL-16 | 20 | DBT | 79 |
| Example 2-24 | NEA | 1 | - | - | 0 | BL-17 | 20 | DBT | 79 |
| Example 2-25 | NEA | 1 | - | - | 0 | BL-18 | 20 | DBT | 79 |
| Example 2-26 | NEA | 1 | - | - | 0 | BL-19 | 20 | DBT | 79 |
| Example 2-27 | NEA | 1 | - | - | 0 | BL-20 | 20 | DBT | 79 |
| Example 2-28 | NEA | 1 | - | - | 0 | BL-21 | 20 | DBT | 79 |
| Example 2-29 | NEA | 1 | - | - | 0 | BL-22 | 20 | DBT | 79 |
| Example 2-30 | NEA | 1 | - | - | 0 | BL-23 | 20 | DBT | 79 |
| Example 2-31 | NEA | 1 | - | - | 0 | BL-24 | 20 | DBT | 79 |
| Example 2-32 | NEA | 1 | - | - | 0 | BL-25 | 20 | DBT | 79 |
| Example 2-33 | NEA | 1 | - | - | 0 | BL-26 | 20 | DBT | 79 |
| Example 2-34 | NEA | 1 | - | - | 0 | BL-27 | 20 | DBT | 79 |
| Example 2-35 | NEA | 1 | - | - | 0 | BL-28 | 20 | DBT | 79 |
| Example 2-36 | NEA | 1 | - | - | 0 | BL-29 | 20 | DBT | 79 |
| Example 2-37 | NEA | 1 | - | - | 0 | BL-30 | 20 | DBT | 79 |

**[Table 2-3]**

| | Secondary amine compound (I) | | Tertiary amine compound (II) | | | BL-NCO | | Solvent | |
|---|---|---|---|---|---|---|---|---|---|
| | type | parts by mass | type | Mw | parts by mass | type | parts by mass | type | parts by mass |
| Example 2-38 | NEA | 1 | - | - | 0 | BL-31 | 20 | DBT | 79 |
| Example 2-39 | NEA | 1 | - | - | 0 | BL-32 | 20 | DBT | 79 |
| Example 2-40 | NEA | 1 | - | - | 0 | BL-33 | 20 | DBT | 79 |
| Example 2-41 | NEA | 1 | - | - | 0 | BL-34 | 20 | DBT | 79 |
| Example 2-42 | NEA | 1 | - | - | 0 | BL-35 | 20 | DBT | 79 |
| Example 2-43 | NEA | 1 | - | - | 0 | BL-36 | 20 | DBT | 79 |
| Example 2-44 | NEA | 1 | - | - | 0 | BL-37 | 20 | DBT | 79 |
| Example 2-45 | NEA | 1 | - | - | 0 | BL-38 | 20 | DBT | 79 |
| Example 2-46 | NEA | 1 | - | - | 0 | BL-39 | 20 | DBT | 79 |
| Example 2-47 | NEA | 1 | - | - | 0 | BL-40 | 20 | DBT | 79 |
| Example 2-48 | - | 0 | NNDEA | 149.24 | 1 | BL-2 | 20 | DBT | 79 |
| Example 2-49 | - | 0 | NNDEA | 149.24 | 1 | BL-3 | 20 | DBT | 79 |
| Example 2-50 | - | 0 | NNDEA | 149.24 | 1 | BL-4 | 20 | DBT | 79 |
| Example 2-51 | - | 0 | NNDEA | 149.24 | 1 | BL-5 | 20 | DBT | 79 |
| Example 2-52 | - | 0 | NNDEA | 149.24 | 1 | BL-6 | 20 | DBT | 79 |
| Example 2-53 | - | 0 | NNDEA | 149.24 | 1 | BL-7 | 20 | DBT | 79 |
| Example 2-54 | - | 0 | NNDEA | 149.24 | 1 | BL-8 | 20 | DBT | 79 |
| Example 2-55 | - | 0 | NNDEA | 149.24 | 1 | BL-9 | 20 | DBT | 79 |
| Example 2-56 | - | 0 | NNDEA | 149.24 | 1 | BL-10 | 20 | DBT | 79 |
| Example 2-57 | - | 0 | NNDEA | 149.24 | 1 | BL-11 | 20 | DBT | 79 |
| Example 2-58 | - | 0 | NNDEA | 149.24 | 1 | BL-12 | 20 | DBT | 79 |
| Example 2-59 | - | 0 | NNDEA | 149.24 | 1 | BL-13 | 20 | DBT | 79 |
| Example 2-60 | - | 0 | NNDEA | 149.24 | 1 | BL-14 | 20 | DBT | 79 |
| Example 2-61 | - | 0 | NNDEA | 149.24 | 1 | BL-15 | 20 | DBT | 79 |
| Example 2-62 | - | 0 | NNDEA | 149.24 | 1 | BL-16 | 20 | DBT | 79 |
| Example 2-63 | - | 0 | NNDEA | 149.24 | 1 | BL-17 | 20 | DBT | 79 |
| Example 2-64 | - | 0 | NNDEA | 149.24 | 1 | BL-18 | 20 | DBT | 79 |
| Example 2-65 | - | 0 | NNDEA | 149.24 | 1 | BL-19 | 20 | DBT | 79 |
| Example 2-66 | - | 0 | NNDEA | 149.24 | 1 | BL-20 | 20 | DBT | 79 |
| Example 2-67 | - | 0 | NNDEA | 149.24 | 1 | BL-21 | 20 | DBT | 79 |
| Example 2-68 | - | 0 | NNDEA | 149.24 | 1 | BL-22 | 20 | DBT | 79 |
| Example 2-69 | - | 0 | NNDEA | 149.24 | 1 | BL-23 | 20 | DBT | 79 |
| Example 2-70 | - | 0 | NNDEA | 149.24 | 1 | BL-24 | 20 | DBT | 79 |
| Example 2-71 | - | 0 | NNDEA | 149.24 | 1 | BL-25 | 20 | DBT | 79 |
| Example 2-72 | - | 0 | NNDEA | 149.24 | 1 | BL-26 | 20 | DBT | 79 |

**[Table 2-4]**

| | Secondary amine compound (I) | | Tertiary amine compound (II) | | | BL-NCO | | Solvent | |
|---|---|---|---|---|---|---|---|---|---|
| | type | parts by mass | type | Mw | parts by mass | type | parts by mass | type | parts by mass |
| Example 2-73 | - | 0 | NNDEA | 149.24 | 1 | BL-27 | 20 | DBT | 79 |
| Example 2-74 | - | 0 | NNDEA | 149.24 | 1 | BL-28 | 20 | DBT | 79 |
| Example 2-75 | - | 0 | NNDEA | 149.24 | 1 | BL-29 | 20 | DBT | 79 |
| Example 2-76 | - | 0 | NNDEA | 149.24 | 1 | BL-30 | 20 | DBT | 79 |
| Example 2-77 | | 0 | NNDEA | 149.24 | 1 | BL-31 | 20 | DBT | 79 |
| Example 2-78 | - | 0 | NNDEA | 149.24 | 1 | BL-32 | 20 | DBT | 79 |
| Example 2-79 | | 0 | NNDEA | 149.24 | 1 | BL-33 | 20 | DBT | 79 |
| Example 2-80 | - | 0 | NNDEA | 149.24 | 1 | BL-34 | 20 | DBT | 79 |
| Example 2-81 | - | 0 | NNDEA | 149.24 | 1 | BL-35 | 20 | DBT | 79 |
| Example 2-82 | - | 0 | NNDEA | 149.24 | 1 | BL-36 | 20 | DBT | 79 |
| Example 2-83 | - | 0 | NNDEA | 149.24 | 1 | BL-37 | 20 | DBT | 79 |
| Example 2-84 | - | 0 | NNDEA | 149.24 | 1 | BL-38 | 20 | DBT | 79 |
| Example 2-85 | - | 0 | NNDEA | 149.24 | 1 | BL-39 | 20 | DBT | 79 |
| Example 2-86 | - | 0 | NNDEA | 149.24 | 1 | BL-40 | 20 | DBT | 79 |
| Example 2-87 | NMA | 0.0001 | - | - | 0 | BL-1 | 20 | DBT | 80 |
| Example 2-88 | - | 0 | NNDMA | 121.18 | 0.0001 | BL-1 | 20 | DBT | 80 |
| Example 2-89 | NMA | 51 | - | - | 0 | BL-1 | 20 | DBT | 29 |
| Example 2-90 | - | 0 | NNDMA | 121.18 | 51 | BL-1 | 20 | DBT | 29 |
| Example 2-91 | NMA | 1 | NNDMA | 121.18 | 1 | BL-1 | 20 | DBT | 78 |
| Example 2-92 | NMA | 1 | NNDMA | 121.18 | 0.0001 | BL-1 | 20 | DBT | 79 |
| Example 2-93 | NMA | 0.0001 | NNDMA | 121.18 | 1 | BL-1 | 20 | DBT | 79 |
| Example 2-94 | NMA | 50 | NNDMA | 121.18 | 0.0001 | BL-1 | 20 | DBT | 30 |
| Example 2-95 | NMA | 49.5 | NNDMA | 121.18 | 0.5 | BL-1 | 20 | DBT | 30 |
| Example 2-96 | NMA | 45 | NNDMA | 121.18 | 5 | BL-1 | 20 | DBT | 30 |
| Example 2-97 | NMA | 25 | NNDMA | 121.18 | 25 | BL-1 | 20 | DBT | 30 |
| Example 2-98 | NMA | 5 | NNDMA | 121.18 | 45 | BL-1 | 20 | DBT | 30 |
| Example 2-99 | NMA | 0.5 | NNDMA | 121.18 | 49.5 | BL-1 | 20 | DBT | 30 |
| Example 2-100 | NMA | 0.0001 | NNDMA | 121.18 | 50 | BL-1 | 20 | DBT | 30 |

**[Table 2-5]**

| | Secondary amine compound (I) | | Tertiary amine compound (II) | | | BL-NCO | | Solvent | |
|---|---|---|---|---|---|---|---|---|---|
| | type | parts by mass | type | Mw | parts by mass | type | parts by mass | type | parts by mass |
| Example 2-101 | NMA | 1 | - | - | 0 | BL-1 | 20 | DBT | 79 |
| Example 2-102 | NMA | 1 | - | - | 0 | BL-1 | 20 | DBT | 79 |
| Example 2-103 | NMA | 1 | - | - | 0 | BL-1 | 20 | DBT | 79 |
| Example 2-104 | NMA | 1 | - | - | 0 | BL-1 | 20 | DBT | 79 |
| Example 2-105 | NMA | 1 | - | - | 0 | BL-1 | 20 | DBT | 79 |
| Example 2-106 | NMA | 1 | - | - | 0 | BL-1 | 20 | DBT | 79 |
| Example 2-107 | - | - | NNDMA | - | 1 | BL-1 | 20 | DBT | 79 |
| Example 2-108 | - | - | NNDMA | - | 1 | BL-1 | 20 | DBT | 79 |
| Example 2-109 | - | - | NNDMA | - | 1 | BL-1 | 20 | DBT | 79 |
| Example 2-110 | - | - | NNDMA | - | 1 | BL-1 | 20 | DBT | 79 |
| Example 2-111 | - | - | NNDMA | - | 1 | BL-1 | 20 | DBT | 79 |
| Example 2-112 | - | - | NNDMA | - | 1 | BL-1 | 20 | DBT | 79 |
| Example 2-113 | NMA | 1 | - | - | 0 | BL-1 | 20 | DBT | 79 |
| Example 2-114 | NMA | 1 | - | - | 0 | BL-1 | 20 | DBT | 79 |
| Example 2-115 | - | 0 | NNDMA | 121.18 | 1 | BL-1 | 20 | DBT | 79 |
| Example 2-116 | - | 0 | NNDMA | 121.18 | 1 | BL-1 | 20 | DBT | 79 |
| Comparative Example 2-1 | - | 0 | - | - | 0 | BL-1 | 20 | DBT | 80 |
| Comparative Example 2-2 | - | 0 | - | - | 0 | BL-2 | 20 | DBT | 80 |
| Comparative Example 2-3 | - | 0 | - | - | 0 | BL-3 | 20 | DBT | 80 |
| Comparative Example 2-4 | - | 0 | - | - | 0 | BL-4 | 20 | DBT | 80 |
| Comparative Example 2-5 | - | 0 | - | - | 0 | BL-5 | 20 | DBT | 80 |
| Comparative Example 2-6 | - | 0 | - | - | 0 | BL-6 | 20 | DBT | 80 |
| Comparative Example 2-7 | - | 0 | - | - | 0 | BL-7 | 20 | DBT | 80 |
| Comparative Example 2-8 | - | 0 | - | - | 0 | BL-8 | 20 | DBT | 80 |
| Comparative Example 2-9 | - | 0 | - | - | 0 | BL-10 | 20 | DBT | 80 |
| Comparative Example 2-10 | - | 0 | - | - | 0 | BL-19 | 20 | DBT | 80 |
| Comparative Example 2-1 | - | 0 | - | - | 0 | BL-28 | 20 | DBT | 80 |
| Comparative Example 2-12 | - | 0 | - | - | 0 | BL-37 | 20 | DBT | 80 |

**[Table 2-6]**

| | Reaction temperature | Reduced pressure | Condenser tube 1 temperature | Condenser tube 2 temperature | Reaction time | Oxygen concentration |
|---|---|---|---|---|---|---|
| | °C | mmHg | °C | °C | hours | vol% |
| Example 2-1 | 250 | 380 | 165 | 70 | 2 | 0.1 |
| Example 2-2 | 250 | 380 | 165 | 70 | 2 | 0.1 |
| Example 2-3 | 250 | 380 | 165 | 70 | 2 | 0.1 |
| Example 2-4 | 250 | 380 | 165 | 70 | 2 | 0.1 |
| Example 2-5 | 250 | 380 | 165 | 70 | 2 | 0.1 |
| Example 2-6 | 250 | 380 | 165 | 70 | 2 | 0.1 |
| Example 2-7 | 250 | 380 | 165 | 70 | 2 | 0.1 |
| Example 2-8 | 250 | 380 | 165 | 70 | 2 | 0.1 |
| Example 2-9 | 250 | 380 | 178 | 70 | 2 | 0.1 |
| Example 2-10 | 250 | 380 | 178 | 70 | 2 | 0.1 |
| Example 2-11 | 250 | 380 | 178 | 70 | 2 | 0.1 |
| Example 2-12 | 250 | 380 | 178 | 70 | 2 | 0.1 |
| Example 2-13 | 250 | 380 | 178 | 70 | 2 | 0.1 |
| Example 2-14 | 250 | 380 | 178 | 70 | 2 | 0.1 |
| Example 2-15 | 250 | 380 | 178 | 70 | 2 | 0.1 |
| Example 2-16 | 250 | 380 | 178 | 70 | 2 | 0.1 |
| Example 2-17 | 250 | 380 | 178 | 70 | 2 | 0.1 |
| Example 2-18 | 250 | 380 | 178 | 70 | 2 | 0.1 |
| Example 2-19 | 250 | 380 | 178 | 70 | 2 | 0.1 |
| Example 2-20 | 250 | 380 | 178 | 70 | 2 | 0.1 |
| Example 2-21 | 250 | 380 | 178 | 70 | 2 | 0.1 |
| Example 2-22 | 250 | 380 | 178 | 70 | 2 | 0.1 |
| Example 2-23 | 250 | 380 | 178 | 70 | 2 | 0.1 |
| Example 2-24 | 250 | 380 | 178 | 70 | 2 | 0.1 |
| Example 2-25 | 250 | 380 | 178 | 70 | 2 | 0.1 |
| Example 2-26 | 250 | 380 | 178 | 70 | 2 | 0.1 |
| Example 2-27 | 250 | 380 | 178 | 70 | 2 | 0.1 |
| Example 2-28 | 250 | 380 | 178 | 70 | 2 | 0.1 |
| Example 2-29 | 250 | 380 | 178 | 70 | 2 | 0.1 |
| Example 2-30 | 250 | 380 | 178 | 70 | 2 | 0.1 |
| Example 2-31 | 250 | 380 | 178 | 70 | 2 | 0.1 |
| Example 2-32 | 250 | 380 | 178 | 70 | 2 | 0.1 |
| Example 2-33 | 250 | 380 | 178 | 70 | 2 | 0.1 |
| Example 2-34 | 250 | 380 | 178 | 70 | 2 | 0.1 |
| Example 2-35 | 250 | 380 | 178 | 70 | 2 | 0.1 |
| Example 2-36 | 250 | 380 | 178 | 70 | 2 | 0.1 |
| Example 2-37 | 250 | 380 | 178 | 70 | 2 | 0.1 |

**[Table 2-7]**

| | Reaction temperature | Reduced pressure | Condenser tube 1 temperature | Condenser tube 2 temperature | Reaction time | Oxygen concentration |
|---|---|---|---|---|---|---|
| | °C | mmHg | °C | °C | hours | vol% |
| Example 2-38 | 250 | 380 | 178 | 70 | 2 | 0.1 |
| Example 2-39 | 250 | 380 | 178 | 70 | 2 | 0.1 |
| Example 2-40 | 250 | 380 | 178 | 70 | 2 | 0.1 |
| Example 2-41 | 250 | 380 | 178 | 70 | 2 | 0.1 |
| Example 2-42 | 250 | 380 | 178 | 70 | 2 | 0.1 |
| Example 2-43 | 250 | 380 | 178 | 70 | 2 | 0.1 |
| Example 2-44 | 250 | 380 | 178 | 70 | 2 | 0.1 |
| Example 2-45 | 250 | 380 | 178 | 70 | 2 | 0.1 |
| Example 2-46 | 250 | 380 | 178 | 70 | 2 | 0.1 |
| Example 2-47 | 250 | 380 | 178 | 70 | 2 | 0.1 |
| Example 2-48 | 250 | 380 | 178 | 70 | 2 | 0.1 |
| Example 2-49 | 250 | 380 | 178 | 70 | 2 | 0.1 |
| Example 2-50 | 250 | 380 | 178 | 70 | 2 | 0.1 |
| Example 2-51 | 250 | 380 | 178 | 70 | 2 | 0.1 |
| Example 2-52 | 250 | 380 | 178 | 70 | 2 | 0.1 |
| Example 2-53 | 250 | 380 | 178 | 70 | 2 | 0.1 |
| Example 2-54 | 250 | 380 | 178 | 70 | 2 | 0.1 |
| Example 2-55 | 250 | 380 | 178 | 70 | 2 | 0.1 |
| Example 2-56 | 250 | 380 | 178 | 70 | 2 | 0.1 |
| Example 2-57 | 250 | 380 | 178 | 70 | 2 | 0.1 |
| Example 2-58 | 250 | 380 | 178 | 70 | 2 | 0.1 |
| Example 2-59 | 250 | 380 | 178 | 70 | 2 | 0.1 |
| Example 2-60 | 250 | 380 | 178 | 70 | 2 | 0.1 |
| Example 2-6 | 250 | 380 | 178 | 70 | 2 | 0.1 |
| Example 2 | 250 | 380 | 178 | 70 | 2 | 0.1 |
| Example 2-63 | 250 | 380 | 178 | 70 | 2 | 0.1 |
| Example 2-64 | 250 | 380 | 178 | 70 | 2 | 0.1 |
| Example 2-65 | 250 | 380 | 178 | 70 | 2 | 0.1 |
| Example 2-66 | 250 | 380 | 178 | 70 | 2 | 0.1 |
| Example 2-67 | 250 | 380 | 178 | 70 | 2 | 0.1 |
| Example 2-68 | 250 | 380 | 178 | 70 | 2 | 0.1 |
| Example 2-69 | 250 | 380 | 178 | 70 | 2 | 0.1 |
| Example 2-70 | 250 | 380 | 178 | 70 | 2 | 0.1 |
| Example 2-71 | 250 | 380 | 178 | 70 | 2 | 0.1 |
| Example 2-72 | 250 | 380 | 178 | 70 | 2 | 0.1 |

**[Table 2-8]**

| | Reaction temperature | Reduced pressure | Condenser tube 1 temperature | Condenser tube 2 temperature | Reaction time | Oxygen concentration |
|---|---|---|---|---|---|---|
| | °C | mmHg | °C | °C | hours | vol% |
| Example 2-73 | 250 | 380 | 178 | 70 | 2 | 0.1 |
| Example 2-74 | 250 | 380 | 178 | 70 | 2 | 0.1 |
| Example 2-75 | 250 | 380 | 178 | 70 | 2 | 0.1 |
| Example 2-76 | 250 | 380 | 178 | 70 | 2 | 0.1 |
| Example 2-77 | 250 | 380 | 178 | 70 | 2 | 0.1 |
| Example 2-78 | 250 | 380 | 178 | 70 | 2 | 0.1 |
| Example 2-79 | 250 | 380 | 178 | 70 | 2 | 0.1 |
| Example 2-80 | 250 | 380 | 178 | 70 | 2 | 0.1 |
| Example 2-81 | 250 | 380 | 178 | 70 | 2 | 0.1 |
| Example 2-82 | 250 | 380 | 178 | 70 | 2 | 0.1 |
| Example 2-83 | 250 | 380 | 178 | 70 | 2 | 0.1 |
| Example 2-84 | 250 | 380 | 178 | 70 | 2 | 0.1 |
| Example 2-85 | 250 | 380 | 178 | 70 | 2 | 0.1 |
| Example 2-86 | 250 | 380 | 178 | 70 | 2 | 0.1 |
| Example 2-87 | 250 | 380 | 165 | 70 | 2 | 0.1 |
| Example 2-88 | 250 | 380 | 165 | 70 | 2 | 0.1 |
| Example 2-89 | 250 | 380 | 178 | 70 | 2 | 0.1 |
| Example 2-90 | 250 | 380 | 165 | 70 | 2 | 0.1 |
| Example 2-91 | 250 | 380 | 165 | 70 | 2 | 0.1 |
| Example 2-92 | 250 | 380 | 165 | 70 | 2 | 0.1 |
| Example 2-93 | 250 | 380 | 165 | 70 | 2 | 0.1 |
| Example 2-94 | 250 | 380 | 178 | 70 | 2 | 0.1 |
| Example 2-95 | 250 | 380 | 178 | 70 | 2 | 0.1 |
| Example 2-96 | 250 | 380 | 178 | 70 | 2 | 0.1 |
| Example 2-97 | 250 | 380 | 178 | 70 | 2 | 0.1 |
| Example 2-98 | 250 | 380 | 178 | 70 | 2 | 0.1 |
| Example 2-99 | 250 | 380 | 165 | 70 | 2 | 0.1 |
| Example 2-100 | 250 | 380 | 165 | 70 | 2 | 0.1 |

**[Table 2-9]**

| | Reaction temperature | Reduced pressure | Condenser tube 1 temperature | Condenser tube 2 temperature | Reaction time | Oxygen concentration |
|---|---|---|---|---|---|---|
| | °C | mmHg | °C | °C | hours | vol% |
| Example 2-101 | 160 | 380 | 165 | 70 | 2 | 0.1 |
| Example 2-102 | 180 | 380 | 165 | 70 | 2 | 0.1 |
| Example 2-103 | 200 | 380 | 165 | 70 | 2 | 0.1 |
| Example 2-104 | 220 | 380 | 165 | 70 | 2 | 0.1 |
| Example 2-105 | 240 | 380 | 165 | 70 | 2 | 0.1 |
| Example 2-106 | 260 | 380 | 165 | 70 | 2 | 0.1 |
| Example 2-107 | 160 | 380 | 165 | 70 | 2 | 0.1 |
| Example 2-108 | 180 | 380 | 165 | 70 | 2 | 0.1 |
| Example 2-109 | 200 | 380 | 165 | 70 | 2 | 0.1 |
| Example 2-110 | 220 | 380 | 165 | 70 | 2 | 0.1 |
| Example 2-111 | 240 | 380 | 165 | 70 | 2 | 0.1 |
| Example 2-112 | 260 | 380 | 165 | 70 | 2 | 0.1 |
| Example 2-113 | 250 | 380 | 165 | 70 | 2 | 1 |
| Example 2-114 | 250 | 380 | 165 | 70 | 2 | 0.01 |
| Example 2-115 | 250 | 380 | 165 | 70 | 2 | 1 |
| Example 2-116 | 250 | 380 | 165 | 70 | 2 | 0.01 |
| Comparative Example 2-1 | 250 | 380 | 165 | 70 | 2 | 0.1 |
| Comparative Example 2-2 | 250 | 380 | 178 | 70 | 2 | 0.1 |
| Comparative Example 2-3 | 250 | 380 | 178 | 70 | 2 | 0.1 |
| Comparative Example 2-4 | 250 | 380 | 178 | 70 | 2 | 0.1 |
| Comparative Example 2-5 | 250 | 380 | 178 | 70 | 2 | 0.1 |
| Comparative Example 2-6 | 250 | 380 | 178 | 70 | 2 | 0.1 |
| Comparative Example 2-7 | 250 | 380 | 178 | 70 | 2 | 0.1 |
| Comparative Example 2-8 | 250 | 380 | 178 | 70 | 2 | 0.1 |
| Comparative Example 2-9 | 250 | 380 | 165 | 70 | 2 | 0.1 |
| Comparative Example 2-10 | 250 | 380 | 165 | 70 | 2 | 0.1 |
| Comparative Example 2-11 | 250 | 380 | 165 | 70 | 2 | 0.1 |
| Comparative Example 2-12 | 250 | 380 | 165 | 70 | 2 | 0.1 |

**[Table 2-10]**

| | Reaction liquid following thermal decomposition | | | | | | TOP | | | |
|---|---|---|---|---|---|---|---|---|---|---|
| | parts by mass | NCO | NCO-BL | NCO-compound (I) | MB | Hazen color number | parts by mass | BL | Compound (I) | Compound (II) |
| | | mol% | mol% | mol% | mol% | APHA | | mass% | mass% | mass% |
| Example 2-1 | 90.5 | 70 | 10 | 8 | 88 | 3 | 9.5 | 100 | 0 | 0 |
| Example 2-2 | 90.5 | 70 | 10 | 7 | 88 | 3 | 9.5 | 100 | 0 | 0 |
| Example 2-3 | 90.5 | 70 | 10 | 6 | 86 | 3 | 9.5 | 100 | 0 | 0 |
| Example 2-4 | 90.4 | 71 | 9 | 5 | 85 | 3 | 9.6 | 100 | 0 | 0 |
| Example 2-5 | 90.6 | 80 | 11 | 0 | 91 | 4 | 9.4 | 100 | 0 | 0 |
| Example 2-6 | 90.6 | 80 | 11 | 0 | 91 | 4 | 9.4 | 100 | 0 | 0 |
| Example 2-7 | 90.5 | 80 | 10 | 0 | 90 | 4 | 9.5 | 100 | 0 | 0 |
| Example 2-8 | 90.6 | 80 | 11 | 0 | 91 | 4 | 9.4 | 100 | 0 | 0 |
| Example 2-9 | 89.6 | 81 | 7 | 8 | 96 | 3 | 10.4 | 100 | 0 | 0 |
| Example 2-10 | 90.2 | 70 | 13 | 8 | 91 | 3 | 9.8 | 100 | 0 | 0 |
| Example 2-11 | 90.1 | 71 | 12 | 8 | 90 | 3 | 9.9 | 100 | 0 | 0 |
| Example 2-12 | 88.1 | 85 | 1 | 9 | 95 | 3 | 11.9 | 100 | 0 | 0 |
| Example 2-13 | 93.8 | 50 | 33 | 7 | 90 | 4 | 6.2 | 100 | 0 | 0 |
| Example 2-14 | 90.5 | 80 | 8 | 7 | 96 | 4 | 9.5 | 100 | 0 | 0 |
| Example 2-15 | 89.3 | 50 | 12 | 9 | 71 | 5 | 10.7 | 100 | 0 | 0 |
| Example 2-16 | 93.0 | 57 | 24 | 8 | 89 | 3 | 7.0 | 100 | 0 | 0 |
| Example 2-17 | 92.3 | 64 | 22 | 9 | 95 | 3 | 7.7 | 100 | 0 | 0 |
| Example 2-18 | 92.4 | 57 | 23 | 9 | 89 | 3 | 7.6 | 100 | 0 | 0 |
| Example 2-19 | 92.5 | 56 | 24 | 9 | 90 | 3 | 7.5 | 100 | 0 | 0 |
| Example 2-20 | 91.1 | 68 | 17 | 10 | 95 | 3 | 8.9 | 100 | 0 | 0 |
| Example 2-21 | 95.2 | 40 | 41 | 8 | 88 | 4 | 4.8 | 100 | 0 | 0 |
| Example 2-22 | 93.0 | 64 | 21 | 8 | 94 | 4 | 7.0 | 100 | 0 | 0 |
| Example 2-23 | 91.4 | 40 | 20 | 10 | 70 | 5 | 8.6 | 100 | 0 | 0 |
| Example 2-24 | 94.2 | 49 | 31 | 9 | 90 | 3 | 5.8 | 100 | 0 | 0 |
| Example 2-25 | 93.6 | 57 | 29 | 10 | 95 | 3 | 6.4 | 100 | 0 | 0 |
| Example 2-26 | 93.8 | 49 | 32 | 10 | 91 | 3 | 6.2 | 100 | 0 | 0 |
| Example 2-27 | 93.8 | 49 | 31 | 10 | 90 | 3 | 6.2 | 100 | 0 | 0 |
| Example 2-28 | 92.6 | 60 | 25 | 11 | 96 | 3 | 7.4 | 100 | 0 | 0 |
| Example 2-29 | 96.0 | 35 | 45 | 8 | 89 | 4 | 4.0 | 100 | 0 | 0 |
| Example 2-30 | 94.3 | 56 | 29 | 9 | 94 | 4 | 5.7 | 100 | 0 | 0 |
| Example 2-31 | 92.6 | 36 | 25 | 11 | 72 | 5 | 7.4 | 100 | 0 | 0 |
| Example 2-32 | 90.1 | 64 | 5 | 7 | 76 | 4 | 9.9 | 100 | 0 | 0 |
| Example 2-33 | 89.2 | 73 | 3 | 8 | 83 | 4 | 10.8 | 100 | 0 | 0 |
| Example 2-34 | 89.4 | 64 | 4 | 8 | 76 | 4 | 10.6 | 100 | 0 | 0 |
| Example 2-35 | 89.5 | 63 | 5 | 8 | 77 | 4 | 10.5 | 100 | 0 | 0 |
| Example 2-36 | 88.0 | 77 | 0 | 9 | 86 | 4 | 12.0 | 100 | 0 | 0 |
| Example 2-37 | 92.1 | 46 | 14 | 7 | 67 | 5 | 7.9 | 100 | 0 | 0 |

**[Table 2-11]**

| | Reaction liquid following thermal decomposition | | | | | | TOP | | | |
|---|---|---|---|---|---|---|---|---|---|---|
| | parts by mass | NCO | NCO-BL | NCO-compound (I) | MB | Hazen color number | parts by mass | BL | Compound (I) | Compound (II) |
| | | mol% | mol% | mol% | mol% | APHA | | mass% | mass% | mass% |
| Example 2-38 | 90.2 | 72 | 3 | 7 | 83 | 5 | 9.8 | 100 | 0 | 0 |
| Example 2-39 | 88.7 | 46 | 5 | 9 | 60 | 6 | 11.3 | 100 | 0 | 0 |
| Example 2-40 | 91.9 | 63 | 6 | 9 | 78 | 3 | 8.1 | 100 | 0 | 0 |
| Example 2-41 | 91.0 | 73 | 2 | 10 | 85 | 3 | 9.0 | 100 | 0 | 0 |
| Example 2-42 | 91.1 | 64 | 4 | 10 | 78 | 3 | 8.9 | 100 | 0 | 0 |
| Example 2-43 | 91.2 | 64 | 5 | 10 | 78 | 3 | 8.8 | 100 | 0 | 0 |
| Example 2-44 | 89.9 | 77 | 0 | 10 | 87 | 3 | 10.1 | 100 | 0 | 0 |
| Example 2-45 | 93.7 | 46 | 15 | 8 | 69 | 4 | 6.3 | 100 | 0 | 0 |
| Example 2-46 | 91.9 | 73 | 2 | 9 | 84 | 4 | 8.1 | 100 | 0 | 0 |
| Example 2-47 | 90.4 | 45 | 5 | 11 | 61 | 5 | 9.6 | 100 | 0 | 0 |
| Example 2-48 | 89.4 | 90 | 5 | 0 | 96 | 4 | 10.6 | 100 | 0 | 0 |
| Example 2-49 | 89.9 | 81 | 10 | 0 | 91 | 4 | 10.1 | 100 | 0 | 0 |
| Example 2-50 | 89.9 | 81 | 10 | 0 | 91 | 4 | 10.1 | 100 | 0 | 0 |
| Example 2-51 | 87.9 | 96 | 0 | 0 | 96 | 4 | 12.1 | 100 | 0 | 0 |
| Example 2-52 | 93.4 | 61 | 30 | 0 | 91 | 5 | 6.6 | 100 | 0 | 0 |
| Example 2-53 | 90.2 | 90 | 5 | 0 | 95 | 5 | 9.8 | 100 | 0 | 0 |
| Example 2-54 | 89.1 | 60 | 10 | 0 | 70 | 6 | 10.9 | 100 | 0 | 0 |
| Example 2-55 | 93.0 | 65 | 23 | 0 | 89 | 4 | 7.0 | 100 | 0 | 0 |
| Example 2-56 | 92.3 | 73 | 22 | 0 | 95 | 4 | 7.7 | 100 | 0 | 0 |
| Example 2-57 | 92.6 | 65 | 25 | 0 | 90 | 4 | 7.4 | 100 | 0 | 0 |
| Example 2-58 | 92.6 | 65 | 25 | 0 | 90 | 4 | 7.4 | 100 | 0 | 0 |
| Example 2-59 | 91.0 | 79 | 16 | 0 | 95 | 4 | 9.0 | 100 | 0 | 0 |
| Example 2-60 | 95.2 | 48 | 40 | 0 | 88 | 5 | 4.8 | 100 | 0 | 0 |
| Example 2-61 | 93.0 | 73 | 21 | 0 | 94 | 5 | 7.0 | 100 | 0 | 0 |
| Example 2-62 | 91.3 | 51 | 19 | 0 | 70 | 6 | 8.7 | 100 | 0 | 0 |
| Example 2-63 | 94.3 | 59 | 32 | 0 | 90 | 4 | 5.7 | 100 | 0 | 0 |
| Example 2-64 | 93.6 | 66 | 29 | 0 | 95 | 4 | 6.4 | 100 | 0 | 0 |
| Example 2-65 | 93.7 | 60 | 31 | 0 | 91 | 4 | 6.3 | 100 | 0 | 0 |
| Example 2-66 | 93.7 | 59 | 31 | 0 | 90 | 4 | 6.3 | 100 | 0 | 0 |
| Example 2-67 | 92.7 | 71 | 26 | 0 | 96 | 4 | 7.3 | 100 | 0 | 0 |
| Example 2-68 | 96.0 | 44 | 44 | 0 | 89 | 5 | 4.0 | 100 | 0 | 0 |
| Example 2-69 | 94.2 | 66 | 28 | 0 | 94 | 5 | 5.8 | 100 | 0 | 0 |
| Example 2-70 | 92.5 | 46 | 25 | 0 | 71 | 6 | 7.5 | 100 | 0 | 0 |
| Example 2-71 | 90.2 | 71 | 6 | 0 | 76 | 5 | 9.8 | 100 | 0 | 0 |
| Example 2-72 | 89.3 | 80 | 3 | 0 | 84 | 5 | 10.7 | 100 | 0 | 0 |

**[Table 2-12]**

| | Reaction liquid following thermal decomposition | | | | | | TOP | | | |
|---|---|---|---|---|---|---|---|---|---|---|
| | parts by mass | NCO | NCO-BL | NCO-compound (I) | MB | Hazen color number | parts by mass | BL | Compound (I) | Compound (II) |
| | | mol% | mol% | mol% | mol% | APHA | | mass% | mass% | mass% |
| Example 2-73 | 89.6 | 71 | 6 | 0 | 77 | 5 | 10.4 | 100 | 0 | 0 |
| Example 2-74 | 89.5 | 71 | 6 | 0 | 77 | 5 | 10.5 | 100 | 0 | 0 |
| Example 2-75 | 88.0 | 86 | 0 | 0 | 86 | 5 | 12.0 | 100 | 0 | 0 |
| Example 2-76 | 92.1 | 53 | 15 | 0 | 67 | 6 | 7.9 | 100 | 0 | 0 |
| Example 2-77 | 90.1 | 80 | 2 | 0 | 83 | 6 | 9.9 | 100 | 0 | 0 |
| Example 2-78 | 88.6 | 54 | 5 | 0 | 59 | 7 | 11.4 | 100 | 0 | 0 |
| Example 2-79 | 91.8 | 73 | 5 | 0 | 77 | 4 | 8.2 | 100 | 0 | 0 |
| Example 2-80 | 90.9 | 82 | 2 | 0 | 84 | 4 | 9.1 | 100 | 0 | 0 |
| Example 2-81 | 91.3 | 73 | 6 | 0 | 79 | 4 | 8.7 | 100 | 0 | 0 |
| Example 2-82 | 91.2 | 73 | 5 | 0 | 78 | 4 | 8.8 | 100 | 0 | 0 |
| Example 2-83 | 89.9 | 87 | 0 | 0 | 88 | 4 | 10.1 | 100 | 0 | 0 |
| Example 2-84 | 93.6 | 54 | 15 | 0 | 69 | 5 | 6.4 | 100 | 0 | 0 |
| Example 2-85 | 91.9 | 81 | 2 | 0 | 84 | 5 | 8.1 | 100 | 0 | 0 |
| Example 2-86 | 90.3 | 56 | 5 | 0 | 61 | 6 | 9.7 | 100 | 0 | 0 |
| Example 2-87 | 90.5 | 71 | 10 | 0 | 81 | 4 | 9.5 | 100 | 0 | 0 |
| Example 2-88 | 90.4 | 81 | 9 | 0 | 90 | 5 | 9.6 | 100 | 0 | 0 |
| Example 2-89 | 40.7 | 60 | 10 | 10 | 80 | 3 | 59.3 | 37 | 31 | 32 |
| Example 2-90 | 90.5 | 71 | 10 | 0 | 80 | 4 | 9.5 | 100 | 0 | 0 |
| Example 2-91 | 90.4 | 71 | 9 | 8 | 88 | 1 | 9.6 | 100 | 0 | 0 |
| Example 2-92 | 90.6 | 70 | 11 | 8 | 89 | 2 | 9.4 | 100 | 0 | 0 |
| Example 2-93 | 90.4 | 79 | 9 | 0 | 89 | 2 | 9.6 | 100 | 0 | 0 |
| Example 2-94 | 41.1 | 55 | 10 | 5 | 70 | 1 | 58.9 | 54 | 46 | 0 |
| Example 2-95 | 41.5 | 56 | 9 | 5 | 70 | 1 | 58.5 | 54 | 46 | 0 |
| Example 2-96 | 46.2 | 55 | 11 | 5 | 71 | 1 | 53.8 | 52 | 43 | 4 |
| Example 2-97 | 66.1 | 66 | 5 | 10 | 80 | 1 | 33.9 | 42 | 29 | 29 |
| Example 2-98 | 85.8 | 70 | 2 | 10 | 82 | 1 | 14.2 | 23 | 6 | 71 |
| Example 2-99 | 89.8 | 70 | 5 | 3 | 78 | 1 | 10.2 | 17 | 0 | 83 |
| Example 2-100 | 89.9 | 71 | 4 | 0 | 75 | 2 | 10.1 | 100 | 0 | 0 |

**[Table 2-13]**

| | Reaction liquid following thermal decomposition | | | | | | TOP | | | |
|---|---|---|---|---|---|---|---|---|---|---|
| | parts by mass | NCO | NCO-BL | NCO-compound (I) | MB | Hazen color number | parts by mass | BL | Compound (I) | Compound (II) |
| | | mol% | mol% | mol% | mol% | APHA | | mass % | mass% | mass% |
| Example 2-101 | 95.3 | 30 | 55 | 8 | 94 | 5 | 4.7 | 100 | 0 | 0 |
| Example 2-102 | 93.6 | 41 | 40 | 8 | 89 | 5 | 6.4 | 100 | 0 | 0 |
| Example 2-103 | 92.6 | 51 | 30 | 8 | 89 | 4 | 7.4 | 100 | 0 | 0 |
| Example 2-104 | 91.5 | 61 | 19 | 8 | 88 | 4 | 8.5 | 100 | 0 | 0 |
| Example 2-105 | 90.5 | 71 | 10 | 8 | 89 | 4 | 9.5 | 100 | 0 | 0 |
| Example 2-106 | 90.0 | 70 | 5 | 8 | 84 | 3 | 10.0 | 100 | 0 | 0 |
| Example 2-107 | 94.2 | 39 | 55 | 0 | 93 | 4 | 5.8 | 100 | 0 | 0 |
| Example 2-108 | 92.6 | 49 | 40 | 0 | 89 | 4 | 7.4 | 100 | 0 | 0 |
| Example 2-109 | 91.6 | 59 | 30 | 0 | 88 | 4 | 8.4 | 100 | 0 | 0 |
| Example 2-110 | 90.5 | 69 | 19 | 0 | 88 | 4 | 9.5 | 100 | 0 | 0 |
| Example 2-111 | 89.5 | 79 | 10 | 0 | 89 | 4 | 10.5 | 100 | 0 | 0 |
| Example 2-112 | 89.0 | 79 | 5 | 0 | 84 | 4 | 11.0 | 100 | 0 | 0 |
| Example 2-113 | 90.5 | 70 | 10 | 8 | 88 | 4 | 9.5 | 100 | 0 | 0 |
| Example 2-114 | 90.5 | 70 | 10 | 8 | 88 | 2 | 9.5 | 100 | 0 | 0 |
| Example 2-115 | 90.6 | 80 | 11 | 0 | 91 | 5 | 9.4 | 100 | 0 | 0 |
| Example 2-116 | 90.6 | 80 | 11 | 0 | 91 | 4 | 9.4 | 100 | 0 | 0 |
| Comparative Example 2-1 | 90.5 | 80 | 10 | 0 | 91 | 8 | 9.5 | 100 | 0 | 0 |
| Comparative Example 2-2 | 89.3 | 90 | 5 | 0 | 95 | 8 | 10.7 | 100 | 0 | 0 |
| Comparative Example 2-3 | 89.9 | 80 | 11 | 0 | 91 | 8 | 10.1 | 100 | 0 | 0 |
| Comparative Example 2-4 | 89.8 | 80 | 10 | 0 | 90 | 8 | 10.2 | 100 | 0 | 0 |
| Comparative Example 2-5 | 88.0 | 95 | 0 | 0 | 96 | 9 | 12.0 | 100 | 0 | 0 |
| Comparative Example 2-6 | 93.4 | 60 | 30 | 0 | 90 | 9 | 6.6 | 100 | 0 | 0 |
| Comparative Example 2-7 | 90.2 | 91 | 5 | 0 | 96 | 9 | 9.8 | 100 | 0 | 0 |
| Comparative Example 2-8 | 89.1 | 60 | 10 | 0 | 70 | 10 | 10.9 | 100 | 0 | 0 |
| Comparative Example 2-9 | 93.1 | 65 | 25 | 0 | 89 | 8 | 6.9 | 100 | 0 | 0 |
| Comparative Example 2-10 | 94.2 | 59 | 31 | 0 | 90 | 8 | 5.8 | 100 | 0 | 0 |
| Comparative Example 2-11 | 90.1 | 71 | 5 | 0 | 76 | 10 | 9.9 | 100 | 0 | 0 |
| Comparative Example 2-12 | 91.9 | 72 | 5 | 0 | 78 | 9 | 8.1 | 100 | 0 | 0 |

As shown in the above tables, the Hazen color numbers (APHA) of the isocyanate compositions obtained by thermal decomposition of blocked isocyanate compositions following addition of at least one compound selected from the group consisting of the secondary amine compounds (I) and the tertiary amine compounds (II) (Examples 2-1 to 2-116) were evaluated as rank of 7 or lower, which were favorable.

In contrast, the Hazen color numbers of the isocyanate compositions obtained by thermal decomposition of blocked isocyanate compositions without the addition of either the secondary amine compound (1) or the tertiary amine compound (II) (Comparative Examples 2-1 to 2-12) were evaluated as rank 8 or higher, which were unfavorable.

Further, among the isocyanate compositions obtained by thermal decomposition, in the presence of the secondary amine compound (I), of blocked isocyanate compositions blocked with a phenol-based, alcohol-based, hydroxylamine-based or amine-based blocking agent (Examples 2-2 and 2-9 to 2-47), the isocyanate compositions obtained by thermal decomposition of blocked isocyanate compositions blocked with a phenol-based blocking agent (Examples 2-2, 2-9 to 2-12, 2-16 to 2-20, 2-24 to 2-28, 2-32 to 2-36, and 2-40 to 2-44) exhibited Hazen color numbers evaluated as the lowest rank, which were particularly favorable.

Furthermore, among the isocyanate compositions obtained by thermal decomposition, in the presence of the tertiary amine compound (II), of blocked isocyanate compositions blocked with a phenol-based, alcohol-based, hydroxylamine-based or amine-based blocking agent (Examples 2-6 and 2-48 to 2-86), the isocyanate compositions obtained by thermal decomposition of blocked isocyanate compositions blocked with a phenol-based blocking agent (Examples 2-6, 2-48 to 2-51, 2-55 to 2-59, 2-63 to 2-67, 2-71 to 2-75, and 2-79 to 2-83) exhibited Hazen color numbers evaluated as the lowest rank, which were particularly favorable.

Furthermore, among the isocyanate compositions in which the amounts added of the secondary amine compound (1) or the tertiary amine compound (II) were different (Examples 2-1, 2-5, and 2-87 to 2-90), the isocyanate compositions obtained by thermal decomposition of blocked isocyanate compositions in which the secondary amine compound (I) or the tertiary amine compound (II) had been added in an amount of at least 1 ppm by mass relative to the total mass of the reaction liquid (Examples 2-87 and 2-88) exhibited Hazen color numbers evaluated as rank 5 or lower, which were favorable.

Furthermore, among the isocyanate compositions in which the amounts added of the secondary amine compound (I) or the tertiary amine compound (II) were different (Examples 2-1, 2-5, and 2-87 to 2-90), the isocyanate compositions obtained by thermal decomposition of blocked isocyanate compositions in which the secondary amine compound (I) or the tertiary amine compound (II) had been added in an amount of at least 1% by mass relative to the total mass of the reaction liquid (Examples 2-1, 2-5, and 2-89 and 2-90) exhibited Hazen color numbers evaluated as rank 4 or lower, which were particularly favorable.

Furthermore, the isocyanate compositions obtained by thermal decomposition of blocked isocyanate compositions to which both the secondary amine compound (I) and the tertiary amine compound (II) had been added (Examples 2-91 to 2-100) exhibited Hazen color numbers evaluated as rank 2 or lower, which were the most favorable.

Furthermore, comparison of blocked isocyanate compositions prepared at different reaction temperatures (Examples 2-1, and 2-101 to 2-106) revealed that the ranks of the Hazen color numbers of isocyanate compositions obtained by thermal decomposition of blocked isocyanate compositions in the presence of the secondary amine compound (I) depended on the reaction temperature, and the coloration reduction effect was improved as the reaction temperature was increased.

On the other hand, comparison of blocked isocyanate compositions prepared at different reaction temperatures (Examples 2-5, and 2-107 to 2-112) revealed that the ranks of the Hazen color numbers of isocyanate compositions obtained by thermal decomposition of blocked isocyanate compositions in the presence of the tertiary amine compound (II) did not significantly depend on the reaction temperature.

Further, comparison of blocked isocyanate compositions prepared under different oxygen concentrations (Examples 2-1, and 2-113 and 2-114) revealed that the ranks of the Hazen color numbers of isocyanate compositions obtained by thermal decomposition of blocked isocyanate compositions in the presence of the secondary amine compound (I) depended on the oxygen concentration, and the coloration reduction effect was improved as the oxygen concentration was decreased.

Further, comparison of blocked isocyanate compositions prepared under different oxygen concentrations (Examples 2-5, and 2-115 and 2-116) revealed that the ranks of the Hazen color numbers of isocyanate compositions obtained by thermal decomposition of blocked isocyanate compositions in the presence of the tertiary amine compound (II) depended on the oxygen concentration, and the coloration reduction effect was improved as the oxygen concentration was decreased.

### <<Third Test>>

### [Physical Property 3-1]

### (Amount of Isocyanate Groups Blocked with First Blocking Agent)

The amount (mol%) of isocyanate groups blocked with the first blocking agent in an obtained blocked isocyanate composition (hereinafter sometimes abbreviated as "NCO-BL(A) mol%") was determined in accordance with the formula shown below, from the molar amount of isocyanate groups blocked with the first blocking agent within the mixture of the first blocked isocyanate compound and the second blocking agent prior to reaction, and the molar amount of residual isocyanate groups blocked with the first blocking agent following the reaction. The molar amount of isocyanate groups blocked with the first blocking agent was determined from an NMR spectrum.[NCO-BL(A) mol%] = (molar amount of isocyanate groups blocked with the first blocking agent following reaction) × 100 / (molar amount of isocyanate groups blocked with the first blocking agent prior to reaction)

### [Physical Property 3-2]

### (Amount of Isocyanate Groups Blocked with Second Blocking Agent)

The amount (mol%) of isocyanate groups blocked with the second blocking agent in an obtained blocked isocyanate composition (hereinafter sometimes abbreviated as "NCO-BL(C) mol%") was determined in accordance with the formula shown below, from the molar amount of isocyanate groups blocked with the first blocking agent within the mixture of the first blocked isocyanate compound and the second blocking agent prior to reaction, and the molar amount of isocyanate groups blocked with the second blocking agent following the reaction. The molar amount of isocyanate groups blocked with the second blocking agent was determined from an NMR spectrum.[NCO-BL(C) mol%] = (molar amount of isocyanate groups blocked with the second blocking agent following reaction) × 100 / (molar amount of isocyanate groups blocked with the first blocking agent prior to reaction)

### [Physical Property 3-3]

### (Amount of Isocyanate Groups Bonded to Secondary Amine Compound (I))

The amount (mol%) of isocyanate groups bonded to the secondary amine compound (I) in an obtained blocked isocyanate composition (hereinafter sometimes abbreviated as "NCO-compound (I) mol%") was determined in accordance with the formula shown below, from the molar amount of isocyanate groups blocked with the first blocking agent within the mixture of the first blocked isocyanate compound within the mixture of the first blocked isocyanate compound and the second blocking agent prior to reaction, and the molar amount of isocyanate groups bonded to the secondary amine compound (I) following the reaction. The molar amount of isocyanate groups bonded to the secondary amine compound (I) was determined from an NMR spectrum.[NCO-BL(I) mol%] = (molar amount of isocyanate groups bonded to the secondary amine compound (I) following reaction) × 100 / (molar amount of isocyanate groups blocked with the first blocking agent prior to reaction)

### [Evaluation 3-1]

### (Mass Balance)

The sum, within the obtained blocked isocyanate composition, of NCO-BL(A) mol%, NCO-BL(C) mol% and NCO-BL(I) mol% was determined as the mass balance (hereinafter sometimes abbreviated as "MB mol%").

### [Evaluation 3-2]

### (Hazen Color Number)

The Hazen color number was determined by dissolving, in 2 g of benzyltoluene, 1 g of the blocked isocyanate composition obtained following removal by distillation of any free compound (I), compound (II), the first blocking agent and the second blocking agent contained in the obtained reaction solution. Based on the value measured with a Hazen meter, the resultant was ranked in accordance with the following evaluation criteria.

### (Evaluation Criteria)

Rank 1: APHA of at least 0 but less than 5
Rank 2: APHA of at least 5 but less than 10
Rank 3: APHA of at least 10 but less than 15
Rank 4: APHA of at least 15 but less than 20
Rank 5: APHA of at least 20 but less than 25
Rank 6: APHA of at least 25 but less than 30
Rank 7: APHA of at least 30 but less than 35
Rank 8: APHA of at least 35 but less than 40
Rank 9: APHA of at least 40 but less than 45
Rank 10: APHA of at least 45 but less than 50

### [Example 3-1]

20 parts by mass of the blocked isocyanate compound BL-1 prepared in Production Example 2-1 of the second test described above, 79 parts by mass of para-cumylphenol, and 1 part by mass of N-methylaniline were placed in a 300 mL glass vessel equipped with a condenser tube 1 packed with a filler (the condenser tube 1 having a structure that allows the condensed liquid from the condenser tube 1 to fall into the 300 mL glass vessel) and a condenser 2 (having a structure that allow the condensed liquid from the condenser tube 2 to be collected as a TOP liquid without entering the 300 mL glass vessel) equipped with a pressure reduction device at the tip thereof. Subsequently, the internal temperature of the 300 mL glass vessel was set to 250°C, the temperature of the condenser tube 1 was set to 200°C, and the temperature of the condenser tube 2 was set to 70°C. Further, a synthesis gas was produced from a nitrogen cylinder and an oxygen cylinder, and the oxygen concentration in the synthesis gas was confirmed to be 0.1 vol% using an oxygen concentration meter, followed by adjusting the absolute pressure inside the reaction vessel to 380 mmHg using the pressure reduction device while supplying the synthesis gas to the 300 mL glass vessel at a flow rate of 20 NmL/min to allow the reaction to proceed for two hours. As a result, 88 parts by mass of a thermally decomposed reaction liquid was obtained inside the 300 mL glass vessel, and 12 parts by mass of the TOP liquid were collected from the condenser tube 2. The result determined from ¹H NMR integral values of the obtained reaction liquid following addition-elimination reaction revealed an NCO-BL (compound I) mol% of 0 mol%, NCO-BL (A) mol% of 0 mol%, NCO-BL (C) mol% of 99 mol%, and MB mol% of 99 mol%. 1 g of the obtained blocked isocyanate composition was dissolved in 2 g of benzyltoluene and the Hazen color number thereof was measured, thereby confirming that the obtained value was evaluated as rank 3.

### [Examples 3-2 to 3-122, and Comparative Examples 3-1 to 3-43]

In each of Examples 3-2 to 3-122 and Comparative Examples 3-1 to 3-43, blocked isocyanate compounds were produced using the same method as that described in Example 3-1, except that the raw materials and reaction conditions were altered as shown in the following tables. As the first blocked isocyanate compound, the compound produced in the second test example mentioned above was used.

In the following tables, the abbreviations used refer to the following compounds.

### (Secondary Amine Compounds (I))

NMA: N-methyl-aniline
NEA: N-ethyl-aniline
NBA: N-butyl-aniline
DPA: N,N-diphenylamine

### (Tertiary Amine Compounds (II))

NNDMA: N,N-dimethylaniline
NNDEA: N,N-diethylaniline
NMDPA: N-methyl-N.N-diphenylamine
TPA: triphenylamine

### (First Blocking Agents)

PhOH: phenol
o-cresol: ortho-cresol
m-cresol: meta-cresol
p-cresol: para-cresol
OCP: o-chlorophenol
n-BuOH: normal butanol
DBA: dibutylamine

### (Second Blocking Agent)

PCP: para-cumylphenol
4-octylPhOH: 4-octylphenol
nonylPhOH: nonylphenol isomeric mixture
DCA: dicyclohexylamine

### (Blocking Agents)

BL(A): first blocking agent derived from first blocked isocyanate
BL(C): second blocking agent

### (Functional Groups)

NCO-compound (I): blocked isocyanate group obtained by bonding of compound (1) to an isocyanate group
NCO-BL(A): blocked isocyanate group obtained by bonding of BL(A) (first blocking agent) to an isocyanate group
NCO-BL(C): blocked isocyanate group obtained by bonding of BL(C) (second blocking agent) to an isocyanate group

**[Table 3-1]**

| | Secondary amine compound (I) | | Tertiary amine compound (II) | | First blocked isocyanate compound | | | Second blocking agent | |
|---|---|---|---|---|---|---|---|---|---|
| | type | parts by mass | type | parts by mass | type | First blocking agent | parts by mass | type | parts by mass |
| Example 3-1 | NMA | 1 | - | 0 | BL-1 | PhOH | 20 | PCP | 79 |
| Example 3-2 | NEA | 1 | - | 0 | BL-1 | PhOH | 20 | PCP | 79 |
| Example 3-3 | NBA | 1 | - | 0 | BL-1 | PhOH | 20 | PCP | 79 |
| Example 3-4 | DPA | 1 | - | 0 | BL-1 | PhOH | 20 | PCP | 79 |
| Example 3-5 | - | 0 | NNDMA | 1 | BL-1 | PhOH | 20 | PCP | 79 |
| Example 3-6 | - | 0 | NNDEA | 1 | BL-1 | PhOH | 20 | PCP | 79 |
| Example 3-7 | - | 0 | NMDPA | 1 | BL-1 | PhOH | 20 | PCP | 79 |
| Example 3-8 | - | 0 | TPA | 1 | BL-1 | PhOH | 20 | PCP | 79 |
| Example 3-9 | NEA | 1 | - | 0 | BL-1 | PhOH | 20 | 4-octylPhOH | 79 |
| Example 3-10 | NEA | 1 | - | 0 | BL-1 | PhOH | 20 | 2,4-di-α-cumyl phenol | 79 |
| Example 3-11 | NEA | 1 | - | 0 | BL-1 | PhOH | 20 | nonylPhOH | 79 |
| Example 3-12 | NEA | 1 | - | 0 | BL-1 | PhOH | 20 | 4-chloro-3,5-dimethylphen ol | 79 |
| Example 3-13 | NEA | 1 | - | 0 | BL-1 | PhOH | 20 | 1-dodecanol | 79 |
| Example 3-14 | NEA | 1 | - | 0 | BL-1 | PhOH | 20 | DCA | 79 |
| Example 3-15 | NEA | 1 | - | 0 | BL-2 | o-cresol | 20 | PCP | 79 |
| Example 3-16 | NEA | 1 | - | 0 | BL-3 | m-cresol | 20 | PCP | 79 |
| Example 3-17 | NEA | 1 | - | 0 | BL-4 | p-cresol | 20 | PCP | 79 |
| Example 3-18 | NEA | 1 | - | 0 | BL-5 | OCP | 20 | PCP | 79 |
| Example 3-19 | NEA | 1 | - | 0 | BL-6 | n-BuOH | 20 | PCP | 79 |
| Example 3-20 | NEA | 1 | - | 0 | BL-8 | DBA | 20 | PCP | 79 |
| Example 3-21 | NEA | 1 | - | 0 | BL-6 | n-BuOH | 20 | 1-dodecanol | 79 |
| Example 3-22 | NEA | 1 | - | 0 | BL-8 | DBA | 20 | DCA | 79 |
| Example 3-23 | NEA | 1 | - | 0 | BL-9 | PhOH | 20 | PCP | 79 |
| Example 3-24 | NEA | 1 | - | 0 | BL-9 | PhOH | 20 | 4-Octyl PhOH | 79 |
| Example 3-25 | NEA | 1 | - | 0 | BL-9 | PhOH | 20 | 2,4-di-α-cumyl phenol | 79 |

**[Table 3-2]**

| | Secondary amine compound (I) | | Tertiary amine compound (II) | | First blocked isocyanate compound | | | Second blocking agent | |
|---|---|---|---|---|---|---|---|---|---|
| | type | parts by mass | type | parts by mass | type | First blocking agent | parts by mass | type | parts by mass |
| Example 3-26 | NEA | 1 | - | 0 | BL-9 | PhOH | 20 | nonylPhOH | 79 |
| Example 3-27 | NEA | 1 | - | 0 | BL-9 | PhOH | 20 | 4-chloro-3,5-dimethylphenol | 79 |
| Example 3-28 | NEA | 1 | - | 0 | BL-9 | PhOH | 20 | 1-dodecanol | 79 |
| Example 3-29 | NEA | 1 | - | 0 | BL-9 | PhOH | 20 | DCA | 79 |
| Example 3-30 | NEA | 1 | - | 0 | BL-17 | PhOH | 20 | PCP | 79 |
| Example 3-31 | NEA | 1 | - | 0 | BL-17 | PhOH | 20 | 4-octylPhOH | 79 |
| Example 3-32 | NEA | 1 | - | 0 | BL-17 | PhOH | 20 | 2,4-di-α-cumyl phenol | 79 |
| Example 3-33 | NEA | 1 | - | 0 | BL-17 | PhOH | 20 | nonylPhOH | 79 |
| Example 3-34 | NEA | 1 | - | 0 | BL-17 | PhOH | 20 | 4-chloro-3,5-dimethylphenol | 79 |
| Example 3-35 | NEA | 1 | - | 0 | BL-17 | PhOH | 20 | 1-dodecanol | 79 |
| Example 3-36 | NEA | 1 | - | 0 | BL-17 | PhOH | 20 | DCA | 79 |
| Example 3-37 | NEA | 1 | - | 0 | BL-25 | PhOH | 20 | PCP | 79 |
| Example 3-38 | NEA | 1 | - | 0 | BL-25 | PhOH | 20 | 4-octylPhOH | 79 |
| Example 3-39 | NEA | 1 | - | 0 | BL-25 | PhOH | 20 | 2,4-di-α-cumyl phenol | 79 |
| Example 3-40 | NEA | 1 | - | 0 | BL-25 | PhOH | 20 | nonylPhOH | 79 |
| Example 3-41 | NEA | 1 | - | 0 | BL-25 | PhOH | 20 | 4-chloro-3,5-dimethylphenol | 79 |
| Example 3-42 | NEA | 1 | - | 0 | BL-25 | PhOH | 20 | 1-dodecanol | 79 |
| Example 3-43 | NEA | 1 | - | 0 | BL-25 | PhOH | 20 | DCA | 79 |
| Example 3-44 | NEA | 1 | - | 0 | BL-33 | PhOH | 20 | PCP | 79 |
| Example 3-45 | NEA | 1 | - | 0 | BL-33 | PhOH | 20 | 4-octylPhOH | 79 |

**[Table 3-3]**

| | Secondary amine compound (I) | | Tertiary amine compound (II) | | First blocked isocyanate compound | | | Second blocking agent | |
|---|---|---|---|---|---|---|---|---|---|
| | type | parts by mass | type | parts by mass | type | First blocking agent | parts by mass | type | parts by mass |
| Example 3-46 | NEA | 1 | - | 0 | BL-33 | PhOH | 20 | 2,4-di-α-cumyl phenol | 79 |
| Example 3-47 | NEA | 1 | - | 0 | BL-33 | PhOH | 20 | nonylPhOH | 79 |
| Example 3-48 | NEA | 1 | - | 0 | BL-33 | PhOH | 20 | 4-chloro-3,5-dimethylphenol | 79 |
| Example 3-49 | NEA | 1 | - | 0 | BL-33 | PhOH | 20 | 1-dodecanol | 79 |
| Example 3-50 | NEA | 1 | - | 0 | BL-33 | PhOH | 20 | DCA | 79 |
| Example 3-51 | - | 0 | NNDEA | 1 | BL-1 | PhOH | 20 | 4-octylPhOH | 79 |
| Example 3-52 | - | 0 | NNDEA | 1 | BL-1 | PhOH | 20 | 2,4-di-α-cumyl phenol | 79 |
| Example 3-53 | - | 0 | NNDEA | 1 | BL-1 | PhOH | 20 | nonylPhOH | 79 |
| Example 3-54 | - | 0 | NNDEA | 1 | BL-1 | PhOH | 20 | 4-chloro-3,5-dimethylphenol | 79 |
| Example 3-55 | - | 0 | NNDEA | 1 | BL-1 | PhOH | 20 | 1-dodecanol | 79 |
| Example 3-56 | - | 0 | NNDEA | 1 | BL-1 | PhOH | 20 | DCA | 79 |
| Example 3-57 | - | 0 | NNDEA | 1 | BL-2 | o-cresol | 20 | PCP | 79 |
| Example 3-58 | - | 0 | NNDEA | 1 | BL-3 | m-cresol | 20 | PCP | 79 |
| Example 3-59 | - | 0 | NNDEA | 1 | BL-4 | p-cresol | 20 | PCP | 79 |
| Example 3-60 | - | 0 | NNDEA | 1 | BL-5 | OCP | 20 | PCP | 79 |
| Example 3-61 | - | 0 | NNDEA | 1 | BL-6 | n-BuOH | 20 | PCP | 79 |
| Example 3-62 | - | 0 | NNDEA | 1 | BL-8 | DBA | 20 | PCP | 79 |
| Example 3-63 | | 0 | NNDEA | 1 | BL-6 | n-BuOH | 20 | 1-dodecanol | 79 |
| Example 3-64 | | 0 | NNDEA | 1 | BL-8 | DBA | 20 | DCA | 79 |
| Example 3-65 | - | 0 | NNDEA | 1 | BL-9 | PhOH | 20 | PCP | 79 |

**[Table 3-4]**

| | Secondary amine compound (I) | | Tertiary amine compound (II) | | First blocked isocyanate compound | | | Second blocking agent | |
|---|---|---|---|---|---|---|---|---|---|
| | type | parts by mass | type | parts by mass | type | First blocking agent | parts by mass | type | parts by mass |
| Example 3-66 | - | 0 | NNDEA | 1 | BL-9 | PhOH | 20 | 4-octylPhOH | 79 |
| Example 3-67 | - | 0 | NNDEA | 1 | BL-9 | PhOH | 20 | 2,4-di-α-cumyl phenol | 79 |
| Example 3-68 | - | 0 | NNDEA | 1 | BL-9 | PhOH | 20 | nonylPhOH | 79 |
| Example 3-69 | - | 0 | NNDEA | 1 | BL-9 | PhOH | 20 | 4-chloro-3,5-dimethylphenol | 79 |
| Example 3-70 | - | 0 | NNDEA | 1 | BL-9 | PhOH | 20 | 1-dodecanol | 79 |
| Example 3-71 | - | 0 | NNDEA | 1 | BL-9 | PhOH | 20 | DCA | 79 |
| Example 3-72 | - | 0 | NNDEA | 1 | BL-17 | PhOH | 20 | PCP | 79 |
| Example 3-73 | - | 0 | NNDEA | 1 | BL-17 | PhOH | 20 | 4-octylPhOH | 79 |
| Example 3-74 | - | 0 | NNDEA | 1 | BL-17 | PhOH | 20 | 2,4-di-α-cumyl phenol | 79 |
| Example 3-75 | - | 0 | NNDEA | 1 | BL-17 | PhOH | 20 | nonylPhOH | 79 |
| Example 3-76 | - | 0 | NNDEA | 1 | BL-17 | PhOH | 20 | 4-chloro-3,5-dimethylphenol | 79 |
| Example 3-77 | - | 0 | NNDEA | 1 | BL-17 | PhOH | 20 | 1-dodecanol | 79 |
| Example 3-78 | - | 0 | NNDEA | 1 | BL-17 | PhOH | 20 | DCA | 79 |
| Example 3-79 | - | 0 | NNDEA | 1 | BL-25 | PhOH | 20 | PCP | 79 |
| Example 3-80 | - | 0 | NNDEA | 1 | BL-25 | PhOH | 20 | 4-octylPhOH | 79 |
| Example 3-81 | - | 0 | NNDEA | 1 | BL-25 | PhOH | 20 | 2,4-di-α-cumyl phenol | 79 |
| Example 3-82 | - | 0 | NNDEA | 1 | BL-25 | PhOH | 20 | nonylPhOH | 79 |
| Example 3-83 | - | 0 | NNDEA | 1 | BL-25 | PhOH | 20 | 4-chloro-3,5-dimethylphenol | 79 |
| Example 3-84 | - | 0 | NNDEA | 1 | BL-25 | PhOH | 20 | 1-dodecanol | 79 |
| Example 3-85 | - | 0 | NNDEA | 1 | BL-25 | PhOH | 20 | DCA | 79 |

**[Table 3-5]**

| | Secondary amine compound (I) | | Tertiary amine compound (II) | | First blocked isocyanate compound | | | Second blocking agent | |
|---|---|---|---|---|---|---|---|---|---|
| | type | parts by mass | type | parts by mass | type | First blocking agent | parts by mass | type | parts by mass |
| Example 3-86 | - | 0 | NNDEA | 1 | BL-33 | PhOH | 20 | PCP | 79 |
| Example 3-87 | - | 0 | NNDEA | 1 | BL-33 | PhOH | 20 | 4-octylPhOH | 79 |
| Example 3-88 | - | 0 | NNDEA | 1 | BL-33 | PhOH | 20 | 2,4-di-α-cumyl phenol | 79 |
| Example 3-89 | - | 0 | NNDEA | 1 | BL-33 | PhOH | 20 | nonylPhOH | 79 |
| Example 3-90 | - | 0 | NNDEA | 1 | BL-33 | PhOH | 20 | 4-chloro-3,5-dimethylphenol | 79 |
| Example 3-91 | - | 0 | NNDEA | 1 | BL-33 | PhOH | 20 | 1-dodecanol | 79 |
| Example 3-92 | - | 0 | NNDEA | 1 | BL-33 | PhOH | 20 | DCA | 79 |
| Example 3-93 | NMA | 0.0001 | - | 0 | BL-1 | PhOH | 20 | PCP | 80 |
| Example 3-94 | - | 0 | NNDMA | 0.0001 | BL-1 | PhOH | 20 | PCP | 80 |
| Example 3-95 | NMA | 51 | - | 0 | BL-1 | PhOH | 20 | PCP | 29 |
| Example 3-96 | - | 0 | NNDMA | 51 | BL-1 | PhOH | 20 | PCP | 29 |
| Example 3-97 | NMA | 1 | NNDMA | 1 | BL-1 | PhOH | 20 | PCP | 78 |
| Example 3-98 | NMA | 1 | NNDMA | 0.0001 | BL-1 | PhOH | 20 | PCP | 79 |
| Example 3-99 | NMA | 0.0001 | NNDMA | 1 | BL-1 | PhOH | 20 | PCP | 79 |

**[Table 3-6]**

| | Secondary amine compound (I) | | Tertiary amine compound (II) | | First blocked isocyanate compound | | | Second blocking agent | |
|---|---|---|---|---|---|---|---|---|---|
| | type | parts by mass | type | parts by mass | type | First blocking agent | parts by mass | type | parts by mass |
| Example 3-100 | NMA | 50 | NNDMA | 0.0001 | BL-1 | PhOH | 20 | PCP | 30 |
| Example 3-101 | NMA | 49.5 | NNDMA | 0.5 | BL-1 | PhOH | 20 | PCP | 30 |
| Example 3-102 | NMA | 45 | NNDMA | 5 | BL-1 | PhOH | 20 | PCP | 30 |
| Example 3-103 | NMA | 25 | NNDMA | 25 | BL-1 | PhOH | 20 | PCP | 30 |
| Example 3-104 | NMA | 5 | NNDMA | 45 | BL-1 | PhOH | 20 | PCP | 30 |
| Example 3-105 | NMA | 0.5 | NNDMA | 49.5 | BL-1 | PhOH | 20 | PCP | 30 |
| Example 3-106 | NMA | 0.0001 | NNDMA | 50 | BL-1 | PhOH | 20 | PCP | 30 |
| Example 3-107 | NMA | 1 | - | 0 | BL-1 | PhOH | 20 | PCP | 79 |
| Example 3-108 | NMA | 1 | - | 0 | BL-1 | PhOH | 20 | PCP | 79 |
| Example 3-109 | NMA | 1 | - | 0 | BL-1 | PhOH | 20 | PCP | 79 |
| Example 3-110 | NMA | 1 | - | 0 | BL-1 | PhOH | 20 | PCP | 79 |
| Example 3-111 | NMA | 1 | - | 0 | BL-1 | PhOH | 20 | PCP | 79 |
| Example 3-112 | NMA | 1 | - | 0 | BL-1 | PhOH | 20 | PCP | 79 |
| Example 3-113 | - | 0 | NNDMA | 1 | BL-1 | PhOH | 20 | PCP | 79 |
| Example 3-114 | - | 0 | NNDMA | 1 | BL-1 | PhOH | 20 | PCP | 79 |
| Example 3-115 | - | 0 | NNDMA | 1 | BL-1 | PhOH | 20 | PCP | 79 |
| Example 3-116 | - | 0 | NNDMA | 1 | BL-1 | PhOH | 20 | PCP | 79 |
| Example 3-117 | - | 0 | NNDMA | 1 | BL-1 | PhOH | 20 | PCP | 79 |
| Example 3-118 | - | 0 | NNDMA | 1 | BL-1 | PhOH | 20 | PCP | 79 |
| Example 3-119 | NMA | 1 | - | 0 | BL-1 | PhOH | 20 | PCP | 79 |
| Example 3-120 | NMA | 1 | - | 0 | BL-1 | PhOH | 20 | PCP | 79 |
| Example 3-121 | - | 0 | NNDMA | 1 | BL-1 | PhOH | 20 | PCP | 79 |
| Example 3-122 | - | 0 | NNDMA | 1 | BL-1 | PhOH | 20 | PCP | 79 |

**[Table 3-7]**

| | Secondary amine compound (I) | | Tertiary amine compound (II) | | First blocked isocyanate compound | | | Second blocking agent | |
|---|---|---|---|---|---|---|---|---|---|
| | type | parts by mass | type | parts by mass | type | First blocking agent | parts by mass | type | parts by mass |
| Comparative Example 3-1 | - | 0 | - | 0 | BL-1 | PhOH | 20 | PCP | 79 |
| Comparative Example 3-2 | - | 0 | - | 0 | BL-1 | PhOH | 20 | 4-octylPhOH | 79 |
| Comparative Example 3-3 | - | 0 | - | 0 | BL-1 | PhOH | 20 | 2,4-di-α-cumyl phenol | 79 |
| Comparative Example 3-4 | - | 0 | - | 0 | BL-1 | PhOH | 20 | nonylPhOH | 79 |
| Comparative Example 3-5 | - | 0 | - | 0 | BL-1 | PhOH | 20 | 4-chloro-3,5-dimethylphenol | 79 |
| Comparative Example 3-6 | - | 0 | - | 0 | BL-1 | PhOH | 20 | 1-dodecanol | 79 |
| Comparative Example 3-7 | - | 0 | - | 0 | BL-1 | PhOH | 20 | DCA | 79 |
| Comparative Example 3-8 | - | 0 | - | 0 | BL-2 | o-cresol | 20 | PCP | 79 |
| Comparative Example 3-9 | - | 0 | - | 0 | BL-3 | m-cresol | 20 | PCP | 79 |
| Comparative Example 3-10 | - | 0 | - | 0 | BL-4 | p-cresol | 20 | PCP | 79 |
| Comparative Example 3-11 | - | 0 | - | 0 | BL-5 | OCP | 20 | PCP | 79 |
| Comparative Example 3-12 | - | 0 | - | 0 | BL-6 | n-BuOH | 20 | PCP | 79 |
| Comparative Example 3-13 | - | 0 | - | 0 | BL-8 | DBA | 20 | PCP | 79 |
| Comparative Example 3-14 | - | 0 | - | 0 | BL-6 | n-BuOH | 20 | 1-dodecanol | 79 |
| Comparative Example 3-15 | - | 0 | - | 0 | BL-8 | DBA | 20 | DCA | 79 |
| Comparative Example 3-16 | - | 0 | - | 0 | BL-9 | PhOH | 20 | PCP | 79 |
| Comparative Example 3-17 | - | 0 | - | 0 | BL-9 | PhOH | 20 | 4-octylPhOH | 79 |
| Comparative Example 3-18 | - | 0 | - | 0 | BL-9 | PhOH | 20 | 2,4-di-α-cumyl phenol | 79 |
| Comparative Example 3-19 | - | 0 | - | 0 | BL-9 | PhOH | 20 | nonylPhOH | 79 |
| Comparative Example 3-20 | - | 0 | - | 0 | BL-9 | PhOH | 20 | 4-chloro-3,5-dimethylphenol | 79 |
| Comparative Example 3-21 | - | 0 | - | 0 | BL-9 | PhOH | 20 | 1-dodecanol | 79 |

**[Table 3-8]**

| | Secondary amine compound (I) | | Tertiary amine compound (II) | | First blocked isocyanate compound | | | Second blocking agent | |
|---|---|---|---|---|---|---|---|---|---|
| | type | parts by mass | type | parts by mass | type | First blocking agent | parts by mass | type | parts by mass |
| Comparative Example 3-22 | - | 0 | - | 0 | BL-9 | PhOH | 20 | DCA | 79 |
| Comparative Example 3-23 | - | 0 | - | 0 | BL-17 | PhOH | 20 | PCP | 79 |
| Comparative Example 3-24 | - | 0 | - | 0 | BL-17 | PhOH | 20 | 4-octylPhOH | 79 |
| Comparative Example 3-25 | - | 0 | - | 0 | BL-17 | PhOH | 20 | 2,4-di-α-cumyl phenol | 79 |
| Comparative Example 3-26 | - | 0 | - | 0 | BL-17 | PhOH | 20 | nonylPhOH | 79 |
| Comparative Example 3-27 | - | 0 | - | 0 | BL-17 | PhOH | 20 | 4-chloro-3,5-dimethylphenol | 79 |
| Comparative Example 3-28 | - | 0 | - | 0 | BL-17 | PhOH | 20 | 1-dodecanol | 79 |
| Comparative Example 3-29 | - | 0 | - | 0 | BL-17 | PhOH | 20 | DCA | 79 |
| Comparative Example 3-30 | - | 0 | - | 0 | BL-25 | PhOH | 20 | PCP | 79 |
| Comparative Example 3-31 | - | 0 | - | 0 | BL-25 | PhOH | 20 | 4-octylPhOH | 79 |
| Comparative Example 3-32 | - | 0 | - | 0 | BL-25 | PhOH | 20 | 2,4-di-α-cumyl phenol | 79 |
| Comparative Example 3-33 | - | 0 | - | 0 | BL-25 | PhOH | 20 | nonylPhOH | 79 |
| Comparative Example 3-34 | - | 0 | - | 0 | BL-25 | PhOH | 20 | 4-chloro-3,5-dimethylphenol | 79 |
| Comparative Example 3-35 | - | 0 | - | 0 | BL-25 | PhOH | 20 | 1-dodecanol | 79 |
| Comparative Example 3-36 | - | 0 | - | 0 | BL-25 | PhOH | 20 | DCA | 79 |
| Comparative Example 3-37 | - | 0 | - | 0 | BL-33 | PhOH | 20 | PCP | 79 |
| Comparative Example 3-38 | - | 0 | - | 0 | BL-33 | PhOH | 20 | 4-octylPhOH | 79 |
| Comparative Example 3-39 | - | 0 | - | 0 | BL-33 | PhOH | 20 | 2,4-di-α-cumyl phenol | 79 |
| Comparative Example 3-40 | - | 0 | - | 0 | BL-33 | PhOH | 20 | nonylPhOH | 79 |
| Comparative Example 3-41 | - | 0 | - | 0 | BL-33 | PhOH | 20 | 4-chloro-3,5-dimethylphenol | 79 |
| Comparative Example 3-42 | - | 0 | - | 0 | BL-33 | PhOH | 20 | 1-dodecanol | 79 |
| Comparative Example 3-43 | - | 0 | - | 0 | BL-33 | PhOH | 20 | DCA | 79 |

**[Table 3-9]**

| | Reaction temperature | Absolute pressure | Condenser tube 1 temperature | Condenser tube 2 temperature | Reaction time | Oxygen concentration | BTM |
|---|---|---|---|---|---|---|---|
| | °C | mmHg | °C | °C | hours | vol% | parts by mass |
| Example 3-1 | 250 | 380 | 200 | 70 | 2 | 0.1 | 88 |
| Example 3-2 | 250 | 380 | 200 | 70 | 2 | 0.1 | 88 |
| Example 3-3 | 250 | 380 | 200 | 70 | 2 | 0.1 | 88 |
| Example 3-4 | 250 | 380 | 200 | 70 | 2 | 0.1 | 88 |
| Example 3-5 | 250 | 380 | 200 | 70 | 2 | 0.1 | 88 |
| Example 3-6 | 250 | 380 | 200 | 70 | 2 | 0.1 | 88 |
| Example 3-7 | 250 | 380 | 200 | 70 | 2 | 0.1 | 88 |
| Example 3-8 | 250 | 380 | 200 | 70 | 2 | 0.1 | 88 |
| Example 3-9 | 250 | 380 | 200 | 70 | 2 | 0.1 | 88 |
| Example 3-10 | 250 | 380 | 200 | 70 | 2 | 0.1 | 88 |
| Example 3-11 | 250 | 380 | 200 | 70 | 2 | 0.1 | 88 |
| Example 3-12 | 250 | 380 | 200 | 70 | 2 | 0.1 | 88 |
| Example 3-13 | 250 | 380 | 200 | 70 | 2 | 0.1 | 88 |
| Example 3-14 | 250 | 380 | 200 | 70 | 2 | 0.1 | 88 |
| Example 3-15 | 250 | 380 | 200 | 70 | 2 | 0.1 | 88 |
| Example 3-16 | 250 | 380 | 200 | 70 | 2 | 0.1 | 88 |
| Example 3-17 | 250 | 380 | 200 | 70 | 2 | 0.1 | 88 |
| Example 3-18 | 250 | 380 | 200 | 70 | 2 | 0.1 | 87 |
| Example 3-19 | 250 | 380 | 200 | 70 | 2 | 0.1 | 90 |
| Example 3-20 | 250 | 380 | 200 | 70 | 2 | 0.1 | 87 |
| Example 3-21 | 250 | 380 | 200 | 70 | 2 | 0.1 | 90 |
| Example 3-22 | 250 | 380 | 200 | 70 | 2 | 0.1 | 87 |
| Example 3-23 | 250 | 380 | 200 | 70 | 2 | 0.1 | 90 |
| Example 3-24 | 250 | 380 | 200 | 70 | 2 | 0.1 | 90 |
| Example 3-25 | 250 | 380 | 200 | 70 | 2 | 0.1 | 90 |

**[Table 3-10]**

| | Reaction temperature | Absolute pressure | Condenser tube 1 temperature | Condenser tube 2 temperature | Reaction time | Oxygen concentration | BTM |
|---|---|---|---|---|---|---|---|
| | °C | mmHg | °C | °C | hours | vol% | parts by mass |
| Example 3-26 | 250 | 380 | 200 | 70 | 2 | 0.1 | 90 |
| Example 3-27 | 250 | 380 | 200 | 70 | 2 | 0.1 | 90 |
| Example 3-28 | 250 | 380 | 200 | 70 | 2 | 0.1 | 90 |
| Example 3-29 | 250 | 380 | 200 | 70 | 2 | 0.1 | 90 |
| Example 3-30 | 250 | 380 | 200 | 70 | 2 | 0.1 | 91 |
| Example 3-31 | 250 | 380 | 200 | 70 | 2 | 0.1 | 91 |
| Example 3-32 | 250 | 380 | 200 | 70 | 2 | 0.1 | 91 |
| Example 3-33 | 250 | 380 | 200 | 70 | 2 | 0.1 | 91 |
| Example 3-34 | 250 | 380 | 200 | 70 | 2 | 0.1 | 91 |
| Example 3-35 | 250 | 380 | 200 | 70 | 2 | 0.1 | 91 |
| Example 3-36 | 250 | 380 | 200 | 70 | 2 | 0.1 | 91 |
| Example 3-37 | 250 | 380 | 200 | 70 | 2 | 0.1 | 89 |
| Example 3-38 | 250 | 380 | 200 | 70 | 2 | 0.1 | 89 |
| Example 3-39 | 250 | 380 | 200 | 70 | 2 | 0.1 | 89 |
| Example 3-40 | 250 | 380 | 200 | 70 | 2 | 0.1 | 89 |
| Example 3-41 | 250 | 380 | 200 | 70 | 2 | 0.1 | 89 |
| Example 3-42 | 250 | 380 | 200 | 70 | 2 | 0.1 | 89 |
| Example 3-43 | 250 | 380 | 200 | 70 | 2 | 0.1 | 89 |
| Example 3-44 | 250 | 380 | 200 | 70 | 2 | 0.1 | 90 |
| Example 3-45 | 250 | 380 | 200 | 70 | 2 | 0.1 | 90 |

**[Table 3-11]**

| | Reaction temperature | Absolute pressure | Condenser tube 1 temperature | Condenser tube 2 temperature | Reaction time | Oxygen concentration | BTM |
|---|---|---|---|---|---|---|---|
| | °C | mmHg | °C | °C | hours | vol% | parts by mass |
| Example 3-46 | 250 | 380 | 200 | 70 | 2 | 0.1 | 90 |
| Example 3-47 | 250 | 380 | 200 | 70 | 2 | 0.1 | 90 |
| Example 3-48 | 250 | 380 | 200 | 70 | 2 | 0.1 | 90 |
| Example 3-49 | 250 | 380 | 200 | 70 | 2 | 0.1 | 90 |
| Example 3-50 | 250 | 380 | 200 | 70 | 2 | 0.1 | 90 |
| Example 3-51 | 250 | 380 | 200 | 70 | 2 | 0.1 | 88 |
| Example 3-52 | 250 | 380 | 200 | 70 | 2 | 0.1 | 88 |
| Example 3-53 | 250 | 380 | 200 | 70 | 2 | 0.1 | 88 |
| Example 3-54 | 250 | 380 | 200 | 70 | 2 | 0.1 | 88 |
| Example 3-55 | 250 | 380 | 200 | 70 | 2 | 0.1 | 88 |
| Example 3-56 | 250 | 380 | 200 | 70 | 2 | 0.1 | 88 |
| Example 3-57 | 250 | 380 | 200 | 70 | 2 | 0.1 | 88 |
| Example 3-58 | 250 | 380 | 200 | 70 | 2 | 0.1 | 88 |
| Example 3-59 | 250 | 380 | 200 | 70 | 2 | 0.1 | 88 |
| Example 3-60 | 250 | 380 | 200 | 70 | 2 | 0.1 | 87 |
| Example 3-61 | 250 | 380 | 200 | 70 | 2 | 0.1 | 90 |
| Example 3-62 | 250 | 380 | 200 | 70 | 2 | 0.1 | 87 |
| Example 3-63 | 250 | 380 | 200 | 70 | 2 | 0.1 | 90 |
| Example 3-64 | 250 | 380 | 200 | 70 | 2 | 0.1 | 87 |
| Example 3-65 | 250 | 380 | 200 | 70 | 2 | 0.1 | 90 |

**[Table 3-12]**

| | Reaction temperature | Absolute pressure | Condenser tube 1 temperature | Condenser tube 2 temperature | Reaction time | Oxygen concentration | BTM |
|---|---|---|---|---|---|---|---|
| | °C | mmHg | °C | °C | hours | vol% | parts by mass |
| Example 3-66 | 250 | 380 | 200 | 70 | 2 | 0.1 | 90 |
| Example 3-67 | 250 | 380 | 200 | 70 | 2 | 0.1 | 90 |
| Example 3-68 | 250 | 380 | 200 | 70 | 2 | 0.1 | 90 |
| Example 3-69 | 250 | 380 | 200 | 70 | 2 | 0.1 | 90 |
| Example 3-70 | 250 | 380 | 200 | 70 | 2 | 0.1 | 90 |
| Example 3-71 | 250 | 380 | 200 | 70 | 2 | 0.1 | 90 |
| Example 3-72 | 250 | 380 | 200 | 70 | 2 | 0.1 | 91 |
| Example 3-73 | 250 | 380 | 200 | 70 | 2 | 0.1 | 91 |
| Example 3-74 | 250 | 380 | 200 | 70 | 2 | 0.1 | 91 |
| Example 3-75 | 250 | 380 | 200 | 70 | 2 | 0.1 | 91 |
| Example 3-76 | 250 | 380 | 200 | 70 | 2 | 0.1 | 91 |
| Example 3-77 | 250 | 380 | 200 | 70 | 2 | 0.1 | 91 |
| Example 3-78 | 250 | 380 | 200 | 70 | 2 | 0.1 | 91 |
| Example 3-79 | 250 | 380 | 200 | 70 | 2 | 0.1 | 89 |
| Example 3-80 | 250 | 380 | 200 | 70 | 2 | 0.1 | 89 |
| Example 3-81 | 250 | 380 | 200 | 70 | 2 | 0.1 | 89 |
| Example 3-82 | 250 | 380 | 200 | 70 | 2 | 0.1 | 89 |
| Example 3-83 | 250 | 380 | 200 | 70 | 2 | 0.1 | 89 |
| Example 3-84 | 250 | 380 | 200 | 70 | 2 | 0.1 | 89 |
| Example 3-85 | 250 | 380 | 200 | 70 | 2 | 0.1 | 89 |

**[Table 3-13]**

| | Reaction temperature | Absolute pressure | Condenser tube 1 temperature | Condenser tube 2 temperature | Reaction time | Oxygen concentration | BTM |
|---|---|---|---|---|---|---|---|
| | °C | mmHg | °C | °C | hours | vol% | parts by mass |
| Example 3-86 | 250 | 380 | 200 | 70 | 2 | 0.1 | 90 |
| Example 3-87 | 250 | 380 | 200 | 70 | 2 | 0.1 | 90 |
| Example 3-88 | 250 | 380 | 200 | 70 | 2 | 0.1 | 90 |
| Example 3-89 | 250 | 380 | 200 | 70 | 2 | 0.1 | 90 |
| Example 3-90 | 250 | 380 | 200 | 70 | 2 | 0.1 | 90 |
| Example 3-91 | 250 | 380 | 200 | 70 | 2 | 0.1 | 90 |
| Example 3-92 | 250 | 380 | 200 | 70 | 2 | 0.1 | 90 |
| Example 3-93 | 250 | 380 | 200 | 70 | 2 | 0.1 | 89 |
| Example 3-94 | 250 | 380 | 200 | 70 | 2 | 0.1 | 89 |
| Example 3-95 | 250 | 380 | 200 | 70 | 2 | 0.1 | 38 |
| Example 3-96 | 250 | 380 | 200 | 70 | 2 | 0.1 | 38 |
| Example 3-97 | 250 | 380 | 200 | 70 | 2 | 0.1 | 87 |
| Example 3-98 | 250 | 380 | 200 | 70 | 2 | 0.1 | 88 |
| Example 3-99 | 250 | 380 | 200 | 70 | 2 | 0.1 | 88 |

**[Table 3-14]**

| | Reaction temperature | Absolute pressure | Condenser tube 1 temperature | Condenser tube 2 temperature | Reaction time | Oxygen concentration | BTM |
|---|---|---|---|---|---|---|---|
| | °C | mmHg | °C | °C | hours | vol% | parts by mass |
| Example 3-100 | 250 | 380 | 200 | 70 | 2 | 0.1 | 39 |
| Example 3-101 | 250 | 380 | 200 | 70 | 2 | 0.1 | 39 |
| Example 3-102 | 250 | 380 | 200 | 70 | 2 | 0.1 | 39 |
| Example 3-103 | 250 | 380 | 200 | 70 | 2 | 0.1 | 39 |
| Example 3-104 | 250 | 380 | 200 | 70 | 2 | 0.1 | 39 |
| Example 3-105 | 250 | 380 | 200 | 70 | 2 | 0.1 | 39 |
| Example 3-106 | 250 | 380 | 200 | 70 | 2 | 0.1 | 39 |
| Example 3-107 | 160 | 380 | 200 | 70 | 2 | 0.1 | 88 |
| Example 3-108 | 180 | 380 | 200 | 70 | 2 | 0.1 | 88 |
| Example 3-109 | 200 | 380 | 200 | 70 | 2 | 0.1 | 88 |
| Example 3-110 | 220 | 380 | 200 | 70 | 2 | 0.1 | 88 |
| Example 3-111 | 240 | 380 | 200 | 70 | 2 | 0.1 | 88 |
| Example 3-112 | 260 | 380 | 200 | 70 | 2 | 0.1 | 88 |
| Example 3-113 | 160 | 380 | 200 | 70 | 2 | 0.1 | 88 |
| Example 3-114 | 180 | 380 | 200 | 70 | 2 | 0.1 | 88 |
| Example 3-115 | 200 | 380 | 200 | 70 | 2 | 0.1 | 88 |
| Example 3-116 | 220 | 380 | 200 | 70 | 2 | 0.1 | 88 |
| Example 3-117 | 240 | 380 | 200 | 70 | 2 | 0.1 | 88 |
| Example 3-118 | 260 | 380 | 200 | 70 | 2 | 0.1 | 88 |
| Example 3-119 | 250 | 380 | 200 | 70 | 2 | 1 | 88 |
| Example 3-120 | 250 | 380 | 200 | 70 | 2 | 0.01 | 88 |
| Example 3-121 | 250 | 380 | 200 | 70 | 2 | 1 | 88 |
| Example 3-122 | 250 | 380 | 200 | 70 | 2 | 0.01 | 88 |

**[Table 3-15]**

| | Reaction temperature | Absolute pressure | Condenser tube 1 temperature | Condenser tube 2 temperature | Reaction time | Oxygen concentration | BTM |
|---|---|---|---|---|---|---|---|
| | °C | mmHg | °C | °C | hours | vol% | parts by mass |
| Comparative Example 3-1 | 250 | 380 | 200 | 70 | 2 | 0.1 | 88 |
| Comparative Example 3-2 | 250 | 380 | 200 | 70 | 2 | 0.1 | 88 |
| Comparative Example 3-3 | 250 | 380 | 200 | 70 | 2 | 0.1 | 88 |
| Comparative Example 3-4 | 250 | 380 | 200 | 70 | 2 | 0.1 | 88 |
| Comparative Example 3-5 | 250 | 380 | 200 | 70 | 2 | 0.1 | 88 |
| Comparative Example 3-6 | 250 | 380 | 200 | 70 | 2 | 0.1 | 88 |
| Comparative Example 3-7 | 250 | 380 | 200 | 70 | 2 | 0.1 | 88 |
| Comparative Example 3-8 | 250 | 380 | 200 | 70 | 2 | 0.1 | 88 |
| Comparative Example 3-9 | 250 | 380 | 200 | 70 | 2 | 0.1 | 88 |
| Comparative Example 3-10 | 250 | 380 | 200 | 70 | 2 | 0.1 | 88 |
| Comparative Example 3-11 | 250 | 380 | 200 | 70 | 2 | 0.1 | 87 |
| Comparative Example 3-12 | 250 | 380 | 200 | 70 | 2 | 0.1 | 90 |
| Comparative Example 3-13 | 250 | 380 | 200 | 70 | 2 | 0.1 | 87 |
| Comparative Example 3-14 | 250 | 380 | 200 | 70 | 2 | 0.1 | 90 |
| Comparative Example 3-15 | 250 | 380 | 200 | 70 | 2 | 0.1 | 87 |
| Comparative Example 3-16 | 250 | 380 | 200 | 70 | 2 | 0.1 | 90 |
| Comparative Example 3-17 | 250 | 380 | 200 | 70 | 2 | 0.1 | 90 |
| Comparative Example 3-18 | 250 | 380 | 200 | 70 | 2 | 0.1 | 90 |
| Comparative Example 3-19 | 250 | 380 | 200 | 70 | 2 | 0.1 | 90 |
| Comparative Example 3-20 | 250 | 380 | 200 | 70 | 2 | 0.1 | 90 |
| Comparative Example 3-21 | 250 | 380 | 200 | 70 | 2 | 0.1 | 90 |

**[Table 3-16]**

| | Reaction temperature | Absolute pressure | Condenser tube 1 temperature | Condenser tube 2 temperature | Reaction time | Oxygen concentration | BTM |
|---|---|---|---|---|---|---|---|
| | °C | mmHg | °C | °C | hours | vol% | parts by mass |
| Comparative Example 3-22 | 250 | 380 | 200 | 70 | 2 | 0.1 | 90 |
| Comparative Example 3-23 | 250 | 380 | 200 | 70 | 2 | 0.1 | 91 |
| Comparative Example 3-24 | 250 | 380 | 200 | 70 | 2 | 0.1 | 91 |
| Comparative Example 3-25 | 250 | 380 | 200 | 70 | 2 | 0.1 | 91 |
| Comparative Example 3-26 | 250 | 380 | 200 | 70 | 2 | 0.1 | 91 |
| Comparative Example 3-27 | 250 | 380 | 200 | 70 | 2 | 0.1 | 91 |
| Comparative Example 3-28 | 250 | 380 | 200 | 70 | 2 | 0.1 | 91 |
| Comparative Example 3-29 | 250 | 380 | 200 | 70 | 2 | 0.1 | 91 |
| Comparative Example 3-30 | 250 | 380 | 200 | 70 | 2 | 0.1 | 89 |
| Comparative Example 3-31 | 250 | 380 | 200 | 70 | 2 | 0.1 | 89 |
| Comparative Example 3-32 | 250 | 380 | 200 | 70 | 2 | 0.1 | 89 |
| Comparative Example 3-33 | 250 | 380 | 200 | 70 | 2 | 0.1 | 89 |
| Comparative Example 3-34 | 250 | 380 | 200 | 70 | 2 | 0.1 | 89 |
| Comparative Example 3-35 | 250 | 380 | 200 | 70 | 2 | 0.1 | 89 |
| Comparative Example 3-36 | 250 | 380 | 200 | 70 | 2 | 0.1 | 89 |
| Comparative Example 3-37 | 250 | 380 | 200 | 70 | 2 | 0.1 | 90 |
| Comparative Example 3-38 | 250 | 380 | 200 | 70 | 2 | 0.1 | 90 |
| Comparative Example 3-39 | 250 | 380 | 200 | 70 | 2 | 0.1 | 90 |
| Comparative Example 3-40 | 250 | 380 | 200 | 70 | 2 | 0.1 | 90 |
| Comparative Example 3-41 | 250 | 380 | 200 | 70 | 2 | 0.1 | 90 |
| Comparative Example 3-42 | 250 | 380 | 200 | 70 | 2 | 0.1 | 90 |
| Comparative Example 3-43 | 250 | 380 | 200 | 70 | 2 | 0.1 | 90 |

**[Table 3-17]**

| | NCO-BL(A) | NCO-BL(C) | NCO-compound (I) | MB | Hazen color number | TOP |
|---|---|---|---|---|---|---|
| | mol% | mol% | mol% | mol% | APHA | parts by mass |
| Example 3-1 | 0 | 99 | 0 | 99 | 3 | 12 |
| Example 3-2 | 0 | 98 | 0 | 98 | 3 | 12 |
| Example 3-3 | 0 | 99 | 0 | 99 | 3 | 12 |
| Example 3-4 | 0 | 98 | 0 | 98 | 3 | 12 |
| Example 3-5 | 0 | 98 | 0 | 98 | 4 | 12 |
| Example 3-6 | 0 | 99 | 0 | 99 | 4 | 12 |
| Example 3-7 | 0 | 98 | 0 | 98 | 4 | 12 |
| Example 3-8 | 0 | 98 | 0 | 98 | 4 | 12 |
| Example 3-9 | 0 | 98 | 0 | 98 | 3 | 12 |
| Example 3-10 | 0 | 98 | 0 | 98 | 3 | 12 |
| Example 3-11 | 0 | 98 | 0 | 98 | 3 | 12 |
| Example 3-12 | 0 | 98 | 0 | 98 | 3 | 12 |
| Example 3-13 | 0 | 98 | 0 | 98 | 3 | 12 |
| Example 3-14 | 0 | 81 | 0 | 81 | 3 | 12 |
| Example 3-15 | 0 | 99 | 0 | 99 | 3 | 12 |
| Example 3-16 | 0 | 98 | 0 | 98 | 3 | 12 |
| Example 3-17 | 0 | 98 | 0 | 98 | 3 | 12 |
| Example 3-18 | 0 | 98 | 0 | 98 | 3 | 13 |
| Example 3-19 | 0 | 92 | 0 | 92 | 3 | 10 |
| Example 3-20 | 0 | 80 | 0 | 80 | 3 | 13 |
| Example 3-21 | 0 | 83 | 0 | 83 | 4 | 10 |
| Example 3-22 | 0 | 65 | 0 | 65 | 5 | 13 |
| Example 3-23 | 0 | 98 | 0 | 98 | 3 | 10 |
| Example 3-24 | 0 | 98 | 0 | 98 | 3 | 10 |
| Example 3-25 | 0 | 99 | 0 | 99 | 3 | 10 |

**[Table 3-18]**

| | NCO-BL(A) | NCO-BL(C) | NCO-compound (I) | MB | Hazen color number | TOP |
|---|---|---|---|---|---|---|
| | mol% | mol% | mol% | mol% | APHA | parts by mass |
| Example 3-26 | 0 | 98 | 0 | 98 | 3 | 10 |
| Example 3-27 | 0 | 98 | 0 | 98 | 3 | 10 |
| Example 3-28 | 0 | 98 | 0 | 98 | 3 | 10 |
| Example 3-29 | 0 | 84 | 0 | 84 | 3 | 10 |
| Example 3-30 | 0 | 98 | 0 | 98 | 3 | 9 |
| Example 3-31 | 0 | 99 | 0 | 99 | 3 | 9 |
| Example 3-32 | 0 | 99 | 0 | 99 | 3 | 9 |
| Example 3-33 | 0 | 98 | 0 | 98 | 3 | 9 |
| Example 3-34 | 0 | 98 | 0 | 98 | 3 | 9 |
| Example 3-35 | 0 | 98 | 0 | 98 | 3 | 9 |
| Example 3-36 | 0 | 86 | 0 | 86 | 3 | 9 |
| Example 3-37 | 0 | 99 | 0 | 99 | 3 | 11 |
| Example 3-38 | 0 | 99 | 0 | 99 | 3 | 11 |
| Example 3-39 | 0 | 98 | 0 | 98 | 3 | 11 |
| Example 3-40 | 0 | 98 | 0 | 98 | 3 | 11 |
| Example 3-41 | 0 | 98 | 0 | 98 | 3 | 11 |
| Example 3-42 | 0 | 98 | 0 | 98 | 3 | 11 |
| Example 3-43 | 0 | 74 | 0 | 74 | 3 | 11 |
| Example 3-44 | 0 | 99 | 0 | 99 | 3 | 10 |
| Example 3-45 | 0 | 99 | 0 | 99 | 3 | 10 |

**[Table 3-19]**

| | NCO-BL(A) | NCO-BL(C) | NCO-compound (I) | MB | Hazen color number | TOP |
|---|---|---|---|---|---|---|
| | mol% | mol% | mol% | mol% | APHA | parts by mass |
| Example 3-46 | 0 | 98 | 0 | 98 | 3 | 10 |
| Example 3-47 | 0 | 98 | 0 | 98 | 3 | 10 |
| Example 3-48 | 0 | 98 | 0 | 98 | 3 | 10 |
| Example 3-49 | 0 | 98 | 0 | 98 | 3 | 10 |
| Example 3-50 | 0 | 75 | 0 | 75 | 3 | 10 |
| Example 3-51 | 0 | 99 | 0 | 99 | 4 | 12 |
| Example 3-52 | 0 | 98 | 0 | 98 | 4 | 12 |
| Example 3-53 | 0 | 99 | 0 | 99 | 4 | 12 |
| Example 3-54 | 0 | 99 | 0 | 99 | 4 | 12 |
| Example 3-55 | 0 | 99 | 0 | 99 | 4 | 12 |
| Example 3-56 | 0 | 81 | 0 | 81 | 4 | 12 |
| Example 3-57 | 0 | 99 | 0 | 99 | 4 | 12 |
| Example 3-58 | 0 | 99 | 0 | 99 | 4 | 12 |
| Example 3-59 | 0 | 99 | 0 | 99 | 4 | 12 |
| Example 3-60 | 0 | 99 | 0 | 99 | 4 | 13 |
| Example 3-61 | 0 | 93 | 0 | 93 | 4 | 10 |
| Example 3-62 | 0 | 80 | 0 | 80 | 4 | 13 |
| Example 3-63 | 0 | 83 | 0 | 83 | 5 | 10 |
| Example 3-64 | 0 | 65 | 0 | 65 | 6 | 13 |
| Example 3-65 | 0 | 98 | 0 | 98 | 4 | 10 |

**[Table 3-20]**

| | NCO-BL(A) | NCO-BL(C) | NCO-compound (I) | MB | Hazen color number | TOP |
|---|---|---|---|---|---|---|
| | mol% | mol% | mol% | mol% | APHA | parts by mass |
| Example 3-66 | 0 | 99 | 0 | 99 | 4 | 10 |
| Example 3-67 | 0 | 98 | 0 | 98 | 4 | 10 |
| Example 3-68 | 0 | 98 | 0 | 98 | 4 | 10 |
| Example 3-69 | 0 | 99 | 0 | 99 | 4 | 10 |
| Example 3-70 | 0 | 98 | 0 | 98 | 4 | 10 |
| Example 3-71 | 0 | 85 | 0 | 85 | 4 | 10 |
| Example 3-72 | 0 | 98 | 0 | 98 | 4 | 9 |
| Example 3-73 | 0 | 99 | 0 | 99 | 4 | 9 |
| Example 3-74 | 0 | 98 | 0 | 98 | 4 | 9 |
| Example 3-75 | 0 | 98 | 0 | 98 | 4 | 9 |
| Example 3-76 | 0 | 99 | 0 | 99 | 4 | 9 |
| Example 3-77 | 0 | 98 | 0 | 98 | 4 | 9 |
| Example 3-78 | 0 | 86 | 0 | 86 | 4 | 9 |
| Example 3-79 | 0 | 99 | 0 | 99 | 4 | 11 |
| Example 3-80 | 0 | 98 | 0 | 98 | 4 | 11 |
| Example 3-81 | 0 | 99 | 0 | 99 | 4 | 11 |
| Example 3-82 | 0 | 99 | 0 | 99 | 4 | 11 |
| Example 3-83 | 0 | 99 | 0 | 99 | 4 | 11 |
| Example 3-84 | 0 | 99 | 0 | 99 | 4 | 11 |
| Example 3-85 | 0 | 74 | 0 | 74 | 4 | 11 |

**[Table 3-21]**

| | NCO-BL(A) | NCO-BL(C) | NCO-compound (I) | MB | Hazen color number | TOP |
|---|---|---|---|---|---|---|
| | mol% | mol% | mol% | mol% | APHA | parts by mass |
| Example 3-86 | 0 | 99 | 0 | 99 | 4 | 10 |
| Example 3-87 | 0 | 98 | 0 | 98 | 4 | 10 |
| Example 3-88 | 0 | 98 | 0 | 98 | 4 | 10 |
| Example 3-89 | 0 | 99 | 0 | 99 | 4 | 10 |
| Example 3-90 | 0 | 98 | 0 | 98 | 4 | 10 |
| Example 3-91 | 0 | 99 | 0 | 99 | 4 | 10 |
| Example 3-92 | 0 | 75 | 0 | 75 | 4 | 10 |
| Example 3-93 | 0 | 98 | 0 | 98 | 4 | 11 |
| Example 3-94 | 0 | 98 | 0 | 98 | 5 | 11 |
| Example 3-95 | 0 | 98 | 0 | 98 | 3 | 62 |
| Example 3-96 | 0 | 98 | 0 | 98 | 4 | 62 |
| Example 3-97 | 0 | 98 | 0 | 98 | 1 | 13 |
| Example 3-98 | 0 | 99 | 0 | 99 | 2 | 12 |
| Example 3-99 | 0 | 98 | 0 | 98 | 2 | 12 |

**[Table 3-22]**

| | NCO-BL(A) | NCO-BL(C) | NCO-compound (I) | MB | Hazen color number | TOP |
|---|---|---|---|---|---|---|
| | mol% | mol% | mol% | mol% | APHA | parts by mass |
| Example 3-100 | 0 | 98 | 0 | 98 | 1 | 61 |
| Example 3-101 | 0 | 98 | 0 | 98 | 1 | 61 |
| Example 3-102 | 0 | 98 | 0 | 98 | 1 | 61 |
| Example 3-103 | 0 | 98 | 0 | 98 | 1 | 61 |
| Example 3-104 | 0 | 98 | 0 | 98 | 1 | 61 |
| Example 3-105 | 0 | 98 | 0 | 98 | 1 | 61 |
| Example 3-106 | 0 | 98 | 0 | 98 | 1 | 61 |
| Example 3-107 | 0 | 98 | 0 | 98 | 5 | 12 |
| Example 3-108 | 0 | 98 | 0 | 98 | 5 | 12 |
| Example 3-109 | 0 | 98 | 0 | 98 | 4 | 12 |
| Example 3-110 | 0 | 98 | 0 | 98 | 4 | 12 |
| Example 3-111 | 0 | 98 | 0 | 98 | 4 | 12 |
| Example 3-112 | 0 | 98 | 0 | 98 | 3 | 12 |
| Example 3-113 | 0 | 98 | 0 | 98 | 4 | 12 |
| Example 3-114 | 0 | 98 | 0 | 98 | 4 | 12 |
| Example 3-115 | 0 | 98 | 0 | 98 | 4 | 12 |
| Example 3-116 | 0 | 98 | 0 | 98 | 4 | 12 |
| Example 3-117 | 0 | 98 | 0 | 98 | 4 | 12 |
| Example 3-118 | 0 | 98 | 0 | 98 | 4 | 12 |
| Example 3-119 | 0 | 98 | 0 | 98 | 4 | 12 |
| Example 3-120 | 0 | 98 | 0 | 98 | 2 | 12 |
| Example 3-121 | 0 | 98 | 0 | 98 | 5 | 12 |
| Example 3-122 | 0 | 98 | 0 | 98 | 4 | 12 |

**[Table 3-23]**

| | NCO-BL(A) | NCO-BL(C) | NCO-compound (I) | MB | Hazen color number | TOP |
|---|---|---|---|---|---|---|
| | mol% | mol% | mol% | mol% | APHA | parts by mass |
| Comparative Example 3-1 | 0 | 98 | 0 | 98 | 8 | 11 |
| Comparative Example 3-2 | 0 | 98 | 0 | 98 | 8 | 11 |
| Comparative Example 3-3 | 0 | 98 | 0 | 98 | 8 | 11 |
| Comparative Example 3-4 | 0 | 98 | 0 | 98 | 8 | 11 |
| Comparative Example 3-5 | 0 | 98 | 0 | 98 | 8 | 11 |
| Comparative Example 3-6 | 0 | 98 | 0 | 98 | 9 | 11 |
| Comparative Example 3-7 | 0 | 81 | 0 | 81 | 10 | 11 |
| Comparative Example 3-8 | 0 | 98 | 0 | 98 | 8 | 11 |
| Comparative Example 3-9 | 0 | 98 | 0 | 98 | 8 | 11 |
| Comparative Example 3-10 | 0 | 98 | 0 | 98 | 8 | 11 |
| Comparative Example 3-11 | 0 | 98 | 0 | 98 | 9 | 12 |
| Comparative Example 3-12 | 0 | 92 | 0 | 92 | 9 | 9 |
| Comparative Example 3-13 | 0 | 79 | 0 | 79 | 10 | 12 |
| Comparative Example 3-14 | 0 | 83 | 0 | 83 | 9 | 10 |
| Comparative Example 3-15 | 0 | 65 | 0 | 65 | 10 | 13 |
| Comparative Example 3-16 | 0 | 98 | 0 | 98 | 8 | 9 |
| Comparative Example 3-17 | 0 | 98 | 0 | 98 | 8 | 9 |
| Comparative Example 3-18 | 0 | 98 | 0 | 98 | 8 | 9 |
| Comparative Example 3-19 | 0 | 98 | 0 | 98 | 8 | 9 |
| Comparative Example 3-20 | 0 | 98 | 0 | 98 | 8 | 9 |
| Comparative Example 3-21 | 0 | 98 | 0 | 98 | 9 | 9 |

**[Table 3-24]**

| | NCO-BL(A) | NCO-BL(C) | NCO-compound (I) | MB | Hazen color number | TOP |
|---|---|---|---|---|---|---|
| | mol% | mol% | mol% | mol% | APHA | parts by mass |
| Comparative Example 3-22 | 0 | 84 | 0 | 84 | 10 | 9 |
| Comparative Example 3-23 | 0 | 98 | 0 | 98 | 8 | 8 |
| Comparative Example 3-24 | 0 | 98 | 0 | 98 | 8 | 8 |
| Comparative Example 3-25 | 0 | 98 | 0 | 98 | 8 | 8 |
| Comparative Example 3-26 | 0 | 98 | 0 | 98 | 8 | 8 |
| Comparative Example 3-27 | 0 | 98 | 0 | 98 | 8 | 8 |
| Comparative Example 3-28 | 0 | 98 | 0 | 98 | 9 | 8 |
| Comparative Example 3-29 | 0 | 86 | 0 | 86 | 10 | 8 |
| Comparative Example 3-30 | 0 | 98 | 0 | 98 | 9 | 10 |
| Comparative Example 3-31 | 0 | 98 | 0 | 98 | 9 | 10 |
| Comparative Example 3-32 | 0 | 98 | 0 | 98 | 9 | 10 |
| Comparative Example 3-33 | 0 | 98 | 0 | 98 | 9 | 10 |
| Comparative Example 3-34 | 0 | 98 | 0 | 98 | 9 | 10 |
| Comparative Example 3-35 | 0 | 98 | 0 | 98 | 10 | 10 |
| Comparative Example 3-36 | 0 | 74 | 0 | 74 | 10 | 10 |
| Comparative Example 3-37 | 0 | 98 | 0 | 98 | 8 | 9 |
| Comparative Example 3-38 | 0 | 98 | 0 | 98 | 8 | 9 |
| Comparative Example 3-39 | 0 | 98 | 0 | 98 | 8 | 9 |
| Comparative Example 3-40 | 0 | 98 | 0 | 98 | 8 | 9 |
| Comparative Example 3-41 | 0 | 98 | 0 | 98 | 8 | 9 |
| Comparative Example 3-42 | 0 | 98 | 0 | 98 | 9 | 9 |
| Comparative Example 3-43 | 0 | 74 | 0 | 74 | 10 | 9 |

As shown in the above tables, the Hazen color numbers (APHA) of the blocked isocyanate compositions obtained by conducting the addition-elimination reaction in a reaction system in whic at least one compound selected from the group consisting of the secondary amine compounds (1) and the tertiary amine compounds (II) had been added (Examples 3-1 to 3-122) were evaluated as rank of 7 or lower, which were favorable.

In contrast, the Hazen color numbers of the blocked isocyanate compositions obtained by conducting the addition-elimination reaction without the addition of either the secondary amine compound (1) or the tertiary amine compound (II) (Comparative Examples 3-1 to 3-43) were evaluated as rank 8 or higher, which were unfavorable.

Further, among the blocked isocyanate compositions obtained by conducting the addition-elimination reaction in the presence of the secondary amine compound (1) (Examples 3-2 and 3-9 to 3-50), the blocked isocyanate compositions obtained by conducting the addition-elimination reaction in a reaction system in which one of the first blocking agent and the second blocking agent was a phenol-based blocking agent (Examples 3-2, 3-9 to 3-20, and 3-23 to 3-50) exhibited Hazen color numbers evaluated as the lowest rank, which were particularly favorable.

Further, among the blocked isocyanate compositions obtained by conducting the addition-elimination reaction in the presence of the tertiary amine compound (II) (Examples 3-6 and 3-51 to 3-92), the blocked isocyanate compositions obtained by conducting the addition-elimination reaction in a reaction system in which one of the first blocking agent and the second blocking agent was a phenol-based blocking agent (Examples 3-6, 3-51 to 3-62, and 3-65 to 3-92) exhibited Hazen color numbers evaluated as the lowest rank, which were particularly favorable.

Further, among the blocked isocyanate compositions in which the amounts added of the secondary amine compound (1) or the tertiary amine compound (ll) were different (Examples 3-1, 3-5, and 3-93 to 3-96), the blocked isocyanate compositions obtained by conducting the addition-elimination reaction in a reaction system in which the secondary amine compound (1) or the tertiary amine compound (ll) had been added in an amount of at least 1 ppm by mass relative to the total mass of the reaction liquid (Examples 3-93 and 3-94) exhibited Hazen color numbers evaluated as rank 5 or lower, which were more favorable.

Furthermore, among the blocked isocyanate compositions in which the amounts added of the secondary amine compound (1) or the tertiary amine compound (ll) were different (Examples 3-1, 3-5, and 3-93 to 3-96), the blocked isocyanate compositions obtained by conducting the addition-elimination reaction in a reaction system in which the secondary amine compound (1) or the tertiary amine compound (ll) had been added in an amount of at least 1 % by mass relative to the total mass of the reaction liquid (Examples 3-1 and 3-5) exhibited Hazen color numbers evaluated as rank 4 or lower, which were particularly favorable.

Furthermore, the blocked isocyanate compositions obtained by conducting the addition-elimination reaction in a reaction system in which both the secondary amine compound (I) and the tertiary amine compound (II) had been added (Examples 3-97 to 3-106) exhibited Hazen color numbers evaluated as rank 2 or lower, which were the most favorable.

Furthermore, comparison of blocked isocyanate compositions prepared at different reaction temperatures (Examples 3-1, and 3-107 to 3-112) revealed that the ranks of the Hazen color numbers of blocked isocyanate compositions obtained by conducting the addition-elimination reaction in the presence of the secondary amine compound (I) depended on the reaction temperature, and the coloration reduction effect was improved as the reaction temperature was increased.

In contrast, comparison of blocked isocyanate compositions prepared at different reaction temperatures (Examples 3-5, and 3-113 to 3-118) revealed that the ranks of the Hazen color numbers of blocked isocyanate compositions obtained by conducting the addition-elimination reaction in the presence of the tertiary amine compound (II) did not significantly depend on the reaction temperature.

Further, comparison of blocked isocyanate compositions prepared under different oxygen concentrations (Examples 3-1, and 3-119 and 3-120) revealed that the ranks of the Hazen color numbers of blocked isocyanate compositions obtained by conducting the addition-elimination reaction in the presence of the secondary amine compound (1) depended on the oxygen concentration, and the coloration reduction effect was improved as the oxygen concentration was decreased.

Further, comparison of blocked isocyanate compositions prepared under different oxygen concentrations (Examples 3-5, and 3-121 and 3-122) revealed that the ranks of the Hazen color numbers of blocked isocyanate compositions obtained by conducting the addition-elimination reaction in the presence of the tertiary amine compound (ll) depended on the oxygen concentration, and the coloration reduction effect was improved as the oxygen concentration was decreased.

### <<Fourth Test>>

### [Physical Property 4-1]

### (Amount of Isocyanate Groups Blocked with First Blocking Agent)

The amount (mol%) of isocyanate groups blocked with the first blocking agent in the blocked isocyanate composition obtained in the first reaction step and the isocyanate composition obtained in the second reaction step (hereinafter sometimes abbreviated as "NCO-BL(A) mol%") was determined in accordance with the formula shown below after the concentration of carbon skeletons derived from the primary amine was determined from an NMR spectrum, and then the molar amount of amino groups of the primary amine compound was calculated from the product of the valency of the primary amine amino groups and the concentration of carbon skeletons derived from the primary amine. The molar amount of isocyanate groups blocked with the first blocking agent was determined from an NMR spectrum.[NCO-BL(A) mol%] = (molar amount of isocyanate groups blocked with the first blocking agent) × 100 / (molar amount of amino groups derived from primary amine)

### [Physical Property 4-2]

### (Amount of Isocyanate Groups Bonded to Secondary Amine Compound (I))

The amount (mol%) of isocyanate groups bonded to the secondary amine compound (I) in the blocked isocyanate composition obtained in the first reaction step and the isocyanate composition obtained in the second reaction step (hereinafter sometimes abbreviated as "NCO-BL(I) mol%") was determined in accordance with the formula shown below after the concentration of carbon skeletons derived from the primary amine was determined from an NMR spectrum, and then the molar amount of amino groups of the primary amine compound was calculated from the product of the valency of the primary amine amino groups and the concentration of carbon skeletons derived from the primary amine. The molar amount of isocyanate groups bonded to the secondary amine compound (I) was determined from an NMR spectrum.[NCO-BL(I) mol%] = (molar amount of isocyanate groups bonded to the secondary amine compound (I)) × 100 / (molar amount of amino groups derived from primary amine)

### [Physical Property 4-3]

### (Ureido Group Content)

The ureido group content (mol%) within the blocked isocyanate composition obtained in the first reaction step and the isocyanate composition obtained in the second reaction step (hereinafter sometimes abbreviated as "ureido group mol%") was determined in accordance with the formula shown below after the concentration of carbon skeletons derived from the primary amine was determined from an NMR spectrum, and then the molar amount of amino groups of the primary amine compound was calculated from the product of the valency of the primary amine amino groups and the concentration of carbon skeletons derived from the primary amine. The molar amount of ureido groups was determined from an NMR spectrum.[Ureido group mol%] = (molar amount of ureido groups) × 100 / (molar amount of amino groups derived from primary amine)

### [Physical Property 4-4]

### (Amino Group Content)

The amino group content (mol%) within the blocked isocyanate composition obtained in the first reaction step and the isocyanate composition obtained in the second reaction step (hereinafter sometimes abbreviated as "amino group mol%") was determined in accordance with the formula shown below after the concentration of carbon skeletons derived from the primary amine was determined from an NMR spectrum, and then the molar amount of amino groups of the primary amine compound was calculated from the product of the valency of the primary amine amino groups and the concentration of carbon skeletons derived from the primary amine. The molar amount of amino groups was determined from an NMR spectrum.[Amino group mol%] = (molar amount of amino groups) / (molar amount of amino groups derived from primary amine)

### [Physical Property 4-5]

### (Isocyanate Group Content)

The isocyanate group content (mol%) within the blocked isocyanate composition obtained in the first reaction step and the isocyanate composition obtained in the second reaction step (hereinafter sometimes abbreviated as "NCO mol%") was determined in accordance with the formula shown below after the concentration of carbon skeletons derived from the primary amine was determined from an NMR spectrum, and then the molar amount of amino groups of the primary amine compound was calculated from the product of the valency of the primary amine amino groups and the concentration of carbon skeletons derived from the primary amine. The molar amount of isocyanate groups was determined from an NMR spectrum.[NCO mol%] = (molar amount of isocyanate groups) / (molar amount of amino groups derived from primary amine)

### [Evaluation 4-1]

### (Mass Balance)

The sum, within the obtained blocked isocyanate composition, of the amino group mol%, ureido group, NCO group and NCO-BL(A) mol%, NCO-BL(C) mol% and NCO-BL(l) mol% was determined as the mass balance (hereinafter sometimes abbreviated as "MB mol%").

### [Evaluation 4-2]

### (Hazen Color Number)

The Hazen color number was determined by dissolving, in 2 g of acetonitrile, 1 g of the blocked isocyanate composition obtained following removal by distillation of any free compound (I), compound (II), the first blocking agent, and solvent contained in the obtained reaction solution. Based on the value measured with a Hazen meter, the resultant was ranked in accordance with the following evaluation criteria.

### (Evaluation Criteria)

Rank 1: APHA of at least 0 but less than 5
Rank 2: APHA of at least 5 but less than 10
Rank 3: APHA of at least 10 but less than 15
Rank 4: APHA of at least 15 but less than 20
Rank 5: APHA of at least 20 but less than 25
Rank 6: APHA of at least 25 but less than 30
Rank 7: APHA of at least 30 but less than 35
Rank 8: APHA of at least 35 but less than 40
Rank 9: APHA of at least 40 but less than 45
Rank 10: APHA of at least 45 but less than 50
Rank 11: APHA of at least 50 but less than 55

### [Evaluation 4-3]

### (Isocyanate group content)

The isocyanate group content was determined by GC analysis.

### [Evaluation 4-4]

### (Isocyanate Yield)

The molar amount of carbon skeletons derived from the primary amine contained within the raw materials in the second reaction step was determined from an NMR spectrum, and the isocyanate yield was then determined from the molar amount of isocyanate compound obtained in the BTM of the condenser tube 2, in accordance with the formula shown below. The molar amount of isocyanate compound was determined from the product of the isocyanate content and the total mass of the isocyanate composition.[Isocyanate yield (mol%)] = (molar amount of isocyanate compound obtained in BTM of condenser tube 2) × 100 / (molar amount of carbon skeletons derived from primary amine)

### [Example 4-1]

10 parts by mass of hexamethylenediamine (HDA), 10.9 parts by mass of urea, 90 parts by mass of phenol, and 1 part by mass of N-methylaniline (NMA) were placed in a 500 mL SUS pressure-resistant vessel equipped with a condenser tube packed with a filler (the condenser tube having a structure that allows the condensed liquid from the condenser tube to fall into the 500 mL SUS pressure-resistant vessel) and an ammonia trap, the internal temperature of the 500 mL pressure-resistant vessel was set to 240°C, and the temperature of the condenser tube was set to 60°C. The nitrogen gas was supplied to the 500 mL SUS pressure-resistant vessel from a nitrogen cylinder to adjust the absolute pressure to 350 kPa, followed by allowing the reaction to proceed for three hours. As a result, 105.7 parts by mass of a reaction liquid 1 was obtained in the 500 mL SUS pressure-resistant vessel. The results of analyses revealed an amino group content of 0 mol%, and, based on ¹H NMR integral values, NCO-BL mol% of 92 mol%, NCO-compound (I) mol% of 5 mol%, ureido group of 1 mol%, and MB mol% of 98 mol%. Further, when he Hazen color number was determined, the result was evaluated as rank 2. Furthermore, free phenol and free N-methylaniline contained in the obtained reaction liquid were removed by distillation under reduced pressure, thereby obtaining a low boiling point component-free blocked isocyanate composition.

Next, 10 parts by mass of the thus obtained low boiling point component-free blocked isocyanate composition, 89 parts by mass of a naphthene-based solvent (EXXSOL^{™} D80) and 1 part by mass of N-methylaniline were placed in a 300 mL glass vessel equipped with a condenser tube 1 packed with a filler (the condenser tube 1 having a structure that allows the condensed liquid from the condenser tube 1 to fall into the 300 mL glass vessel), a condenser tube 2 (the condenser tube having a structure that allow the condensed liquid from the condenser tube 2 to be collected as a TOP liquid without enteringe the 300 mL glass vessel) and a pressure reduction device provided at the tip thereof. Subsequently, the internal temperature of the 300 mL glass vessel was set to 220°C, the temperature of the condenser tube 1 was set to 220°C, and the temperature of the condenser tube 2 was set to 50°C, under a nitrogen gas atmosphere. Further, the pressure inside the reaction vessel was adjusted to an absolute pressure of 700 mmHg with the pressure reduction device, and then the reaction was allowed to proceed for two hours. As a result, 50 parts by mass of a thermally decomposed reaction liquid was obtained in the 300 mL glass vessel, and 50 parts by mass of the TOP liquid were collected from the condenser tube 2. The obtained thermally decomposed reaction liquid had an NCO mol% of 92 mol%, an amino group mol% of 0 mol%, NCO-BL(A) mol% of 0 mol%, NCO-compound (1) mol% of 1 mol%, an ureido group mol% of 0 mol%, and MB mol% of 93 mol%. When the Hazen color number of the obtained isocyanate composition was measured, the resulta was evaluated as rank 2. After the isocyanate functional groups contained in the thermally decomposed reaction liquid were quenched with aniline, hexamethylene diisocyanate was quantified as 1,1'-(hexane-1,6-diyl)bis(3-phenylurea) by LC analysis, thereby confirming that hexamethylene diisocyanate had been produced with a yield of 85 mol% relative to the concentration of skeletons derived from the primary amine compound.

### [Examples 4-2 to 4-58, and Comparative Examples 4-1 to 4-18]

In each of Examples 4-2 to 4-58 and Comparative Examples 4-1 to 4-18, isocyanate compounds were produced using the same method as that described in Example 4-1, except that the raw materials and reaction conditions were altered as shown in the following tables.

In the following tables, the abbreviations used refer to the following compounds.

### (Secondary Amine Compounds (I))

NMA: N-methyl-aniline
NEA: N-ethyl-aniline
NBA: N-butyl-aniline
DPA: N,N-diphenylamine

### (Tertiary Amine Compounds (II))

NNDMA: N,N-dimethylaniline
NNDEA: N,N-diethylaniline
NMDPA: N-methyl-N.N-diphenylamine
TPA: triphenylamine

### (Blocked Isocyanate Compound)

BL-NCO: blocked isocyanate compound

### (Blocking Agents A)

PhOH: phenol
PhOH: phenol
2-ETH: 2-ethylhexanol
ECHA: N-ethyl-cyclohexylamine

### (Primary Amine Compounds)

HDA: 1,6-hexamethylenediamine
IPDA: isophoronediamine (isomeric mixture)
MBCA: 4,4'-methylenebis(cyclohexylamine) (isomeric mixture)
DAT: diaminotoluene (isomeric mixture)
DPM: 4,4'-diphenylmethanediamine
TAN: 4-aminomethyl-1,8-octanediamine

### (Solvents)

Naphthene: naphthene-based solvent (EXXSOL^{™} D80)
BP: benzophenone
DPE: diphenyl ether

### (Functional Groups)

NCO-compound (A): blocked isocyanate group obtained by bonding of first blocking agent (compound (A)) to isocyanate group
NCO-compound (I): blocked isocyanate group obtained by bonding of secondary amine compound (I) to isocyanate group

**[Table 4-1]**

| | First reaction step - Raw materials | | | | | | | | | |
|---|---|---|---|---|---|---|---|---|---|---|
| | Secondary amine compound (I) | | Tertiary amine compound (II) | | Primary amine compound | | First blocking agent | | Carbonic acid derivative | |
| | type | parts by mass | type | parts by mass | type | parts by mass | type | parts by mass | type | parts by mass |
| Example 4-1 | NMA | 1 | - | 0 | HDA | 10 | PhOH | 90 | urea | 10.9 |
| Example 4-2 | NEA | 1 | - | 0 | HDA | 10 | PhOH | 90 | urea | 10.9 |
| Example 4-3 | NBA | 1 | - | 0 | HDA | 10 | PhOH | 90 | urea | 10.9 |
| Example 4-4 | DPA | 1 | - | 0 | HDA | 10 | PhOH | 90 | urea | 10.9 |
| Example 4-5 | - | 0 | NNDMA | 1 | HDA | 10 | PhOH | 90 | urea | 10.9 |
| Example 4-6 | - | 0 | NNDEA | 1 | HDA | 10 | PhOH | 90 | urea | 10.9 |
| Example 4-7 | - | 0 | NMDPA | 1 | HDA | 10 | PhOH | 90 | urea | 10.9 |
| Example 4-8 | - | 0 | TPA | 1 | HDA | 10 | PhOH | 90 | urea | 10.9 |
| Example 4-9 | NEA | 1 | - | 0 | HDA | 10 | 2-ETH | 90 | urea | 10.9 |
| Example 4-10 | NEA | 1 | - | 0 | HDA | 10 | ECHA | 90 | urea | 10.9 |
| Example 4-11 | NEA | 1 | - | 0 | TAN | 10 | PhOH | 90 | urea | 7.3 |
| Example 4-12 | NEA | 1 | - | 0 | TAN | 10 | 2-ETH | 90 | urea | 7.3 |
| Example 4-13 | NEA | 1 | - | 0 | TAN | 10 | ECHA | 90 | urea | 7.3 |
| Example 4-14 | NEA | 1 | - | 0 | IPDA | 10 | PhOH | 90 | urea | 7.4 |
| Example 4-15 | NEA | 1 | - | 0 | IPDA | 10 | 2-ETH | 90 | urea | 7.4 |
| Example 4-16 | NEA | 1 | - | 0 | IPDA | 10 | ECHA | 90 | urea | 7.4 |
| Example 4-17 | NEA | 1 | - | 0 | MBCA | 10 | PhOH | 90 | urea | 6.0 |
| Example 4-18 | NEA | 1 | - | 0 | MBCA | 10 | 2-ETH | 90 | urea | 6.0 |
| Example 4-19 | NEA | 1 | - | 0 | MBCA | 10 | ECHA | 90 | urea | 6.0 |
| Example 4-20 | NEA | 1 | - | 0 | DAT | 10 | PhOH | 90 | urea | 10.3 |
| Example 4-21 | NEA | 1 | - | 0 | DAT | 10 | 2-ETH | 90 | urea | 10.3 |
| Example 4-22 | NEA | 1 | - | 0 | DAT | 10 | ECHA | 90 | urea | 10.3 |
| Example 4-23 | NEA | 1 | - | 0 | DPM | 10 | PhOH | 90 | urea | 6.4 |
| Example 4-24 | NEA | 1 | - | 0 | DPM | 10 | 2-ETH | 90 | urea | 6.4 |
| Example 4-25 | NEA | 1 | - | 0 | DPM | 10 | ECHA | 90 | urea | 6.4 |

**[Table 4-2]**

| | First reaction step - Raw materials | | | | | | | | | |
|---|---|---|---|---|---|---|---|---|---|---|
| | Secondary amine compound (I) | | Tertiary amine compound (II) | | Primary amine compound | | First blocking agent | | Carbonic acid derivative | |
| | type | parts by mass | type | parts by mass | type | parts by mass | type | parts by mass | type | parts by mass |
| Example 4-26 | - | 0 | NNDEA | 1 | HDA | 10 | 2-ETH | 90 | urea | 10.9 |
| Example 4-27 | - | 0 | NNDEA | 1 | HDA | 10 | ECHA | 90 | urea | 10.9 |
| Example 4-28 | - | 0 | NNDEA | 1 | TAN | 10 | PhOH | 90 | urea | 7.3 |
| Example 4-29 | - | 0 | NNDEA | 1 | TAN | 10 | 2-ETH | 90 | urea | 7.3 |
| Example 4-30 | - | 0 | NNDEA | 1 | TAN | 10 | ECHA | 90 | urea | 7.3 |
| Example 4-31 | - | 0 | NNDEA | 1 | IPDA | 10 | PhOH | 90 | urea | 7.4 |
| Example 4-32 | - | 0 | NNDEA | 1 | IPDA | 10 | 2-ETH | 90 | urea | 7.4 |
| Example 4-33 | - | 0 | NNDEA | 1 | IPDA | 10 | ECHA | 90 | urea | 7.4 |
| Example 4-34 | - | 0 | NNDEA | 1 | MBCA | 10 | PhOH | 90 | urea | 6.0 |
| Example 4-35 | - | 0 | NNDEA | 1 | MBCA | 10 | 2-ETH | 90 | urea | 6.0 |
| Example 4-36 | - | 0 | NNDEA | 1 | MBCA | 10 | ECHA | 90 | urea | 6.0 |
| Example 4-37 | - | 0 | NNDEA | 1 | DAT | 10 | PhOH | 90 | urea | 10.3 |
| Example 4-38 | - | 0 | NNDEA | 1 | DAT | 10 | 2-ETH | 90 | urea | 10.3 |
| Example 4-39 | - | 0 | NNDEA | 1 | DAT | 10 | ECHA | 90 | urea | 10.3 |
| Example 4-40 | - | 0 | NNDEA | 1 | DPM | 10 | PhOH | 90 | urea | 6.4 |
| Example 4-41 | - | 0 | NNDEA | 1 | DPM | 10 | 2-ETH | 90 | urea | 6.4 |
| Example 4-42 | - | 0 | NNDEA | 1 | DPM | 10 | ECHA | 90 | urea | 6.4 |
| Example 4-43 | NMA | 100 | NNDMA | 1 | HDA | 10 | PhOH | 90 | urea | 10.9 |
| Example 4-44 | NMA | 1 | NNDMA | 100 | HDA | 10 | PhOH | 90 | urea | 10.9 |
| Example 4-45 | NMA | 100 | NNDMA | 1 | HDA | 10 | 2-ETH | 90 | urea | 10.9 |
| Example 4-46 | NMA | 1 | NNDMA | 100 | HDA | 10 | 2-ETH | 90 | urea | 10.9 |
| Example 4-47 | NMA | 100 | NNDMA | 1 | HDA | 10 | ECHA | 90 | urea | 10.9 |
| Example 4-48 | NMA | 1 | NNDMA | 100 | HDA | 10 | ECHA | 90 | urea | 10.9 |
| Example 4-49 | NMA | 100 | NNDMA | 1 | TAN | 10 | PhOH | 90 | urea | 10.9 |
| Example 4-50 | NMA | 1 | NNDMA | 100 | TAN | 10 | PhOH | 90 | urea | 10.9 |
| Example 4-51 | NMA | 100 | NNDMA | 1 | IPDA | 10 | PhOH | 90 | urea | 7.4 |
| Example 4-52 | NMA | 1 | NNDMA | 100 | IPDA | 10 | PhOH | 90 | urea | 7.4 |
| Example 4-53 | NMA | 100 | NNDMA | 1 | MBCA | 10 | PhOH | 90 | urea | 6.0 |
| Example 4-54 | NMA | 1 | NNDMA | 100 | MBCA | 10 | PhOH | 90 | urea | 6.0 |
| Example 4-55 | NMA | 100 | NNDMA | 1 | DAT | 10 | PhOH | 90 | urea | 10.3 |
| Example 4-56 | NMA | 1 | NNDMA | 100 | DAT | 10 | PhOH | 90 | urea | 10.3 |
| Example 4-57 | NMA | 100 | NNDMA | 1 | DPM | 10 | PhOH | 90 | urea | 6.4 |
| Example 4-58 | NMA | 1 | NNDMA | 100 | DPM | 10 | PhOH | 90 | urea | 6.4 |

**[Table 4-3]**

| | First reaction step - Raw materials | | | | | | | | | |
|---|---|---|---|---|---|---|---|---|---|---|
| | Secondary amine compound (I) | | Tertiary amine compound (II) | | Primary amine compound | | First blocking agent | | Carbonic acid derivative | |
| | type | parts by mass | type | parts by mass | type | parts by mass | type | parts by mass | type | parts by mass |
| Comparative Example 4-1 | - | - | - | - | HDA | 10 | PhOH | 90 | urea | 10.9 |
| Comparative Example 4-2 | - | - | - | - | HDA | 10 | 2-ETH | 90 | urea | 10.9 |
| Comparative Example 4-3 | - | - | - | - | HDA | 10 | ECHA | 90 | urea | 10.9 |
| Comparative Example 4-4 | - | - | - | - | TAN | 10 | PhOH | 90 | urea | 7.3 |
| Comparative Example 4-5 | - | - | - | - | TAN | 10 | 2-ETH | 90 | urea | 7.3 |
| Comparative Example 4-6 | - | - | - | - | TAN | 10 | ECHA | 90 | urea | 7.3 |
| Comparative Example 4-7 | - | - | - | - | IPDA | 10 | PhOH | 90 | urea | 7.4 |
| Comparative Example 4-8 | - | - | - | - | IPDA | 10 | 2-ETH | 90 | urea | 7.4 |
| Comparative Example 4-9 | - | - | - | - | IPDA | 10 | ECHA | 90 | urea | 7.4 |
| Comparative Example 4-10 | - | - | - | - | MBCA | 10 | PhOH | 90 | urea | 6.0 |
| Comparative Example 4-11 | - | - | - | - | MBCA | 10 | 2-ETH | 90 | urea | 6.0 |
| Comparative Example 4-12 | - | - | - | - | MBCA | 10 | ECHA | 90 | urea | 6.0 |
| Comparative Example 4-13 | - | - | - | - | DAT | 10 | PhOH | 90 | urea | 10.3 |
| Comparative Example 4-14 | - | - | - | - | DAT | 10 | 2-ETH | 90 | urea | 10.3 |
| Comparative Example 4-15 | - | - | - | - | DAT | 10 | ECHA | 90 | urea | 10.3 |
| Comparative Example 4-16 | - | - | - | - | DPM | 10 | PhOH | 90 | urea | 6.4 |
| Comparative Example 4-17 | - | - | - | - | DPM | 10 | 2-ETH | 90 | urea | 6.4 |
| Comparative Example 4-18 | - | - | - | - | DPM | 10 | ECHA | 90 | urea | 6.4 |

**[Table 4-4]**

| | First reaction step - Reaction conditions | | | | | |
|---|---|---|---|---|---|---|
| | Reaction temperature | Absolute pressure | Condenser tube temperature | Reaction time | Oxygen concentration | BTM |
| | °C | kPa | °C | Hours | vol% | parts by mass |
| Example 4-1 | 240 | 350 | 60 | 3 | 0 | 105.7 |
| Example 4-2 | 240 | 350 | 60 | 3 | 0 | 105.7 |
| Example 4-3 | 240 | 350 | 60 | 3 | 0 | 105.7 |
| Example 4-4 | 240 | 350 | 60 | 3 | 0 | 105.7 |
| Example 4-5 | 240 | 350 | 60 | 3 | 0 | 105.7 |
| Example 4-6 | 240 | 350 | 60 | 3 | 0 | 105.7 |
| Example 4-7 | 240 | 350 | 60 | 3 | 0 | 105.7 |
| Example 4-8 | 240 | 350 | 60 | 3 | 0 | 105.7 |
| Example 4-9 | 240 | 350 | 60 | 3 | 0 | 105.7 |
| Example 4-10 | 240 | 350 | 60 | 3 | 0 | 105.7 |
| Example 4-11 | 240 | 350 | 60 | 3 | 0 | 104.2 |
| Example 4-12 | 240 | 350 | 60 | 3 | 0 | 104.2 |
| Example 4-13 | 240 | 350 | 60 | 3 | 0 | 104.2 |
| Example 4-14 | 240 | 350 | 60 | 3 | 0 | 104.2 |
| Example 4-15 | 240 | 350 | 60 | 3 | 0 | 104.2 |
| Example 4-16 | 240 | 350 | 60 | 3 | 0 | 104.2 |
| Example 4-17 | 240 | 350 | 60 | 3 | 0 | 103.6 |
| Example 4-18 | 240 | 350 | 60 | 3 | 0 | 103.6 |
| Example 4-19 | 240 | 350 | 60 | 3 | 0 | 103.6 |
| Example 4-20 | 240 | 350 | 60 | 3 | 0 | 105.5 |
| Example 4-21 | 240 | 350 | 60 | 3 | 0 | 105.5 |
| Example 4-22 | 240 | 350 | 60 | 3 | 0 | 105.5 |
| Example 4-23 | 240 | 350 | 60 | 3 | 0 | 103.8 |
| Example 4-24 | 240 | 350 | 60 | 3 | 0 | 103.8 |
| Example 4-25 | 240 | 350 | 60 | 3 | 0 | 103.8 |

**[Table 4-5]**

| | First reaction step - Reaction conditions | | | | | |
|---|---|---|---|---|---|---|
| | Reaction temperature | Absolute pressure | Condenser tube temperature | Reaction time | Oxygen concentration | BTM |
| | °C | kPa | °C | Hours | vol% | parts by mass |
| Example 4-26 | 240 | 350 | 60 | 3 | 0 | 105.7 |
| Example 4-27 | 240 | 350 | 60 | 3 | 0 | 105.7 |
| Example 4-28 | 240 | 350 | 60 | 3 | 0 | 104.2 |
| Example 4-29 | 240 | 350 | 60 | 3 | 0 | 104.2 |
| Example 4-30 | 240 | 350 | 60 | 3 | 0 | 104.2 |
| Example 4-31 | 240 | 350 | 60 | 3 | 0 | 104.2 |
| Example 4-32 | 240 | 350 | 60 | 3 | 0 | 104.2 |
| Example 4-33 | 240 | 350 | 60 | 3 | 0 | 104.2 |
| Example 4-34 | 240 | 350 | 60 | 3 | 0 | 103.6 |
| Example 4-35 | 240 | 350 | 60 | 3 | 0 | 103.6 |
| Example 4-36 | 240 | 350 | 60 | 3 | 0 | 103.6 |
| Example 4-37 | 240 | 350 | 60 | 3 | 0 | 105.5 |
| Example 4-38 | 240 | 350 | 60 | 3 | 0 | 105.5 |
| Example 4-39 | 240 | 350 | 60 | 3 | 0 | 105.5 |
| Example 4-40 | 240 | 350 | 60 | 3 | 0 | 103.8 |
| Example 4-41 | 240 | 350 | 60 | 3 | 0 | 103.8 |
| Example 4-42 | 240 | 350 | 60 | 3 | 0 | 103.8 |
| Example 4-43 | 240 | 350 | 60 | 3 | 0 | 205.7 |
| Example 4-44 | 240 | 350 | 60 | 3 | 0 | 205.7 |
| Example 4-45 | 240 | 350 | 60 | 3 | 0 | 205.7 |
| Example 4-46 | 240 | 350 | 60 | 3 | 0 | 205.7 |
| Example 4-47 | 240 | 350 | 60 | 3 | 0 | 205.7 |
| Example 4-48 | 240 | 350 | 60 | 3 | 0 | 205.7 |
| Example 4-49 | 240 | 350 | 60 | 3 | 0 | 205.7 |
| Example 4-50 | 240 | 350 | 60 | 3 | 0 | 205.7 |
| Example 4-51 | 240 | 350 | 60 | 3 | 0 | 204.2 |
| Example 4-52 | 240 | 350 | 60 | 3 | 0 | 204.2 |
| Example 4-53 | 240 | 350 | 60 | 3 | 0 | 203.6 |
| Example 4-54 | 240 | 350 | 60 | 3 | 0 | 203.6 |
| Example 4-55 | 240 | 350 | 60 | 3 | 0 | 205.5 |
| Example 4-56 | 240 | 350 | 60 | 3 | 0 | 205.5 |
| Example 4-57 | 240 | 350 | 60 | 3 | 0 | 203.8 |
| Example 4-58 | 240 | 350 | 60 | 3 | 0 | 203.8 |

**[Table 4-6]**

| | First reaction step - Reaction conditions | | | | | |
|---|---|---|---|---|---|---|
| | Reaction temperature | Absolute pressure | Condenser tube temperature | Reaction time | Oxygen concentration | BTM |
| | °C | kPa | °C | Hours | vol% | parts by mass |
| Comparative Example 4-1 | 240 | 350 | 60 | 3 | 0 | 104.7 |
| Comparative Example 4-2 | 240 | 350 | 60 | 3 | 0 | 104.7 |
| Comparative Example 4-3 | 240 | 350 | 60 | 3 | 0 | 104.7 |
| Comparative Example 4-4 | 240 | 350 | 60 | 3 | 0 | 103.2 |
| Comparative Example 4-5 | 240 | 350 | 60 | 3 | 0 | 103.2 |
| Comparative Example 4-6 | 240 | 350 | 60 | 3 | 0 | 103.2 |
| Comparative Example 4-7 | 240 | 350 | 60 | 3 | 0 | 103.2 |
| Comparative Example 4-8 | 240 | 350 | 60 | 3 | 0 | 103.2 |
| Comparative Example 4-9 | 240 | 350 | 60 | 3 | 0 | 103.2 |
| Comparative Example 4-10 | 240 | 350 | 60 | 3 | 0 | 102.6 |
| Comparative Example 4-11 | 240 | 350 | 60 | 3 | 0 | 102.6 |
| Comparative Example 4-12 | 240 | 350 | 60 | 3 | 0 | 102.6 |
| Comparative Example 4-13 | 240 | 350 | 60 | 3 | 0 | 104.5 |
| Comparative Example 4-14 | 240 | 350 | 60 | 3 | 0 | 104.5 |
| Comparative Example 4-15 | 240 | 350 | 60 | 3 | 0 | 104.5 |
| Comparative Example 4-16 | 240 | 350 | 60 | 3 | 0 | 102.8 |
| Comparative Example 4-17 | 240 | 350 | 60 | 3 | 0 | 102.8 |
| Comparative Example 4-18 | 240 | 350 | 60 | 3 | 0 | 102.8 |

**[Table 4-7]**

| | First reaction step - Evaluation Results | | | | | |
|---|---|---|---|---|---|---|
| | Amino groups | NCO-BL(A) | NCO-compound (1) | Ureido groups | MB | Hazen color number |
| | mol% | mol% | mol% | mol% | mol% | APHA |
| Example 4-1 | 0 | 92 | 5 | 1 | 98 | 2 |
| Example 4-2 | 0 | 91 | 5 | 1 | 97 | 2 |
| Example 4-3 | 0 | 93 | 4 | 1 | 98 | 2 |
| Example 4-4 | 0 | 92 | 3 | 2 | 97 | 2 |
| Example 4-5 | 0 | 91 | 0 | 3 | 94 | 3 |
| Example 4-6 | 0 | 91 | 0 | 3 | 94 | 3 |
| Example 4-7 | 0 | 91 | 0 | 3 | 94 | 3 |
| Example 4-8 | 0 | 92 | 0 | 3 | 95 | 3 |
| Example 4-9 | 0 | 92 | 5 | 1 | 98 | 3 |
| Example 4-10 | 0 | 83 | 2 | 1 | 86 | 4 |
| Example 4-11 | 0 | 90 | 7 | 1 | 98 | 2 |
| Example 4-12 | 0 | 89 | 7 | 1 | 97 | 3 |
| Example 4-13 | 0 | 82 | 2 | 1 | 85 | 4 |
| Example 4-14 | 0 | 89 | 7 | 1 | 97 | 2 |
| Example 4-15 | 0 | 89 | 7 | 1 | 97 | 3 |
| Example 4-16 | 0 | 83 | 2 | 1 | 86 | 4 |
| Example 4-17 | 0 | 88 | 9 | 1 | 98 | 2 |
| Example 4-18 | 0 | 88 | 9 | 1 | 98 | 3 |
| Example 4-19 | 0 | 81 | 2 | 1 | 84 | 4 |
| Example 4-20 | 0 | 91 | 5 | 1 | 97 | 3 |
| Example 4-21 | 0 | 91 | 5 | 1 | 97 | 4 |
| Example 4-22 | 0 | 79 | 2 | 1 | 82 | 5 |
| Example 4-23 | 0 | 88 | 8 | 1 | 97 | 2 |
| Example 4-24 | 0 | 89 | 8 | 1 | 98 | 3 |
| Example 4-25 | 0 | 77 | 2 | 1 | 80 | 4 |

**[Table 4-8]**

| | First reaction step - Evaluation Results | | | | | |
|---|---|---|---|---|---|---|
| | Amino groups | NCO-BL(A) | NCO-compound (I) | Ureido groups | MB | Hazen color number |
| | mol% | mol% | mol% | mol% | mol% | APHA |
| Example 4-26 | 0 | 92 | 0 | 3 | 95 | 4 |
| Example 4-27 | 0 | 85 | 0 | 1 | 86 | 5 |
| Example 4-28 | 0 | 89 | 0 | 3 | 92 | 3 |
| Example 4-29 | 0 | 89 | 0 | 3 | 92 | 4 |
| Example 4-30 | 0 | 83 | 0 | 1 | 84 | 5 |
| Example 4-31 | 0 | 91 | 0 | 3 | 94 | 3 |
| Example 4-32 | 0 | 92 | 0 | 3 | 95 | 4 |
| Example 4-33 | 0 | 85 | 0 | 1 | 86 | 5 |
| Example 4-34 | 0 | 91 | 0 | 3 | 94 | 3 |
| Example 4-35 | 0 | 91 | 0 | 3 | 94 | 4 |
| Example 4-36 | 0 | 84 | 0 | 1 | 85 | 5 |
| Example 4-37 | 0 | 91 | 0 | 3 | 94 | 4 |
| Example 4-38 | 0 | 92 | 0 | 3 | 95 | 5 |
| Example 4-39 | 0 | 80 | 0 | 1 | 81 | 6 |
| Example 4-40 | 0 | 92 | 0 | 3 | 95 | 3 |
| Example 4-41 | 0 | 92 | 0 | 3 | 95 | 4 |
| Example 4-42 | 0 | 80 | 0 | 1 | 81 | 5 |
| Example 4-43 | 0 | 18 | 79 | 1 | 98 | 1 |
| Example 4-44 | 0 | 96 | 0 | 1 | 97 | 1 |
| Example 4-45 | 0 | 17 | 79 | 1 | 97 | 1 |
| Example 4-46 | 0 | 95 | 2 | 1 | 98 | 1 |
| Example 4-47 | 0 | 57 | 30 | 2 | 88 | 1 |
| Example 4-48 | 0 | 84 | 2 | 2 | 87 | 1 |
| Example 4-49 | 0 | 17 | 79 | 1 | 97 | 1 |
| Example 4-50 | 0 | 97 | 0 | 1 | 98 | 1 |
| Example 4-51 | 0 | 18 | 79 | 1 | 98 | 1 |
| Example 4-52 | 0 | 97 | 0 | 1 | 98 | 1 |
| Example 4-53 | 0 | 17 | 79 | 1 | 97 | 1 |
| Example 4-54 | 0 | 97 | 0 | 1 | 98 | 1 |
| Example 4-55 | 0 | 17 | 79 | 1 | 97 | 1 |
| Example 4-56 | 0 | 97 | 0 | 1 | 98 | 1 |
| Example 4-57 | 0 | 18 | 79 | 1 | 98 | 1 |
| Example 4-58 | 0 | 97 | 0 | 1 | 98 | 1 |

**[Table 4-9]**

| | First reaction step - Evaluation Results | | | | | |
|---|---|---|---|---|---|---|
| | Amino groups | NCO-BL(A) | NCO-compound (I) | Ureido groups | MB | Hazen color number |
| | mol% | mol% | mol% | mol% | mol% | APHA |
| Comparative Example 4-1 | 0 | 92 | 0 | 3 | 95 | 7 |
| Comparative Example 4-2 | 0 | 91 | 0 | 3 | 94 | 8 |
| Comparative Example 4-3 | 0 | 84 | 0 | 1 | 85 | 9 |
| Comparative Example 4-4 | 0 | 92 | 0 | 3 | 95 | 7 |
| Comparative Example 4-5 | 0 | 91 | 0 | 3 | 94 | 8 |
| Comparative Example 4-6 | 0 | 84 | 0 | 1 | 85 | 9 |
| Comparative Example 4-7 | 0 | 90 | 0 | 3 | 93 | 7 |
| Comparative Example 4-8 | 0 | 91 | 0 | 3 | 94 | 8 |
| Comparative Example 4-9 | 0 | 85 | 0 | 1 | 86 | 9 |
| Comparative Example 4-10 | 0 | 92 | 0 | 3 | 95 | 7 |
| Comparative Example 4-11 | 0 | 91 | 0 | 3 | 94 | 8 |
| Comparative Example 4-12 | 0 | 85 | 0 | 1 | 86 | 9 |
| Comparative Example 4-13 | 0 | 91 | 0 | 3 | 94 | 8 |
| Comparative Example 4-14 | 0 | 92 | 0 | 3 | 95 | 9 |
| Comparative Example 4-15 | 0 | 80 | 0 | 1 | 81 | 10 |
| Comparative Example 4-16 | 0 | 92 | 0 | 3 | 95 | 7 |
| Comparative Example 4-17 | 0 | 91 | 0 | 3 | 94 | 8 |
| Comparative Example 4-18 | 0 | 79 | 0 | 1 | 80 | 9 |

**[Table 4-10]**

| | Second reaction step - Raw materials | | | | | | |
|---|---|---|---|---|---|---|---|
| | Low boiling point component-free blocked isocyanate composition | Secondary amine compound (I) | | Tertiary amine compound (II) | | Solvent | |
| | parts by mass | type | parts by mass | type | parts by mass | type | parts by mass |
| Example 4-1 | 10 | NMA | 1 | - | 0 | naphthene | 89 |
| Example 4-2 | 10 | NEA | 1 | - | 0 | naphthene | 89 |
| Example 4-3 | 10 | NBA | 1 | - | 0 | naphthene | 89 |
| Example 4-4 | 10 | DPA | 1 | - | 0 | naphthene | 89 |
| Example 4-5 | 10 | - | 0 | NNDMA | 1 | naphthene | 89 |
| Example 4-6 | 10 | - | 0 | NNDEA | 1 | naphthene | 89 |
| Example 4-7 | 10 | - | 0 | NMDPA | 1 | naphthene | 89 |
| Example 4-8 | 10 | - | 0 | TPA | 1 | naphthene | 89 |
| Example 4-9 | 10 | NEA | 1 | - | 0 | naphthene | 89 |
| Example 4-10 | 10 | NEA | 1 | - | 0 | naphthene | 89 |
| Example 4-11 | 10 | NEA | 1 | - | 0 | BP | 89 |
| Example 4-12 | 10 | NEA | 1 | - | 0 | BP | 89 |
| Example 4-13 | 10 | NEA | 1 | - | 0 | BP | 89 |
| Example 4-14 | 10 | NEA | 1 | - | 0 | DPE | 89 |
| Example 4-15 | 10 | NEA | 1 | - | 0 | DPE | 89 |
| Example 4-16 | 10 | NEA | 1 | - | 0 | DPE | 89 |
| Example 4-17 | 10 | NEA | 1 | - | 0 | BP | 89 |
| Example 4-18 | 10 | NEA | 1 | - | 0 | BP | 89 |
| Example 4-19 | 10 | NEA | 1 | - | 0 | BP | 89 |
| Example 4-20 | 10 | NEA | 1 | - | 0 | naphthene | 89 |
| Example 4-21 | 10 | NEA | 1 | - | 0 | naphthene | 89 |
| Example 4-22 | 10 | NEA | 1 | - | 0 | naphthene | 89 |
| Example 4-23 | 10 | NEA | 1 | - | 0 | DPE | 89 |
| Example 4-24 | 10 | NEA | 1 | - | 0 | DPE | 89 |
| Example 4-25 | 10 | NEA | 1 | - | 0 | DPE | 89 |

**[Table 4-11]**

| | Second reaction step - Raw materials | | | | | | |
|---|---|---|---|---|---|---|---|
| | Low boiling point component-free blocked isocyanate composition | Secondary amine compound (I) | | Tertiary amine compound (II) | | Solvent | |
| | parts by mass | type | parts by mass | type | parts by mass | type | parts by mass |
| Example 4-26 | 10 | - | 0 | NNDEA | 1 | naphthene | 89 |
| Example 4-27 | 10 | - | 0 | NNDEA | 1 | naphthene | 89 |
| Example 4-28 | 10 | - | 0 | NNDMA | 1 | BP | 89 |
| Example 4-29 | 10 | - | 0 | NNDEA | 1 | BP | 89 |
| Example 4-30 | 10 | - | 0 | NNDEA | 1 | BP | 89 |
| Example 4-31 | 10 | - | 0 | NNDEA | 1 | DPE | 89 |
| Example 4-32 | 10 | - | 0 | NNDEA | 1 | DPE | 89 |
| Example 4-33 | 10 | - | 0 | NNDEA | 1 | DPE | 89 |
| Example 4-34 | 10 | - | 0 | NNDEA | 1 | BP | 89 |
| Example 4-35 | 10 | - | 0 | NNDEA | 1 | BP | 89 |
| Example 4-36 | 10 | - | 0 | NNDEA | 1 | BP | 89 |
| Example 4-37 | 10 | - | 0 | NNDEA | 1 | naphthene | 89 |
| Example 4-38 | 10 | - | 0 | NNDEA | 1 | naphthene | 89 |
| Example 4-39 | 10 | - | 0 | NNDEA | 1 | naphthene | 89 |
| Example 4-40 | 10 | - | 0 | NNDEA | 1 | DPE | 89 |
| Example 4-41 | 10 | - | 0 | NNDEA | 1 | DPE | 89 |
| Example 4-42 | 10 | - | 0 | NNDEA | 1 | DPE | 89 |
| Example 4-43 | 10 | NMA | 100 | NNDMA | 1 | naphthene | 89 |
| Example 4-44 | 10 | NMA | 1 | NNDMA | 100 | naphthene | 89 |
| Example 4-45 | 10 | NMA | 100 | NNDMA | 1 | naphthene | 89 |
| Example 4-46 | 10 | NMA | 1 | NNDMA | 100 | naphthene | 89 |
| Example 4-47 | 10 | NMA | 100 | NNDMA | 1 | naphthene | 89 |
| Example 4-48 | 10 | NMA | 1 | NNDMA | 100 | naphthene | 89 |
| Example 4-49 | 10 | NMA | 100 | NNDMA | 1 | BP | 89 |
| Example 4-50 | 10 | NMA | 1 | NNDMA | 100 | BP | 89 |
| Example 4-51 | 10 | NMA | 100 | NNDMA | 1 | DPE | 89 |
| Example 4-52 | 10 | NMA | 1 | NNDMA | 100 | DPE | 89 |
| Example 4-53 | 10 | NMA | 100 | NNDMA | 1 | BP | 89 |
| Example 4-54 | 10 | NMA | 1 | NNDMA | 100 | BP | 89 |
| Example 4-55 | 10 | NMA | 100 | NNDMA | 1 | naphthene | 89 |
| Example 4-56 | 10 | NMA | 1 | NNDMA | 100 | naphthene | 89 |
| Example 4-57 | 10 | NMA | 100 | NNDMA | 1 | DPE | 89 |
| Example 4-58 | 10 | NMA | 1 | NNDMA | 100 | DPE | 89 |

**[Table 4-12]**

| | Second reaction step - Raw materials | | | | | | |
|---|---|---|---|---|---|---|---|
| | Low boiling point component-free blocked isocyanate composition | Secondary amine compound (1) | | Tertiary amine compound (II) | | Solvent | |
| | parts by mass | type | parts by mass | type | parts by mass | type | parts by mass |
| Comparative Example 4-1 | 10 | - | - | - | - | naphthene | 90 |
| Comparative Example 4-2 | 10 | - | - | - | - | naphthene | 90 |
| Comparative Example 4-3 | 10 | - | - | - | - | naphthene | 90 |
| Comparative Example 4-4 | 10 | - | - | - | - | BP | 90 |
| Comparative Example 4-5 | 10 | - | - | - | - | BP | 90 |
| Comparative Example 4-6 | 10 | - | - | - | - | BP | 90 |
| Comparative Example 4-7 | 10 | - | - | - | - | DPE | 90 |
| Comparative Example 4-8 | 10 | - | - | - | - | DPE | 90 |
| Comparative Example 4-9 | 10 | - | - | - | - | DPE | 90 |
| Comparative Example 4-10 | 10 | - | - | - | - | BP | 90 |
| Comparative Example 4-11 | 10 | - | - | - | - | BP | 90 |
| Comparative Example 4-12 | 10 | - | - | - | - | BP | 90 |
| Comparative Example 4-13 | 10 | - | - | - | - | naphthene | 90 |
| Comparative Example 4-14 | 10 | - | - | - | - | naphthene | 90 |
| Comparative Example 4-15 | 10 | - | - | - | - | naphthene | 90 |
| Comparative Example 4-16 | 10 | - | - | - | - | DPE | 90 |
| Comparative Example 4-17 | 10 | - | - | - | - | DPE | 90 |
| Comparative Example 4-18 | 10 | - | - | - | - | DPE | 90 |

**Table 4-13]**

| | Second reaction step - Reaction conditions | | | | | | |
|---|---|---|---|---|---|---|---|
| | Reaction temperature | Reduced pressure | Condenser tube 1 temperature | Condenser tube 2 temperature | Reaction time | Oxygen concentration | BTM |
| | °C | mmHg | °C | °C | hours | vol% | parts by mass |
| Example 4-1 | 220 | 700 | 220 | 50 | 2 | 0 | 50 |
| Example 4-2 | 220 | 700 | 220 | 50 | 2 | 0 | 50 |
| Example 4-3 | 220 | 700 | 220 | 50 | 2 | 0 | 50 |
| Example 4-4 | 220 | 700 | 220 | 50 | 2 | 0 | 50 |
| Example 4-5 | 220 | 700 | 220 | 50 | 2 | 0 | 50 |
| Example 4-6 | 220 | 700 | 220 | 50 | 2 | 0 | 50 |
| Example 4-7 | 220 | 700 | 220 | 50 | 2 | 0 | 50 |
| Example 4-8 | 220 | 700 | 220 | 50 | 2 | 0 | 50 |
| Example 4-9 | 220 | 700 | 220 | 50 | 2 | 0 | 50 |
| Example 4-10 | 220 | 700 | 220 | 50 | 2 | 0 | 50 |
| Example 4-11 | 250 | 210 | 250 | 50 | 2 | 0 | 50 |
| Example 4-12 | 250 | 210 | 250 | 50 | 2 | 0 | 50 |
| Example 4-13 | 250 | 210 | 250 | 50 | 2 | 0 | 50 |
| Example 4-14 | 250 | 600 | 250 | 50 | 2 | 0 | 50 |
| Example 4-15 | 250 | 600 | 250 | 50 | 2 | 0 | 50 |
| Example 4-16 | 250 | 600 | 250 | 50 | 2 | 0 | 50 |
| Example 4-17 | 250 | 210 | 250 | 50 | 2 | 0 | 50 |
| Example 4-18 | 250 | 210 | 250 | 50 | 2 | 0 | 50 |
| Example 4-19 | 250 | 210 | 250 | 50 | 2 | 0 | 50 |
| Example 4-20 | 220 | 700 | 220 | 50 | 2 | 0 | 50 |
| Example 4-21 | 220 | 700 | 220 | 50 | 2 | 0 | 50 |
| Example 4-22 | 220 | 700 | 220 | 50 | 2 | 0 | 50 |
| Example 4-23 | 250 | 600 | 250 | 50 | 2 | 0 | 50 |
| Example 4-24 | 250 | 600 | 250 | 50 | 2 | 0 | 50 |
| Example 4-25 | 250 | 600 | 250 | 50 | 2 | 0 | 50 |

**[Table 4-14]**

| | Second reaction step - Reaction conditions | | | | | | |
|---|---|---|---|---|---|---|---|
| | Reaction temperature | Reduced pressure | Condenser tube 1 temperature | Condenser tube 2 temperature | Reaction time | Oxygen concentration | BTM |
| | °C | mmHg | °C | °C | hours | vol% | parts by mass |
| Example 4-26 | 220 | 700 | 220 | 50 | 2 | 0 | 50 |
| Example 4-27 | 220 | 700 | 220 | 50 | 2 | 0 | 50 |
| Example 4-28 | 250 | 210 | 250 | 50 | 2 | 0 | 50 |
| Example 4-29 | 250 | 210 | 250 | 50 | 2 | 0 | 50 |
| Example 4-30 | 250 | 210 | 250 | 50 | 2 | 0 | 50 |
| Example 4-31 | 250 | 600 | 250 | 50 | 2 | 0 | 50 |
| Example 4-32 | 250 | 600 | 250 | 50 | 2 | 0 | 50 |
| Example 4-33 | 250 | 600 | 250 | 50 | 2 | 0 | 50 |
| Example 4-34 | 250 | 210 | 250 | 50 | 2 | 0 | 50 |
| Example 4-35 | 250 | 210 | 250 | 50 | 2 | 0 | 50 |
| Example 4-36 | 250 | 210 | 250 | 50 | 2 | 0 | 50 |
| Example 4-37 | 220 | 700 | 220 | 50 | 2 | 0 | 50 |
| Example 4-38 | 220 | 700 | 220 | 50 | 2 | 0 | 50 |
| Example 4-39 | 220 | 700 | 220 | 50 | 2 | 0 | 50 |
| Example 4-40 | 250 | 600 | 250 | 50 | 2 | 0 | 50 |
| Example 4-41 | 250 | 600 | 250 | 50 | 2 | 0 | 50 |
| Example 4-42 | 250 | 600 | 250 | 50 | 2 | 0 | 50 |
| Example 4-43 | 220 | 700 | 220 | 50 | 2 | 0 | 50 |
| Example 4-44 | 220 | 700 | 220 | 50 | 2 | 0 | 50 |
| Example 4-45 | 220 | 700 | 220 | 50 | 2 | 0 | 50 |
| Example 4-46 | 220 | 700 | 220 | 50 | 2 | 0 | 50 |
| Example 4-47 | 220 | 700 | 220 | 50 | 2 | 0 | 50 |
| Example 4-48 | 220 | 700 | 220 | 50 | 2 | 0 | 50 |
| Example 4-49 | 250 | 210 | 250 | 50 | 2 | 0 | 50 |
| Example 4-50 | 250 | 210 | 250 | 50 | 2 | 0 | 50 |
| Example 4-51 | 250 | 600 | 250 | 50 | 2 | 0 | 50 |
| Example 4-52 | 250 | 600 | 250 | 50 | 2 | 0 | 50 |
| Example 4-53 | 250 | 210 | 250 | 50 | 2 | 0 | 50 |
| Example 4-54 | 250 | 210 | 250 | 50 | 2 | 0 | 50 |
| Example 4-55 | 220 | 700 | 220 | 50 | 2 | 0 | 50 |
| Example 4-56 | 220 | 700 | 220 | 50 | 2 | 0 | 50 |
| Example 4-57 | 250 | 600 | 250 | 50 | 2 | 0 | 50 |
| Example 4-58 | 250 | 600 | 250 | 50 | 2 | 0 | 50 |

**[Table 4-15]**

| | Second reaction step - Reaction conditions | | | | | | |
|---|---|---|---|---|---|---|---|
| | Reaction temperature | Reduced pressure | Condenser tube 1 temperature | Condenser tube 2 temperature | Reaction time | Oxygen concentration | BTM |
| | °C | mmHg | °C | °C | hours | vol% | parts by mass |
| Comparative Example 4-1 | 220 | 700 | 220 | 50 | 2 | 0 | 50 |
| Comparative Example 4-2 | 220 | 700 | 220 | 50 | 2 | 0 | 50 |
| Comparative Example 4-3 | 220 | 700 | 220 | 50 | 2 | 0 | 50 |
| Comparative Example 4-4 | 250 | 210 | 250 | 50 | 2 | 0 | 50 |
| Comparative Example 4-5 | 250 | 210 | 250 | 50 | 2 | 0 | 50 |
| Comparative Example 4-6 | 250 | 210 | 250 | 50 | 2 | 0 | 50 |
| Comparative Example 4-7 | 250 | 600 | 250 | 50 | 2 | 0 | 50 |
| Comparative Example 4-8 | 250 | 600 | 250 | 50 | 2 | 0 | 50 |
| Comparative Example 4-9 | 250 | 600 | 250 | 50 | 2 | 0 | 50 |
| Comparative Example 4-10 | 250 | 210 | 250 | 50 | 2 | 0 | 50 |
| Comparative Example 4-11 | 250 | 210 | 250 | 50 | 2 | 0 | 50 |
| Comparative Example 4-12 | 250 | 210 | 250 | 50 | 2 | 0 | 50 |
| Comparative Example 4-13 | 220 | 700 | 220 | 50 | 2 | 0 | 50 |
| Comparative Example 4-14 | 220 | 700 | 220 | 50 | 2 | 0 | 50 |
| Comparative Example 4-15 | 220 | 700 | 220 | 50 | 2 | 0 | 50 |
| Comparative Example 4-16 | 250 | 600 | 250 | 50 | 2 | 0 | 50 |
| Comparative Example 4-17 | 250 | 600 | 250 | 50 | 2 | 0 | 50 |
| Comparative Example 4-18 | 250 | 600 | 250 | 50 | 2 | 0 | 50 |

**[Table 4-16]**

| | Second reaction step - Evaluation results | | | | | | | | |
|---|---|---|---|---|---|---|---|---|---|
| | NCO | Amino groups | NCO-BL(A) | NCO-compound (I) | Ureido groups | MB | Hazen color number | TOP | Isocyanate yield |
| | mol% | mol% | mol% | mol% | mol% | mol% | APHA | parts by mass | mass% |
| Example 4-1 | 92 | 0 | 0 | 1 | 0 | 93 | 3 | 50 | 85 |
| Example 4-2 | 93 | 0 | 0 | 1 | 0 | 94 | 3 | 50 | 86 |
| Example 4-3 | 92 | 0 | 0 | 1 | 0 | 93 | 3 | 50 | 84 |
| Example 4-4 | 93 | 0 | 0 | 1 | 0 | 94 | 3 | 50 | 85 |
| Example 4-5 | 87 | 0 | 1 | 0 | 0 | 88 | 4 | 50 | 76 |
| Example 4-6 | 87 | 0 | 1 | 0 | 0 | 88 | 4 | 50 | 75 |
| Example 4-7 | 87 | 0 | 1 | 0 | 0 | 88 | 4 | 50 | 75 |
| Example 4-8 | 87 | 0 | 1 | 0 | 0 | 88 | 4 | 50 | 75 |
| Example 4-9 | 77 | 0 | 0 | 1 | 0 | 78 | 4 | 50 | 60 |
| Example 4-10 | 67 | 0 | 1 | 0 | 0 | 68 | 5 | 50 | 46 |
| Example 4-11 | 92 | 0 | 0 | 1 | 0 | 93 | 3 | 50 | 77 |
| Example 4-12 | 80 | 0 | 0 | 1 | 0 | 81 | 4 | 50 | 50 |
| Example 4-13 | 71 | 0 | 1 | 0 | 0 | 72 | 5 | 50 | 35 |
| Example 4-14 | 92 | 0 | 0 | 1 | 0 | 93 | 3 | 50 | 84 |
| Example 4-15 | 77 | 0 | 0 | 1 | 0 | 78 | 4 | 50 | 61 |
| Example 4-16 | 66 | 0 | 1 | 0 | 0 | 67 | 5 | 50 | 45 |
| Example 4-17 | 92 | 0 | 0 | 1 | 0 | 93 | 3 | 50 | 85 |
| Example 4-18 | 77 | 0 | 0 | 1 | 0 | 78 | 4 | 50 | 60 |
| Example 4-19 | 67 | 0 | 1 | 0 | 0 | 68 | 5 | 50 | 44 |
| Example 4-20 | 89 | 0 | 0 | 1 | 0 | 90 | 4 | 50 | 80 |
| Example 4-21 | 74 | 0 | 0 | 1 | 0 | 75 | 5 | 50 | 56 |
| Example 4-22 | 62 | 0 | 1 | 0 | 0 | 63 | 6 | 50 | 40 |
| Example 4-23 | 90 | 0 | 0 | 1 | 0 | 91 | 3 | 50 | 80 |
| Example 4-24 | 74 | 0 | 0 | 1 | 0 | 75 | 4 | 50 | 56 |
| Example 4-25 | 62 | 0 | 1 | 0 | 0 | 63 | 5 | 50 | 40 |

**[Table 4-17]**

| | Second reaction step - Evaluation results | | | | | | | | |
|---|---|---|---|---|---|---|---|---|---|
| | NCO | Amino groups | NCO-BL(A) | NCO-compound (I) | Ureido groups | MB | Hazen color number | TOP | Isocyanate yield |
| | mol% | mol% | mol% | mol% | mol% | mol% | APHA | parts by mass | mass% |
| Example 4-26 | 71 | 0 | 1 | 0 | 0 | 72 | 5 | 50 | 49 |
| Example 4-27 | 64 | 0 | 1 | 0 | 0 | 65 | 6 | 50 | 35 |
| Example 4-28 | 88 | 0 | 1 | 0 | 0 | 89 | 4 | 50 | 67 |
| Example 4-29 | 74 | 0 | 1 | 0 | 0 | 75 | 5 | 50 | 41 |
| Example 4-30 | 68 | 0 | 1 | 0 | 0 | 69 | 6 | 50 | 25 |
| Example 4-31 | 86 | 0 | 1 | 0 | 0 | 87 | 4 | 50 | 76 |
| Example 4-32 | 70 | 0 | 1 | 0 | 0 | 71 | 5 | 50 | 51 |
| Example 4-33 | 63 | 0 | 1 | 0 | 0 | 64 | 6 | 50 | 35 |
| Example 4-34 | 87 | 0 | 1 | 0 | 0 | 88 | 4 | 50 | 74 |
| Example 4-35 | 71 | 0 | 1 | 0 | 0 | 72 | 5 | 50 | 49 |
| Example 4-36 | 64 | 0 | 1 | 0 | 0 | 65 | 6 | 50 | 35 |
| Example 4-37 | 84 | 0 | 1 | 0 | 0 | 85 | 5 | 50 | 69 |
| Example 4-38 | 66 | 0 | 1 | 0 | 0 | 67 | 6 | 50 | 46 |
| Example 4-39 | 60 | 0 | 1 | 0 | 0 | 61 | 7 | 50 | 30 |
| Example 4-40 | 84 | 0 | 1 | 0 | 0 | 85 | 4 | 50 | 69 |
| Example 4-41 | 67 | 0 | 1 | 0 | 0 | 68 | 5 | 50 | 44 |
| Example 4-42 | 61 | 0 | 1 | 0 | 0 | 62 | 6 | 50 | 31 |
| Example 4-43 | 92 | 0 | 0 | 1 | 0 | 93 | 1 | 50 | 84 |
| Example 4-44 | 92 | 0 | 0 | 1 | 0 | 93 | 1 | 50 | 85 |
| Example 4-45 | 81 | 0 | 0 | 1 | 0 | 82 | 2 | 50 | 65 |
| Example 4-46 | 81 | 0 | 0 | 1 | 0 | 82 | 2 | 50 | 66 |
| Example 4-47 | 70 | 0 | 1 | 0 | 0 | 71 | 2 | 50 | 51 |
| Example 4-48 | 71 | 0 | 1 | 0 | 0 | 72 | 2 | 50 | 50 |
| Example 4-49 | 92 | 0 | 0 | 1 | 0 | 93 | 1 | 50 | 77 |
| Example 4-50 | 92 | 0 | 0 | 1 | 0 | 93 | 1 | 50 | 77 |
| Example 4-51 | 92 | 0 | 0 | 1 | 0 | 93 | 1 | 50 | 86 |
| Example 4-52 | 93 | 0 | 0 | 1 | 0 | 94 | 1 | 50 | 85 |
| Example 4-53 | 93 | 0 | 0 | 1 | 0 | 94 | 1 | 50 | 85 |
| Example 4-54 | 92 | 0 | 0 | 1 | 0 | 93 | 1 | 50 | 86 |
| Example 4-55 | 89 | 0 | 0 | 1 | 0 | 90 | 1 | 50 | 80 |
| Example 4-56 | 89 | 0 | 0 | 1 | 0 | 90 | 1 | 50 | 80 |
| Example 4-57 | 89 | 0 | 0 | 1 | 0 | 90 | 1 | 50 | 80 |
| Example 4-58 | 89 | 0 | 0 | 1 | 0 | 90 | 1 | 50 | 81 |

**[Table 4-18]**

| | Second reaction step - Evaluation results | | | | | | | | |
|---|---|---|---|---|---|---|---|---|---|
| | NCO | Amino groups | NCO-BL(A) | NCO-compound (I) | Ureido groups | MB | Hazen color number | TOP | Isocyanate yield |
| | mol% | mol% | mol% | mol% | mol% | mol% | APHA | parts by mass | mass% |
| Comparative Example 4-1 | 81 | 0 | 1 | 0 | 0 | 82 | 8 | 50 | 65 |
| Comparative Example 4-2 | 63 | 0 | 1 | 0 | 0 | 64 | 9 | 50 | 40 |
| Comparative Example 4-3 | 62 | 0 | 1 | 0 | 0 | 63 | 10 | 50 | 34 |
| Comparative Example 4-4 | 83 | 0 | 1 | 0 | 0 | 84 | 8 | 50 | 57 |
| Comparative Example 4-5 | 66 | 0 | 1 | 0 | 0 | 67 | 9 | 50 | 31 |
| Comparative Example 4-6 | 68 | 0 | 1 | 0 | 0 | 69 | 10 | 50 | 25 |
| Comparative Example 4-7 | 81 | 0 | 1 | 0 | 0 | 82 | 8 | 50 | 64 |
| Comparative Example 4-8 | 63 | 0 | 1 | 0 | 0 | 64 | 9 | 50 | 41 |
| Comparative Example 4-9 | 64 | 0 | 1 | 0 | 0 | 65 | 10 | 50 | 34 |
| Comparative Example 4-10 | 81 | 0 | 1 | 0 | 0 | 82 | 8 | 50 | 65 |
| Comparative Example 4-11 | 62 | 0 | 1 | 0 | 0 | 63 | 9 | 50 | 40 |
| Comparative Example 4-12 | 63 | 0 | 1 | 0 | 0 | 64 | 10 | 50 | 35 |
| Comparative Example 4-13 | 77 | 0 | 1 | 0 | 0 | 78 | 9 | 50 | 59 |
| Comparative Example 4-14 | 58 | 0 | 1 | 0 | 0 | 59 | 10 | 50 | 34 |
| Comparative Example 4-15 | 60 | 0 | 1 | 0 | 0 | 61 | 11 | 50 | 29 |
| Comparative Example 4-16 | 77 | 0 | 1 | 0 | 0 | 78 | 8 | 50 | 60 |
| Comparative Example 4-17 | 60 | 0 | 1 | 0 | 0 | 61 | 9 | 50 | 34 |
| Comparative Example 4-18 | 59 | 0 | 1 | 0 | 0 | 60 | 10 | 50 | 29 |

As shown in the above tables, the isocyanate compositions each obtained by producing a blocked isocyanate composition from an amine, a carbonic acid derivative and a blocking agent in the presence of at least one added compound selected from the group consisting of the secondary amine compounds (1) and the tertiary amine compounds (II), and then thermally decomposing the obtained blocked isocyanate composition in the presence of at least one added compound selected from the group consisting of the secondary amine compounds (1) and the tertiary amine compounds (ll) (Examples 4-1 to 4-58) exhibited Hazen color numbers (APHA) evaluated as rank 7 or lower, which were favorable.

In contrast, the isocyanate compositions each obtained by producing a blocked isocyanate composition from an amine, a carbonic acid derivative and a blocking agent without the addition of either the secondary amine compound (I) or the tertiary amine compound (II), and then thermally decomposing the obtained blocked isocyanate composition without the addition of either the secondary amine compound (I) or the tertiary amine compound (II) (Comparative Examples 4-1 to 4-18) exhibited Hazen color numbers (APHA) evaluated as rank 8 or higher, which were unfavorable.

Further, among the isocyanate compositions each obtained by producing a blocked isocyanate composition in the presence of the secondary amine compound (1) using a phenol-based, alcohol-based, or aliphatic amine-based blocking agent, and then thermally decomposing the blocked isocyanate composition in the presence of the secondary amine compound (1) (Examples 4-2 and 4-9 to 4-25), the blocked isocyanate compositions obtained using a phenol-based blocking agent (Examples 4-2, 4-11, 4-14, 4-17, 4-20 and 4-23) exhibited Hazen color numbers evaluated as the lowest rank, which were particularly favorable.

Furthermore, among the isocyanate compositions each obtained by producing a blocked isocyanate composition in the presence of the tertiary amine compound (II) using a phenol-based, alcohol-based, aliphatic amine-based, or aromatic amine-based blocking agent, and then thermally decomposing the blocked isocyanate composition in the presence of the secondary amine compound (1) (Examples 4-6 and 4-26 to 4-42), the blocked isocyanate compositions obtained using a phenol-based blocking agent (Examples 4-6, 4-28, 4-31, 4-34, 4-37 and 4-40) exhibited Hazen color numbers evaluated as the lowest rank, which were particularly favorable.

Furthermore, the isocyanate compositions each obtained by producing a blocked isocyanate composition in the presence of both the secondary amine compound (I) and the tertiary amine compound (II), and then thermal decomposing the blocked isocyanate composition in the presence of both the secondary amine compound (I) and the tertiary amine compound (II), (Examples 4-43 to 4-58) exhibited Hazen color numbers evaluated as rank 2 or lower, which were the most favorable.

### <<Fifth Test>>

### [Physical Property 5-1]

### (Amount of Isocyanate Groups Blocked with First Blocking Agent)

The amount (mol%) of isocyanate groups blocked with the first blocking agent in an obtained blocked isocyanate composition (hereinafter sometimes abbreviated as "NCO-BL(A) mol%") was determined in accordance with the formula shown below, from the molar amount of amino groups derived from the primary amine within the raw materials in the first reaction step, and the molar amount of isocyanate groups blocked with the first blocking agent. The molar amount of isocyanate groups blocked with the first blocking agent was determined from an NMR spectrum.

Further, in the addition-elimination step, the concentration of carbon skeletons derived from the primary amine was determined from an NMR spectrum, and the molar amount of amino groups of the primary amine compound was calculated from the product of the valency of the primary amine amino group and the concentration of carbon skeletons derived from the primary amine. Subsequently, NCO-BL(A) mol% was calculated in accordance with the formula shown below, from the molar amount of amino groups from the primary amine compound calculated above, and the molar amount of isocyanate groups blocked with the first blocking agent. The molar amount of isocyanate groups blocked with the first blocking agent was determined from an NMR spectrum.[NCO-BL(A) mol%] = (molar amount of blocked isocyanate groups) × 100 / (molar amount of amino groups of the primary amine compound)

### [Physical Property 5-2]

### (Ureido Group Content)

The ureido group content (mol%) within the blocked isocyanate composition was determined in accordance with the formula shown below, from the molar amount of amino groups derived from the primary amine within the raw materials in the first reaction step, and the molar amount of ureido groups. The molar amount of ureido groups was determined from an NMR spectrum.

Further, in the addition-elimination reaction step, the concentration of carbon skeletons from the primary amine was determined from an NMR spectrum, and the molar amount of amino groups of the primary amine compound was calculated from the product of the valency of the primary amine amino group and the concentration of carbon skeletons from the primary amine. Subsequently, the ureido group content was calculated in accordance with the formula shown below, from the molar amount of amino groups of the primary amine compound calculated above, and the molar amount of ureido groups. The molar amount of ureido groups was determined from an NMR spectrum.[Ureido group mol%] = (molar amount ureido groups following reaction) × 100 / (molar amount of amino groups derived from primary amine prior to reaction)

### [Physical Property 5-3]

### (Amount of Isocyanate Groups bonded to Secondary Amine Compound (I))

The amount (mol%) of isocyanate groups bonded to the secondary amine compound (I) within the blocked isocyanate composition obtained in the first reaction step (hereinafter sometimes abbreviated as "NCO-compound (I) mol%") was determined in accordance with the formula shown below, from the molar amount of amino groups derived from the primary amine within the raw materials in the first reaction step, and the molar amount of isocyanate groups bonded to the secondary amine compound (I). The molar amount of isocyanate groups bonded to the secondary amine compound (I) was determined from an NMR spectrum.

Further, in the addition-elimination reaction step, the concentration of carbon skeletons from the primary amine was determined from an NMR spectrum, and the molar amount of amino groups of the primary amine compound was calculated from the product of the valency of the primary amine amino group and the concentration of carbon skeletons from the primary amine. Subsequently, the NCO-compound (1) mol% was calculated in accordance with the formula shown below, from the molar amount of amino groups of the primary amine compound calculated above, and the molar amount of isocyanate groups bonded to the secondary amine compound (I). The molar amount of isocyanate groups bonded to the secondary amine compound (I) was determined from an NMR spectrum.[NCO-compound (I) mol%] = (molar amount of NCO-compound (I)) × 100 / (molar amount of amino groups derived from primary amine compound)

### [Physical Property 5-4]

### (Amino Group Content)

The amino group content (mol%) within the blocked isocyanate composition obtained in the first reaction step (hereinafter sometimes abbreviated as "amino group mol%") was determined in accordance with the formula shown below, from the molar amount of amino groups derived from the primary amine contained within the raw materials in the first reaction step, and the molar amount of residual amino groups following the reaction. The molar amount of amino groups was determined from an NMR spectrum.

Further, in the addition-elimination reaction step, the concentration of carbon skeletons from the primary amine was determined from an NMR spectrum, and the molar amount of amino groups of the primary amine compound was calculated from the product of the valency of the primary amine amino group and the concentration of carbon skeletons from the primary amine. Subsequently, the amino group mol% was calculated in accordance with the formula shown below, from the molar amount of amino groups from the primary amine compound calculated above, and the molar amount of residual amino groups following the reaction. The molar amount of amino groups was determined from an NMR spectrum.[Amino group mol%] = (molar amount of amino groups) × 100 / (molar amount of amino groups from primary amine)

### [Physical Property 5-5]

### (Amount of Isocyanate Groups Blocked with Second Blocking Agent)

The amount (mol%) of isocyanate groups blocked with the second blocking agent in the blocked isocyanate composition obtained in the addition-elimination reaction step (hereinafter sometimes abbreviated as "NCO-BL(C) mol%") was determined in accordance with the formula shown below, from the molar amount of amino groups derived from the primary amine contained within the raw materials in the addition-elimination reaction, and the molar amount of isocyanate groups blocked with the second blocking agent. The molar amount of isocyanate groups blocked with the second blocking agent was determined from an NMR spectrum. Further, the concentration of carbon skeletons from the primary amine within the raw materials in the addition-elimination reaction step was determined from an NMR spectrum, and the molar amount of amino groups of the primary amine compound was calculated from the product of the valency of the primary amine amino group and the concentration of carbon skeletons from the primary amine.[NCO-BL(C) mol%] = (molar amount of blocked isocyanate groups) × 100 / (molar amount of amino groups of the primary amine compound)

### [Evaluation 5-1]

### (Mass Balance (MB))

In the first reaction step, the sum, within the obtained blocked isocyanate composition, of the amino group mol%, NCO-BL(A) mol%, NCO-compound (1) mol% and ureido group mol% was determined as the mass balance.

On the other hand, in the addition-elimination reaction step, the sum, within the obtained blocked isocyanate composition, of the amino group mol%, NCO-BL(A) mol%, NCO-BL(C) mol%, NCO-compound (I) mol% and ureido group mol% was determined as the mass balance.

### [Evaluation 5-2]

### (Hazen Color Number)

The Hazen color number was determined by dissolving, in 2 g of acetonitrile, 1 g of the blocked isocyanate composition obtained following removal by distillation of any free compound (I), compound (II), and the first blocking agent (A) contained in the reaction solution obtained in the first reaction step. Based on the value measured with a Hazen meter, the resultant was ranked in accordance with the following evaluation criteria.

On the other hand, in the case of the reaction liquid obtained in the addition-elimination reaction step, the concentration (mmol/g) of skeletons derived from the second blocked isocyanate compound was determined from ¹H NMR. Then, acetonitrile was added to the reaction liquid obtained following the addition-elimination reaction based on the concentration (mmol/g) of skeletons derived from the second blocked isocyanate compound within the addition-elimination reaction liquid to adjust the concentration (mmol/g) of skeletons derived from the blocked isocyanate compound to 0.40 (mmol/g), thereby preparing a Hazen color number measurement sample to carry out a Hazen color number measurement.

In the case of the reaction liquid containing a high purity of isocyanate obtained following the second reaction step, a Hazen color number measurement was conducted directly using the reaction liquid.

The Hazen color number was evaluated in accordance with the evaluation criteria shown below based on the measured values.

### (Evaluation Criteria)

Rank 1: APHA of at least 0 but less than 5
Rank 2: APHA of at least 5 but less than 10
Rank 3: APHA of at least 10 but less than 15
Rank 4: APHA of at least 15 but less than 20
Rank 5: APHA of at least 20 but less than 25
Rank 6: APHA of at least 25 but less than 30
Rank 7: APHA of at least 30 but less than 35
Rank 8: APHA of at least 35 but less than 40
Rank 9: APHA of at least 40 but less than 45
Rank 10: APHA of at least 45 but less than 50
Rank 11: APHA of at least 50 but less than 55

### [Evaluation 5-3]

### (Isocyanate Content)

The isocyanate content was determined by GC analysis.

### [Evaluation 5-4]

### (Isocyanate Yield)

The molar amount of carbon skeletons derived from the primary amine contained within the raw material in the second reaction step was determined from an NMR spectrum, and the isocyanate yield was then determined from the molar amount of isocyanate compound obtained in the BTM of the condenser tube 2, in accordance with the formula shown below.[Isocyanate yield] = (molar amount of isocyanate compound obtained in BTM of condenser tube 2) × 100 / (molar amount of carbon skeletons derived from primary amine contained in raw material in the second reaction step)

### [Example 5-1]

200 parts by mass of hexamethylenediamine (HDA), 217 parts by mass of urea, 1,800 parts by mass of phenol, and 20 parts by mass of N-methylaniline (NMA) were placed in an SUS pressure-resistant vessel equipped with a condenser tube packed with a filler (the condenser tube having a structure that allows the condensed liquid from the condenser tube to fall into the SUS pressure-resistant vessel) and an ammonia trap, followed by setting the internal temperature of the SUS pressure-resistant vessel to 240°C and the temperature of the condenser tube to 60°C. Nitrogen gas was supplied to the SUS pressure-resistant vessel from a nitrogen cylinder to adjust the absolute pressure to 350 kPa, followed by allowing the reaction to proceed for three hours. As a result, 2,114 parts by mass of a reaction liquid was obtained inside the SUS pressure-resistant vessel. The results of analyses of the reaction liquid revealed an amino group content of 0 mol%, and, based on ¹H NMR integral values, NCO-BL(A) mol% of 92 mol%, NCO-compound (1) mol% of 5 mol%, ureido group mol% of 1 mol%, and MB mol% of 98 mol%. Further, the Hazen color number of the obtained reaction liquid was determined and evaluated as rank 3. Furthermore, the free phenol and free N-methylaniline contained in the reaction liquid were removed by distillation under reduced pressure, thereby obtaining a low boiling point component-free first blocked isocyanate composition.

200 parts by mass of the low boiling point component-free first blocked isocyanate composition, 790 parts by mass of para-cumylphenol, and 10 part by mass of N-methylaniline were placed in an SUS pressure-resistant vessel equipped with a condenser tube 1 packed with a filler (the condenser tube 1 having a structure that allows the condensed liquid from the condenser tube 1 to fall into the SUS pressure-resistant vessel) and a condenser tube 2 (having a structure that allows the condensed liquid from the condenser tube 2 to be collected as a TOP liquid without entering the SUS pressure-resistant vessel) equipped with a pressure reduction device at the tip thereof. Subsequently, the internal temperature of the SUS pressure-resistant vessel was set to 250°C, the temperature of the condenser tube 1 was set to 250°C, and the temperature of the condenser tube 2 was set to 50°C. Further, the pressure inside the reaction vessel was adjusted under a nitrogen atmosphere to an absolute pressure of 100 mmHg using the pressure reduction device, followed by allowing the reaction to proceed for one hour. As a result, 500 parts by mass of a reaction liquid obtained following the addition-elimination reaction was obtained in the SUS pressure-resistant vessel, and 500 parts by mass of the TOP liquid were collected from the condenser tube 2. Based on ¹H NMR integral values, the obtained reaction liquid obtained following the addition-elimination reaction had an amino group mol% of 0 mol%, NCO-BL(A) mol% of 0 mol%, NCO-BL(C) mol% of 96 mol%, NCO-compound (I) mol% of 0 mol%, and ureido group mol% of 0 mol%. Furthermore, the Hazen color number of the obtained second blocked isocyanate composition was determined and evaluated as rank 3.

Further, 495 parts by mass of the reaction liquid obtained following the addition-elimination reaction was heated as a raw material to 250°C and then supplied to a thin-film evaporator set to a reduced pressure of 100 mmHg, and the resulting vapor was subjected to distillation separation in a distillation column 1. Following separation of the isocyanate compound, the secondary amine compound (I) and the tertiary amine compound (II) from the high-boiling point components (such as para-cumylphenol, and components formed by reaction of the isocyanate compound and para-cumylphenol), the isocyanate compound was collected from the bottom of the distillation column 2. The feed rate to the thin-film evaporator was set to 1,000 parts by mass per hour, and the liquid containing the high-boiling point components collected at the bottom of the distillation column 1 was returned to the raw material line. Following 7 hours of reaction, 70 parts by mass of liquid had been recovered from the bottom of the distillation column 2. Analysis of this liquid revealed an isocyanate content at the bottom of the distillation column 2 of 99 wt%, and an isocyanate yield of 82 mol%.

### [Examples 5-2 to 5-72 and Comparative Examples 5-1 to 5-25]

In each of Examples 5-2 to 5-72 and Comparative Examples 5-1 to 5-25, isocyanate compounds were produced using the same method as that described in Example 5-1, except that the raw materials and reaction conditions were altered as shown in the following tables.

In the following tables, the abbreviations used refer to the following compounds.

### (Secondary Amine Compounds (I))

NMA: N-methyl-aniline
NEA: N-ethyl-aniline
NBA: N-butyl-aniline
DPA: N,N-diphenylamine

### (Tertiary Amine Compounds (II))

NNDMA: N,N-dimethylaniline
NNDEA: N,N-diethylaniline
NMDPA: N-methyl-N.N-diphenylamine
TPA: triphenylamine

### (First Blocking Agents)

PhOH: phenol
n-BuOH: normal butanol
DBA: dibutylamine

### (Primary Amine Compounds)

PDA: 1,5-pentamethylenediamine
HDA: 1,6-hexamethylenediamine
IPDA: isophoronediamine (isomeric mixture)
MBCA: 4,4'-methylenebis(cyclohexylamine) (isomeric mixture)
DAT: diaminotoluene (isomeric mixture)
DPM: 4,4'-diphenylmethanediamine
TAN: 4-aminomethyl-1,8-octanediamine

### (Second Blocking Agent)

PCP: para-cumylphenol

### (Blocked Isocyanate Compound)

BL-NCO: blocked isocyanate compound

### (Functional Groups)

NCO-compound (I): blocked isocyanate group obtained by bonding of compound (I) to an isocyanate group
NCO-BL(A): blocked isocyanate group obtained by bonding of first blocking agent to an isocyanate group
NCO-BL(C): blocked isocyanate group obtained by bonding of second blocking agent to an isocyanate group

**[Table 5-1]**

| | First reaction step - Raw materials | | | | | | | | | |
|---|---|---|---|---|---|---|---|---|---|---|
| | Secondary amine compound (I) | | Tertiary amine compound (II) | | Primary amine compound | | First blocking agent | | Carbonic acid derivative | |
| | type | parts by mass | type | parts by mass | type | parts by mass | type | parts by mass | type | parts by mass |
| Example 5-1 | NMA | 20 | - | - | HDA | 200 | PhOH | 1800 | urea | 217 |
| Example 5-2 | NEA | 20 | - | - | HDA | 200 | PhOH | 1800 | urea | 217 |
| Example 5-3 | NBA | 20 | - | - | HDA | 200 | PhOH | 1800 | urea | 217 |
| Example 5-4 | DPA | 20 | - | - | HDA | 200 | PhOH | 1800 | urea | 217 |
| Example 5-5 | - | - | NNDMA | 20 | HDA | 200 | PhOH | 1800 | urea | 217 |
| Example 5-6 | - | - | NNDEA | 20 | HDA | 200 | PhOH | 1800 | urea | 217 |
| Example 5-7 | - | - | NMDPA | 20 | HDA | 200 | PhOH | 1800 | urea | 217 |
| Example 5-8 | - | - | TPA | 20 | HDA | 200 | PhOH | 1800 | urea | 217 |
| Example 5-9 | NEA | 20 | - | - | HDA | 200 | PhOH | 1800 | urea | 217 |
| Example 5-10 | NEA | 20 | - | - | HDA | 200 | PhOH | 1800 | urea | 217 |
| Example 5-11 | NEA | 20 | - | - | HDA | 200 | n-BuOH | 1800 | urea | 217 |
| Example 5-12 | NEA | 20 | - | - | HDA | 200 | DBA | 1800 | urea | 217 |
| Example 5-13 | NEA | 20 | - | - | HDA | 200 | n-BuOH | 1800 | urea | 217 |
| Example 5-14 | NEA | 20 | - | - | HDA | 200 | DBA | 1800 | urea | 217 |
| Example 5-15 | NEA | 20 | - | - | PDA | 200 | PhOH | 1800 | urea | 247 |
| Example 5-16 | NEA | 20 | - | - | PDA | 200 | n-BuOH | 1800 | urea | 247 |
| Example 5-17 | NEA | 20 | - | - | PDA | 200 | DBA | 1800 | urea | 247 |
| Example 5-18 | NEA | 20 | - | - | TAN | 200 | PhOH | 1800 | urea | 218 |
| Example 5-19 | NEA | 20 | - | - | TAN | 200 | n-BuOH | 1800 | urea | 218 |
| Example 5-20 | NEA | 20 | - | - | TAN | 200 | DBA | 1800 | urea | 218 |
| Example 5-21 | NEA | 20 | - | - | 1PDA | 200 | PhOH | 1800 | urea | 148 |
| Example 5-22 | NEA | 20 | - | - | 1PDA | 200 | n-BuOH | 1800 | urea | 148 |
| Example 5-23 | NEA | 20 | - | - | 1PDA | 200 | DBA | 1800 | urea | 148 |
| Example 5-24 | NEA | 20 | - | - | MBCA | 200 | PhOH | 1800 | urea | 120 |
| Example 5-25 | NEA | 20 | - | - | MBCA | 200 | n-BuOH | 1800 | urea | 120 |

**[Table 5-2]**

| | First reaction step - Raw materials | | | | | | | | | |
|---|---|---|---|---|---|---|---|---|---|---|
| | Secondary amine compound (I) | | Tertiary amine compound (II) | | Primary amine compound | | First blocking agent | | Carbonic acid derivative | |
| | type | parts by mass | type | parts by mass | type | parts by mass | type | parts by mass | type | parts by mass |
| Example 5-26 | NEA | 20 | - | - | MBCA | 200 | DBA | 1800 | urea | 120 |
| Example 5-27 | NEA | 20 | - | - | DAT | 200 | PhOH | 1800 | urea | 206 |
| Example 5-28 | NEA | 20 | - | - | DAT | 200 | n-BuOH | 1800 | urea | 206 |
| Example 5-29 | NEA | 20 | - | - | DAT | 200 | DBA | 1800 | urea | 206 |
| Example 5-30 | NEA | 20 | - | - | DPM | 200 | PhOH | 1800 | urea | 127 |
| Example 5-31 | NEA | 20 | - | - | DPM | 200 | n-BuOH | 1800 | urea | 127 |
| Example 5-32 | NEA | 20 | - | - | DPM | 200 | DBA | 1800 | urea | 127 |
| Example 5-33 | - | - | NNDEA | 20 | HDA | 200 | PhOH | 1800 | urea | 217 |
| Example 5-34 | - | - | NNDEA | 20 | HDA | 200 | PhOH | 1800 | urea | 217 |
| Example 5-35 | - | - | NNDEA | 20 | HDA | 200 | n-BuOH | 1800 | urea | 217 |
| Example 5-36 | - | - | NNDEA | 20 | HDA | 200 | DBA | 1800 | urea | 217 |
| Example 5-37 | - | - | NNDEA | 20 | HDA | 200 | n-BuOH | 1800 | urea | 217 |
| Example 5-38 | - | - | NNDEA | 20 | HDA | 200 | DBA | 1800 | urea | 217 |
| Example 5-39 | - | - | NNDEA | 20 | PDA | 200 | PhOH | 1800 | urea | 247 |
| Example 5-40 | - | - | NNDEA | 20 | PDA | 200 | n-BuOH | 1800 | urea | 247 |
| Example 5-41 | - | - | NNDEA | 20 | PDA | 200 | DBA | 1800 | urea | 247 |
| Example 5-42 | - | - | NNDEA | 20 | TAN | 200 | PhOH | 1800 | urea | 146 |
| Example 5-43 | - | - | NNDEA | 20 | TAN | 200 | n-BuOH | 1800 | urea | 146 |
| Example 5-44 | - | - | NNDEA | 20 | TAN | 200 | DBA | 1800 | urea | 146 |
| Example 5-45 | - | - | NNDEA | 20 | IPDA | 200 | PhOH | 1800 | urea | 148 |
| Example 5-46 | - | - | NNDEA | 20 | IPDA | 200 | n-BuOH | 1800 | urea | 148 |
| Example 5-47 | - | - | NNDEA | 20 | IPDA | 200 | DBA | 1800 | urea | 148 |
| Example 5-48 | - | - | NNDEA | 20 | MBCA | 200 | PhOH | 1800 | urea | 120 |
| Example 5-49 | - | - | NNDEA | 20 | MBCA | 200 | n-BuOH | 1800 | urea | 120 |
| Example 5-50 | - | - | NNDEA | 20 | MBCA | 200 | DBA | 1800 | urea | 120 |

**[Table 5-3]**

| | First reaction step - Raw materials | | | | | | | | | |
|---|---|---|---|---|---|---|---|---|---|---|
| | Secondary amine compound (I) | | Tertiary amine compound (II) | | Primary amine compound | | First blocking agent | | Carbonic acid derivative | |
| | type | parts by mass | type | parts by mass | type | parts by mass | type | parts by mass | type | parts by mass |
| Example 5-51 | - | - | NNDEA | 20 | DAT | 200 | PhOH | 1800 | urea | 206 |
| Example 5-52 | - | - | NNDEA | 20 | DAT | 200 | n-BuOH | 1800 | urea | 206 |
| Example 5-53 | - | - | NNDEA | 20 | DAT | 200 | DBA | 1800 | urea | 206 |
| Example 5-54 | - | - | NNDEA | 20 | DPM | 200 | PhOH | 1800 | urea | 127 |
| Example 5-55 | - | - | NNDEA | 20 | DPM | 200 | n-BuOH | 1800 | urea | 127 |
| Example 5-56 | - | - | NNDEA | 20 | DPM | 200 | DBA | 1800 | urea | 127 |
| Example 5-57 | NMA | 2000 | NNDMA | 20 | HDA | 200 | PhOH | 1800 | urea | 217 |
| Example 5-58 | NMA | 20 | NNDMA | 2000 | HDA | 200 | PhOH | 1800 | urea | 217 |
| Example 5-59 | NMA | 2000 | NNDMA | 20 | HDA | 200 | n-BuOH | 1800 | urea | 217 |
| Example 5-60 | NMA | 20 | NNDMA | 2000 | HDA | 200 | n-BuOH | 1800 | urea | 217 |
| Example 5-61 | NMA | 2000 | NNDMA | 20 | HDA | 200 | DBA | 1800 | urea | 217 |
| Example 5-62 | NMA | 20 | NNDMA | 2000 | HDA | 200 | DBA | 1800 | urea | 217 |
| Example 5-63 | NMA | 2000 | NNDMA | 20 | TAN | 200 | PhOH | 1800 | urea | 217 |
| Example 5-64 | NMA | 20 | NNDMA | 2000 | TAN | 200 | PhOH | 1800 | urea | 217 |
| Example 5-65 | NMA | 2000 | NNDMA | 20 | IPDA | 200 | PhOH | 1800 | urea | 148 |
| Example 5-66 | NMA | 20 | NNDMA | 2000 | IPDA | 200 | PhOH | 1800 | urea | 148 |
| Example 5-67 | NMA | 2000 | NNDMA | 20 | MBCA | 200 | PhOH | 1800 | urea | 120 |
| Example 5-68 | NMA | 20 | NNDMA | 2000 | MBCA | 200 | PhOH | 1800 | urea | 120 |
| Example 5-69 | NMA | 2000 | NNDMA | 20 | DAT | 200 | PhOH | 1800 | urea | 206 |
| Example 5-70 | NMA | 20 | NNDMA | 2000 | DAT | 200 | PhOH | 1800 | urea | 206 |
| Example 5-71 | NMA | 2000 | NNDMA | 20 | DPM | 200 | PhOH | 1800 | urea | 127 |
| Example 5-72 | NMA | 20 | NNDMA | 2000 | DPM | 200 | PhOH | 1800 | urea | 127 |

**[Table 5-4]**

| | First reaction step - Raw materials | | | | | | | | | |
|---|---|---|---|---|---|---|---|---|---|---|
| | Secondary amine compound (I) | | Tertiary amine compound (II) | | Primary amine compound | | First blocking agent | | Carbonic acid derivative | |
| | type | parts by mass | type | parts by mass | type | parts by mass | type | parts by mass | type | parts by mass |
| Comparative Example 5-1 | - | - | - | - | HDA | 200 | PhOH | 1800 | urea | 217 |
| Comparative Example 5-2 | - | - | - | - | HDA | 200 | n-BuOH | 1800 | urea | 217 |
| Comparative Example 5-3 | - | - | - | - | HDA | 200 | DBA | 1800 | urea | 217 |
| Comparative Example 5-4 | - | - | - | - | HDA | 200 | n-BuOH | 1800 | urea | 217 |
| Comparative Example 5-5 | - | - | - | - | HDA | 200 | DBA | 1800 | urea | 217 |
| Comparative Example 5-6 | - | - | - | - | HDA | 200 | PhOH | 1800 | urea | 217 |
| Comparative Example 5-7 | - | - | - | - | HDA | 200 | PhOH | 1800 | urea | 217 |
| Comparative Example 5-8 | - | - | - | - | PDA | 200 | PhOH | 1800 | urea | 247 |
| Comparative Example 5-9 | - | - | - | - | PDA | 200 | n-BuOH | 1800 | urea | 247 |
| Comparative Example 5-10 | - | - | - | - | PDA | 200 | DBA | 1800 | urea | 247 |
| Comparative Example 5-11 | - | - | - | - | TAN | 200 | PhOH | 1800 | urea | 146 |
| Comparative Example 5-12 | - | - | - | - | TAN | 200 | n-BuOH | 1800 | urea | 146 |
| Comparative Example 5-13 | - | - | - | - | TAN | 200 | DBA | 1800 | urea | 146 |
| Comparative Example 5-14 | - | - | - | - | IPDA | 200 | PhOH | 1800 | urea | 148 |
| Comparative Example 5-15 | - | - | - | - | IPDA | 200 | n-BuOH | 1800 | urea | 148 |
| Comparative Example 5-16 | - | - | - | - | IPDA | 200 | DBA | 1800 | urea | 148 |
| Comparative Example 5-17 | - | - | - | - | MBCA | 200 | PhOH | 1800 | urea | 120 |
| Comparative Example 5-18 | - | - | - | - | MBCA | 200 | n-BuOH | 1800 | urea | 120 |
| Comparative Example 5-19 | - | - | - | - | MBCA | 200 | DBA | 1800 | urea | 120 |
| Comparative Example 5-20 | - | - | - | - | DAT | 200 | PhOH | 1800 | urea | 206 |
| Comparative Example 5-21 | - | - | - | - | DAT | 200 | n-BuOH | 1800 | urea | 206 |
| Comparative Example 5-22 | - | - | - | - | DAT | 200 | DBA | 1800 | urea | 206 |
| Comparative Example 5-23 | - | - | - | - | DPM | 200 | PhOH | 1800 | urea | 127 |
| Comparative Example 5-24 | - | - | - | - | DPM | 200 | n-BuOH | 1800 | urea | 127 |
| Comparative Example 5-25 | - | - | - | - | DPM | 200 | DBA | 1800 | urea | 127 |

**[Table 5-5]**

| | First reaction step - Reaction conditions | | | | | |
|---|---|---|---|---|---|---|
| | Reaction temperature | Absolute pressure | Condenser tube temperature | Reaction time | Oxygen concentration | BTM |
| | °C | kPa | °C | hours | vol% | parts by mass |
| Example 5-1 | 240 | 350 | 60 | 3 | 0 | 2114 |
| Example 5-2 | 240 | 350 | 60 | 3 | 0 | 2114 |
| Example 5-3 | 240 | 350 | 60 | 3 | 0 | 2114 |
| Example 5-4 | 240 | 350 | 60 | 3 | 0 | 2114 |
| Example 5-5 | 240 | 350 | 60 | 3 | 0 | 2115 |
| Example 5-6 | 240 | 350 | 60 | 3 | 0 | 2115 |
| Example 5-7 | 240 | 350 | 60 | 3 | 0 | 2115 |
| Example 5-8 | 240 | 350 | 60 | 3 | 0 | 2115 |
| Example 5-9 | 240 | 350 | 60 | 3 | 0 | 2114 |
| Example 5-10 | 240 | 350 | 60 | 3 | 0 | 2114 |
| Example 5-11 | 240 | 350 | 60 | 3 | 0 | 2114 |
| Example 5-12 | 240 | 350 | 60 | 3 | 0 | 2114 |
| Example 5-13 | 240 | 350 | 60 | 3 | 0 | 2114 |
| Example 5-14 | 240 | 350 | 60 | 3 | 0 | 2114 |
| Example 5-15 | 240 | 350 | 60 | 3 | 0 | 2127 |
| Example 5-16 | 240 | 350 | 60 | 3 | 0 | 2127 |
| Example 5-17 | 240 | 350 | 60 | 3 | 0 | 2127 |
| Example 5-18 | 240 | 350 | 60 | 3 | 0 | 2053 |
| Example 5-19 | 240 | 350 | 60 | 3 | 0 | 2053 |
| Example 5-20 | 240 | 350 | 60 | 3 | 0 | 2053 |
| Example 5-21 | 240 | 350 | 60 | 3 | 0 | 2084 |
| Example 5-22 | 240 | 350 | 60 | 3 | 0 | 2084 |
| Example 5-23 | 240 | 350 | 60 | 3 | 0 | 2084 |
| Example 5-24 | 240 | 350 | 60 | 3 | 0 | 2072 |
| Example 5-25 | 240 | 350 | 60 | 3 | 0 | 2072 |

**[Table 5-6]**

| | First reaction step - Reaction conditions | | | | | |
|---|---|---|---|---|---|---|
| | Reaction temperature | Absolute pressure | Condenser tube temperature | Reaction time | Oxygen concentration | BTM |
| | °C | kPa | °C | hours | vol% | parts by mass |
| Example 5-26 | 240 | 350 | 60 | 3 | 0 | 2072 |
| Example 5-27 | 240 | 350 | 60 | 3 | 0 | 2110 |
| Example 5-28 | 240 | 350 | 60 | 3 | 0 | 2110 |
| Example 5-29 | 240 | 350 | 60 | 3 | 0 | 2110 |
| Example 5-30 | 240 | 350 | 60 | 3 | 0 | 2075 |
| Example 5-31 | 240 | 350 | 60 | 3 | 0 | 2075 |
| Example 5-32 | 240 | 350 | 60 | 3 | 0 | 2075 |
| Example 5-33 | 240 | 350 | 60 | 3 | 0 | 2115 |
| Example 5-34 | 240 | 350 | 60 | 3 | 0 | 2115 |
| Example 5-35 | 240 | 350 | 60 | 3 | 0 | 2115 |
| Example 5-36 | 240 | 350 | 60 | 3 | 0 | 2114 |
| Example 5-37 | 240 | 350 | 60 | 3 | 0 | 2115 |
| Example 5-38 | 240 | 350 | 60 | 3 | 0 | 2114 |
| Example 5-39 | 240 | 350 | 60 | 3 | 0 | 2128 |
| Example 5-40 | 240 | 350 | 60 | 3 | 0 | 2128 |
| Example 5-41 | 240 | 350 | 60 | 3 | 0 | 2127 |
| Example 5-42 | 240 | 350 | 60 | 3 | 0 | 2083 |
| Example 5-43 | 240 | 350 | 60 | 3 | 0 | 2083 |
| Example 5-44 | 240 | 350 | 60 | 3 | 0 | 2083 |
| Example 5-45 | 240 | 350 | 60 | 3 | 0 | 2085 |
| Example 5-46 | 240 | 350 | 60 | 3 | 0 | 2085 |
| Example 5-47 | 240 | 350 | 60 | 3 | 0 | 2085 |
| Example 5-48 | 240 | 350 | 60 | 3 | 0 | 2071 |
| Example 5-49 | 240 | 350 | 60 | 3 | 0 | 2072 |
| Example 5-50 | 240 | 350 | 60 | 3 | 0 | 2072 |

**[Table 5-7]**

| | First reaction step - Reaction conditions | | | | | |
|---|---|---|---|---|---|---|
| | Reaction temperature | Absolute pressure | Condenser tube temperature | Reaction time | Oxygen concentration | BTM |
| | °C | kPa | °C | hours | vol% | parts by mass |
| Example 5-51 | 240 | 350 | 60 | 3 | 0 | 2110 |
| Example 5-52 | 240 | 350 | 60 | 3 | 0 | 2110 |
| Example 5-53 | 240 | 350 | 60 | 3 | 0 | 2110 |
| Example 5-54 | 240 | 350 | 60 | 3 | 0 | 2076 |
| Example 5-55 | 240 | 350 | 60 | 3 | 0 | 2076 |
| Example 5-56 | 240 | 350 | 60 | 3 | 0 | 2075 |
| Example 5-57 | 240 | 350 | 60 | 3 | 0 | 4114 |
| Example 5-58 | 240 | 350 | 60 | 3 | 0 | 4114 |
| Example 5-59 | 240 | 350 | 60 | 3 | 0 | 4114 |
| Example 5-60 | 240 | 350 | 60 | 3 | 0 | 4114 |
| Example 5-61 | 240 | 350 | 60 | 3 | 0 | 4114 |
| Example 5-62 | 240 | 350 | 60 | 3 | 0 | 4114 |
| Example 5-63 | 240 | 350 | 60 | 3 | 0 | 4114 |
| Example 5-64 | 240 | 350 | 60 | 3 | 0 | 4114 |
| Example 5-65 | 240 | 350 | 60 | 3 | 0 | 4084 |
| Example 5-66 | 240 | 350 | 60 | 3 | 0 | 4084 |
| Example 5-67 | 240 | 350 | 60 | 3 | 0 | 4072 |
| Example 5-68 | 240 | 350 | 60 | 3 | 0 | 4072 |
| Example 5-69 | 240 | 350 | 60 | 3 | 0 | 4110 |
| Example 5-70 | 240 | 350 | 60 | 3 | 0 | 4110 |
| Example 5-71 | 240 | 350 | 60 | 3 | 0 | 4075 |
| Example 5-72 | 240 | 350 | 60 | 3 | 0 | 4075 |

**[Table 5-8]**

| | First reaction step - Reaction conditions | | | | | |
|---|---|---|---|---|---|---|
| | Reaction temperature | Absolute pressure | Condenser tube temperature | Reaction time | Oxygen concentration | BTM |
| | °C | kPa | °C | hours | vol% | parts by mass |
| Comparative Example 5-1 | 240 | 350 | 60 | 3 | 0 | 2095 |
| Comparative Example 5-2 | 240 | 350 | 60 | 3 | 0 | 2095 |
| Comparative Example 5-3 | 240 | 350 | 60 | 3 | 0 | 2095 |
| Comparative Example 5-4 | 240 | 350 | 60 | 3 | 0 | 2095 |
| Comparative Example 5-5 | 240 | 350 | 60 | 3 | 0 | 2095 |
| Comparative Example 5-6 | 240 | 350 | 60 | 3 | 0 | 2095 |
| Comparative Example 5-7 | 240 | 350 | 60 | 3 | 0 | 2095 |
| Comparative Example 5-8 | 240 | 350 | 60 | 3 | 0 | 2108 |
| Comparative Example 5-9 | 240 | 350 | 60 | 3 | 0 | 2108 |
| Comparative Example 5-10 | 240 | 350 | 60 | 3 | 0 | 2107 |
| Comparative Example 5-11 | 240 | 350 | 60 | 3 | 0 | 2064 |
| Comparative Example 5-12 | 240 | 350 | 60 | 3 | 0 | 2064 |
| Comparative Example 5-13 | 240 | 350 | 60 | 3 | 0 | 2063 |
| Comparative Example 5-14 | 240 | 350 | 60 | 3 | 0 | 2065 |
| Comparative Example 5-15 | 240 | 350 | 60 | 3 | 0 | 2065 |
| Comparative Example 5-16 | 240 | 350 | 60 | 3 | 0 | 2064 |
| Comparative Example 5-17 | 240 | 350 | 60 | 3 | 0 | 2052 |
| Comparative Example 5-18 | 240 | 350 | 60 | 3 | 0 | 2052 |
| Comparative Example 5-19 | 240 | 350 | 60 | 3 | 0 | 2052 |
| Comparative Example 5-20 | 240 | 350 | 60 | 3 | 0 | 2090 |
| Comparative Example 5-21 | 240 | 350 | 60 | 3 | 0 | 2090 |
| Comparative Example 5-22 | 240 | 350 | 60 | 3 | 0 | 2090 |
| Comparative Example 5-23 | 240 | 350 | 60 | 3 | 0 | 2056 |
| Comparative Example 5-24 | 240 | 350 | 60 | 3 | 0 | 2056 |
| Comparative Example 5-25 | 240 | 350 | 60 | 3 | 0 | 2055 |

**[Table 5-9]**

| | First reaction step - Evaluation results | | | | | |
|---|---|---|---|---|---|---|
| | Amino groups | NCO-BL(A) | NCO-compound (I) | Ureido groups | MB | Hazen color number |
| | mol% | mol% | mol% | mol% | mol% | APHA |
| Example 5-1 | 0 | 92 | 5 | 1 | 98 | 2 |
| Example 5-2 | 0 | 92 | 5 | 1 | 98 | 2 |
| Example 5-3 | 0 | 92 | 4 | 1 | 97 | 2 |
| Example 5-4 | 0 | 93 | 3 | 1 | 97 | 2 |
| Example 5-5 | 0 | 92 | 0 | 3 | 95 | 3 |
| Example 5-6 | 0 | 92 | 0 | 3 | 95 | 3 |
| Example 5-7 | 0 | 92 | 0 | 3 | 95 | 3 |
| Example 5-8 | 0 | 92 | 0 | 3 | 95 | 3 |
| Example 5-9 | 0 | 92 | 5 | 1 | 98 | 2 |
| Example 5-10 | 0 | 92 | 5 | 1 | 98 | 2 |
| Example 5-11 | 0 | 92 | 5 | 1 | 98 | 3 |
| Example 5-12 | 0 | 84 | 2 | 1 | 87 | 4 |
| Example 5-13 | 0 | 92 | 5 | 1 | 98 | 3 |
| Example 5-14 | 0 | 83 | 2 | 1 | 86 | 4 |
| Example 5-15 | 0 | 92 | 5 | 1 | 98 | 2 |
| Example 5-16 | 0 | 91 | 5 | 1 | 97 | 3 |
| Example 5-17 | 0 | 83 | 2 | 1 | 86 | 4 |
| Example 5-18 | 0 | 89 | 7 | 1 | 97 | 2 |
| Example 5-19 | 0 | 90 | 7 | 1 | 98 | 3 |
| Example 5-20 | 0 | 84 | 2 | 1 | 87 | 4 |
| Example 5-21 | 0 | 89 | 7 | 1 | 97 | 2 |
| Example 5-22 | 0 | 90 | 7 | 1 | 98 | 3 |
| Example 5-23 | 0 | 83 | 2 | 1 | 86 | 4 |
| Example 5-24 | 0 | 88 | 9 | 1 | 98 | 2 |
| Example 5-25 | 0 | 88 | 9 | 1 | 98 | 3 |

**[Table 5-10]**

| | First reaction step - Evaluation results | | | | | |
|---|---|---|---|---|---|---|
| | Amino groups | NCO-BL(A) | NCO-compound (I) | Ureido groups | MB | Hazen color number |
| | mol% | mol% | mol% | mol% | mol% | APHA |
| Example 5-26 | 0 | 84 | 2 | 1 | 87 | 4 |
| Example 5-27 | 0 | 91 | 5 | 1 | 97 | 3 |
| Example 5-28 | 0 | 92 | 5 | 1 | 98 | 4 |
| Example 5-29 | 0 | 80 | 2 | 1 | 83 | 5 |
| Example 5-30 | 0 | 89 | 8 | 1 | 98 | 2 |
| Example 5-31 | 0 | 89 | 8 | 1 | 98 | 3 |
| Example 5-32 | 0 | 79 | 2 | 1 | 82 | 4 |
| Example 5-33 | 0 | 92 | 0 | 3 | 95 | 3 |
| Example 5-34 | 0 | 92 | 0 | 3 | 95 | 3 |
| Example 5-35 | 0 | 92 | 0 | 3 | 95 | 4 |
| Example 5-36 | 0 | 81 | 0 | 1 | 82 | 5 |
| Example 5-37 | 0 | 91 | 0 | 3 | 94 | 4 |
| Example 5-38 | 0 | 81 | 0 | 1 | 82 | 5 |
| Example 5-39 | 0 | 92 | 0 | 3 | 95 | 3 |
| Example 5-40 | 0 | 91 | 0 | 3 | 94 | 4 |
| Example 5-41 | 0 | 81 | 0 | 1 | 82 | 5 |
| Example 5-42 | 0 | 91 | 0 | 3 | 94 | 3 |
| Example 5-43 | 0 | 90 | 0 | 3 | 93 | 4 |
| Example 5-44 | 0 | 81 | 0 | 1 | 82 | 5 |
| Example 5-45 | 0 | 92 | 0 | 3 | 95 | 3 |
| Example 5-46 | 0 | 92 | 0 | 3 | 95 | 4 |
| Example 5-47 | 0 | 81 | 0 | 1 | 82 | 5 |
| Example 5-48 | 0 | 93 | 0 | 3 | 96 | 3 |
| Example 5-49 | 0 | 92 | 0 | 3 | 95 | 4 |
| Example 5-50 | 0 | 81 | 0 | 1 | 82 | 5 |

**[Table 5-11]**

| | First reaction step - Evaluation results | | | | | |
|---|---|---|---|---|---|---|
| | Amino groups | NCO-BL(A) | NCO-compound (I) | Ureido groups | MB | Hazen color number |
| | mol% | mol% | mol% | mol% | mol% | APHA |
| Example 5-51 | 0 | 91 | 0 | 3 | 94 | 4 |
| Example 5-52 | 0 | 92 | 0 | 3 | 95 | 5 |
| Example 5-53 | 0 | 80 | 0 | 1 | 81 | 6 |
| Example 5-54 | 0 | 92 | 0 | 3 | 95 | 3 |
| Example 5-55 | 0 | 91 | 0 | 3 | 94 | 4 |
| Example 5-56 | 0 | 80 | 0 | 1 | 81 | 5 |
| Example 5-57 | 0 | 18 | 79 | 1 | 98 | 1 |
| Example 5-58 | 0 | 96 | 0 | 1 | 97 | 1 |
| Example 5-59 | 0 | 18 | 79 | 1 | 98 | 1 |
| Example 5-60 | 0 | 96 | 0 | 1 | 97 | 1 |
| Example 5-61 | 0 | 7 | 79 | 1 | 87 | 2 |
| Example 5-62 | 0 | 83 | 2 | 1 | 86 | 2 |
| Example 5-63 | 0 | 18 | 79 | 1 | 98 | 1 |
| Example 5-64 | 0 | 97 | 0 | 1 | 98 | 1 |
| Example 5-65 | 0 | 17 | 79 | 1 | 97 | 1 |
| Example 5-66 | 0 | 97 | 0 | 1 | 98 | 1 |
| Example 5-67 | 0 | 18 | 79 | 1 | 98 | 1 |
| Example 5-68 | 0 | 97 | 0 | 1 | 98 | 1 |
| Example 5-69 | 0 | 18 | 79 | 1 | 98 | 1 |
| Example 5-70 | 0 | 96 | 0 | 1 | 97 | 1 |
| Example 5-71 | 0 | 18 | 79 | 1 | 98 | 1 |
| Example 5-72 | 0 | 96 | 0 | 1 | 97 | 1 |

**[Table 5-12]**

| | First reaction step - Evaluation results | | | | | |
|---|---|---|---|---|---|---|
| | Amino groups | NCO-BL(A) | NCO-compound (I) | Ureido groups | MB | Hazen color number |
| | mol% | mol% | mol% | mol% | mol% | APHA |
| Comparative Example 5-1 | 0 | 92 | 0 | 3 | 95 | 7 |
| Comparative Example 5-2 | 0 | 92 | 0 | 3 | 95 | 8 |
| Comparative Example 5-3 | 0 | 81 | 0 | 3 | 84 | 9 |
| Comparative Example 5-4 | 0 | 92 | 0 | 3 | 95 | 8 |
| Comparative Example 5-5 | 0 | 82 | 0 | 3 | 85 | 9 |
| Comparative Example 5-6 | 0 | 92 | 0 | 3 | 95 | 7 |
| Comparative Example 5-7 | 0 | 92 | 0 | 3 | 95 | 7 |
| Comparative Example 5-8 | 0 | 92 | 0 | 3 | 95 | 7 |
| Comparative Example 5-9 | 0 | 92 | 0 | 3 | 95 | 8 |
| Comparative Example 5-10 | 0 | 85 | 0 | 1 | 86 | 9 |
| Comparative Example 5-11 | 0 | 92 | 0 | 3 | 95 | 7 |
| Comparative Example 5-12 | 0 | 91 | 0 | 3 | 94 | 8 |
| Comparative Example 5-13 | 0 | 85 | 0 | 1 | 86 | 9 |
| Comparative Example 5-14 | 0 | 92 | 0 | 3 | 95 | 7 |
| Comparative Example 5-15 | 0 | 91 | 0 | 3 | 94 | 8 |
| Comparative Example 5-16 | 0 | 85 | 0 | 1 | 86 | 9 |
| Comparative Example 5-17 | 0 | 92 | 0 | 3 | 95 | 7 |
| Comparative Example 5-18 | 0 | 91 | 0 | 3 | 94 | 8 |
| Comparative Example 5-19 | 0 | 85 | 0 | 1 | 86 | 9 |
| Comparative Example 5-20 | 0 | 91 | 0 | 3 | 94 | 8 |
| Comparative Example 5-21 | 0 | 92 | 0 | 3 | 95 | 9 |
| Comparative Example 5-22 | 0 | 80 | 0 | 1 | 81 | 10 |
| Comparative Example 5-23 | 0 | 91 | 0 | 3 | 94 | 7 |
| Comparative Example 5-24 | 0 | 92 | 0 | 3 | 95 | 8 |
| Comparative Example 5-25 | 0 | 79 | 0 | 1 | 80 | 9 |

**[Table 5-13]**

| | Addition-elimination reaction step - Raw materials | | | | | | |
|---|---|---|---|---|---|---|---|
| | First blocked isocyanate composition | Secondary amine compound (I) | | Tertiary amine compound (II) | | Second blocking agent | |
| | parts by mass | type | parts by mass | type | parts by mass | type | parts by mass |
| Example 5-1 | 200 | NMA | 10 | - | - | PCP | 790 |
| Example 5-2 | 200 | NEA | 10 | - | - | PCP | 790 |
| Example 5-3 | 200 | NBA | 10 | - | - | PCP | 790 |
| Example 5-4 | 200 | DPA | 10 | - | - | PCP | 790 |
| Example 5-5 | 200 | - | - | NNDMA | 10 | PCP | 790 |
| Example 5-6 | 200 | - | - | NNDEA | 10 | PCP | 790 |
| Example 5-7 | 200 | - | - | NMDPA | 10 | PCP | 790 |
| Example 5-8 | 200 | - | - | TPA | 10 | PCP | 790 |
| Example 5-9 | 200 | NEA | 10 | - | - | triethylene glycol monobutyl ether | 790 |
| Example 5-10 | 200 | NEA | 10 | - | - | bis(2-ethyl hexylamine) | 790 |
| Example 5-11 | 200 | NEA | 10 | - | - | PCP | 790 |
| Example 5-12 | 200 | NEA | 10 | - | - | PCP | 790 |
| Example 5-13 | 200 | NEA | 10 | - | - | triethylene glycol monobutyl ether | 790 |
| Example 5-14 | 200 | NEA | 10 | - | - | bis(2-ethyl hexylamine) | 790 |
| Example 5-15 | 200 | NEA | 10 | - | - | PCP | 790 |
| Example 5-16 | 200 | NEA | 10 | - | - | PCP | 790 |
| Example 5-17 | 200 | NEA | 10 | - | - | PCP | 790 |
| Example 5-18 | 200 | NEA | 10 | - | - | 2,4-di-α-cumylphenol | 790 |
| Example 5-19 | 200 | NEA | 10 | - | - | 2,4-di-α-cumylphenol | 790 |
| Example 5-20 | 200 | NEA | 10 | - | - | 2,4-di-α-cumylphenol | 790 |
| Example 5-21 | 200 | NEA | 10 | - | - | 2,4-di-α-cumylphenol | 790 |
| Example 5-22 | 200 | NEA | 10 | - | - | 2,4-di-α-cumylphenol | 790 |
| Example 5-23 | 200 | NEA | 10 | - | - | 2,4-di-α-cumylphenol | 790 |
| Example 5-24 | 200 | NEA | 10 | - | - | 2,4-di-α-cumylphenol | 790 |
| Example 5-25 | 200 | NEA | 10 | - | - | 2,4-di-α-cumylphenol | 790 |

**[Table 5-14]**

| | Addition-elimination reaction step - Raw materials | | | | | | |
|---|---|---|---|---|---|---|---|
| | First blocked isocyanate composition | Secondary amine compound (I) | | Tertiary amine compound (II) | | Second blocking agent | |
| | parts by mass | type | parts by mass | type | parts by mass | type | parts by mass |
| Example 5-26 | 200 | NEA | 10 | - | - | 2,4-di-α-cumylphenol | 790 |
| Example 5-27 | 200 | NEA | 10 | - | - | PCP | 790 |
| Example 5-28 | 200 | NEA | 10 | - | - | PCP | 790 |
| Example 5-29 | 200 | NEA | 10 | - | - | PCP | 790 |
| Example 5-30 | 200 | NEA | 10 | - | - | 2,4-di-α-cumylphenol | 790 |
| Example 5-31 | 200 | NEA | 10 | - | - | 2,4-di-α-cumylphenol | 790 |
| Example 5-32 | 200 | NEA | 10 | - | - | 2,4-di-α-cumylphenol | 790 |
| Example 5-33 | 200 | - | - | NNDEA | 10 | triethylene glycol monobutyl ether | 790 |
| Example 5-34 | 200 | - | - | NNDEA | 10 | bis(2-ethyl hexylamine) | 790 |
| Example 5-35 | 200 | - | - | NNDEA | 10 | PCP | 790 |
| Example 5-36 | 200 | - | - | NNDEA | 10 | PCP | 790 |
| Example 5-37 | 200 | - | - | NNDEA | 10 | triethylene glycol monobutyl ether | 790 |
| Example 5-38 | 200 | - | - | NNDEA | 10 | bis(2-ethyl hexylamine) | 790 |
| Example 5-39 | 200 | - | - | NNDEA | 10 | PCP | 790 |
| Example 5-40 | 200 | - | - | NNDEA | 10 | PCP | 790 |
| Example 5-41 | 200 | - | - | NNDEA | 10 | PCP | 790 |
| Example 5-42 | 200 | - | - | NNDMA | 10 | 2,4-di-α-cumylphenol | 790 |
| Example 5-43 | 200 | - | - | NNDEA | 10 | 2,4-di-α-cumylphenol | 790 |
| Example 5-44 | 200 | - | - | NNDEA | 10 | 2,4-di-α-cumylphenol | 790 |
| Example 5-45 | 200 | - | - | NNDEA | 10 | 2,4-di-α-cumylphenol | 790 |
| Example 5-46 | 200 | - | - | NNDEA | 10 | 2,4-di-α-cumylphenol | 790 |
| Example 5-47 | 200 | - | - | NNDEA | 10 | 2,4-di-α-cumylphenol | 790 |
| Example 5-48 | 200 | - | - | NNDEA | 10 | 2,4-di-α-cumylphenol | 790 |
| Example 5-49 | 200 | - | - | NNDEA | 10 | 2,4-di-α-cumylphenol | 790 |
| Example 5-50 | 200 | - | - | NNDEA | 10 | 2,4-di-α-cumylphenol | 790 |

**[Table 5-15]**

| | Addition-elimination reaction step - Raw materials | | | | | | |
|---|---|---|---|---|---|---|---|
| | First blocked isocyanate composition | Secondary amine compound (1) | | Tertiary amine compound (11) | | Second blocking agent | |
| | parts by mass | type | parts by mass | type | parts by mass | type | parts by mass |
| Example 5-51 | 200 | - | - | NNDEA | 10 | PCP | 790 |
| Example 5-52 | 200 | - | - | NNDEA | 10 | PCP | 790 |
| Example 5-53 | 200 | - | - | NNDEA | 10 | PCP | 790 |
| Example 5-54 | 200 | - | - | NNDEA | 10 | 2,4-di-α-cumylphenol | 790 |
| Example 5-55 | 200 | - | - | NNDEA | 10 | 2.4-di-α-cumylphenol | 790 |
| Example 5-56 | 200 | - | - | NNDEA | 10 | 2,4-di-α-cumylphenol | 790 |
| Example 5-57 | 200 | NMA | 1000 | NNDMA | 10 | PCP | 790 |
| Example 5-58 | 200 | NMA | 10 | NNDMA | 1000 | PCP | 790 |
| Example 5-59 | 200 | NMA | 1000 | NNDMA | 10 | PCP | 790 |
| Example 5-60 | 200 | NMA | 10 | NNDMA | 1000 | PCP | 790 |
| Example 5-61 | 200 | NMA | 1000 | NNDMA | 10 | PCP | 790 |
| Example 5-62 | 200 | NMA | 10 | NNDMA | 1000 | PCP | 790 |
| Example 5-63 | 200 | NMA | 1000 | NNDMA | 10 | 2.4-di-α-cumylphenol | 790 |
| Example 5-64 | 200 | NMA | 10 | NNDMA | 1000 | 2,4-di-α-cumylphenol | 790 |
| Example 5-65 | 200 | NMA | 1000 | NNDMA | 10 | 2,4-di-α-cumylphenol | 790 |
| Example 5-66 | 200 | NMA | 10 | NNDMA | 1000 | 2,4-di-α-cumylphenol | 790 |
| Example 5-67 | 200 | NMA | 1000 | NNDMA | 10 | 2,4-di-α-cumylphenol | 790 |
| Example 5-68 | 200 | NMA | 10 | NNDMA | 1000 | 2,4-di-α-cumylphenol | 790 |
| Example 5-69 | 200 | NMA | 1000 | NNDMA | 10 | PCP | 790 |
| Example 5-70 | 200 | NMA | 10 | NNDMA | 1000 | PCP | 790 |
| Example 5-71 | 200 | NMA | 1000 | NNDMA | 10 | 2,4-di-α-cumylphenol | 790 |
| Example 5-72 | 200 | NMA | 10 | NNDMA | 1000 | 2,4-di-α-cumylphenol | 790 |

**[Table 5-16]**

| | Addition-elimination reaction step - Raw materials | | | | | | |
|---|---|---|---|---|---|---|---|
| | First blocked isocyanate composition | Secondary amine compound (I) | | Tertiary amine compound (II) | | Second blocking agent | |
| | parts by mass | type | parts by mass | type | parts by mass | type | parts by mass |
| Comparative Example 5-1 | 200 | - | - | - | - | PCP | 790 |
| Comparative Example 5-2 | 200 | - | - | - | - | PCP | 790 |
| Comparative Example 5-3 | 200 | - | - | - | - | PCP | 790 |
| Comparative Example 5-4 | 200 | - | - | - | - | triethylene glycol monobutyl ether | 790 |
| Comparative Example 5-5 | 200 | - | - | - | - | bis(2-ethyl hexylamine) | 790 |
| Comparative Example 5-6 | 200 | - | - | - | - | triethylene glycol monobutyl ether | 790 |
| Comparative Example 5-7 | 200 | - | - | - | - | bis(2-ethyl hexylamine) | 790 |
| Comparative Example 5-8 | 200 | - | - | - | - | PCP | 790 |
| Comparative Example 5-9 | 200 | - | - | - | - | PCP | 790 |
| Comparative Example 5-10 | 200 | - | - | - | - | PCP | 790 |
| Comparative Example 5-11 | 200 | - | - | - | - | 2,4-di-α-cumylphenol | 790 |
| Comparative Example 5-12 | 200 | - | - | - | - | 2,4-di-α-cumylphenol | 790 |
| Comparative Example 5-13 | 200 | - | - | - | - | 2,4-di-α-cumylphenol | 790 |
| Comparative Example 5-14 | 200 | - | - | - | - | 2,4-di-α-cumylphenol | 790 |
| Comparative Example 5-15 | 200 | - | - | - | - | 2,4-di-α-cumylphenol | 790 |
| Comparative Example 5-16 | 200 | - | - | - | - | 2,4-di-α-cumylphenol | 790 |
| Comparative Example 5-17 | 200 | - | - | - | - | 2,4-di-α-cumylphenol | 790 |
| Comparative Example 5-18 | 200 | - | - | - | - | 2,4-di-α-cumylphenol | 790 |
| Comparative Example 5-19 | 200 | - | - | - | - | 2,4-di-α-cumylphenol | 790 |
| Comparative Example 5-20 | 200 | - | - | - | - | PCP | 790 |
| Comparative Example 5-21 | 200 | - | - | - | - | PCP | 790 |
| Comparative Example 5-22 | 200 | - | - | - | - | PCP | 790 |
| Comparative Example 5-23 | 200 | - | - | - | - | 2,4-di-α-cumylphenol | 790 |
| Comparative Example 5-24 | 200 | - | - | - | - | 2,4-di-α-cumylphenol | 790 |
| Comparative Example 5-25 | 200 | - | - | - | - | 2,4-di-α-cumylphenol | 790 |

**[Table 5-17]**

| | Addition-elimination reaction step - Reaction conditions | | | | | | |
|---|---|---|---|---|---|---|---|
| | Reaction temperature | Absolute pressure | Condenser tube 1 temperature | Condenser tube 2 temperature | Reaction time | Oxygen concentration | BTM |
| | °C | mmHg | °C | °C | hours | vol% | parts by mass |
| Example 5-1 | 250 | 100 | 250 | 50 | 1 | 0 | 500 |
| Example 5-2 | 250 | 100 | 250 | 50 | 1 | 0 | 500 |
| Example 5-3 | 250 | 100 | 250 | 50 | 1 | 0 | 500 |
| Example 5-4 | 250 | 100 | 250 | 50 | 1 | 0 | 500 |
| Example 5-5 | 250 | 100 | 250 | 50 | 1 | 0 | 500 |
| Example 5-6 | 250 | 100 | 250 | 50 | 1 | 0 | 500 |
| Example 5-7 | 250 | 100 | 250 | 50 | 1 | 0 | 500 |
| Example 5-8 | 250 | 100 | 250 | 50 | 1 | 0 | 500 |
| Example 5-9 | 250 | 400 | 250 | 50 | 1 | 0 | 500 |
| Example 5-10 | 250 | 430 | 250 | 50 | 1 | 0 | 500 |
| Example 5-11 | 250 | 100 | 250 | 50 | 1 | 0 | 500 |
| Example 5-12 | 250 | 100 | 250 | 50 | 1 | 0 | 500 |
| Example 5-13 | 250 | 400 | 250 | 50 | 1 | 0 | 500 |
| Example 5-14 | 250 | 430 | 250 | 50 | 1 | 0 | 500 |
| Example 5-15 | 250 | 100 | 250 | 50 | 1 | 0 | 500 |
| Example 5-16 | 250 | 100 | 250 | 50 | 1 | 0 | 500 |
| Example 5-17 | 250 | 100 | 250 | 50 | 1 | 0 | 500 |
| Example 5-18 | 250 | 30 | 250 | 25 | 1 | 0 | 500 |
| Example 5-19 | 250 | 30 | 250 | 25 | 1 | 0 | 500 |
| Example 5-20 | 250 | 30 | 250 | 25 | 1 | 0 | 500 |
| Example 5-21 | 250 | 30 | 250 | 25 | 1 | 0 | 500 |
| Example 5-22 | 250 | 30 | 250 | 25 | 1 | 0 | 500 |
| Example 5-23 | 250 | 30 | 250 | 25 | 1 | 0 | 500 |
| Example 5-24 | 250 | 30 | 250 | 25 | 1 | 0 | 500 |
| Example 5-25 | 250 | 30 | 250 | 25 | 1 | 0 | 500 |

**[Table 5-18]**

| | Addition-elimination reaction step - Reaction conditions | | | | | | |
|---|---|---|---|---|---|---|---|
| | Reaction temperature | Absolute pressure | Condenser tube 1 temperature | Condenser tube 2 temperature | Reaction time | Oxygen concentration | BTM |
| | °C | mmHg | °C | °C | hours | vol% | parts by mass |
| Example 5-26 | 250 | 30 | 250 | 25 | 1 | 0 | 500 |
| Example 5-27 | 250 | 100 | 250 | 50 | 1 | 0 | 500 |
| Example 5-28 | 250 | 100 | 250 | 50 | 1 | 0 | 500 |
| Example 5-29 | 250 | 100 | 250 | 50 | 1 | 0 | 500 |
| Example 5-30 | 250 | 30 | 250 | 25 | 1 | 0 | 500 |
| Example 5-31 | 250 | 30 | 250 | 25 | 1 | 0 | 500 |
| Example 5-32 | 250 | 30 | 250 | 25 | 1 | 0 | 500 |
| Example 5-33 | 250 | 400 | 250 | 50 | 1 | 0 | 500 |
| Example 5-34 | 250 | 430 | 250 | 50 | 1 | 0 | 500 |
| Example 5-35 | 250 | 100 | 250 | 50 | 1 | 0 | 500 |
| Example 5-36 | 250 | 100 | 250 | 50 | 1 | 0 | 500 |
| Example 5-37 | 250 | 400 | 250 | 50 | 1 | 0 | 500 |
| Example 5-38 | 250 | 430 | 250 | 50 | 1 | 0 | 500 |
| Example 5-39 | 250 | 100 | 250 | 50 | 1 | 0 | 500 |
| Example 5-40 | 250 | 100 | 250 | 50 | 1 | 0 | 500 |
| Example 5-41 | 250 | 100 | 250 | 50 | 1 | 0 | 500 |
| Example 5-42 | 250 | 30 | 250 | 25 | 1 | 0 | 500 |
| Example 5-43 | 250 | 30 | 250 | 25 | 1 | 0 | 500 |
| Example 5-44 | 250 | 30 | 250 | 25 | 1 | 0 | 500 |
| Example 5-45 | 250 | 30 | 250 | 25 | 1 | 0 | 500 |
| Example 5-46 | 250 | 30 | 250 | 25 | 1 | 0 | 500 |
| Example 5-47 | 250 | 30 | 250 | 25 | 1 | 0 | 500 |
| Example 5-48 | 250 | 30 | 250 | 25 | 1 | 0 | 500 |
| Example 5-49 | 250 | 30 | 250 | 25 | 1 | 0 | 500 |
| Example 5-50 | 250 | 30 | 250 | 25 | 1 | 0 | 500 |

**[Table 5-19]**

| | Addition-elimination reaction step - Reaction conditions | | | | | | |
|---|---|---|---|---|---|---|---|
| | Reaction temperature | Absolute pressure | Condenser tube 1 temperature | Condenser tube 2 temperature | Reaction time | Oxygen concentration | BTM |
| | °C | mmHg | °C | °C | hours | vol% | parts by mass |
| Example 5-51 | 250 | 100 | 250 | 50 | 1 | 0 | 500 |
| Example 5-52 | 250 | 100 | 250 | 50 | 1 | 0 | 500 |
| Example 5-53 | 250 | 100 | 250 | 50 | 1 | 0 | 500 |
| Example 5-54 | 250 | 30 | 250 | 25 | 1 | 0 | 500 |
| Example 5-55 | 250 | 30 | 250 | 25 | 1 | 0 | 500 |
| Example 5-56 | 250 | 30 | 250 | 25 | 1 | 0 | 500 |
| Example 5-57 | 250 | 100 | 250 | 50 | 1 | 0 | 500 |
| Example 5-58 | 250 | 100 | 250 | 50 | 1 | 0 | 500 |
| Example 5-59 | 250 | 100 | 250 | 50 | 1 | 0 | 500 |
| Example 5-60 | 250 | 100 | 250 | 50 | 1 | 0 | 500 |
| Example 5-61 | 250 | 100 | 250 | 50 | 1 | 0 | 500 |
| Example 5-62 | 250 | 100 | 250 | 50 | 1 | 0 | 500 |
| Example 5-63 | 250 | 30 | 250 | 25 | 1 | 0 | 500 |
| Example 5-64 | 250 | 30 | 250 | 25 | 1 | 0 | 500 |
| Example 5-65 | 250 | 30 | 250 | 25 | 1 | 0 | 500 |
| Example 5-66 | 250 | 30 | 250 | 25 | 1 | 0 | 500 |
| Example 5-67 | 250 | 30 | 250 | 25 | 1 | 0 | 500 |
| Example 5-68 | 250 | 30 | 250 | 25 | 1 | 0 | 500 |
| Example 5-69 | 250 | 100 | 250 | 50 | 1 | 0 | 500 |
| Example 5-70 | 250 | 100 | 250 | 50 | 1 | 0 | 500 |
| Example 5-71 | 250 | 30 | 250 | 25 | 1 | 0 | 500 |
| Example 5-72 | 250 | 30 | 250 | 25 | 1 | 0 | 500 |

**[Table 5-20]**

| | Addition-elimination reaction step - Reaction conditions | | | | | | |
|---|---|---|---|---|---|---|---|
| | Reaction temperature | Absolute pressure | Condenser tube 1 temperature | Condenser tube 2 temperature | Reaction time | Oxygen concentration | BTM |
| | °C | mmHg | °C | °C | hours | vol% | parts by mass |
| Comparative Example 5-1 | 250 | 100 | 250 | 50 | 1 | 0 | 500 |
| Comparative Example 5-2 | 250 | 100 | 250 | 50 | 1 | 0 | 500 |
| Comparative Example 5-3 | 250 | 100 | 250 | 50 | 1 | 0 | 500 |
| Comparative Example 5-4 | 250 | 400 | 250 | 50 | 1 | 0 | 500 |
| Comparative Example 5-5 | 250 | 430 | 250 | 50 | 1 | 0 | 500 |
| Comparative Example 5-6 | 250 | 400 | 250 | 50 | 1 | 0 | 500 |
| Comparative Example 5-7 | 250 | 430 | 250 | 50 | 1 | 0 | 500 |
| Comparative Example 5-8 | 250 | 100 | 250 | 50 | 1 | 0 | 500 |
| Comparative Example 5-9 | 250 | 100 | 250 | 50 | 1 | 0 | 500 |
| Comparative Example 5-10 | 250 | 100 | 250 | 50 | 1 | 0 | 500 |
| Comparative Example 5-11 | 250 | 30 | 250 | 25 | 1 | 0 | 500 |
| Comparative Example 5-12 | 250 | 30 | 250 | 25 | 1 | 0 | 500 |
| Comparative Example 5-13 | 250 | 30 | 250 | 25 | 1 | 0 | 500 |
| Comparative Example 5-14 | 250 | 30 | 250 | 25 | 1 | 0 | 500 |
| Comparative Example 5-15 | 250 | 30 | 250 | 25 | 1 | 0 | 500 |
| Comparative Example 5-16 | 250 | 30 | 250 | 25 | 1 | 0 | 500 |
| Comparative Example 5-17 | 250 | 30 | 250 | 25 | 1 | 0 | 500 |
| Comparative Example 5-18 | 250 | 30 | 250 | 25 | 1 | 0 | 500 |
| Comparative Example 5-19 | 250 | 30 | 250 | 25 | 1 | 0 | 500 |
| Comparative Example 5-20 | 250 | 100 | 250 | 50 | 1 | 0 | 500 |
| Comparative Example 5-21 | 250 | 100 | 250 | 50 | 1 | 0 | 500 |
| Comparative Example 5-22 | 250 | 100 | 250 | 50 | 1 | 0 | 500 |
| Comparative Example 5-23 | 250 | 30 | 250 | 25 | 1 | 0 | 500 |
| Comparative Example 5-24 | 250 | 30 | 250 | 25 | 1 | 0 | 500 |
| Comparative Example 5-25 | 250 | 30 | 250 | 25 | 1 | 0 | 500 |

**[Table 5-21]**

| | Addition-elimination reaction step - Evaluation results | | | | | | | |
|---|---|---|---|---|---|---|---|---|
| | Amino groups | NCO -BL(A) | NCO -BL(C) | Compound (I) | Ureido groups | MB | Hazen color number | TOP |
| | mol% | mol% | mol% | mol% | mol% | mol% | APHA | parts by mass |
| Example 5-1 | 0 | 0 | 96 | 0 | 0 | 96 | 3 | 500 |
| Example 5-2 | 0 | 0 | 96 | 0 | 0 | 96 | 3 | 500 |
| Example 5-3 | 0 | 0 | 95 | 0 | 0 | 95 | 3 | 500 |
| Example 5-4 | 0 | 0 | 95 | 0 | 0 | 95 | 3 | 500 |
| Example 5-5 | 0 | 0 | 90 | 0 | 0 | 90 | 4 | 500 |
| Example 5-6 | 0 | 0 | 90 | 0 | 0 | 90 | 4 | 500 |
| Example 5-7 | 0 | 0 | 89 | 0 | 0 | 89 | 4 | 500 |
| Example 5-8 | 0 | 0 | 90 | 0 | 0 | 90 | 4 | 500 |
| Example 5-9 | 0 | 0 | 96 | 0 | 0 | 96 | 3 | 500 |
| Example 5-10 | 0 | 0 | 81 | 0 | 0 | 81 | 3 | 500 |
| Example 5-11 | 0 | 0 | 90 | 0 | 0 | 90 | 4 | 500 |
| Example 5-12 | 0 | 0 | 72 | 0 | 0 | 72 | 5 | 500 |
| Example 5-13 | 0 | 0 | 91 | 0 | 0 | 91 | 5 | 500 |
| Example 5-14 | 0 | 0 | 55 | 0 | 0 | 55 | 6 | 500 |
| Example 5-15 | 0 | 0 | 96 | 0 | 0 | 96 | 3 | 500 |
| Example 5-16 | 0 | 0 | 90 | 0 | 0 | 90 | 4 | 500 |
| Example 5-17 | 0 | 0 | 71 | 0 | 0 | 71 | 5 | 500 |
| Example 5-18 | 0 | 0 | 95 | 0 | 0 | 95 | 3 | 500 |
| Example 5-19 | 0 | 0 | 90 | 0 | 0 | 90 | 4 | 500 |
| Example 5-20 | 0 | 0 | 71 | 0 | 0 | 71 | 5 | 500 |
| Example 5-21 | 0 | 0 | 95 | 0 | 0 | 95 | 3 | 500 |
| Example 5-22 | 0 | 0 | 91 | 0 | 0 | 91 | 4 | 500 |
| Example 5-23 | 0 | 0 | 71 | 0 | 0 | 71 | 5 | 500 |
| Example 5-24 | 0 | 0 | 96 | 0 | 0 | 96 | 3 | 500 |
| Example 5-25 | 0 | 0 | 91 | 0 | 0 | 91 | 4 | 500 |

**[Table 5-22]**

| | Addition-elimination reaction step - Evaluation results | | | | | | | |
|---|---|---|---|---|---|---|---|---|
| | Amino groups | NCO -BL(A) | NCO -BL(C) | Compound (I) | Ureido groups | MB | Hazen color number | TOP |
| | mol% | mol% | mol% | mol% | mol% | mol% | APHA | parts by mass |
| Example 5-26 | 0 | 0 | 71 | 0 | 0 | 71 | 5 | 500 |
| Example 5-27 | 0 | 0 | 95 | 0 | 0 | 95 | 4 | 500 |
| Example 5-28 | 0 | 0 | 90 | 0 | 0 | 90 | 5 | 500 |
| Example 5-29 | 0 | 0 | 68 | 0 | 0 | 68 | 6 | 500 |
| Example 5-30 | 0 | 0 | 96 | 0 | 0 | 96 | 3 | 500 |
| Example 5-31 | 0 | 0 | 91 | 0 | 0 | 91 | 4 | 500 |
| Example 5-32 | 0 | 0 | 68 | 0 | 0 | 68 | 5 | 500 |
| Example 5-33 | 0 | 0 | 89 | 0 | 0 | 89 | 4 | 500 |
| Example 5-34 | 0 | 0 | 75 | 0 | 0 | 75 | 4 | 500 |
| Example 5-35 | 0 | 0 | 84 | 0 | 0 | 84 | 5 | 500 |
| Example 5-36 | 0 | 0 | 65 | 0 | 0 | 65 | 6 | 500 |
| Example 5-37 | 0 | 0 | 83 | 0 | 0 | 83 | 6 | 500 |
| Example 5-38 | 0 | 0 | 49 | 0 | 0 | 49 | 7 | 500 |
| Example 5-39 | 0 | 0 | 89 | 0 | 0 | 89 | 4 | 500 |
| Example 5-40 | 0 | 0 | 84 | 0 | 0 | 84 | 5 | 500 |
| Example 5-41 | 0 | 0 | 64 | 0 | 0 | 64 | 6 | 500 |
| Example 5-42 | 0 | 0 | 88 | 0 | 0 | 88 | 4 | 500 |
| Example 5-43 | 0 | 0 | 82 | 0 | 0 | 82 | 5 | 500 |
| Example 5-44 | 0 | 0 | 64 | 0 | 0 | 64 | 6 | 500 |
| Example 5-45 | 0 | 0 | 90 | 0 | 0 | 90 | 4 | 500 |
| Example 5-46 | 0 | 0 | 84 | 0 | 0 | 84 | 5 | 500 |
| Example 5-47 | 0 | 0 | 65 | 0 | 0 | 65 | 6 | 500 |
| Example 5-48 | 0 | 0 | 90 | 0 | 0 | 90 | 4 | 500 |
| Example 5-49 | 0 | 0 | 84 | 0 | 0 | 84 | 5 | 500 |
| Example 5-50 | 0 | 0 | 64 | 0 | 0 | 64 | 6 | 500 |

**[Table 5-23]**

| | Addition-elimination reaction step - Evaluation results | | | | | | | |
|---|---|---|---|---|---|---|---|---|
| | Amino groups | NCO -BL(A) | NCO -BL(C) | Compound (I) | Ureido groups | MB | Hazen color number | TOP |
| | mol% | mol% | mol% | mol% | mol% | mol% | APHA | parts by mass |
| Example 5-51 | 0 | 0 | 89 | 0 | 0 | 89 | 5 | 500 |
| Example 5-52 | 0 | 0 | 85 | 0 | 0 | 85 | 6 | 500 |
| Example 5-53 | 0 | 0 | 63 | 0 | 0 | 63 | 7 | 500 |
| Example 5-54 | 0 | 0 | 90 | 0 | 0 | 90 | 4 | 500 |
| Example 5-55 | 0 | 0 | 84 | 0 | 0 | 84 | 5 | 500 |
| Example 5-56 | 0 | 0 | 64 | 0 | 0 | 64 | 6 | 500 |
| Example 5-57 | 0 | 0 | 97 | 0 | 0 | 97 | 1 | 1500 |
| Example 5-58 | 0 | 0 | 96 | 0 | 0 | 96 | 1 | 1500 |
| Example 5-59 | 0 | 0 | 91 | 0 | 0 | 91 | 1 | 1500 |
| Example 5-60 | 0 | 0 | 89 | 0 | 0 | 89 | 1 | 1500 |
| Example 5-61 | 0 | 0 | 72 | 0 | 0 | 72 | 2 | 1500 |
| Example 5-62 | 0 | 0 | 71 | 0 | 0 | 71 | 2 | 1500 |
| Example 5-63 | 0 | 0 | 96 | 0 | 0 | 96 | 1 | 1500 |
| Example 5-64 | 0 | 0 | 97 | 0 | 0 | 97 | 1 | 1500 |
| Example 5-65 | 0 | 0 | 95 | 0 | 0 | 95 | 1 | 1500 |
| Example 5-66 | 0 | 0 | 97 | 0 | 0 | 97 | 1 | 1500 |
| Example 5-67 | 0 | 0 | 96 | 0 | 0 | 96 | 1 | 1500 |
| Example 5-68 | 0 | 0 | 96 | 0 | 0 | 96 | 1 | 1500 |
| Example 5-69 | 0 | 0 | 96 | 0 | 0 | 96 | 1 | 1500 |
| Example 5-70 | 0 | 0 | 95 | 0 | 0 | 95 | 1 | 1500 |
| Example 5-71 | 0 | 0 | 96 | 0 | 0 | 96 | 1 | 1500 |
| Example 5-72 | 0 | 0 | 96 | 0 | 0 | 96 | 1 | 1500 |

**[Table 5-24]**

| | Addition-elimination reaction step - Evaluation results | | | | | | | |
|---|---|---|---|---|---|---|---|---|
| | Amino groups | NCO -BL(A) | NCO -BL(C) | Compound (I) | Ureido groups | MB | Hazen color number | TOP |
| | mol% | mol% | mol% | mol% | mol% | mol% | APHA | parts by mass |
| Comparative Example 5-1 | 0 | 0 | 86 | 0 | 0 | 86 | 8 | 490 |
| Comparative Example 5-2 | 0 | 0 | 86 | 0 | 0 | 86 | 9 | 490 |
| Comparative Example 5-3 | 0 | 0 | 66 | 0 | 0 | 66 | 10 | 490 |
| Comparative Example 5-4 | 0 | 0 | 81 | 0 | 0 | 81 | 10 | 490 |
| Comparative Example 5-5 | 0 | 0 | 57 | 0 | 0 | 57 | 11 | 490 |
| Comparative Example 5-6 | 0 | 0 | 86 | 0 | 0 | 86 | 8 | 490 |
| Comparative Example 5-7 | 0 | 0 | 74 | 0 | 0 | 74 | 8 | 490 |
| Comparative Example 5-8 | 0 | 0 | 86 | 0 | 0 | 86 | 8 | 490 |
| Comparative Example 5-9 | 0 | 0 | 81 | 0 | 0 | 81 | 9 | 490 |
| Comparative Example 5-10 | 0 | 0 | 71 | 0 | 0 | 71 | 10 | 490 |
| Comparative Example 5-11 | 0 | 0 | 86 | 0 | 0 | 86 | 8 | 490 |
| Comparative Example 5-12 | 0 | 0 | 86 | 0 | 0 | 86 | 9 | 490 |
| Comparative Example 5-13 | 0 | 0 | 67 | 0 | 0 | 67 | 10 | 490 |
| Comparative Example 5-14 | 0 | 0 | 86 | 0 | 0 | 86 | 8 | 490 |
| Comparative Example 5-15 | 0 | 0 | 86 | 0 | 0 | 86 | 9 | 490 |
| Comparative Example 5-16 | 0 | 0 | 67 | 0 | 0 | 67 | 10 | 490 |
| Comparative Example 5-17 | 0 | 0 | 86 | 0 | 0 | 86 | 8 | 490 |
| Comparative Example 5-18 | 0 | 0 | 86 | 0 | 0 | 86 | 9 | 490 |
| Comparative Example 5-19 | 0 | 0 | 67 | 0 | 0 | 67 | 10 | 490 |
| Comparative Example 5-20 | 0 | 0 | 86 | 0 | 0 | 86 | 9 | 490 |
| Comparative Example 5-21 | 0 | 0 | 86 | 0 | 0 | 86 | 10 | 490 |
| Comparative Example 5-22 | 0 | 0 | 64 | 0 | 0 | 64 | 11 | 490 |
| Comparative Example 5-23 | 0 | 0 | 86 | 0 | 0 | 86 | 8 | 490 |
| Comparative Example 5-24 | 0 | 0 | 86 | 0 | 0 | 86 | 9 | 490 |
| Comparative Example 5-25 | 0 | 0 | 63 | 0 | 0 | 63 | 10 | 490 |

**[Table 5-25]**

| | Second reaction step - Raw materials | | | | |
|---|---|---|---|---|---|
| | BTM following addition-elimination step | Secondary amine compound (I) | | Tertiary amine compound (II) | |
| | parts by mass | type | parts by mass | type | parts by mass |
| Example 5-1 | 495 | NMA | 5 | - | - |
| Example 5-2 | 495 | NEA | 5 | - | - |
| Example 5-3 | 495 | NBA | 5 | - | - |
| Example 5-4 | 495 | DPA | 5 | - | - |
| Example 5-5 | 495 | - | - | NNDMA | 5 |
| Example 5-6 | 495 | - | - | NNDEA | 5 |
| Example 5-7 | 495 | - | - | NMDPA | 5 |
| Example 5-8 | 495 | - | - | TPA | 5 |
| Example 5-9 | 495 | NEA | 5 | - | - |
| Example 5-10 | 495 | NEA | 5 | - | - |
| Example 5-11 | 495 | NEA | 5 | - | - |
| Example 5-12 | 495 | NEA | 5 | - | - |
| Example 5-13 | 495 | NEA | 5 | - | - |
| Example 5-14 | 495 | NEA | 5 | - | - |
| Example 5-15 | 495 | NEA | 5 | - | - |
| Example 5-16 | 495 | NEA | 5 | - | - |
| Example 5-17 | 495 | NEA | 5 | - | - |
| Example 5-18 | 495 | NEA | 5 | - | - |
| Example 5-19 | 495 | NEA | 5 | - | - |
| Example 5-20 | 495 | NEA | 5 | - | - |
| Example 5-21 | 495 | NEA | 5 | - | - |
| Example 5-22 | 495 | NEA | 5 | - | - |
| Example 5-23 | 495 | NEA | 5 | - | - |
| Example 5-24 | 495 | NEA | 5 | - | - |
| Example 5-25 | 495 | NEA | 5 | - | - |

**[Table 5-26]**

| | Second reaction step - Raw materials | | | | |
|---|---|---|---|---|---|
| | BTM following addition-elimination step | Secondary amine compound (I) | | Tertiary amine compound (II) | |
| | parts by mass | type | parts by mass | type | parts by mass |
| Example 5-26 | 495 | NEA | 5 | - | - |
| Example 5-27 | 495 | NEA | 5 | - | - |
| Example 5-28 | 495 | NEA | 5 | - | - |
| Example 5-29 | 495 | NEA | 5 | - | - |
| Example 5-30 | 495 | NEA | 5 | - | - |
| Example 5-31 | 495 | NEA | 5 | - | - |
| Example 5-32 | 495 | NEA | 5 | - | - |
| Example 5-33 | 495 | - | - | NNDEA | 5 |
| Example 5-34 | 495 | - | - | NNDEA | 5 |
| Example 5-35 | 495 | - | - | NNDEA | 5 |
| Example 5-36 | 495 | - | - | NNDEA | 5 |
| Example 5-37 | 495 | - | - | NNDEA | 5 |
| Example 5-38 | 495 | - | - | NNDEA | 5 |
| Example 5-39 | 495 | - | - | NNDEA | 5 |
| Example 5-40 | 495 | - | - | NNDEA | 5 |
| Example 5-41 | 495 | - | - | NNDEA | 5 |
| Example 5-42 | 495 | - | - | NNDMA | 5 |
| Example 5-43 | 495 | - | - | NNDEA | 5 |
| Example 5-44 | 495 | - | - | NNDEA | 5 |
| Example 5-45 | 495 | - | - | NNDEA | 5 |
| Example 5-46 | 495 | - | - | NNDEA | 5 |
| Example 5-47 | 495 | - | - | NNDEA | 5 |
| Example 5-48 | 495 | - | - | NNDEA | 5 |
| Example 5-49 | 495 | - | - | NNDEA | 5 |
| Example 5-50 | 495 | - | - | NNDEA | 5 |

**[Table 5-27]**

| | Second reaction step - Raw materials | | | | |
|---|---|---|---|---|---|
| | BTM following addition-elimination step | Secondary amine compound (I) | | Tertiary amine compound (II) | |
| | parts by mass | type | parts by mass | type | parts by mass |
| Example 5-51 | 495 | - | - | NNDEA | 5 |
| Example 5-52 | 495 | - | - | NNDEA | 5 |
| Example 5-53 | 495 | - | - | NNDEA | 5 |
| Example 5-54 | 495 | - | - | NNDEA | 5 |
| Example 5-55 | 495 | - | - | NNDEA | 5 |
| Example 5-56 | 495 | - | - | NNDEA | 5 |
| Example 5-57 | 495 | NMA | 500 | NNDMA | 5 |
| Example 5-58 | 495 | NMA | 5 | NNDMA | 500 |
| Example 5-59 | 495 | NMA | 500 | NNDMA | 5 |
| Example 5-60 | 495 | NMA | 5 | NNDMA | 500 |
| Example 5-61 | 495 | NMA | 500 | NNDMA | 5 |
| Example 5-62 | 495 | NMA | 5 | NNDMA | 500 |
| Example 5-63 | 495 | NMA | 500 | NNDMA | 5 |
| Example 5-64 | 495 | NMA | 5 | NNDMA | 500 |
| Example 5-65 | 495 | NMA | 500 | NNDMA | 5 |
| Example 5-66 | 495 | NMA | 5 | NNDMA | 500 |
| Example 5-67 | 495 | NMA | 500 | NNDMA | 5 |
| Example 5-68 | 495 | NMA | 5 | NNDMA | 500 |
| Example 5-69 | 495 | NMA | 500 | NNDMA | 5 |
| Example 5-70 | 495 | NMA | 5 | NNDMA | 500 |
| Example 5-71 | 495 | NMA | 500 | NNDMA | 5 |
| Example 5-72 | 495 | NMA | 5 | NNDMA | 500 |

**[Table 5-28]**

| | Second reaction step - Raw materials | | | | |
|---|---|---|---|---|---|
| | BTM following addition-elimination step | Secondary amine compound (I) | | Tertiary amine compound (II) | |
| | parts by mass | type | parts by mass | type | parts by mass |
| Comparative Example 5-1 | 495 | - | - | - | - |
| Comparative Example 5-2 | 495 | - | - | - | - |
| Comparative Example 5-3 | 495 | - | - | - | - |
| Comparative Example 5-4 | 495 | - | - | - | - |
| Comparative Example 5-5 | 495 | - | - | - | - |
| Comparative Example 5-6 | 495 | - | - | - | - |
| Comparative Example 5-7 | 495 | - | - | - | - |
| Comparative Example 5-8 | 495 | - | - | - | - |
| Comparative Example 5-9 | 495 | - | - | - | - |
| Comparative Example 5-10 | 495 | - | - | - | - |
| Comparative Example 5-11 | 495 | - | - | - | - |
| Comparative Example 5-12 | 495 | - | - | - | - |
| Comparative Example 5-13 | 495 | - | - | - | - |
| Comparative Example 5-14 | 495 | - | - | - | - |
| Comparative Example 5-15 | 495 | - | - | - | - |
| Comparative Example 5-16 | 495 | - | - | - | - |
| Comparative Example 5-17 | 495 | - | - | - | - |
| Comparative Example 5-18 | 495 | - | - | - | - |
| Comparative Example 5-19 | 495 | - | - | - | - |
| Comparative Example 5-20 | 495 | - | - | - | - |
| Comparative Example 5-21 | 495 | - | - | - | - |
| Comparative Example 5-22 | 495 | - | - | - | - |
| Comparative Example 5-23 | 495 | - | - | - | - |
| Comparative Example 5-24 | 495 | - | - | - | - |
| Comparative Example 5-25 | 495 | - | - | - | - |

**[Table 5-29]**

| | Second reaction step - Reaction conditions | | | | | |
|---|---|---|---|---|---|---|
| | Feed rate | Feed time | Thin-film apparatus temperature | Reduced pressure | Oxygen concentration | Distillation column 2 BTM |
| | parts by mass per hour | h | °C | mmHg | vol% | parts by mass |
| Example 5-1 | 1000 | 7 | 250 | 100 | 0 | 70.0 |
| Example 5-2 | 1000 | 7 | 250 | 100 | 0 | 71.5 |
| Example 5-3 | 1000 | 7 | 250 | 100 | 0 | 69.0 |
| Example 5-4 | 1000 | 7 | 250 | 100 | 0 | 68.1 |
| Example 5-5 | 1000 | 7 | 250 | 100 | 0 | 54.3 |
| Example 5-6 | 1000 | 7 | 250 | 100 | 0 | 53.5 |
| Example 5-7 | 1000 | 7 | 250 | 100 | 0 | 52.8 |
| Example 5-8 | 1000 | 7 | 250 | 100 | 0 | 53.5 |
| Example 5-9 | 1000 | 7 | 250 | 100 | 0 | 54.5 |
| Example 5-10 | 1000 | 7 | 250 | 100 | 0 | 28.1 |
| Example 5-11 | 1000 | 7 | 250 | 100 | 0 | 70.5 |
| Example 5-12 | 1000 | 7 | 250 | 100 | 0 | 36.5 |
| Example 5-13 | 1000 | 7 | 250 | 100 | 0 | 54.5 |
| Example 5-14 | 1000 | 7 | 250 | 100 | 0 | 11.7 |
| Example 5-15 | 1000 | 7 | 250 | 100 | 0 | 68.0 |
| Example 5-16 | 1000 | 7 | 250 | 100 | 0 | 67.6 |
| Example 5-17 | 1000 | 7 | 250 | 100 | 0 | 34.1 |
| Example 5-18 | 1000 | 7 | 250 | 8 | 0 | 46.8 |
| Example 5-19 | 1000 | 7 | 250 | 8 | 0 | 43.9 |
| Example 5-20 | 1000 | 7 | 250 | 8 | 0 | 17.8 |
| Example 5-21 | 1000 | 7 | 250 | 100 | 0 | 81.1 |
| Example 5-22 | 1000 | 7 | 250 | 100 | 0 | 80.4 |
| Example 5-23 | 1000 | 7 | 250 | 100 | 0 | 41.2 |
| Example 5-24 | 1000 | 7 | 250 | 8 | 0 | 87.2 |
| Example 5-25 | 1000 | 7 | 250 | 8 | 0 | 83.7 |

**[Table 5-30]**

| | Second reaction step - Reaction conditions | | | | | |
|---|---|---|---|---|---|---|
| | Feed rate | Feed time | Thin-film apparatus temperature | Reduced pressure | Oxygen concentration | Distillation column 2 BTM |
| | parts by mass per hour | h | °C | mmHg | vol% | parts by mass |
| Example 5-26 | 1000 | 7 | 250 | 8 | 0 | 45.6 |
| Example 5-27 | 1000 | 7 | 250 | 100 | 0 | 70.6 |
| Example 5-28 | 1000 | 7 | 250 | 100 | 0 | 68.0 |
| Example 5-29 | 1000 | 7 | 250 | 100 | 0 | 34.2 |
| Example 5-30 | 1000 | 7 | 250 | 30 | 0 | 88.5 |
| Example 5-31 | 1000 | 7 | 250 | 30 | 0 | 85.6 |
| Example 5-32 | 1000 | 7 | 250 | 30 | 0 | 42.8 |
| Example 5-33 | 1000 | 7 | 250 | 100 | 0 | 40.9 |
| Example 5-34 | 1000 | 7 | 250 | 100 | 0 | 20.8 |
| Example 5-35 | 1000 | 7 | 250 | 100 | 0 | 51.1 |
| Example 5-36 | 1000 | 7 | 250 | 100 | 0 | 31.2 |
| Example 5-37 | 1000 | 7 | 250 | 100 | 0 | 40.7 |
| Example 5-38 | 1000 | 7 | 250 | 100 | 0 | 9.6 |
| Example 5-39 | 1000 | 7 | 250 | 100 | 0 | 50.7 |
| Example 5-40 | 1000 | 7 | 250 | 100 | 0 | 49.5 |
| Example 5-41 | 1000 | 7 | 250 | 100 | 0 | 27.4 |
| Example 5-42 | 1000 | 7 | 250 | 8 | 0 | 45.4 |
| Example 5-43 | 1000 | 7 | 250 | 8 | 0 | 41.6 |
| Example 5-44 | 1000 | 7 | 250 | 8 | 0 | 18.4 |
| Example 5-45 | 1000 | 7 | 250 | 100 | 0 | 63.1 |
| Example 5-46 | 1000 | 7 | 250 | 100 | 0 | 59.6 |
| Example 5-47 | 1000 | 7 | 250 | 100 | 0 | 35.6 |
| Example 5-48 | 1000 | 7 | 250 | 8 | 0 | 69.6 |
| Example 5-49 | 1000 | 7 | 250 | 8 | 0 | 63.7 |
| Example 5-50 | 1000 | 7 | 250 | 8 | 0 | 36.6 |

**[Table 5-31]**

| | Second reaction step - Reaction conditions | | | | | |
|---|---|---|---|---|---|---|
| | Feed rate | Feed time | Thin-film apparatus temperature | Reduced pressure | Oxygen concentration | Distillation column 2 BTM |
| | parts by mass per hour | h | °C | mmHg | vol% | parts by mass |
| Example 5-51 | 1000 | 7 | 250 | 100 | 0 | 54.2 |
| Example 5-52 | 1000 | 7 | 250 | 100 | 0 | 54.6 |
| Example 5-53 | 1000 | 7 | 250 | 100 | 0 | 29.6 |
| Example 5-54 | 1000 | 7 | 250 | 30 | 0 | 67.2 |
| Example 5-55 | 1000 | 7 | 250 | 30 | 0 | 63.4 |
| Example 5-56 | 1000 | 7 | 250 | 30 | 0 | 37.0 |
| Example 5-57 | 1000 | 7 | 250 | 100 | 0 | 69.1 |
| Example 5-58 | 1000 | 7 | 250 | 100 | 0 | 69.7 |
| Example 5-59 | 1000 | 7 | 250 | 100 | 0 | 61.3 |
| Example 5-60 | 1000 | 7 | 250 | 100 | 0 | 67.9 |
| Example 5-61 | 1000 | 7 | 250 | 100 | 0 | 40.6 |
| Example 5-62 | 1000 | 7 | 250 | 100 | 0 | 36.0 |
| Example 5-63 | 1000 | 7 | 250 | 8 | 0 | 45.4 |
| Example 5-64 | 1000 | 7 | 250 | 8 | 0 | 52.2 |
| Example 5-65 | 1000 | 7 | 250 | 100 | 0 | 78.1 |
| Example 5-66 | 1000 | 7 | 250 | 100 | 0 | 85.4 |
| Example 5-67 | 1000 | 7 | 250 | 8 | 0 | 86.5 |
| Example 5-68 | 1000 | 7 | 250 | 8 | 0 | 90.2 |
| Example 5-69 | 1000 | 7 | 250 | 100 | 0 | 68.9 |
| Example 5-70 | 1000 | 7 | 250 | 100 | 0 | 69.4 |
| Example 5-71 | 1000 | 7 | 250 | 30 | 0 | 82.5 |
| Example 5-72 | 1000 | 7 | 250 | 30 | 0 | 88.6 |

**[Table 5-32]**

| | Second reaction step - Reaction conditions | | | | | |
|---|---|---|---|---|---|---|
| | Feed rate | Feed time | Thin-film apparatus temperature | Reduced pressure | Oxygen concentration | Distillation column 2 BTM |
| | parts by mass per hour | h | °C | mmHg | vol% | parts by mass |
| Comparative Example 5-1 | 1000 | 7 | 250 | 100 | 0 | 49.8 |
| Comparative Example 5-2 | 1000 | 7 | 250 | 100 | 0 | 55.1 |
| Comparative Example 5-3 | 1000 | 7 | 250 | 100 | 0 | 27.5 |
| Comparative Example 5-4 | 1000 | 7 | 250 | 100 | 0 | 38.1 |
| Comparative Example 5-5 | 1000 | 7 | 250 | 100 | 0 | 10.9 |
| Comparative Example 5-6 | 1000 | 7 | 250 | 100 | 0 | 38.7 |
| Comparative Example 5-7 | 1000 | 7 | 250 | 100 | 0 | 20.8 |
| Comparative Example 5-8 | 1000 | 7 | 250 | 100 | 0 | 47.2 |
| Comparative Example 5-9 | 1000 | 7 | 250 | 100 | 0 | 44.7 |
| Comparative Example 5-10 | 1000 | 7 | 250 | 100 | 0 | 34.1 |
| Comparative Example 5-11 | 1000 | 7 | 250 | 8 | 0 | 37.4 |
| Comparative Example 5-12 | 1000 | 7 | 250 | 8 | 0 | 41.4 |
| Comparative Example 5-13 | 1000 | 7 | 250 | 8 | 0 | 19.5 |
| Comparative Example 5-14 | 1000 | 7 | 250 | 100 | 0 | 57.8 |
| Comparative Example 5-15 | 1000 | 7 | 250 | 100 | 0 | 64.5 |
| Comparative Example 5-16 | 1000 | 7 | 250 | 100 | 0 | 36.6 |
| Comparative Example 5-17 | 1000 | 7 | 250 | 8 | 0 | 62.2 |
| Comparative Example 5-18 | 1000 | 7 | 250 | 8 | 0 | 69.0 |
| Comparative Example 5-19 | 1000 | 7 | 250 | 8 | 0 | 39.9 |
| Comparative Example 5-20 | 1000 | 7 | 250 | 100 | 0 | 49.6 |
| Comparative Example 5-21 | 1000 | 7 | 250 | 100 | 0 | 56.3 |
| Comparative Example 5-22 | 1000 | 7 | 250 | 100 | 0 | 30.4 |
| Comparative Example 5-23 | 1000 | 7 | 250 | 30 | 0 | 61.8 |
| Comparative Example 5-24 | 1000 | 7 | 250 | 30 | 0 | 67.3 |
| Comparative Example 5-25 | 1000 | 7 | 250 | 30 | 0 | 35.1 |

**[Table 5-33]**

| | Second reaction step - Evaluation results | | |
|---|---|---|---|
| | Isocyanate content | Isocyanate yield | Hazen color number |
| | wt% | mol% | APHA |
| Example 5-1 | 99 | 82 | 2 |
| Example 5-2 | 99 | 84 | 2 |
| Example 5-3 | 99 | 81 | 2 |
| Example 5-4 | 99 | 80 | 2 |
| Example 5-5 | 99 | 73 | 3 |
| Example 5-6 | 99 | 72 | 3 |
| Example 5-7 | 99 | 71 | 3 |
| Example 5-8 | 99 | 72 | 3 |
| Example 5-9 | 99 | 64 | 2 |
| Example 5-10 | 99 | 33 | 2 |
| Example 5-11 | 99 | 75 | 3 |
| Example 5-12 | 99 | 47 | 4 |
| Example 5-13 | 99 | 58 | 4 |
| Example 5-14 | 99 | 15 | 5 |
| Example 5-15 | 99 | 84 | 2 |
| Example 5-16 | 99 | 75 | 3 |
| Example 5-17 | 99 | 46 | 4 |
| Example 5-18 | 99 | 55 | 2 |
| Example 5-19 | 99 | 47 | 3 |
| Example 5-20 | 99 | 23 | 4 |
| Example 5-21 | 99 | 82 | 2 |
| Example 5-22 | 99 | 75 | 3 |
| Example 5-23 | 99 | 45 | 4 |
| Example 5-24 | 99 | 82 | 2 |
| Example 5-25 | 99 | 73 | 3 |

**[Table 5-34]**

| | Second reaction step - Evaluation results | | |
|---|---|---|---|
| | Isocyanate content | Isocyanate yield | Hazen color number |
| | wt% | mol% | APHA |
| Example 5-26 | 99 | 46 | 4 |
| Example 5-27 | 99 | 81 | 3 |
| Example 5-28 | 99 | 71 | 4 |
| Example 5-29 | 99 | 42 | 5 |
| Example 5-30 | 99 | 85 | 2 |
| Example 5-31 | 99 | 76 | 3 |
| Example 5-32 | 99 | 43 | 4 |
| Example 5-33 | 99 | 55 | 3 |
| Example 5-34 | 99 | 28 | 3 |
| Example 5-35 | 99 | 63 | 4 |
| Example 5-36 | 99 | 39 | 5 |
| Example 5-37 | 99 | 50 | 5 |
| Example 5-38 | 99 | 12 | 6 |
| Example 5-39 | 99 | 72 | 3 |
| Example 5-40 | 99 | 64 | 4 |
| Example 5-41 | 99 | 36 | 5 |
| Example 5-42 | 99 | 61 | 3 |
| Example 5-43 | 99 | 51 | 4 |
| Example 5-44 | 99 | 23 | 5 |
| Example 5-45 | 99 | 72 | 3 |
| Example 5-46 | 99 | 63 | 4 |
| Example 5-47 | 99 | 38 | 5 |
| Example 5-48 | 99 | 73 | 3 |
| Example 5-49 | 99 | 62 | 4 |
| Example 5-50 | 99 | 36 | 5 |

**[Table 5-35]**

| | Second reaction step - Evaluation results | | |
|---|---|---|---|
| | Isocyanate content | Isocyanate yield | Hazen color number |
| | wt% | mol% | APHA |
| Example 5-51 | 99 | 71 | 4 |
| Example 5-52 | 99 | 66 | 5 |
| Example 5-53 | 99 | 36 | 6 |
| Example 5-54 | 99 | 72 | 3 |
| Example 5-55 | 99 | 63 | 4 |
| Example 5-56 | 99 | 37 | 5 |
| Example 5-57 | 99 | 85 | 1 |
| Example 5-58 | 99 | 81 | 1 |
| Example 5-59 | 99 | 74 | 1 |
| Example 5-60 | 99 | 71 | 1 |
| Example 5-61 | 99 | 48 | 2 |
| Example 5-62 | 99 | 46 | 2 |
| Example 5-63 | 99 | 56 | 1 |
| Example 5-64 | 99 | 61 | 1 |
| Example 5-65 | 99 | 82 | 1 |
| Example 5-66 | 99 | 86 | 1 |
| Example 5-67 | 99 | 84 | 1 |
| Example 5-68 | 99 | 84 | 1 |
| Example 5-69 | 99 | 83 | 1 |
| Example 5-70 | 99 | 79 | 1 |
| Example 5-71 | 99 | 82 | 1 |
| Example 5-72 | 99 | 84 | 1 |

**[Table 5-36]**

| | Second reaction step - Evaluation results | | |
|---|---|---|---|
| | Isocyanate content | Isocyanate yield | Hazen color number |
| | wt% | mol% | APHA |
| Comparative Example 5-1 | 99 | 67 | 8 |
| Comparative Example 5-2 | 99 | 68 | 9 |
| Comparative Example 5-3 | 99 | 40 | 10 |
| Comparative Example 5-4 | 99 | 47 | 5 |
| Comparative Example 5-5 | 99 | 16 | 6 |
| Comparative Example 5-6 | 99 | 52 | 8 |
| Comparative Example 5-7 | 99 | 28 | 8 |
| Comparative Example 5-8 | 99 | 67 | 3 |
| Comparative Example 5-9 | 99 | 58 | 3 |
| Comparative Example 5-10 | 99 | 46 | 3 |
| Comparative Example 5-11 | 99 | 40 | 10 |
| Comparative Example 5-12 | 99 | 41 | 8 |
| Comparative Example 5-13 | 99 | 20 | 9 |
| Comparative Example 5-14 | 99 | 66 | 8 |
| Comparative Example 5-15 | 99 | 68 | 8 |
| Comparative Example 5-16 | 99 | 40 | 8 |
| Comparative Example 5-17 | 99 | 65 | 8 |
| Comparative Example 5-18 | 99 | 67 | 8 |
| Comparative Example 5-19 | 99 | 40 | 8 |
| Comparative Example 5-20 | 99 | 65 | 9 |
| Comparative Example 5-21 | 99 | 68 | 9 |
| Comparative Example 5-22 | 99 | 37 | 9 |
| Comparative Example 5-23 | 99 | 66 | 9 |
| Comparative Example 5-24 | 99 | 67 | 9 |
| Comparative Example 5-25 | 99 | 35 | 9 |

As shown in the above tables, the isocyanate compositions each obtained by producing a blocked isocyanate composition from an amine, a carbonic acid derivative and a blocking agent in the presence of at least one added compound selected from the group consisting of the secondary amine compounds (I) and the tertiary amine compounds (II), subsequently producing a blocked isocyanate composition containing a second blocked isocyanate compound different from the first blocked isocyanate compound by conducting an addition-elimination reaction, and then thermally decomposing the obtained blocked isocyanate composition (Examples 5-1 to 5-72) exhibited the Hazen color numbers (APHA) evaluated as rank 6 or lower, which were favorable.

In contrast, the isocyanate compositions each obtained by producing a blocked isocyanate composition from an amine, a carbonic acid derivative and a blocking agent without the addition of either the secondary amine compound (I) or the tertiary amine compound (II), subsequently producing a blocked isocyanate composition containing a second blocked isocyanate compound different from the first blocked isocyanate compound by conducting an addition-elimination reaction, and then thermally decomposing the obtained blocked isocyanate composition (Comparative Examples 5-1 to 5-25) exhibited the Hazen color numbers (APHA) evaluated as rank 8 or higher, which were unfavorable.

The isocyanate compositions each obtained by producing a fist blocked isocyanate composition using a phenol-based, alcohol-based or aliphatic amine-based first blocking agent in the presence of the secondary amine compound (I), subsequently producing a second blocked isocyanate composition containing a second blocked isocyanate compound by conducting an addition-elimination reaction using a phenol-based, alcohol-based or aliphatic amine-based second blocking agent different from the first blocking agent in the presence of the secondary amine compound (I), and then conducting the second reaction step (Examples 5-2, and 5-9 to 5-32) were compared. The results revealed that the isocyanate compositions obtained under conditions in which at least one of the first blocking agent and the second blocking agent was a phenol-based blocking agent (Examples 5-2, 5-9 to 5-12, and 5-15 to 5-32) exhibited the Hazen color numbers evaluated as the lowest rank, which were particularly favorable.

Further, the isocyanate compositions each obtained by producing a blocked isocyanate composition using a phenol-based, alcohol-based or aliphatic amine-based first blocking agent in the presence of the tertiary amine compound (II), subsequently producing a blocked isocyanate composition containing a second blocked isocyanate compound by conducting an addition-elimination reaction using a phenol-based, alcohol-based or aliphatic amine-based second blocking agent different from the first blocking agent in the presence of the secondary amine compound (I), and then conducting the second reaction step (Examples 5-6, and 5-33 to 5-56) were compared. The results revealed that the isocyanate compositions obtained under conditions in which at least one of the first blocking agent and the second blocking agent was a phenol-based blocking agent (Examples 5-6, 5-33 to 5-36, and 5-39 to 5-56) exhibited the Hazen color numbers evaluated as the lowest rank, which were particularly favorable.

Furthermore, the isocyanate compositions each obtained by producing a first blocked isocyanate composition in the presence of both the secondary amine compound (I) and the tertiary amine compound (II), subsequently producing a second blocked isocyanate composition containing a second blocked isocyanate compound different from the first blocked isocyanate compound by conducting an addition-elimination reaction in the presence of both the secondary amine compound (I) and the tertiary amine compound (II), and then conducting the second reaction step (Examples 5-57 to 5-72) exhibited the Hazen color numbers evaluated as rank 1 or lower, which were the most favorable.

### [Industrial Applicability]

The method for producing a blocked isocyanate compound according to the present embodiment makes it possible to provide a method for producing a blocked isocyanate compound in which thermal denaturation and coloration are suppressed.

Further, the method for producing an isocyanate compound according to the present embodiment makes it possible to provide a method for producing an isocyanate compound in which thermal denaturation and coloration are suppressed.

## Claims

1. A method for producing a blocked isocyanate compound, the method comprising: a reaction step of reacting a primary amine compound, a carbonic acid derivative and a blocking agent by conducting a heat treatment in presence of at least one compound selected from the group consisting of secondary amine compounds of general formula (I) and tertiary amine compounds of general formula (II), thus obtaining a blocked isocyanate compound, 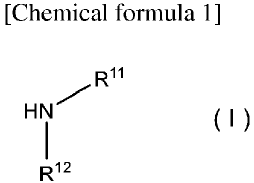 (wherein in general formula (I), R¹¹ and R¹² are each independently a monovalent organic group, R¹¹ and R¹² may be bonded together to form a cyclic structure via a carbon-carbon bond, a carbon-oxygen-carbon bond, or a carbon-nitrogen-carbon bond, and at least one of R¹¹ and R¹² has an aromatic group), 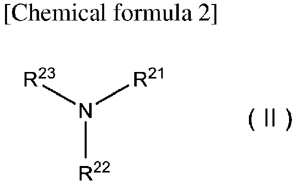 (wherein in general formula (II), R²¹, R²² and R²³ are each independently a monovalent organic group, each combination of R²¹ and R²², R²² and R²³, and R²³ and R²¹ may be independently bonded together to form a cyclic structure via a carbon-carbon bond, a carbon-oxygen-carbon bond, or a carbon-nitrogen-carbon bond, and at least one of R²¹, R²² and R²³ has an aromatic group).

2. The method for producing a blocked isocyanate compound according to Claim 1, wherein the blocking agent comprises at least one compound selected from the group consisting of hydroxy compounds, amine compounds and ammonia.

3. The method for producing a blocked isocyanate compound according to Claim 1 or 2, wherein the primary amine compound is an amine compound of general formula (III): 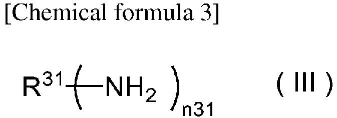 (wherein in general formula (III), R³¹ is an n31-valent organic group, and n31 is an integer of 1 or more but not more than 12).

4. The method for producing a blocked isocyanate compound according to Claim 1 or 2, wherein the blocking agent is an aromatic hydroxy compound of general formula (IV-1): 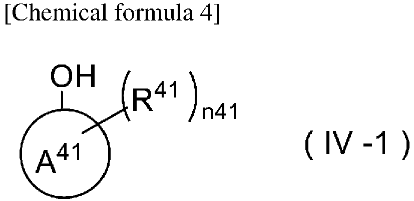 (wherein in general formula (IV-1), a ring A⁴¹ is an aromatic hydrocarbon ring of 6 or more but not more than 20 carbon atoms, R⁴¹ is a hydrogen atom, a halogen atom, a carboxy group, an alkyl group of 1 or more but not more than 20 carbon atoms, an alkoxy group of 1 or more but not more than 20 carbon atoms, an alkyloxycarbonyl group of 1 or more but not more than 20 carbon atoms, an alkylcarbonyloxy group of 1 or more but not more than 20 carbon atoms, an aryl group of 6 or more but not more than 20 carbon atoms, an aryloxy group of 6 or more but not more than 20 carbon atoms, an aralkyl group of 7 or more but not more than 20 carbon atoms, or an aralkyloxy group of 7 or more but not more than 20 carbon atoms, R⁴¹ may be bonded to the ring A⁴¹ to form a cyclic structure, and n41 is an integer of 1 or more but not more than 10).

5. The method for producing a blocked isocyanate compound according to Claim 1 or 2, wherein the blocking agent is an aliphatic hydroxy compound of general formula (IV-2):
[Chemical formula 5]
R⁴²-OH ( IV-2 )
(wherein in general formula (IV-2), R⁴² is a substituted or unsubstituted aliphatic hydrocarbon group of 1 or more but not more than 24 carbon atoms which may have an ether group, a carbonyl group or an ester group).

6. The method for producing a blocked isocyanate compound according to Claim 1 or 2, wherein the blocking agent is a secondary amine compound of general formula (V): 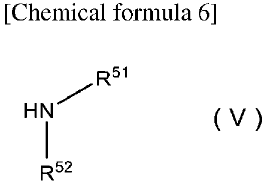 (wherein in general formula (V), R⁵¹ and R⁵² are each independently a monovalent organic group, and R⁵¹ and R⁵² may be bonded together to form a cyclic structure via a carbon-carbon bond, a carbon-oxygen-carbon bond, or a carbon-nitrogen-carbon bond).

7. The method for producing a blocked isocyanate compound according to Claim 1 or 2, wherein the carbonic acid derivative is a compound of general formula (VI): 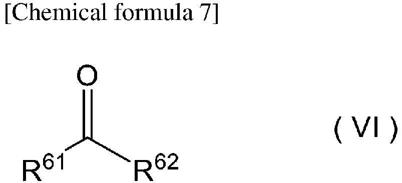 (wherein in general formula (VI), R⁶¹ and R⁶² are each independently an amino group, a substituted or unsubstituted alkoxy group of 1 or more but not more than 20 carbon atoms, a substituted or unsubstituted aryloxy group of 6 or more but not more than 20 carbon atoms, a substituted or unsubstituted alkylamino group of 1 or more but not more than 20 carbon atoms, or a substituted or unsubstituted arylamino group of 6 or more but not more than 20 carbon atoms).

8. The method for producing a blocked isocyanate compound according to Claim 1 or 2, wherein in the reaction step, an oxygen concentration within a supplied gas is not more than 21% by volume relative to a total volume of the gas.

9. The method for producing a blocked isocyanate compound according to Claim 1 or 2, wherein in the reaction step, an amount present of at least one compound selected from the group consisting of the secondary amine compounds of the general formula (I) and the tertiary amine compounds of the general formula (II) is at least 1 ppm by mass relative to a total mass of the primary amine compound and the blocking agent.

10. The method for producing a blocked isocyanate compound according to Claim 1 or 2, wherein in the reaction step, the secondary amine compound of the general formula (1) and the tertiary amine compound of the general formula (II) are incorporated simultaneously.

11. A method for producing an isocyanate compound, the method comprising a reaction step of decomposing a blocked isocyanate compound into a blocking agent and an isocyanate compound by conducting a heat treatment in presence of at least one compound selected from the group consisting of secondary amine compounds of general formula (I) and tertiary amine compounds of general formula (II), thus obtaining the isocyanate compound: 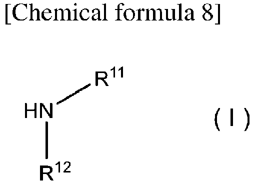 (wherein in general formula (I), R¹¹ and R¹² are each independently a monovalent organic group, R¹¹ and R¹² may be bonded together to form a cyclic structure via a carbon-carbon bond, a carbon-oxygen-carbon bond, or a carbon-nitrogen-carbon bond, and at least one of R¹¹ and R¹² has an aromatic group), 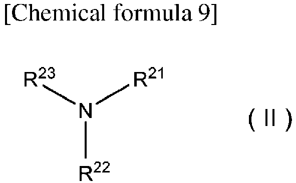 (wherein in general formula (II), R²¹, R²² and R²³ are each independently a monovalent organic group, each combination of R²¹ and R²², R²² and R²³, and R²³ and R²¹ may be independently bonded together to form a cyclic structure via a carbon-carbon bond, a carbon-oxygen-carbon bond, or a carbon-nitrogen-carbon bond, and at least one of R²¹, R²² and R²³ has an aromatic group).

12. The method for producing an isocyanate compound according to Claim 11, wherein the blocking agent comprises at least one compound selected from the group consisting of hydroxy compounds, amine compounds and ammonia.

13. The method for producing an isocyanate compound according to Claim 11 or 12, wherein the isocyanate compound is an isocyanate compound of general formula (VII): 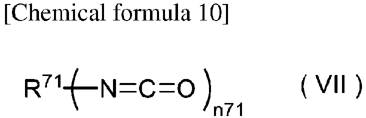 (wherein in general formula (VII), R⁷¹ is an n71-valent organic group, and n71 is an integer of 1 or more but not more than 12).

14. The method for producing an isocyanate compound according to Claim 11 or 12, wherein the blocking agent is an aromatic hydroxy compound of general formula (IV-1): 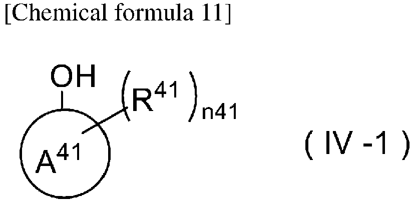 (wherein in general formula (IV-1), a ring A⁴¹ is an aromatic hydrocarbon ring of 6 or more but not more than 20 carbon atoms, R⁴¹ is a hydrogen atom, a halogen atom, a carboxy group, an alkyl group of 1 or more but not more than 20 carbon atoms, an alkoxy group of 1 or more but not more than 20 carbon atoms, an alkyloxycarbonyl group of 1 or more but not more than 20 carbon atoms, an alkylcarbonyloxy group of 1 or more but not more than 20 carbon atoms, an aryl group of 6 or more but not more than 20 carbon atoms, an aryloxy group of 6 or more but not more than 20 carbon atoms, an aralkyl group of 7 or more but not more than 20 carbon atoms, or an aralkyloxy group of 7 or more but not more than 20 carbon atoms, R⁴¹ may be bonded to the ring A⁴¹ to form a cyclic structure, and n41 is an integer of 1 or more but not more than 10).

15. The method for producing an isocyanate compound according to Claim 11 or 12, wherein the blocking agent is an aliphatic hydroxy compound of general formula (IV-2):
[Chemical formula 12]
R⁴²-OH ( IV-2 )
(wherein in general formula (IV-2), R⁴² is a substituted or unsubstituted aliphatic hydrocarbon group of 1 or more but not more than 24 carbon atoms which may have an ether group, a carbonyl group or an ester group).

16. The method for producing an isocyanate compound according to Claim 11 or 12, wherein the blocking agent is a secondary amine compound of general formula (V): 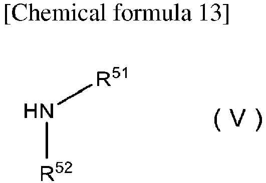 (wherein in general formula (V), R⁵¹ and R⁵² are each independently a monovalent organic group, and R⁵¹ and R⁵² may be bonded together to form a cyclic structure via a carbon-carbon bond, a carbon-oxygen-carbon bond, or a carbon-nitrogen-carbon bond).

17. The method for producing an isocyanate compound according to Claim 11 or 12, wherein in the reaction step, an oxygen concentration within a supplied gas is not more than 21 % by volume relative to a total volume of the gas.

18. The method for producing an isocyanate compound according to Claim 11 or 12, wherein in the reaction step, an amount present of at least one compound selected from the group consisting of the secondary amine compounds of the general formula (I) and the tertiary amine compounds of the general formula (II) is at least 1 ppm by mass relative to a mass of a reaction liquid.

19. The method for producing an isocyanate compound according to Claim 11 or 12, wherein in the reaction step, the secondary amine compound of the general formula (I) and the tertiary amine compound of the general formula (II) are incorporated simultaneously.

20. A method for producing an isocyanate compound, the method comprising:
a first reaction step of reacting a primary amine compound, a carbonic acid derivative and a first blocking agent by conducting a heat treatment in presence of at least one compound selected from the group consisting of secondary amine compounds of general formula (I) and tertiary amine compounds of general formula (II), thus obtaining a blocked isocyanate compound, and
a reaction step of decomposing the blocked isocyanate compound into the first blocking agent and an isocyanate compound by conducting a heat treatment in presence or absence of at least one compound selected from the group consisting of secondary amine compounds of general formula (I) and tertiary amine compounds of general formula (II), thus obtaining the isocyanate compound: 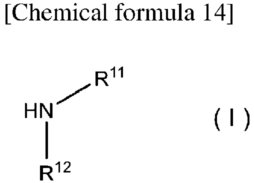
(wherein in general formula (I), R¹¹ and R¹² are each independently a monovalent organic group, R¹¹ and R¹² may be bonded together to form a cyclic structure via a carbon-carbon bond, a carbon-oxygen-carbon bond, or a carbon-nitrogen-carbon bond, and at least one of R¹¹ and R¹² has an aromatic group), 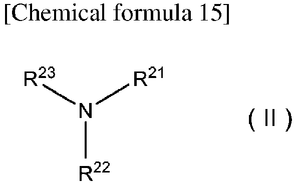
(wherein in general formula (II), R²¹, R²² and R²³ are each independently a monovalent organic group, each combination of R²¹ and R²², R²² and R²³, and R²³ and R²¹ may be independently bonded together to form a cyclic structure via a carbon-carbon bond, a carbon-oxygen-carbon bond, or a carbon-nitrogen-carbon bond, and at least one of R²¹, R²² and R²³ has an aromatic group).

21. A method for producing a blocked isocyanate compound, the method comprising an addition-elimination reaction step of reacting a first blocked isocyanate compound and a second blocking agent by an addition-elimination reaction in presence of at least one compound selected from the group consisting of secondary amine compounds of general formula (I) and tertiary amine compounds of general formula (II), thus obtaining a second blocked isocyanate compound, wherein
the second blocking agent is a compound different from a first blocking agent used in blocking isocyanate groups of the first blocked isocyanate compound, and
the first blocked isocyanate compound and the second blocked isocyanate compound have structures different from each other, 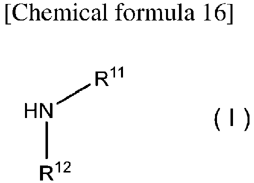
(wherein in general formula (I), R¹¹ and R¹² are each independently a monovalent organic group, R¹¹ and R¹² may be bonded together to form a cyclic structure via a carbon-carbon bond, a carbon-oxygen-carbon bond, or a carbon-nitrogen-carbon bond, and at least one of R¹¹ and R¹² has an aromatic group), 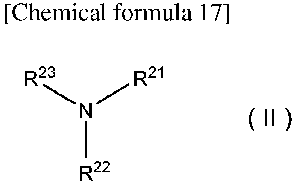
(wherein in general formula (II), R²¹, R²² and R²³ are each independently a monovalent organic group, each combination of R²¹ and R²², R²² and R²³, and R²³ and R²¹ may be independently bonded together to form a cyclic structure via a carbon-carbon bond, a carbon-oxygen-carbon bond, or a carbon-nitrogen-carbon bond, and at least one of R²¹, R²² and R²³ has an aromatic group).

22. The method for producing a blocked isocyanate compound according to Claim 21, wherein an isocyanate compound used in producing the first blocked isocyanate compound comprises an isocyanate compound of general formula (VII): 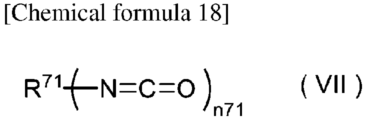 (wherein in general formula (VII), R⁷¹ is an n71-valent organic group, and n71 is an integer of 1 or more but not more than 12).

23. The method for producing a blocked isocyanate compound according to Claim 21 or 22, wherein the first blocking agent comprises at least one compound selected from the group consisting of hydroxy compounds, amine compounds and ammonia.

24. The method for producing a blocked isocyanate compound according to Claim 21 or 22, wherein the first blocking agent comprises an aromatic hydroxy compound of general formula (IV-1): 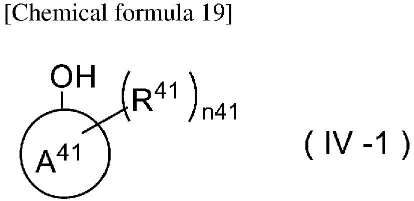 (wherein in general formula (IV-1), a ring A⁴¹ is an aromatic hydrocarbon ring of 6 or more but not more than 20 carbon atoms, R⁴¹ is a hydrogen atom, a halogen atom, a carboxy group, an alkyl group of 1 or more but not more than 20 carbon atoms, an alkoxy group of 1 or more but not more than 20 carbon atoms, an alkyloxycarbonyl group of 1 or more but not more than 20 carbon atoms, an alkylcarbonyloxy group of 1 or more but not more than 20 carbon atoms, an aryl group of 6 or more but not more than 20 carbon atoms, an aryloxy group of 6 or more but not more than 20 carbon atoms, an aralkyl group of 7 or more but not more than 20 carbon atoms, or an aralkyloxy group of 7 or more but not more than 20 carbon atoms, R⁴¹ may be bonded to the ring A⁴¹ to form a cyclic structure, and n41 is an integer of 1 or more but not more than 10).

25. The method for producing a blocked isocyanate compound according to Claim 21 or 22, wherein the first blocking agent comprises an aliphatic hydroxy compound of general formula (IV-2):
[Chemical formula 20]
R⁴²-OH ( IV-2 )
(wherein in general formula (IV-2), R⁴² is a substituted or unsubstituted aliphatic hydrocarbon group of 1 or more but not more than 24 carbon atoms which may have an ether group, a carbonyl group or an ester group).

26. The method for producing a blocked isocyanate compound according to Claim 21 or 22, wherein the first blocking agent comprises a secondary amine compound of general formula (V): 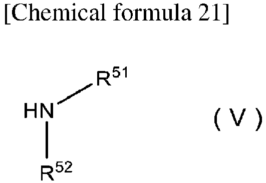 (wherein in general formula (V), R⁵¹ and R⁵² are each independently a monovalent organic group, and R⁵¹ and R⁵² may be bonded together to form a cyclic structure via a carbon-carbon bond, a carbon-oxygen-carbon bond, or a carbon-nitrogen-carbon bond).

27. The method for producing a blocked isocyanate compound according to Claim 21 or 22, wherein the second blocking agent comprises at least one compound selected from the group consisting of hydroxy compounds and amine compounds.

28. The method for producing a blocked isocyanate compound according to Claim 21 or 22, wherein the second blocking agent comprises an aromatic hydroxy compound of general formula (IV-1): 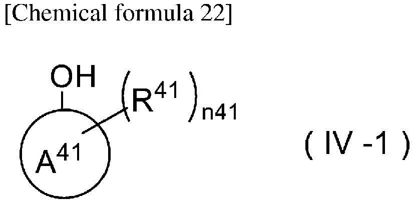 (wherein in general formula (IV-1), a ring A⁴¹ is an aromatic hydrocarbon ring of 6 or more but not more than 20 carbon atoms, R⁴¹ is a hydrogen atom, a halogen atom, a carboxy group, an alkyl group of 1 or more but not more than 20 carbon atoms, an alkoxy group of 1 or more but not more than 20 carbon atoms, an alkyloxycarbonyl group of 1 or more but not more than 20 carbon atoms, an alkylcarbonyloxy group of 1 or more but not more than 20 carbon atoms, an aryl group of 6 or more but not more than 20 carbon atoms, an aryloxy group of 6 or more but not more than 20 carbon atoms, an aralkyl group of 7 or more but not more than 20 carbon atoms, or an aralkyloxy group of 7 or more but not more than 20 carbon atoms, R⁴¹ may be bonded to the ring A⁴¹ to form a cyclic structure, and n41 is an integer of 1 or more but not more than 10).

29. The method for producing a blocked isocyanate compound according to Claim 21 or 22, wherein the second blocking agent comprises an aliphatic hydroxy compound of general formula (IV-2):
[Chemical formula 23]
R⁴²-OH ( IV-2 )
(wherein in general formula (IV-2), R⁴² is a substituted or unsubstituted aliphatic hydrocarbon group of 1 or more but not more than 24 carbon atoms which may have an ether group, a carbonyl group or an ester group).

30. The method for producing a blocked isocyanate compound according to Claim 21 or 22, wherein the second blocking agent comprises a secondary amine compound of general formula (V): 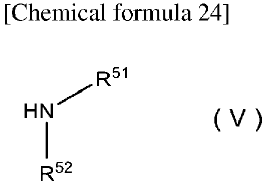 (wherein in general formula (V), R⁵¹ and R⁵² are each independently a monovalent organic group, and R⁵¹ and R⁵² may be bonded together to form a cyclic structure via a carbon-carbon bond, a carbon-oxygen-carbon bond, or a carbon-nitrogen-carbon bond).

31. The method for producing a blocked isocyanate compound according to Claim 21 or 22, wherein in the addition-elimination reaction step, an oxygen concentration within a supplied gas is not more than 21% by volume relative to a total volume of the gas.

32. The method for producing a blocked isocyanate compound according to Claim 21 or 22, wherein in the addition-elimination reaction step, an amount present of at least one compound selected from the group consisting of the secondary amine compounds of the general formula (I) and the tertiary amine compounds of the general formula (II) is at least 1 ppm by mass relative to a total mass of a reaction liquid.

33. The method for producing a blocked isocyanate compound according to Claim 21 or 22, wherein in the addition-elimination reaction step, the secondary amine compound of the general formula (I) and the tertiary amine compound of the general formula (II) are incorporated simultaneously.

34. A method for producing a blocked isocyanate compound, the method comprising:
a reaction step of reacting a primary amine compound, a carbonic acid derivative and a first blocking agent by conducting a heat treatment in presence of at least one compound selected from the group consisting of secondary amine compounds of general formula (I) and tertiary amine compounds of general formula (II), thus obtaining a first blocked isocyanate compound, and
an addition-elimination reaction step of reacting the first blocked isocyanate compound and a second blocking agent by an addition-elimination reaction in presence or absence of at least one compound selected from the group consisting of secondary amine compounds of the general formula (I) and tertiary amine compounds of the general formula (II), thus obtaining a second blocked isocyanate compound, wherein
the second blocking agent is a compound different from the first blocking agent and a third blocking agent derived from the carbonic acid derivative, and
the first blocked isocyanate compound and the second blocked isocyanate compound have structures different from each other, 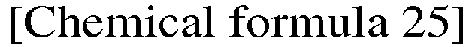 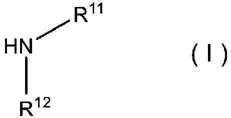
(wherein in general formula (I), R¹¹ and R¹² are each independently a monovalent organic group, R¹¹ and R¹² may be bonded together to form a cyclic structure via a carbon-carbon bond, a carbon-oxygen-carbon bond, or a carbon-nitrogen-carbon bond, and at least one of R¹¹ and R¹² has an aromatic group), 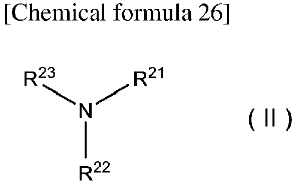
(wherein in general formula (II), R²¹, R²² and R²³ are each independently a monovalent organic group, each combination of R²¹ and R²², R²² and R²³, and R²³ and R²¹ may be independently bonded together to form a cyclic structure via a carbon-carbon bond, a carbon-oxygen-carbon bond, or a carbon-nitrogen-carbon bond, and at least one of R²¹, R²² and R²³ has an aromatic group.

35. A method for producing an isocyanate compound, the method comprising:
a first reaction step of reacting a primary amine compound, a carbonic acid derivative and a first blocking agent by conducting a heat treatment in presence of at least one compound selected from the group consisting of secondary amine compounds of general formula (I) and tertiary amine compounds of general formula (II), thus obtaining a blocked isocyanate compound, and
an addition-elimination reaction step of reacting the first blocked isocyanate compound and a second blocking agent by an addition-elimination reaction in presence or absence of at least one compound selected from the group consisting of secondary amine compounds of the general formula (I) and tertiary amine compounds of the general formula (II), thus obtaining a second blocked isocyanate compound, and
a second reaction step of decomposing the second blocked isocyanate compound into the second blocking agent and an isocyanate compound by conducting a heat treatment in presence or absence of at least one compound selected from the group consisting of secondary amine compounds of the general formula (I) and tertiary amine compounds of the general formula (II), thus obtaining the isocyanate compound, wherein
the second blocking agent is a compound different from the first blocking agent and a third blocking agent derived from the carbonic acid derivative, and
the first blocked isocyanate compound and the second blocked isocyanate compound have structures different from each other, 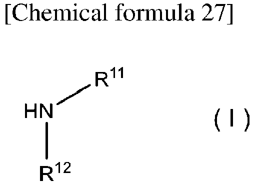
(wherein in general formula (I), R¹¹ and R¹² are each independently a monovalent organic group, R¹¹ and R¹² may be bonded together to form a cyclic structure via a carbon-carbon bond, a carbon-oxygen-carbon bond, or a carbon-nitrogen-carbon bond, and at least one of R¹¹ and R¹² has an aromatic group), 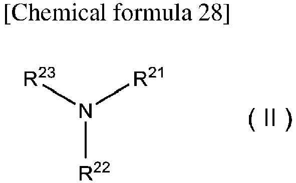
(wherein in general formula (II), R²¹, R²² and R²³ are each independently a monovalent organic group, each combination of R²¹ and R²², R²² and R²³, and R²³ and R²¹ may be independently bonded together to form a cyclic structure via a carbon-carbon bond, a carbon-oxygen-carbon bond, or a carbon-nitrogen-carbon bond, and at least one of R²¹, R²² and R²³ has an aromatic group).
